# EUROPÄISCHE PATENTSCHRIFT

(11) **EP 1 966 217 B1**
(45) Veröffentlichungstag und Bekanntmachung des Hinweises auf die Patenterteilung: **08.05.2013**
(21) Anmeldenummer: 06829427.1
(22) Anmeldetag: 08.12.2006
(51) Int. Cl.: C07D 491/04, A61K 31/519, A61P 9/00

(54) **NEUE, ACYCLISCH SUBSTITUIERTE FUROPYRIMIDIN-DERIVATE UND IHRE VERWENDUNG ZUR BEHANDLUNG VON KARDIOVASKULÄREN ERKRANKUNGEN**
NOVEL, ACYCLIC SUBSTITUTED FUROPYRIMIDINE DERIVATIVES AND USE THEREOF FOR TREATING CARDIOVASCULAR DISEASES
NOUVEAUX DERIVES DE FUROPYRIMIDINES A SUBSTITUTION ACYCLIQUE ET LEUR UTILISATION POUR TRAITER DES MALADIES CARDIOVASCULAIRES

(30) Priorität: 21.12.2005 DE 102005061170
(43) Veröffentlichungstag der Anmeldung: 10.09.2008
(73) Patentinhaber: Bayer Intellectual Property GmbH, 40789 Monheim (DE)
(72) Erfinder: LAMPE, Thomas, 40545 Düsseldorf (DE); BECKER, Eva-Maria, 42117 Wuppertal (DE); KAST, Raimund, 42349 Wuppertal (DE); BECK, Hartmut, 50733 Köln (DE); JESKE, Mario, 42699 Solingen (DE); SCHUHMACHER, Joachim, 42113 Wuppertal (DE); STOLL, Friederike, 40215 Düsseldorf (DE); KLEIN, Martina, 40489 Düsseldorf (DE); AKBABA, Metin, 40880 Ratingen (DE); KNORR, Andreas, 40699 Erkrath (DE); STASCH, Johannes-Peter, 42651 Solingen (DE); BÄRFACKER, Lars, 46047 Oberhausen (DE); HILLISCH, Alexander, 42651 Solingen (DE); KARIG, Gunter, 65719 Hofheim am Taunus (DE); MEININGHAUS, Mark, 42327 Wuppertal (DE); SCHLEMMER, Karl-Heinz, 42113 Wuppertal (DE); SCHOHE-LOOP, Rudolf, 42327 Wuppertal (DE)
(74) Vertreter: BIP Patents
(86) Internationale Anmeldenummer: PCT/EP2006/011826
(87) Internationale Veröffentlichungsnummer: WO 2007/079862

(56) Entgegenhaltungen:
- WO-A-03/018589
- WO-A-2006/004658
- DE-A1- 10 148 883
- US-A1- 2003 225 098
- FOLOPPE, NICOLAS ET AL: "Structure-Based Design of Novel Chk1 Inhibitors: Insights into Hydrogen Bonding and Protein-Ligand Affinity" JOURNAL OF MEDICINAL CHEMISTRY , 48(13), 4332-4345 CODEN: JMCMAR; ISSN: 0022-2623, 2005, XP002427056

## Beschreibung

Die vorliegende Anmeldung betrifft neue, acyclisch substituierte Furopyrimidin-Derivate, Verfahren zu ihrer Herstellung, besagte Verbindungen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Prostacyclin (PGI₂) gehört zur Familie der bioaktiven Prostaglandine, die Derivate der Arachidonsäure darstellen. PGI₂ ist das Hauptprodukt des Arachidonsäure-Stoffwechsels in Endothelzellen und hat potente gefäßerweiternde und anti-aggregatorische Eigenschaften. PGI₂ ist der physiologische Gegenspieler von Thromboxan A₂ (TxA₂), einem starken Vasokonstriktor und Stimulator der Thrombozytenaggregation, und trägt somit zur Aufrechterhaltung der vaskulären Homeostase bei. Eine Reduktion der PGI₂-Spiegel ist vermutlich mitverantwortlich für die Entstehung verschiedener kardiovaskulärer Erkrankungen [Dusting, G.J. et al., Pharmac. Ther. 1990, 48: 323-344; Vane, J. et al., Eur. J. Vasc. Endovasc. Surg. 2003, 26: 571-578].

Nach Freisetzung der Arachidonsäure aus Phospholipiden über Phospholipasen A₂ wird PGI₂ durch Cyclooxygenasen und anschließend durch die PGI₂-Synthase synthetisiert. PGI₂ wird nicht gespeichert, sondern nach Synthese sofort freigesetzt, wodurch es lokal seine Wirkungen entfaltet. PGI₂ ist ein instabiles Molekül, welches schnell (Halbwertszeit ca. 3 Minuten) nicht-enzymatisch zu einem inaktiven Metaboliten, 6-Keto-Prostaglandin-F1alpha, umgelagert wird [Dusting, G.J. et al., Pharmac. Ther. 1990, 48: 323-344].

Die biologischen Effekte von PGI₂ kommen durch die Bindung an einen membranständigen Rezeptor, den sogenannten Prostacyclin- oder IP-Rezeptor [Narumiya, S. et al., Physiol. Rev. 1999, 79: 1193-1226], zustande. Der IP-Rezeptor gehört zu den G-Protein-gekoppelten Rezeptoren, die durch sieben Transmembrandomänen charakterisiert sind. Neben dem humanen IP-Rezeptor sind auch noch die Prostacyclin-Rezeptoren aus Ratte und Maus kloniert worden [Vane, J. et al., Eur. J. Vasc. Endovasc. Surg. 2003, 26: 571-578]. In den Glattmuskelzellen führt die Aktivierung des IP-Rezeptors zur Stimulation der Adenylatzyklase, die die Bildung von cAMP aus ATP katalysiert. Die Erhöhung der intrazellulären cAMP-Konzentration ist für die Prostacyclin-induzierte Vasodilatation sowie die Hemmung der Thrombozytenaggregation verantwortlich. Neben den vasoaktiven Eigenschaften wurden für PGI₂ noch anti-proliferative [Schroer, K. et al., Agents Actions Suppl. 1997, 48: 63-91; Kothapalli, D. et al., Mol. Pharmacol. 2003, 64: 249-258; Planchon, P. et al., Life Sci. 1995, 57: 1233-1240] und anti-arteriosklerotische Wirkungen beschrieben [Rudic, R.D. et al., Circ. Res. 2005, 96: 1240-1247; Egan K.M. et al., Science 2004, 114: 784-794]. Darüber hinaus wird die Metastasenbildung durch PGI₂ gehemmt [Schneider, M.R. et al., Cancer Metastasis Rev. 1994, 13: 349-64). Ob diese Effekte durch Stimulation der cAMP-Bildung oder durch eine IP-Rezeptor-vermittelte Aktivierung anderer Signaltransduktionswege in der jeweiligen Zielzelle [Wise, H. et al. TIPS 1996, 17: 17-21], wie z.B. der Phosphoinositidkaskade sowie von Kaliumkanälen, zustande kommen, ist unklar.

Obwohl die Wirkungen von PGI₂ insgesamt therapeutisch von Nutzen sind, ist ein klinische Verwendung von PGI₂ durch seine chemische und metabolische Instabilität stark eingeschränkt. Stabilere PGI₂-Analoga wie z.B. Iloprost [Badesch, D.B. et al., J. Am. Coll. Cardiol. 2004, 43: 56S-61 S] und Treprostinil [Chattaraj, S.C., Curr. Opion. Invest. Drugs 2002, 3: 582-586] konnten zwar zur Verfügung gestellt werden, allerdings ist die Wirkdauer dieser Verbindungen nach wie vor sehr kurz. Auch können die Substanzen nur über komplizierte Applikationswege dem Patienten verabreicht werden, wie z.B. durch Dauerinfusion, subkutan oder über mehrmalige Inhalationen. Diese Applikationswege können zudem zu zusätzlichen Nebenwirkungen, wie z.B. Infektionen oder Schmerzen an der Injektionsstelle, führen. Die Verwendung des bisher einzigen für den Patienten oral verfügbaren PGI₂-Derivates, Beraprost [Barst, R.J. et al., J. Am. Coll. Cardiol. 2003, 41: 2119-2125], ist wiederum durch seine kurze Wirkdauer limitiert.

Die in der vorliegenden Anmeldung beschriebenen Verbindungen sind im Vergleich zu PGI₂ chemisch und metabolisch stabile, nicht-prostanoide Aktivatoren des IP-Rezeptors, die die biologische Wirkung von PGI₂ nachahmen und somit zur Behandlung von Erkrankungen, insbesondere von kardiovaskulären Erkrankungen, eingesetzt werden können.

In DE 1 817 146, EP 1 018 514, EP 1 132 093, WO 02/092603, WO 03/022852, WO 2005/092896, WO 2005/121149 und WO 2006/004658 werden verschiedene 4-Oxy-, 4-Thio- und/oder 4-Amino-furo[2,3-d]pyrimidin-Derivate und ihre Verwendung zur Behandlung von Erkrankungen beschrieben. In WO 03/018589 werden 4-Aminofuropyrimidine als Adenosinkinase-Inhibitoren zur Behandlung kardiovaskulärer Erkrankungen offenbart. Die Herstellung bestimmter 4-Aminofuropyrimidin-Derivate ist in Chemica Scripta 1986, 26 (2): 337-342, Yakugaku Zasshi 1969, 89 (10): 1434-1439 sowie Yakugaku Zasshi 1977, 97 (9): 1022-1033 publiziert. In WO 00/75145 werden Verbindungen mit einer bicyclischen Heteroaryl-Kernstruktur als Inhibitoren der Zelladhäsion beansprucht.

Die im Rahmen der vorliegenden Anmeldung beanspruchten Verbindungen zeichnen sich im Vergleich zu den Verbindungen aus dem Stand der Technik durch eine 5,6-Diphenylfuro[2,3-d]pyrimidin-Kemstruktur aus, die über die 4-Position in einem bestimmten räumlichen Abstand mit einer Carbonsäure- oder Carbonsäure-ähnlichen Funktionalität verknüpft ist.

Gegenstand der vorliegenden Erfindung sind Verbindungen der allgemeinen Formel (I) 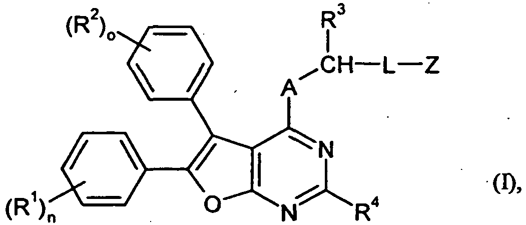 in welcher
- A: für O, S oder N-R⁵ steht, worin
R⁵ Wasserstoff, (C₁-C₆)-Alkyl, (C₃-C₇)-Cycloalkyl oder (C₄-C₇)Cycloalkenyl bedeutet,
- L: für (C₁-C₇)Alkandiyl oder (C₂-C₇)-Alkendiyl, welche ein- oder zweifach mit Fluor substituiert sein können, oder für eine Gruppe der Formel *-L¹-Q-L² steht, worin
* die Verknüpfungsstelle mit der CHR³-Gruppe,
L¹ (C₁-C₅)-Alkandiyl, welches mit (C₁-C₄)-Alkyl oder (C₁-C₄)-Alkoxy substituiert sein kann,
L² eine Bindung oder (C₁-C₃)-Alkandiyl, welches ein- oder zweifach mit Fluor substituiert sein kann, und
Q O oder N-R⁶, worin
R⁶ Wasserstoff, (C₁-C₆)-Alkyl oder (C₃-C₇)-Cycloalkyl darstellt, bedeuten,
- Z: für eine Gruppe der Formel 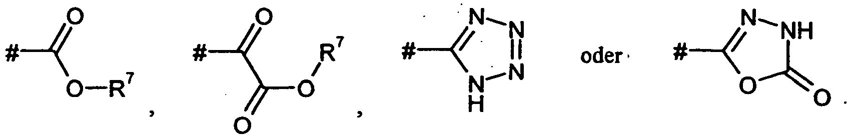 steht, worin
# die Verknüpfungsstelle mit der Gruppe L und
R⁷ Wasserstoff oder (C₁-C₄)-Alkyl bedeutet,
- R¹ und R²: unabhängig voneinander für einen Substituenten ausgewählt aus der Reihe Halogen, Cyano, Nitro, (C₁-C₆)-Alkyl, (C₂-C₆)-Alkenyl, (C₂-C₄)-Alkinyl, (C₃-C₇)-Cycloalkyl, (C₄-C₇)-Cycloalkenyl, (C₁-C₆)-Alkoxy, Trifluormethyl, Trifluormethoxy, (C₁-C₆)-Alkylthio, (C₁-C₆)-Acyl, Amino, Mono-(C₁-C₆)-alkylamino, Di-(C₁-C₆)-alkylamino und (C₁-C₆)-Acylamino stehen,
wobei (C₁-C₆)-Alkyl und (C₁-C₆)-Alkoxy ihrerseits jeweils mit Cyano, Hydroxy, (C₁-C₄)-Alkoxy, (C₁-C₄)-Alkylthio, Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂-O-, -O-CH₂-CH₂-O- oder -O-CF₂-CF₂-O- bilden,
- n und o: unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
- R³: für Wasserstoff oder (C₁-C₄)-Alkyl, welches mit Hydroxy oder Amino substituiert sein kann, steht
und
- R⁴: für Wasserstoff, (C₁-C₄)-Alkyl oder Cyclopropyl steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausfuhrungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Insbesondere umfasst die vorliegende Erfindung bei den Verbindungen der Formel (I), in welcher
- Z: für eine Gruppe der Formel 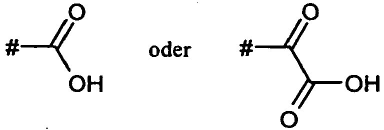 steht,
auch hydrolysierbare Ester-Derivate dieser Verbindungen. Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkytester, in welchen die Alkylgruppe geradkettig oder verzweigt sein kann, bevorzugt. Besonders bevorzugt sind Methyl- oder Ethylester (siehe auch entsprechende Definitionen des Restes R⁷).

Im Rahmen der vorliegenden Erfindung haben die Substituenten, soweit nicht anders spezifiziert, die folgende Bedeutung:
(C₁-C₆)-Alkyl, (C₁-C₅)-Alkyl, (C₁-C₄)-Alkyl und (C₁-C₃)-Alkyl stehen im Rahmen der Erfindung für einen geradkettigen oder verzweigten Alkylrest mit 1 bis 6, 1 bis 5, 1 bis 4 bzw. 1 bis 3 Kohlenstoffatomen. Bevorzugt ist ein geradkettiger oder verzweigter Alkylrest mit 1 bis 4, besonders bevorzugt mit 1 bis 3 Kohlenstoffatomen. Beispielhaft und vorzugsweise seien genannt: Methyl, Ethyl, n-Propyl, Isopropyl, n-Butyl, iso-Butyl, *sec*.-Butyl, *tert*.-Butyl, 1-Ethylpropyl, n-Pentyl und n-Hexyl.
(C₂-C₆)-Alkenyl und (C₂-C₅)-Alkenyl stehen im Rahmen der Erfindung für einen geradkettigen oder verzweigten Alkenylrest mit 2 bis 6 bzw. 2 bis 5 Kohlenstoffatomen und einer oder zwei Doppelbindungen. Bevorzugt ist ein geradkettiger oder verzweigter Alkenylrest mit 2 bis 5 Kohlenstoffatomen und einer Doppelbindung. Beispielhaft und vorzugsweise seien genannt: Vinyl, Allyl, Isopropenyl und n-But-2-en-1-yl.
(C₂-C₄)-Alkinyl steht im Rahmen der Erfindung für einen geradkettigen oder verzweigten Alkinylrest mit 2 bis 4 Kohlenstoffatomen und einer Dreifachbindung. Bevorzugt ist ein geradkettiger Alkinylrest mit 2 bis 4 Kohlenstoffatomen. Beispielhaft und vorzugsweise seien genannt: Ethinyl, n-Prop-1-in-1-yl, n-Prop-2-in-1-yl, n-But-2-in-1-yl und n-But-3-in-1-yl.
(C₁-C₇)-Alkandiyl, (C₁-C₅)-Alkandiyl, (C₁-C₃)-Alkandiyl und (C₁-C₇)-Alkandiyl stehen im Rahmen der Erfindung für einen geradkettigen oder verzweigten divalenten Alkylrest mit 1 bis 7, 1 bis 5, 1 bis 3 bzw. 3 bis 7 Kohlenstoffatomen. Bevorzugt ist ein geradkettiger oder verzweigter Alkandiylrest mit 1 bis 5, 1 bis 3 bzw. 3 bis 7 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl, Pentan-1,5-diyl, Pentan-2,4-diyl, 3-Methyl-pentan-2,4-diyl und Hexan-1,6-diyl.
(C₂-C₇)-Alkendiyl und (C₃-C₇)-Alkendiyl stehen im Rahmen der Erfindung für einen geradkettigen oder verzweigten divalenten Alkenylrest mit 2 bis 7 bzw. 3 bis 7 Kohlenstoffatomen und bis zu 3 Doppelbindungen. Bevorzugt ist ein geradkettiger oder verzweigter Alkendiylrest mit 3 bis 7 Kohlenstoffatomen und einer Doppelbindung. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
(C₁-C₆)-Alkylthio und-(C₁-C₄)-Alkylthio stehen im Rahmen der Erfindung für einen geradkettigen oder verzweigten Alkylthiorest mit 1 bis 6 bzw. 1 bis 4 Kohlenstoffatomen. Bevorzugt ist ein geradkettiger oder verzweigter Alkylthiorest mit 1 bis 4 Kohlenstoffatomen. Beispielhaft und vorzugsweise seien genannt: Methylthio, Ethylthio, n-Propylthio, Isopropylthio, n-Butylthio, tert.-Butylthio, n-Pentylthio und n-Hexylthio.
(C₁-C₆)-Acyl [(C₁-C₆)-Alkanoyl], (C₁-C₅)-Acyl [(C₁-C₅)-Alkanoyl] und (C₁-C₄)-Acyl [(C₁-C₄)-Alkanoyl] stehen im Rahmen der Erfindung für einen geradkettigen oder verzweigten Alkylrest mit 1 bis 6, 1 bis 5 bzw. 1 bis 4 Kohlenstoffatomen, der in der 1-Position ein doppelt gebundenes Sauerstoffatom trägt und über die 1-Position verknüpft ist. Bevorzugt ist ein geradkettiger oder verzweigter Acylrest mit 1 bis 4 Kohlenstoffatomen. Beispielhaft und vorzugsweise seien genannt: Formyl, Acetyl, Propionyl, n-Butyryl, iso-Butyryl und Pivaloyl.
Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino und tert.-Butylamino.
Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N-*Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
(C₁-C₆)-Acylamino und (C₁-C₄)-Acylamino stehen im Rahmen der Erfindung für eine Amino-Gruppe mit einem geradkettigen oder verzweigten Acyl-Substituenten, der 1 bis 6 bzw. 1 bis 4 Kohlenstoffatome aufweist und über die Carbonylgruppe verknüpft ist. Bevorzugt ist ein Acylamino-Rest mit 1 bis 4 Kohlenstoffatomen. Beispielhaft und vorzugsweise seien genannt: Formamido, Acetamido, Propionamido, n-Butyramido und Pivaloylamido.
(C₃-C₇)-Cycloalkyl und (C₃-C₆)-Cycloakyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₄-C₇)-Cycloalkenyl und (C₄-C₆)Cycloalkenyl stehen im Rahmen der Erfmdung für eine monocyclische Cycloalkylgruppe mit 4 bis 7 bzw. 4 bis 6 Kohlenstoffatomen und einer Doppelbindung. Bevorzugt ist ein Cycloalkenylrest mit 4 bis 6 Kohlenstoffatomen. Beispielhaft und vorzugsweise seien genannt: Cyclobutenyl, Cyclopentenyl, Cyclohexenyl und Cycloheptenyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I), in welcher
- A: für O, S oder N-R⁵ steht, worin
R⁵ Wasserstoff, (C₁-C₆)-Alkyl, (C₃-C₇)-Cycloalkyl oder (C₄-C₇)-Cycloalkenyl bedeutet,
- L: für (C₁-C₇)-Alkandiyl oder (C₂-C₇)-Alkendiyl, welche ein- oder zweifach mit Fluor substituiert sein können, oder für eine Gruppe der Formel *-L¹-Q-L² steht, worin
* die Verknüpfungsstelle mit der CHR³-Gruppe,
L¹ (C₁-C₅)-Alkandiyl,
L² eine Bindung oder (C₁-C₃)-Alkandiyl, welches ein- oder zweifach mit Fluor substituiert sein kann, und
Q O oder N-R⁶, worin
R⁶ Wasserstoff, (C₁-C₆)-Alkyl oder (C₃-C₇)-Cycloalkyl darstellt, bedeuten,
- Z: für eine Gruppe der Formel 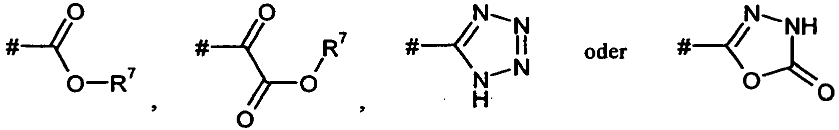 steht, worin
# die Verknüpfungsstelle mit der Gruppe L und
R⁷ Wasserstoff oder (C₁-C₄)-Alkyl bedeutet,
- R¹ und R²: unabhängig voneinander für einen Substituenten ausgewählt aus der Reihe Halogen, Cyano, Nitro, (C₁-C₆)-Alkyl, (C₂-C₆)-Alkenyl, (C₂-C₄)-Alkinyl, (C₃-C₇)-Cycloalkyl, (C₄-C₇)-Cycloalkenyl, (C₁-C₆)-Alkoxy, Trifluormethyl, Trifluormethoxy, (C₁-C₆)-Alkylthio, (C₁-C₆)-Acyl, Amino, Mono-(C₁-C₆)-alkylamino, Di-(C₁-C₆)-alkylamino und (C₁-C₆)-Acylamino stehen,
wobei (C₁-C₆)-Alkyl und (C₁-C₆)-Alkoxy ihrerseits jeweils mit Hydroxy, (C₁-C₄)-Alkoxy Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂-O-, -O-CH₂-CH₂-O- oder -O-CF₂-CF₂-O- bilden,
- n und o: unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
- R³: für Wasserstoff oder (C₁-C₄)-Alkyl, welches mit Hydroxy oder Amino substituiert sein kann, steht
und
- R⁴: für Wasserstoff, (C₁-C₄)-Alkyl oder Cyclopropyl steht,
sowie ihre Salze, Solvate und Solvate der Salze.

Besonders bevorzugt im Rahmen der vorliegenden Erfindung sind Verbindungen der Formel (I), in welcher
- A: für O oder N-R⁵ steht, worin
R⁵ Wasserstoff, (C₁-C₄)-Akyl oder (C₃-C₆)-Cycloalkyl bedeutet,
- L: für (C₃-C₇)-Alkandiyl oder (C₃-C₇)-Alkendiyl, welche ein- oder zweifach mit Fluor substituiert sein können, oder für eine Gruppe der Formel *-L¹-Q-L² steht, worin
* die Verknüpfungsstelle mit der CHR³-Gruppe,
L¹ (C₁-C₃)-Alkandiyl,
L² (C₁-C₃)-Alkandiyl, welches ein- oder zweifach mit Fluor substituiert sein kann, und
Q O oder N-R⁶, worin
R⁶ Wasserstoff, (C₁-C₃)-Alkyl oder Cyclopropyl darstellt, bedeuten,
- Z: für eine Gruppe der Formel 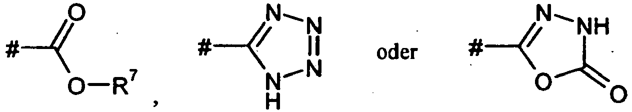 steht, worin
# die Verknüpfungsstelle mit der Gruppe L und
R⁷ Wasserstoff, Methyl oder Ethyl bedeutet,
- R¹ und R²: unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄)-Alkoxy, Trifluormethyl, Trifluormethoxy, (C₁-C₄)-Alkylthio, (C₁-C₅)-Acyl, Amino, Mono-(C₁-C₄)-alkylamino, Di-(C₁-C₄)-alkylamino und (C₁-C₄)-Acylamino stehen
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O-bilden,
- n und o: unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
- R³: für Wasserstoff oder (C₁-C₃)-Alkyl, welches mit Hydroxy oder Amino substituiert sein kann, steht
und
- R⁴: für Wasserstoff oder (C₁-C₃)-Alkyl steht,
sowie ihre Salze, Solvate und Solvate der Salze.

Ganz besonders bevorzugt im Rahmen der vorliegenden Erfindung sind Verbindungen der Formel (I), in welcher
- A: für O oder NH steht,
- L: für (C₃-C₇)-Alkandiyl, (C₃-C₇)-Alkendiyl oder für eine Gruppe der Formel *-L¹-O-L² steht, worin
* die Verknüpfungsstelle mit der CHR³-Gruppe und
L¹ und L² unabhängig voneinander (C₁-C₃)-Alkandiyl bedeuten,
- Z: für eine Gruppe der Formel 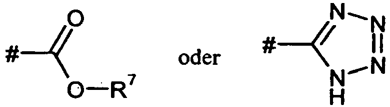 steht, worin
# die Verknüpfungsstelle mit der Gruppe L und
R⁷ Wasserstoff, Methyl oder Ethyl bedeutet,
- R¹ und R²: unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄)-Alkoxy, Trifluormethyl, Trifluormethoxy, (C₁-C₄)-Alkylthio, (C₁₋C₅)-Acyl, Amino, Mono-(C₁-C₄)-alkylamino, Di-(C₁-C₄)-alkylamino und (C₁-C₄)-Acylamino stehen
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O-bilden,
- n und o: unabhängig voneinander für die Zahl 0, 1 oder 2 stehen,
wobei für den Fall, dass R¹ oder R² zweifach auftreten, ihre Bedeutungen jeweils gleich oder verschieden sein können,
- R³: für Wasserstoff, Methyl oder Ethyl steht
und
- R⁴: für Wasserstoff steht,
sowie ihre Salze, Solvate und Solvate der Salze.

Ganz besonders bevorzugt im Rahmen der vorliegenden Erfindung sind auch Verbindungen der Formel (I), in welcher
- A: für O oder NH steht,
- L: für eine Gruppe der Formel *-L¹-N(CH₃)-L² steht, worin
* die Verknüpfungsstelle mit der CHR³-Gruppe und
L¹ und L² unabhängig voneinander (C₁-C₃)-Alkandiyl bedeuten,
- Z: für eine Gruppe der Formel 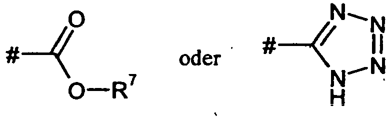 steht, worin
# die Verknüpfungsstelle mit der Gruppe L und
R⁷ Wasserstoff, Methyl oder Ethyl bedeutet,
- R¹ und R²: unabhängig voneinander für einen Substituenten ausgewählt aus der Reihe Fluor, Chlor, Cyano, (C₁-C₅)-Alkyl, (C₂-C₆)-Alkenyl, (C₃-C₆)-Cycloalkyl, (C₄-C₆)-Cycloalkenyl, (C₁-C₄)-Alkoxy, Trifluormethyl, Trifluormethoxy, (C₁-C₄)-Alkylthio, (C₁-C₅)-Acyl, Amino, Mono-(C₁-C₄)-alkylamino, Di-(C₁-C₄)-alkylamino und (C₁-C₄)-Acylamino stehen
oder
zwei an benachbarte Kohlenstoffatome des jeweiligen Phenylrings gebundene Reste R¹ und/oder R² zusammen eine Gruppe der Formel -O-CH₂-O-, -O-CHF-O- oder -O-CF₂-O-bilden,
- n und o: unabhängig voneinander für die Zahl 0, 1 oder 2 stehen,
wobei für den Fall, dass R¹ oder R² zweifach auftreten, ihre Bedeutungen jeweils gleich oder verschieden sein können,
- R³: für Wasserstoff, Methyl oder Ethyl steht
und
- R⁴: für Wasserstoff steht,
sowie ihre Salze, Solvate und Solvate der Salze.

Vor allem bevorzugt im Rahmen der vorliegenden Erfindung sind Verbindungen der Formel (I), in welcher
- A: für O oder NH steht,
- L: für (C₃-C₇)-Alkandiyl, (C₃-C₇)-Alkendiyl oder für eine Gruppe der Formel *-L¹-Q-L² steht, worin
* die Verknüpfungsstelle mit der CHR³-Gruppe,
L¹ und L² unabhängig voneinander (C₁-C₃)-Akandiyl und
Q O oder N(CH₃) bedeuten,
- Z: für eine Gruppe der Formel 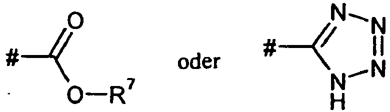 steht, worin
# die Verknüpfungsstelle mit der Gruppe L und
R⁷ Wasserstoff, Methyl oder Ethyl bedeutet,
- R¹: für einen Substituenten ausgewählt aus der Reihe Fluor, Chlor, Methyl, Ethyl, Vinyl, Trifluormethyl und Methoxy steht,
- R²: für einen Substituenten ausgewählt aus der Reihe Fluor, Chlor, Cyano, Methyl, Ethyl, n-Propyl, Vinyl, Trifluormethyl, Methoxy, Ethoxy, Trifluormethoxy, Methylthio, Ethylthio, Amino, Methylamino und Ethylamino steht,
- n und o: unabhängig voneinander für die Zahl 0, 1 oder 2 stehen,
wobei für den Fall, dass R¹ oder R² zweifach auftreten, ihre Bedeutungen jeweils gleich oder verschieden sein können,
- R³: für Wasserstoff, Methyl oder Ethyl steht
und
- R⁴: für Wasserstoff steht,
sowie ihre Salze, Solvate und Solvate der Salze.

Von besonderer Bedeutung im Rahmen der vorliegenden Erfindung sind Verbindungen der Formel (I), in welcher
- A: für O steht,
sowie ihre Salze, Solvate und Solvate der Salze.

Von besonderer Bedeutung im Rahmen der vorliegenden Erfindung sind auch Verbindungen der Formel (I), in welcher
- L: für eine Gruppe der Formel *-L¹-Q-L² steht, worin
* die Verknüpfungsstelle mit der CHR³-Gruppe,
L¹ (C₁-C₅)-Alkandiyl,
L² eine Bindung oder (C₁-C₃)-Alkandiyl, welches ein- oder zweifach mit Fluor substituiert sein kann, und
Q O oder N-R⁶, worin
R⁶ Wasserstoff, (C₁-C₆)-Alkyl oder (C₃-C₇)-Cycloalkyl darstellt, bedeuten,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man entweder
[A] Verbindungen der Formel (II) 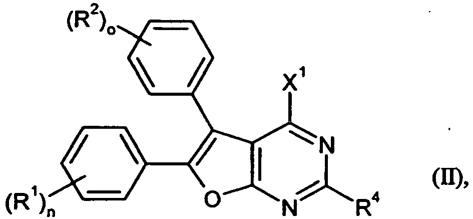 in welcher R¹, R², R⁴, n und o jeweils die oben angegebenen Bedeutungen haben
   und
   - X¹: für eine Abgangsgruppe wie beispielsweise Halogen, insbesondere für Chlor steht,
   in Gegenwart einer Base gegebenenfalls in einem inerten Lösungsmittel mit einer Verbindung der Formel (III) 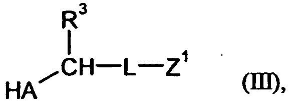 in welcher A, L und R³ jeweils die oben angegebenen Bedeutungen haben
   und
   - Z¹: für Cyano oder eine Gruppe der Formel -[C(O)]_{y}-COOR^{7A} steht, worin
   y die Zahl 0 oder 1 und
   R^{7A} (C₁-C₄)-Alkyl bedeutet,
   zu Verbindungen der Formel (IV) 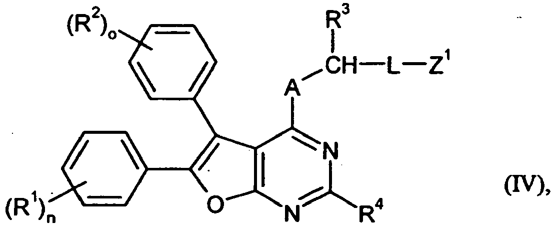 in welcher A, L, Z¹, R¹, R², R³, R⁴, n und o jeweils die oben angegebenen Bedeutungen haben,
   umsetzt
   oder
[B] Verbindungen der Formel (V-1) 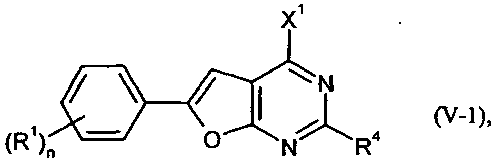 in welcher R¹, R⁴, X¹ und n jeweils die oben angegebenen Bedeutungen haben,
   in Gegenwart einer Base gegebenenfalls in einem inerten Lösungsmittel mit einer Verbindung der Formel (III) zu Verbindungen der Formel (VI-1) 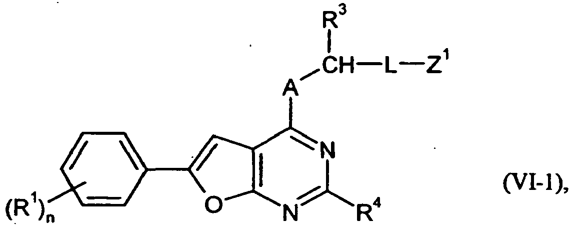 in welcher A, L, Z¹, R¹, R³, R⁴ und n jeweils die oben angegebenen Bedeutungen haben,
   umsetzt, dann in einem inerten Lösungsmittel beispielsweise mit N-Bromsuccinimid zu Verbindungen der Formel (VII-1) 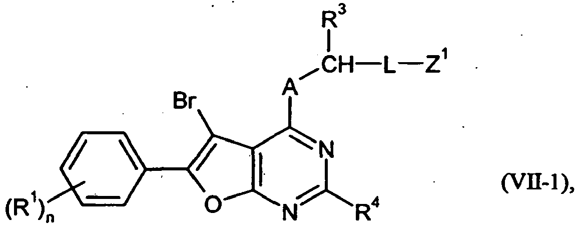 in welcher A, L, Z¹, R¹, R³, R⁴ und n jeweils die oben angegebenen Bedeutungen haben,
   bromiert und diese anschließend in einem inerten Lösungsmittel in Gegenwart einer Base und eines geeigneten Palladium-Katalysators mit einer Phenylboronsäure der Formel (VIII-1) 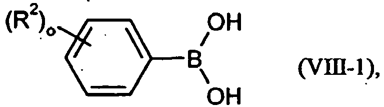 in welcher R² und o die oben angegebenen Bedeutungen haben,
   zu Verbindungen der Formel (IV) kuppelt
   oder
[C] Verbindungen der Formel (V-2) 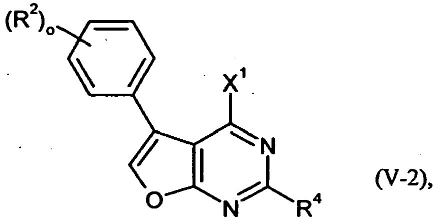 in welcher R², R⁴, X¹ und o jeweils die oben angegebenen Bedeutungen haben,
   in Gegenwart einer Base gegebenenfalls in einem inerten Lösungsmittel mit einer Verbindung der Formel (III) zu Verbindungen der Formel (VI-2) 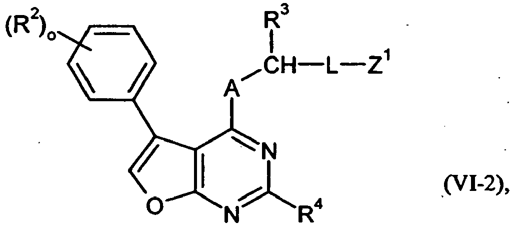 in welcher A, L, Z¹, R², R³, R⁴ und o jeweils die oben angegebenen Bedeutungen haben,
   umsetzt, dann in einem inerten Lösungsmittel beispielsweise mit N-Bromsuccinimid zu Verbindungen der Formel (VII-2) 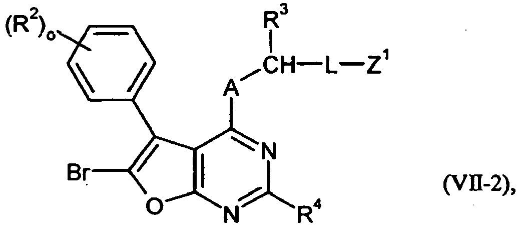 in welcher A, L, Z¹, R², R³, R⁴ und o jeweils die oben angegebenen Bedeutungen haben, bromiert und diese anschließend in einem inerten Lösungsmittel in Gegenwart einer Base und eines geeigneten Palladium-Katalysators mit einer Phenylboronsäure der Formel (VIII-2) 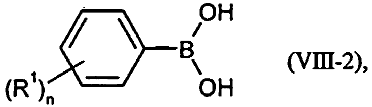 in welcher R¹ und n die oben angegebenen Bedeutungen haben,
   zu Verbindungen der Formel (IV) kuppelt,
und die jeweils resultierenden Verbindungen der Formel (IV) dann durch Hydrolyse der Ester- bzw. Cyano-Gruppe Z¹ in die Carbonsäuren der Formel (I-A) 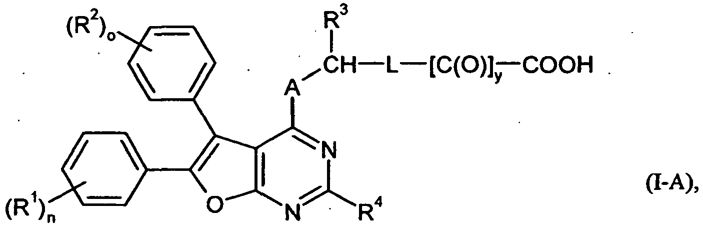 in welcher A, L, R¹, R², R³, R⁴, n, o und y jeweils die oben angegebenen Bedeutungen haben,
überführt und diese gegebenenfalls mit den entsprechenden (i) Lösungsmitteln und/oder (ii) Basen oder Säuren zu ihren Solvaten, Salzen und/oder Solvaten der Salze umsetzt.

Inerte Lösungsmittel für die Verfahrensschritte (II) + (III) → (IV), (V-1) + (III) → (VI-1) und (V-2) + (III) → (VI-2) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethan, Tetrachlorethan, Trichlorethylen, Chlorbenzol oder Chlortoluol, oder andere Lösungsmittel wie Dimethylformamid (DMF), Dimethylsulfoxid (DMSO), *N,N*'-Dimethylpropylenharnstoff (DMPU), *N-*Methylpyrrolidon (NMP) oder Acetonitril. Ebenso ist es möglich, Gemische der genannten Lösungsmittel einzusetzen. Bevorzugt werden Tetrahydrofuran, Dimethylformamid, Dimethylsulfoxid oder Gemische aus diesen verwendet.

Gegebenenfalls können die Verfahrensschritte (II) + (III) → (IV), (V-1) + (III) → (VI-1) und (V-2) + (III) → (VI-2) jedoch auch ohne Lösungsmittel durchgeführt werden.

Als Basen für die Verfahrensschritte (II) + (III) → (IV), (V-1) + (III) → (VI-1) und (V-2) + (III) → (VI-2) eignen sich übliche anorganische oder organische Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kalium-*tert*.-butylat, Alkalihydride wie Natrium- oder Kaliumhydrid, Amide wie Lithium- oder Kalium-bis(trimethylsilyl)amid oder Lithiumdiisopropylamid, metallorganische Verbindungen wie Butyllithium oder Phenyllithium, oder organische Amine wie Triethylamin, *N*-Methylmorpholin, *N*-Methylpiperidin, *N,N*-Diisopropylethylamin oder Pyridin.

Im Falle der Umsetzung mit Alkoholderivaten [A in (III) = O] sind auch Phosphazen-Basen (so genannte "Schwesinger-Basen") wie beispielsweise P2-t-Bu oder P4-t-Bu zweckmäßig [vgl. z.B. R Schwesinger, H. Schlemper, Angew. Chem. Int. Ed. Engl. 26, 1167 (1987); T. Pietzonka, D. Seebach, Chem. Ber. 124, 1837 (1991)].

Bei der Umsetzung mit Aminderivaten [A in (III) = N] werden vorzugsweise tertiäre Amine, wie insbesondere *N,N*-Diisopropylethylamin, als Base verwendet. Gegebenenfalls können diese Umsetzungen aber auch - bei Verwendung eines Überschusses der Aminkomponente (III) - ohne Zusatz einer Hilfsbase erfolgen. Bei der Reaktion mit Alkoholderivaten [A in (III) = O] sind Kalium- oder Cäsiumcarbonat oder die Phosphazen-Basen P2-t-Bu und P4-t-Bu bevorzugt.

Die Verfahrensschritte (II) + (III) → (IV), (V-1) + (III) → (VI-1) und (V-2) + (III) → (VI-2) können gegebenenfalls vorteilhaft unter Zusatz eines Kronenethers durchgeführt werden.

In einer Verfahrensvariante können die Reaktionen (II) + (III) → (IV), (V-1) + (III) → (VI-1) und (V-2) + (III) → (VI-2) auch in einem Zwei-Phasen-Gemisch, bestehend aus einer wässrigen Alkalihydroxid-Lösung als Base und einem der oben genannten Kohlenwasserstoffe oder Halogenkohlenwasserstoffe als weiterem Lösungsmittel, unter Verwendung eines Phasentransfer-Katalysators wie Tetrabutylammoniumhydrogensulfat oder Tetrabutylammoniumbromid durchgeführt werden.

Die Verfahrensschritte (II) + (III) → (IV), (V-1) + (III) → (VI-1) und (V-2) + (III) → (VI-2) erfolgen bei der Umsetzung mit Aminderivaten [A in (III) = N] im Allgemeinen in einem Temperaturbereich von +50°C bis +150°C. Bei der Umsetzung mit Alkohölderivaten [A in (III) = O] werden die Reaktionen im Allgemeinen in einem Temperaturbereich von -20°C bis +120°C, bevorzugt bei 0°C bis +60°C durchgeführt.

Die Bromierung in den Verfahrensschritten (VI-1) → (VII-1) bzw. (VI-2) → (VII-2) wird vorzugsweise in einem Halogenkohlenwasserstoff als Lösungsmittel, insbesondere in Tetrachlormethan, in einem Temperaturbereich von +50°C bis +100°C durchgeführt. Als Bromierungsmittel eignen sich elementares Brom sowie insbesondere *N*-Bromsuccinimid (NBS), gegebenenfalls unter Zusatz von α,α'-Azobis(isobutyronitril) (AIBN) als Initiator.

Inerte Lösungsmittel für die Verfahrensschritte (VII-1) + (VIII-1) → (IV) und (VII-2) + (VIII-2) → (IV) sind beispielsweise Alkohole wie Methanol, Ethanol, n-Propanol, Isopropanol, n-Butanol oder *tert*.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylsulfoxid, *N,N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist ein Gemisch aus Dimethylsulfoxid und Wasser.

Als Basen für die Verfahrensschritte (VII-1) + (VIII-1) → (IV) und (VII-2) + (VIII-2) → (IV) eignen sich übliche anorganische Basen. Hierzu gehören insbesondere Alkalihydroxide wie beispielsweise Lithium-, Natrium- oder Kaliumhydroxid, Alkalihydrogencarbonate wie Natrium- oder Kaliumhydrogencarbonat, Alkali- oder Erdalkalicarbonate wie Lithium-, Natrium-, Kalium-, Calcium- oder Cäsiumcarbonat, oder Alkalihydrogenphosphate wie Dinatrium- oder Dikaliumhydrogenphosphat. Bevorzugt wird Natrium- oder Kaliumcarbonat verwendet.

Als Palladium-Katalysator für die Verfahrensschritte (VII-1) + (VIII-1) → (IV) und (VII-2) + (VIII-2) → (IV) ["Suzuki-Kupplung"] sind beispielsweise Palladium auf Aktivkohle, Palladium-(II)-acetat, Tetrakis-(triphenylphosphin)-palladium(0), Bis-(triphenylphosphin)-palladium(II)-chlorid, Bis-(acetonitril)-palladium(II)-chlorid und [1,1'-Bis(diphenylphosphino)ferrocen]dichlor-palladium(II)-Dichlormethan-Komplex geeignet [vgl. z.B. J. Hassan et al., Chem. Rev. 102, 1359-1469 (2002)].

Die Reaktionen (VII-1) + (VIII-1) → (IV) und (VII-2) + (VIII-2) → (IV) werden im Allgemeinen in einem Temperaturbereich von +20°C bis +150°C, bevorzugt bei +50°C bis +100°C durchgeführt.

Die Hydrolyse der Ester- bzw. Nitril-Gruppe Z¹ im Verfahrensschritt (IV) → (I-A) erfolgt nach üblichen Methoden, indem man die Ester bzw. Nitrile in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol, bei der Nitril-Hydrolyse bevorzugt Wasser und/oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von 0°C bis +100°C, bevorzugt bei +0°C bis +50°C. Die Nitril-Hydrolyse wird im Allgemeinen in einem Temperaturbereich von +50°C bis +150°C, bevorzugt bei +80°C bis +120°C durchgeführt.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die erfindungsgemäßen Verbindungen der Formel (I), in welcher Z für eine Gruppe der Formel 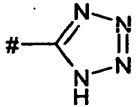 steht,
können hergestellt werden, indem man Verbindungen der Formel (IV), in welcher Z¹ für Cyano steht, in einem inerten Lösungsmittel mit einem Alkali-Azid in Gegenwart von Ammoniumchlorid oder mit Trimethylsilylazid gegebenenfalls in Gegenwart eines Katalysators umsetzt.

Inerte Lösungsmittel für diese Umsetzung sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Dimethylsulfoxid, Dimethylformamid, *N,N'*-Dimethylpropylenharnstoff (DMPU) oder *N-*Methylpyrrolidon (NMP). Ebenso ist es möglich, Gemische der genannten Lösungsmittel einzusetzen. Bevorzugt wird Toluol verwendet.

Als Azid-Reagenz ist insbesondere Natriumazid in Gegenwart von Ammoniumchlorid oder Trimethylsilylazid geeignet. Letztere Reaktion kann vorteilhafterweise in Gegenwart eines Katalysators durchgeführt werden. Hiefür eignen sich insbesondere Verbindungen wie Di-n-butylzinnoxid, Trimethylaluminium oder Zinkbromid. Bevorzugt wird Trimethylsilylazid in Kombination mit Din-butylzinnoxid verwendet.

Die Reaktion wird im Allgemeinen in einem Temperaturbereich von +50°C bis +150°C, bevorzugt bei +60°C bis +110°C durchgeführt. Die Umsetzung kann bei normalem, erhöhtem oder erniedrigtem Druck durchgeführt werden (z.B. von 0.5 bis 5 bar). Im Allgemeinen arbeitet man bei Normaldruck.

Die erfindungsgemäßen Verbindungen der Formel (I), in welcher Z für eine Gruppe der Formel 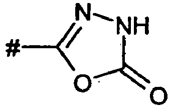 steht,
können hergestellt werden, indem man Verbindungen der Formel (IV), in welcher Z¹ für Methoxy-oder Ethoxycarbonyl steht, zunächst in einem inerten Lösungsmittel mit Hydrazin in Verbindungen der Formel (IX) 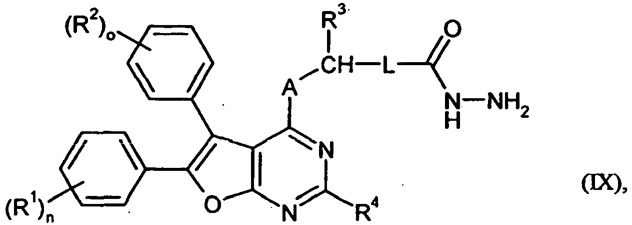 in welcher A, L, R¹, R², R³, R⁴, n und o jeweils die oben angegebenen Bedeutungen haben,
überführt und dann in einem inerten Lösungsmittel mit Phosgen oder einem Phosgen-Äquivalent, wie beispielsweise *N,N'*-Carbonyldiimidazol, umsetzt.

Als inerte Lösungsmittel sind für den ersten Schritt dieser Reaktionsfolge insbesondere Alkohole wie Methanol, Ethanol, n-Propanol, Isopropanol, n-Butanol oder *tert*.-Butanol, oder Ether wie Diethylether, Dioxan, Tetrahydrofuran, Glykoldimethylether oder Diethylenglykoldimethylether geeignet. Ebenso ist es möglich, Gemische dieser Lösungsmittel einzusetzen. Bevorzugt wird ein Gemisch aus Methanol und Tetrahydrofuran verwendet. Der zweite Reaktionsschritt wird vorzugsweise in einem Ether, insbesondere in Tetrahydrofuran durchgeführt. Die Umsetzungen erfolgen im Allgemeinen in einem Temperaturbereich von 0°C bis +70°C unter Normaldruck.

Die erfindungsgemäßen Verbindungen der Formel (I), in welcher L für eine Gruppe der Formel *-L¹-Q-L² steht, worin L¹, L² und Q die oben angegebenen Bedeutungen haben, können alternativ auch dadurch hergestellt werden, dass man Verbindungen der Formel (X) 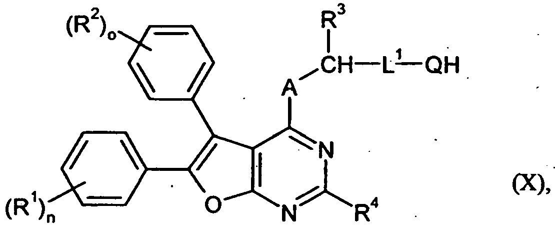 in welcher A, L¹, Q, R¹, R², R³, R⁴, n und o jeweils die oben angegebenen Bedeutungen haben,
in Gegenwart einer Base gegebenenfalls in einem inerten Lösungsmittel mit einer Verbindung der Formel (XI)

X²-L²-Z¹ (XI),

in welcher L² und Z¹ die oben angegebenen Bedeutungen haben
und
- X²: für eine Abgangsgruppe, wie beispielsweise Halogen, Mesylat oder Tosylat, steht,
oder im Falle, dass L² für -CH₂CH₂- steht, mit einer Verbindung der Formel (XII) 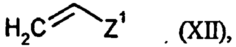 in welcher Z¹ die oben angegebene Bedeutung hat,
in Verbindungen der Formel (IV-A) 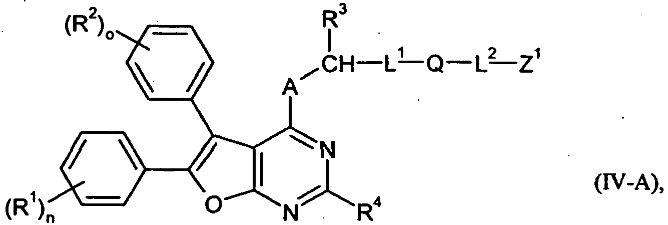 in welcher A, L¹, L², Q, Z¹, R¹, R², R³, R⁴, n und o jeweils die oben angegebenen Bedeutungen haben,
überführt und diese dann entsprechend dem zuvor beschriebenen Verfahren weiter umsetzt.

Die Verbindungen der Formel (X) können ausgehend von einer Verbindung der Formel (II), (V-1) oder (V-2) durch basenkatalysierte Reaktion mit einer Verbindung der Formel (XIII) 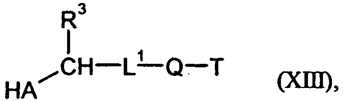 in welcher A, L¹, Q und R³ jeweils die oben angegebenen Bedeutungen haben
und
- T: für Wasserstoff oder eine temporäre O- bzw. N-Schutzgruppe steht,
sowie entsprechend weitere Umsetzung analog zu den zuvor beschriebenen Verfahrensvarianten [B] bzw. [C] erhalten werden, wobei im Falle der Reaktionssequenz (V-1) bzw. (V-2) → (IV-A) die Reihenfolge der einzelnen Verfahrensschritte, falls zweckmäßig, auch variiert werden kann (vgl. auch die nachfolgenden Reaktionsschemata 2-8).

Für die Verfahrensschritte (X) + (XI) bzw. (XII) → (IV-A) und (II) + (XIII) → (X) finden die zuvor für die Umsetzungen (II) + (III) → (IV), (V-1) + (III) → (VI-1) bzw. (V-2) + (III) → (VI-2) beschriebenen Reaktionsparameter wie Lösungsmittel, Basen und Reaktionstemperaturen in analoger Weise Anwendung.

Die Verbindungen der Formeln (II), (III), (V-1), (VIII-1), (V-2), (VIII-2), (XI), (XII) und (XIII) sind kommerziell erhältlich, literaturbekannt oder können in Analogie zu literaturbekannten Verfahren hergestellt werden (vgl. z.B. WO 03/018589; siehe auch Reaktionsschema 1).

Die Herstellung der erfindungsgemäßen Verbindungen kann durch die folgenden Syntheseschemata veranschaulicht werden:

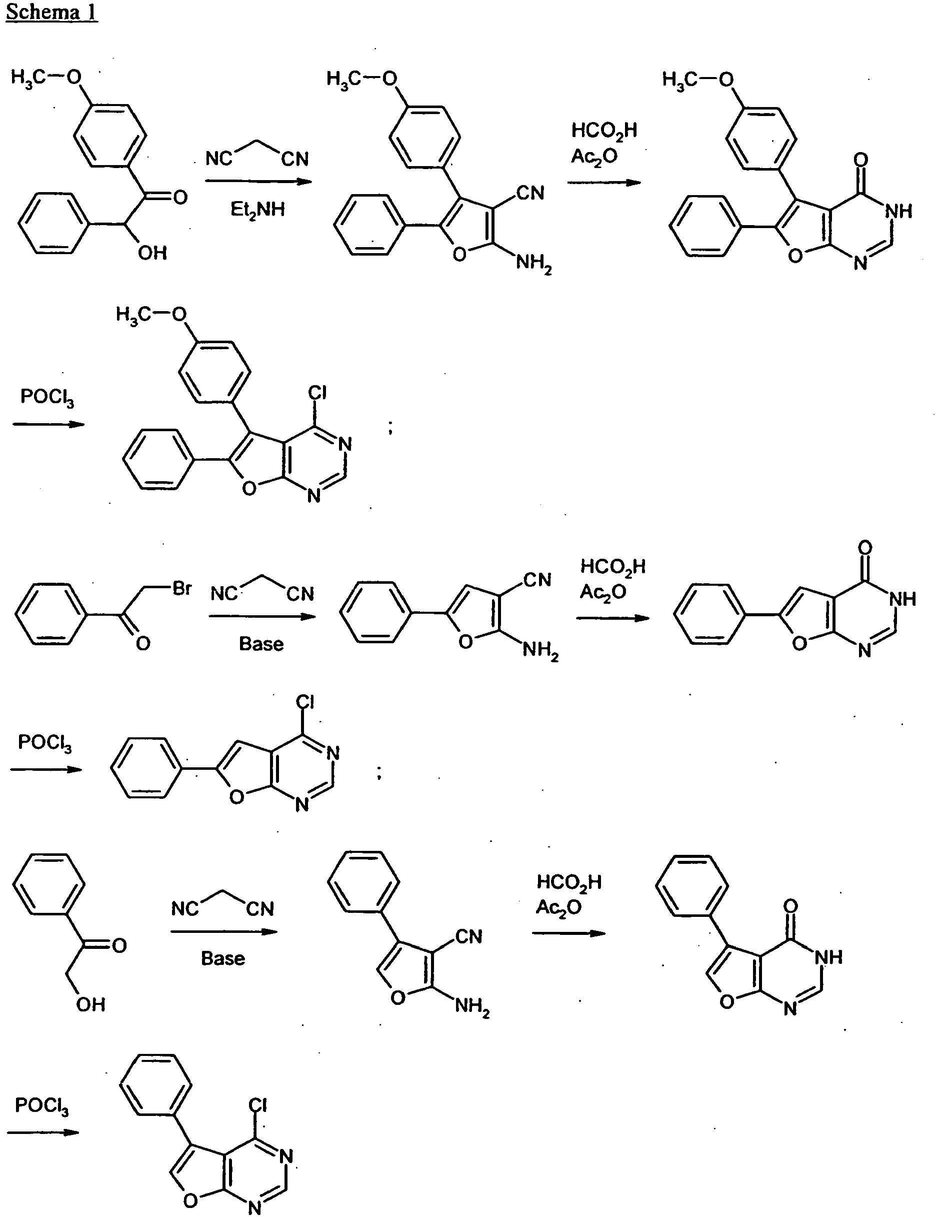

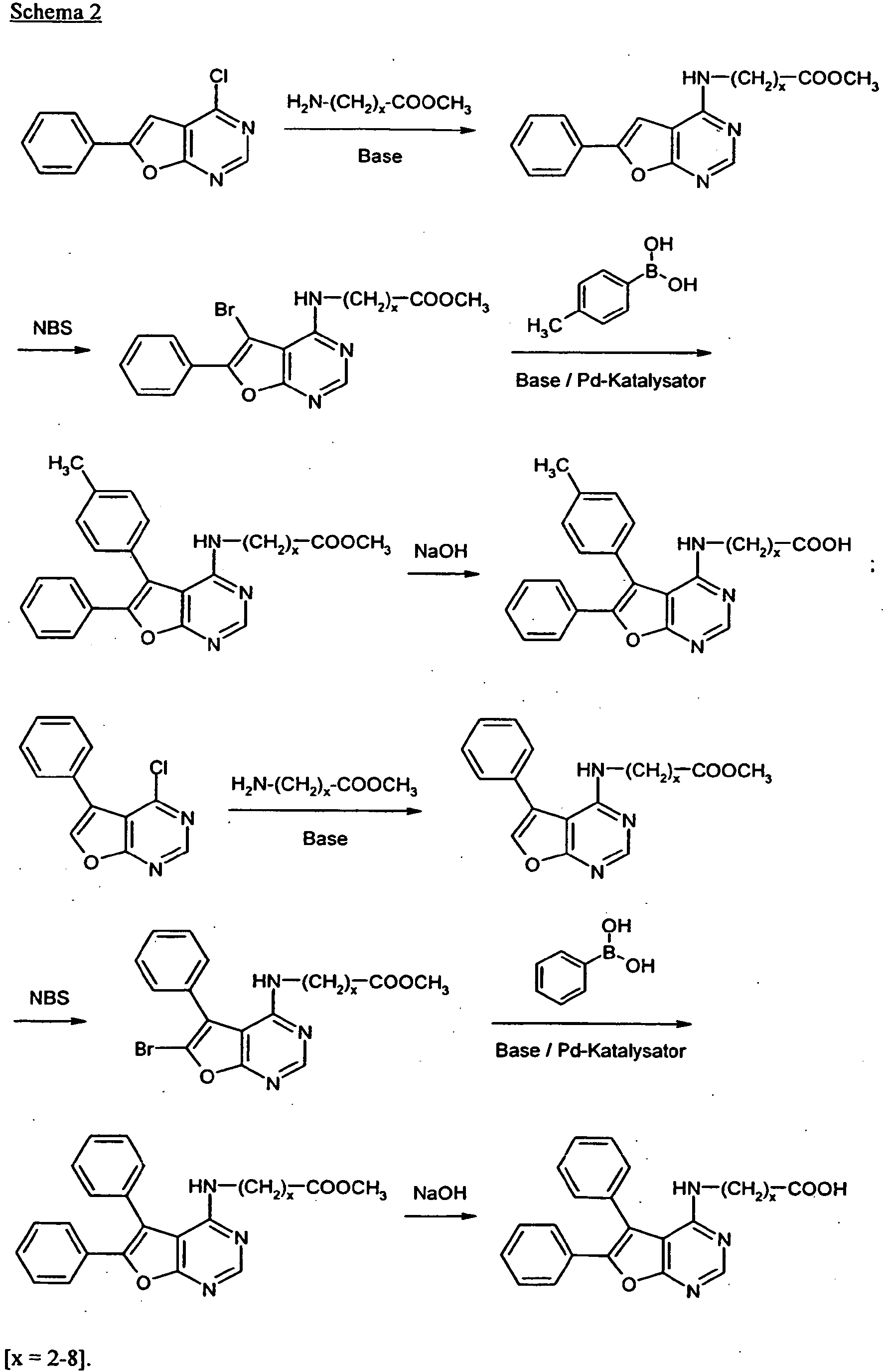

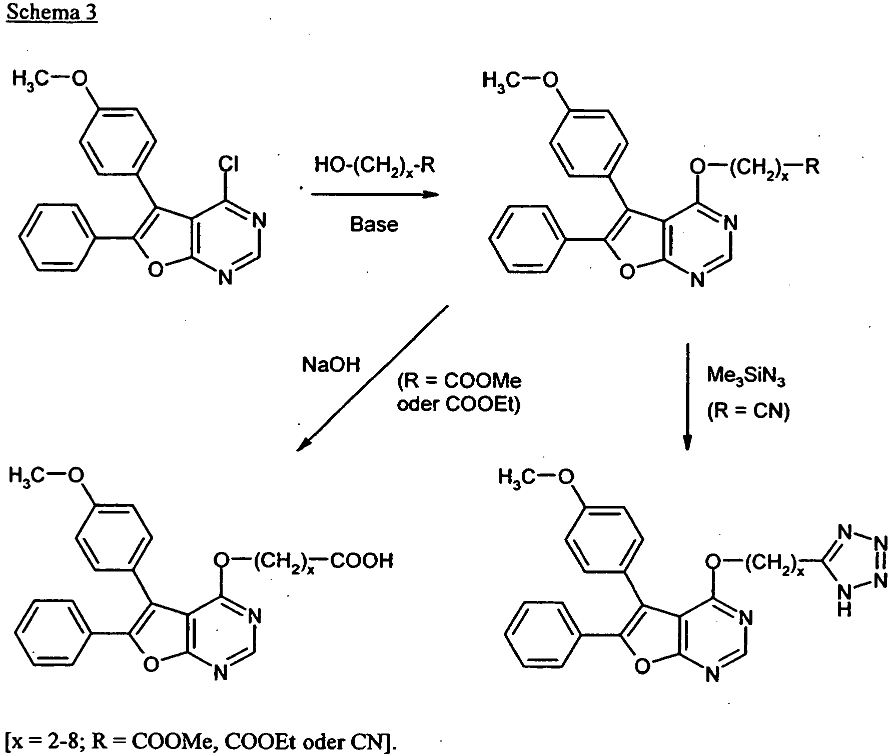

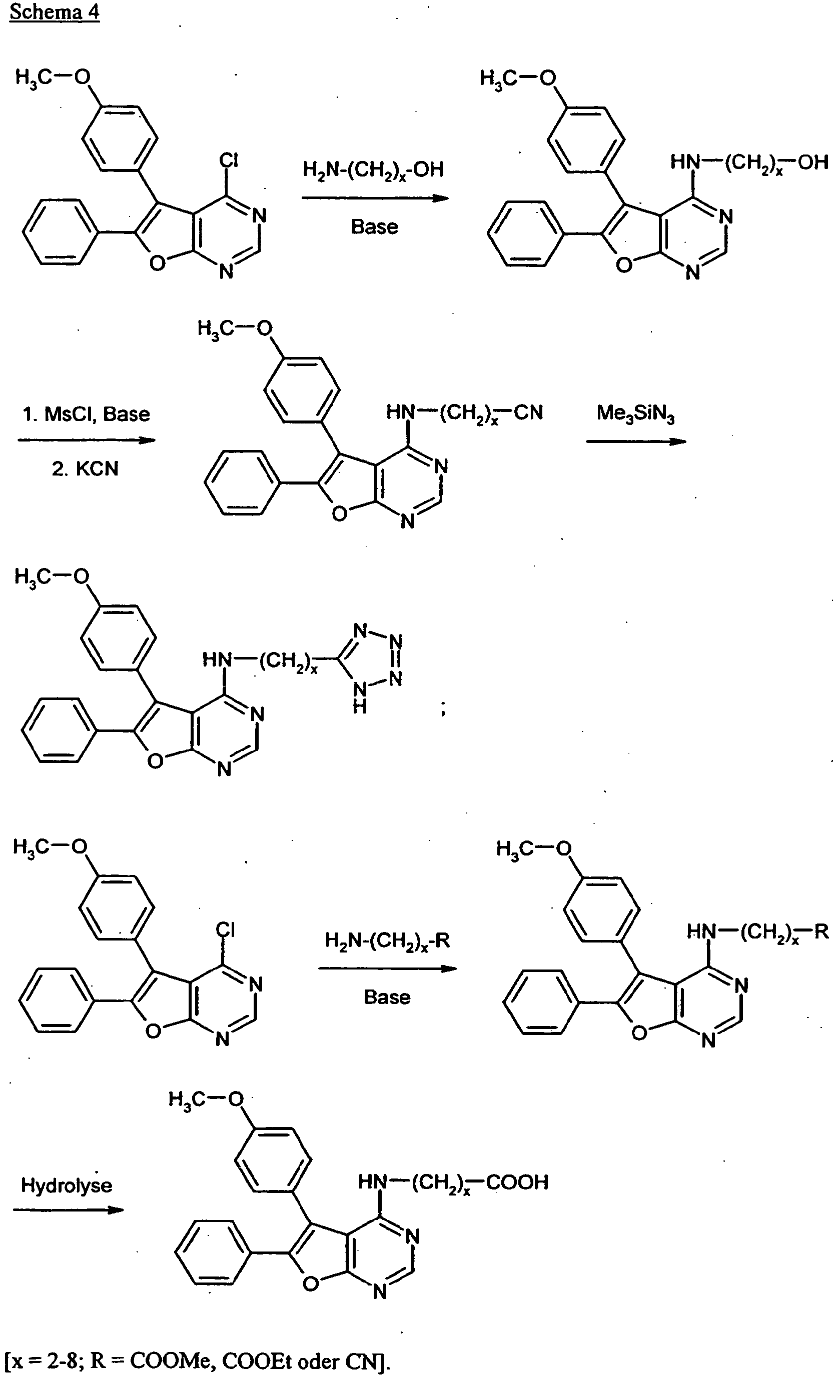

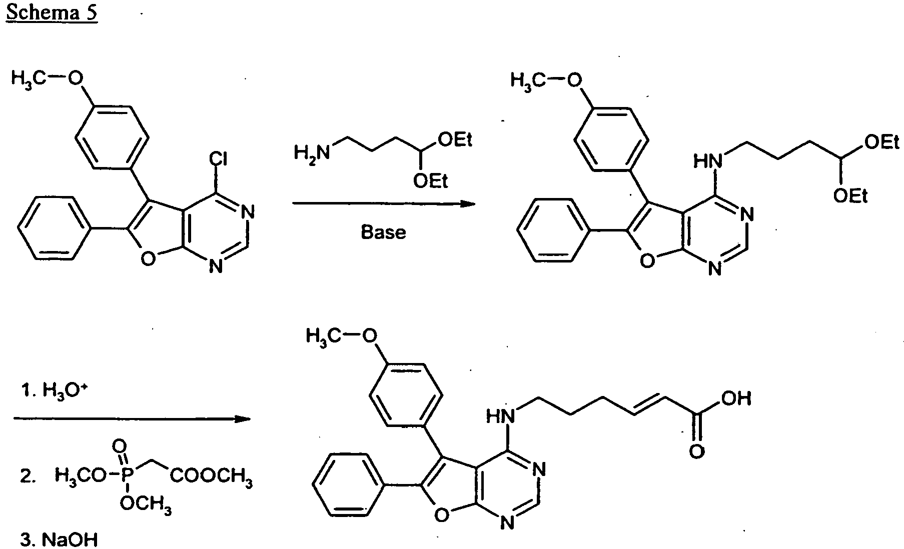

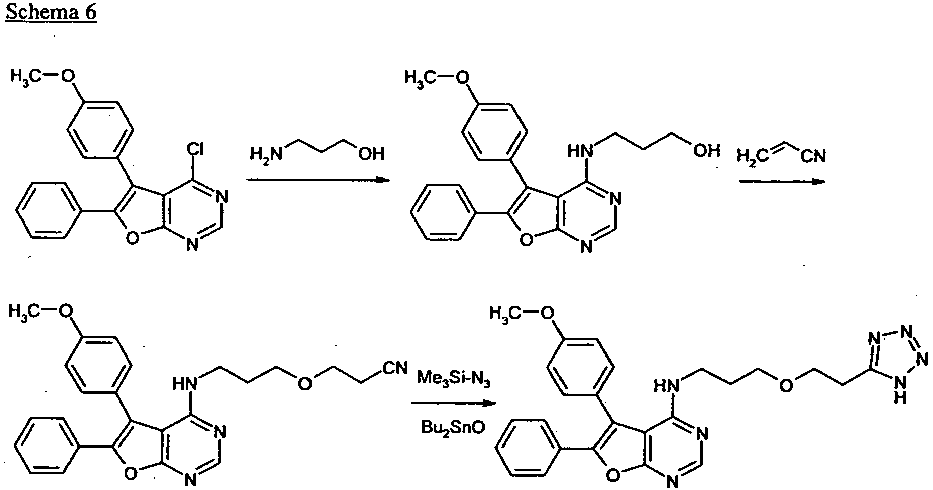

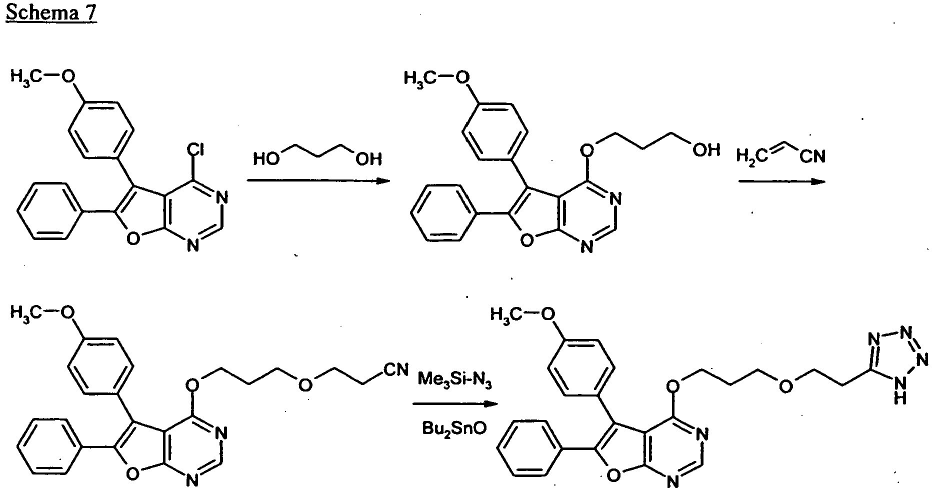

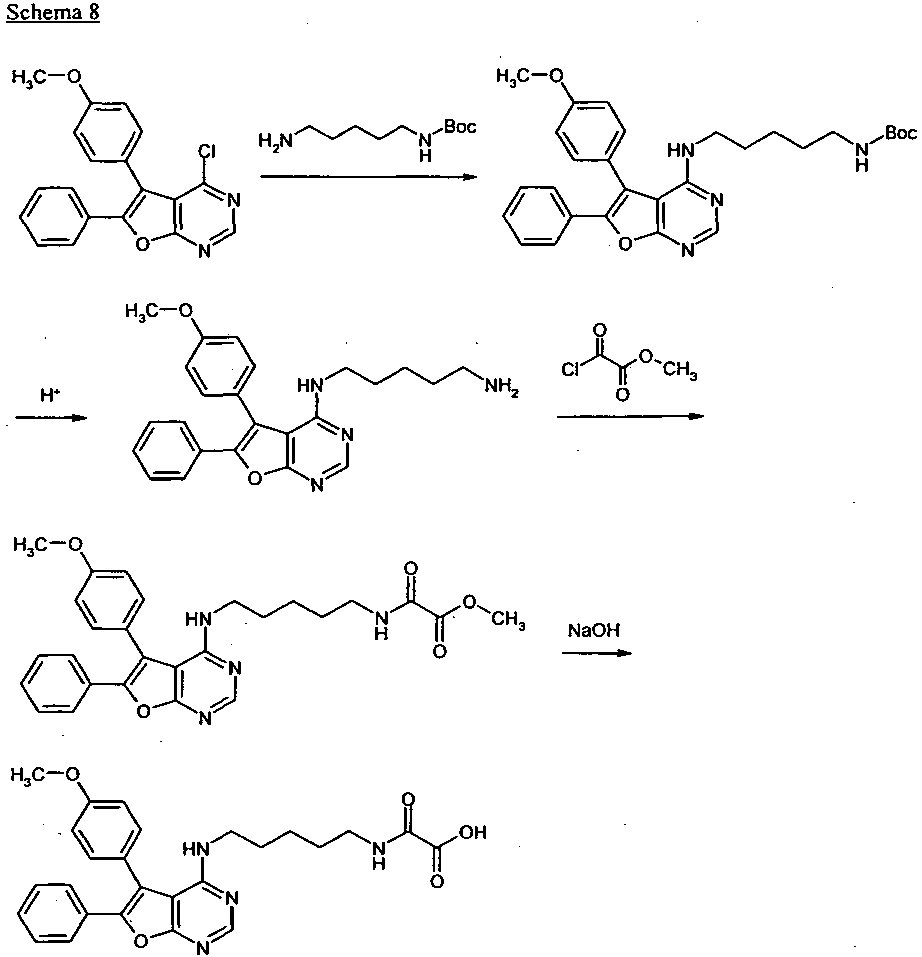

Die erfindungsgemäßen Verbindungen besitzen wertvolle pharmakologische Eigenschaften und können zur Vorbeugung und Behandlung von Erkrankungen bei Menschen und Tieren verwendet werden.

Sie eignen sich insbesondere zur Prophylaxe und/oder Behandlung von kardiovaskulären Erkrankungen wie beispielsweise der stabilen und instabilen Angina pectoris, von peripheren und kardialen Gefäßerkrankungen, des Bluthochdruckes und der Herzinsuffizienz, der pulmonalen Hypertonie, von peripheren Durchblutungsstörungen, zur Prophylaxe und/oder Behandlung von thromboembolischen Erkrankungen und Ischämien wie Myokardinfarkt, Hirnschlag, transistorischen und ischämischen Attacken sowie Subarachnoidalblutungen, und zur Verhinderung von Restenosen wie beispielsweise nach Thrombolysetherapien, percutan-transluminalen Angioplastien (PTA), Koronarangioplastien (PTCA) und Bypass.

Darüber hinaus können die erfindungsgemäßen Verbindungen zur Behandlung von Arteriosklerose, Hepatitis, asthmatischen Erkrankungen, chronisch-obstruktiven Atemwegserkrankungen (COPD), fibrosierenden Lungenerkrankungen wie idiopathische pulmonale Fibrose (IPF) und ARDS, entzündlichen vaskulären Erkrankungen wie Sklerodermie und Lupus erythematodes, Nierenversagen, Arthritis und Osteoporose verwendet werden.

Weiterhin können die erfindungsgemäßen Verbindungen zur Prophylaxe und/oder Behandlung von Krebserkrankungen, insbesondere von metastasierenden Tumoren eingesetzt werden.

Ferner können die erfindungsgemäßen Verbindungen auch als Zusatz zum Konservierungsmedium eines Organtransplantates wie z.B. Nieren, Lungen, Herz oder Inselzellen verwendet werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- Verbindungen, die die humane neutrophile Elastase inhibieren, wie beispielsweise Sivelestat oder DX-890 (Reltran);
- die Signaltransduktionskaskade inhibierende Verbindungen, wie beispielsweise Tyrosinkinase- und/oder Serin/Threoninkinase-Inhibitoren, insbesondere Imatinib, Gefitinib, Erlotinib, Sorafenib und Sunitinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise BAY 59-7939, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin All-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- abs.: absolut
- Ac: Acetyl
- Ac₂O: Essigsäureanhydrid
- Boc: *tert*.-Butoxycarbonyl
- Bu: Butyl
- c: Konzentration
- DC: Dünnschicht-Chromatographie
- DCI: direkte chemische Ionisation (bei MS)
- DIBAH: Diisobutylaluminiumhydrid
- DIEA: Diisopropylethylamin ("Hünig-Base")
- DMAP: 4-*N,N*-Dimethylaminopyridin
- DME: 1,2-Dimethoxyethan
- DMF: *N,N*-Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ESI: Elektrospray-Ionisation (bei MS)
- Et: Ethyl
- Fp.: Schmelzpunkt
- GC: Gaschromatographie
- ges.: gesättigt
- h: Stunde(n)
- HPLC: Hochdruckflüssigchromatographie
- konz.: konzentriert
- LC-MS: Flüssigchromatographie-gekoppelte Massenspektrometrie
- Me: Methyl
- min: Minute(n)
- Ms: Methansulfonyl (Mesyl)
- MS: Massenspektrometrie
- NBS: *N*-Bromsuccinimid
- NMR: Kernresonanzspektrometrie
- Pd/C: Palladium auf Aktivkohle

- rac.: racemisch
- RP: reverse phase (Umkehrphase, bei HPLC)
- RT: Raumtemperatur
- Rₜ: Retentionszeit (bei HPLC)
- TFA: Trifluoressigsäure
- THF: Tetrahydrofuran

### LC-MS-, HPLC- und GC-Methoden:

### Methode 1 (HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 2 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS):

Instrument: Micromass Plattform LCZ mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 4 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 6 (HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 7 (LC-MS):

Instrument: Micromass Quattro LCZ mit HPLC Agilent Serie 1100; Säule: Phenomenex Onyx Monolithic C18, 100 mm x 3 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 8 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 9 (LC-MS):

Gerätetyp MS: Waters ZQ; Gerätetyp HPLC: Waters Alliance 2795; Säule: Merck Chromolith RP18e, 100 mm x 3 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 10 (LC-MS):

Instrument: Micromass Quattro LCZ mit HPLC Agilent Serie 1100; Säule: Phenomenex Gemini 3µ, 30 mm x 3.00 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 11 (LC-MS):

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B → 7.0 min 95% B → 9.0 min 95% B; Fluss: 0.0 min 1.0 ml/min → 7.0 min 2.0 ml/min → 9.0 min 2.0 ml/min; Ofen: 35°C; UV-Detektion: 210 nm.

### Methode 12 (GC):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Ausgangsverbindungen und Intermediate:

### Beispiel 1A

### (4-Methoxyphenyl)[(trimethylsilyl)oxy]acetonitril

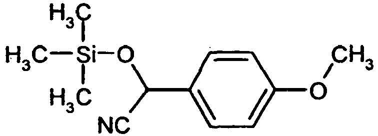

Analog Literaturvorschrift [*J*. *Chem. Soc. Perkin Trans. I*, 1992, 2409-2417] wird zu einer Mischung von 290.0 g (2130 mmol) 4-Methoxybenzaldehyd und 1.156 g (3.622 mmol) Zinkiodid in 37.5 Liter Benzol bei RT unter Kühlung innerhalb von ca. 5 min eine Lösung von 221.88 g (2236 mmol) Trimethylsilylcyanid in 25 Liter Benzol gegeben. Die Mischung wird 90 min bei RT gerührt und dann im Vakuum eingeengt. Der Rückstand wird durch Säulenfiltration an Kieselgel (Laufmittel: Cyclohexan/Ethylacetat 4:1) gereinigt. Es werden 442.4 g (88.3% d. Th.) der Zielverbindung erhalten.
HPLC (Methode 1): Rₜ = 3.76 min
MS (DCI): m/z = 253 (M+NH₄)⁺
¹H-NMR (400 MHz, CDCl₃): δ = 7.49 (d, 2H), 6.92 (d, 2H), 5.42 (s, 1H) 3.81 (s, 3H).

### Beispiel 2A

### 2-Hydroxy-1-(4-methoxyphenyl)-2-phenylethanon

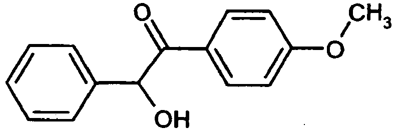

Gemäß Literaturvorschrift [*J*. *Chem. Soc. Perkin Trans. I*, 1992, 2409-2417] werden 292 ml (2.08 mol) Diisopropylamin in 3.6 Liter 1,2-Dimethoxyethan gelöst und auf -78°C abgekühlt. 826 ml n-Butyllithium-Lösung (2.5 M in n-Hexan, 2.066 mol) werden unterhalb von -60°C zudosiert. Die Mischung wird 15 min bei < -60°C nachgerührt und dann eine Lösung von 442 g (1.877 mol) (4-Methoxyphenyl)[(trimethylsilyl)oxy]acetonitril in 1.41 Liter 1,2-Dimethoxyethan bei < -60°C zugetropft. Nach weiterem Rühren für 30 min bei -60°C wird eine Lösung von 199.3 g (1.878 mol) Benzaldehyd in 1.4 Liter 1,2-Dimethoxyethan innerhalb von 20 min bei -60°C zugegeben. Anschließend wird das Reaktionsgemisch langsam über 4 h auf RT erwärmt. Man setzt 7 Liter gesättigte Ammoniumchlorid-Lösung hinzu und extrahiert mit Ethylacetat. Die organische Phase wird mit gesättigter Ammoniumchlorid-Lösung gewaschen, getrocknet und im Vakuum konzentriert. Der Rückstand wird in 7 Liter Dioxan und 5 Liter Methanol aufgenommen und mit 6 Liter 1 N Salzsäure versetzt. Die Mischung wird 3 h bei RT gerührt, dann werden 3 Liter gesättigte Natriumchlorid-Lösung zugesetzt und das Gemisch mit 6.5 Liter Ethylacetat extrahiert. Die organische Phase wird mit 1.0 Liter 1 N Natronlauge und mit gesättigter Natriumchlorid-Lösung gewaschen, getrocknet und im Vakuum konzentriert. Der Rückstand wird in 2 Liter Diisopropylether aufgenommen, vom Unlöslichen abdekantiert und mit Kristallen angeimpft. Die entstehende Suspension wird 2 h bei RT gerührt und die Kristalle dann abgesaugt. Man wäscht mit 300 ml Diisopropylether und Petrolether und trocknet im Vakuum. Es werden 236.8 g (47.8% d. Th.) der Zielverbindung erhalten.
HPLC (Methode 1): Rₜ = 4.23 min
MS (DCI): m/z = 260 (M+NH₄)⁺, 243 (M+H)⁺
¹H-NMR (400 MHz, CDCl₃): δ = 7.92 (d, 2H), 7.38-7.28 (m, 5H), 6.88 (d, 2H), 5.90 (d, 1H), 4.64 (d, 1H), 3.82 (s, 3H).

### Beispiel 3A

### 2-Amino-4-(4-methoxyphenyl)-5-phenyl-3-furonitril

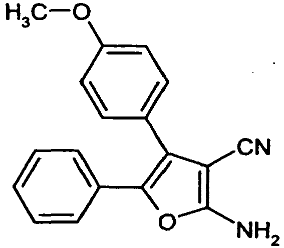

236 g (974 mmol) 2-Hydroxy-1-(4-methoxyphenyl)-2-phenylethanon und 83.66 g (1266 mmol) Malonsäuredinitril werden in 470 ml DMF gelöst und unter Eisbad-Kühlung mit 86.6 ml (836.7 mmol) Diethylamin versetzt. Nach 1 h wird die Mischung auf RT erwärmt und noch 4 h bei RT nachgerührt, bevor 2.5 Liter Wasser und einige Impfkristalle zugefügt werden. Nach 30 min wird überstehendes Wasser abdekantiert und durch 1.25 Liter frisches Wasser ersetzt. Die Suspension wird durchgemischt und überstehendes Wasser erneut abdekantiert. Der klebrig-kristalline Rückstand wird in Ethylacetat aufgenommen und dann im Vakuum beinahe vollständig eingeengt. Man verrührt den Rückstand mit 730 ml Diisopropylether und lässt die Suspension über Nacht bei RT stehen. Der Feststoff wird dann abgesaugt und im Vakuum getrocknet. Es werden 211.5 g (57.6% d. Th.) der Titelverbindung erhalten.
HPLC (Methode 1): Rₜ = 4.60 min
MS (DCI): m/z = 308 (M+NH₄)⁺, 291 (M+H)⁺
¹H-NMR (400 MHz, CDCl₃): δ = 7.39-7.33 (m, 5H), 7.28-7.18 (m, 3H), 6.93 (d, 2H), 5.02 (s, 2H), 3.85 (s, 3H).

### Beispiel 4A

### 5-(4-Methoxyphenyl)-6-phenylfuro[2,3-d]pyrimidin-4(3H)-on

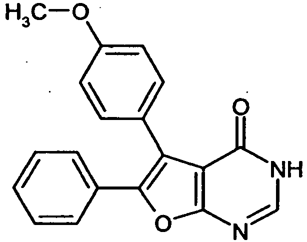

Zu 1600 ml (16.96 mol) Essigsäureanhydrid werden bei 0°C 800 ml (21.21 mol) Ameisensäure getropft. Die Mischung wird 30 min bei 0°C gerührt und dann 211 g (727 mmol) 2-Amino-4-(4-methoxyphenyl)-5-phenyl-3-furonitril zugesetzt. Die Kühlung wird entfernt und die Mischung erwärmt; bei ca. 80°C setzt Gasentwicklung ein, die nach ca. 3 h aufhört. Man rührt insgesamt 24 h unter Rückfluss (Badtemperatur ca. 130°C). Nach Abkühlen auf RT wird 2 h bei 10°C gerührt und der entstandene Feststoff abfiltriert. Man wäscht den Rückstand mit Diethylether und trocknet im Hochvakuum. Es werden 135.6 g (58.6% d. Th.) der Titelverbindung erhalten.
HPLC (Methode 1): Rₜ = 4.38 min
MS (DCI): m/z = 336 (M+NH₄)⁺, 319 (M+H)⁺
¹H-NMR (400 MHz, CDCl₃): δ = 10.3 (br. s, 1H), 7.95 (s, 1H), 7.58-7.53 (m, 2H), 7.47 (d, 2H), 7.33-7.27 (m, 3H), 6.95 (d, 2H), 3.86 (s, 3H).

### Beispiel 5A

### 4-Chlor-5-(4-methoxyphenyl)-6-phenylfuro[2,3-d]pyrimidin

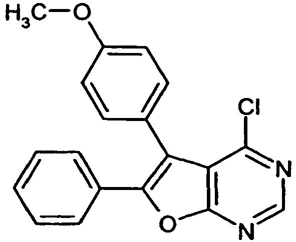

135 g (424 mmol) 5-(4-Methoxyphenyl)-6-phenylfuro[2,3-d]pyrimidin-4(3*H*)-on werden bei RT in 675 ml (7241 mmol) Phosphoroxychlorid suspendiert und die Mischung dann zum Sieden erhitzt (HCl-Entwicklung). Nach 1 h wird die dunkle Lösung auf RT abgekühlt und zu einer kräftig gerührten Mischung von 2.25 Liter Wasser und 4.05 Liter konz. Ammoniak-Lösung (25 Gew.%) getropft (Erwärmung auf 55-75°C, pH >9). Nach Ende der Zugabe wird auf RT abgekühlt und die Mischung dreimal mit je 1.0 Liter Dichlormethan extrahiert. Die vereinigten organischen Phasen werden getrocknet und im Vakuum eingeengt. Der Rückstand wird mit Diethylether verrührt, abgesaugt und im Hochvakuum getrocknet. Es werden 134.4 g (94.1% d. Th.) der Titelverbindung erhalten.
HPLC (Methode 1): Rₜ = 4.96 min
MS (DCI): m/z = 354 (M+NH₄)⁺, 337 (M+H)⁺
¹H-NMR (400 MHz, CDCl₃): δ = 8.76 (s, 1H), 7.62 (d, 2H), 7.40-7.30 (m, 5H), 7.03 (d, 2H), 3.90 (s, 3H).

### Beispiel 6A

### 2-Amino-5-phenyl-3-furonitril

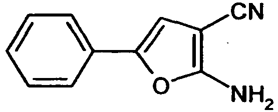

Zu einer Mischung von 60.0 g (301 mmol) Bromacetophenon und 25.89 g (391.86 mmol) Malonsäuredinitril in 130 ml DMF werden bei RT 68.6 ml (663 mmol) Diethylamin getropft (Kühlung erforderlich, um die Temperatur zu halten). Gegen Ende der Zugabe wird die Kühlung entfernt, die Mischung 1 h bei RT gerührt und dann auf 385 ml Wasser gegeben. Es wird mit weiteren 125 ml Wasser verdünnt und 20 min bei RT gerührt. Man saugt den ausgefallenen Feststoff ab, wäscht zweimal mit je 125 ml Wasser, saugt trocken und wäscht mit Petrolether. Der Rückstand wird im Hochvakuum getrocknet. Es werden 33.3 g (50.1% d. Th.) der Zielverbindung als gelb-braune Kristalle erhalten.
HPLC (Methode 1): Rₜ = 4.27 min
MS (DCI): m/z = 202 (M+NH₄)⁺, 185 (M+H)⁺
¹H-NMR (400 MHz, CDCl₃): δ = 7.51-7.45 (m, 2H), 7.39-7.32 (m, 3H), 6.54 (s, 1H), 4.89 (br. s, 1H).

### Beispiel 7A

### 6-Phenylfuro[2,3-d]pyrimidin-4(3H)-on

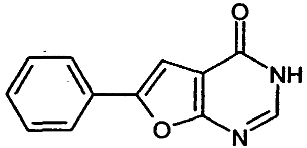

Zu 884.9 ml (9.378 mol) Essigsäureanhydrid werden bei 0°C 424.5 ml (11.25 mol) Ameisensäure getropft. Die Mischung wird 30 min bei 0°C gerührt und dann 69.1 g (0.375 mol) 2-Amino-5-phenyl-3-furonitril zugesetzt. Die Kühlung wird entfernt und die Mischung erwärmt; bei ca. 80°C setzt Gasentwicklung ein, die nach ca. 3 h aufhört. Man rührt insgesamt 24 h unter Rückfluss (Badtemperatur ca. 130°C). Nach Abkühlen der Suspension auf RT wird mit 750 ml Diisopropylether versetzt, auf 0°C gekühlt und abfiltriert. Man wäscht den Rückstand mit Diisopropylether und trocknet im Hochvakuum. Erhalten werden 50.83 g (58.7% d. Th.) der Zielverbindung als brauner Feststoff.
HPLC (Methode 1): Rₜ = 3.92 min
MS: m/z = 213 (M+H)⁺
¹H-NMR (400 MHz, DMSO-d₆): δ = 12.68 (br. s, 1H), 8.17 (s, 1H), 7.88 (d, 2H), 7.52-7.48 (m, 3H), 7.42-7.38 (m, 1H).

### Beispiel 8A

### 4-Chlor-6-phenylfuro[2,3-d]pyrimidin

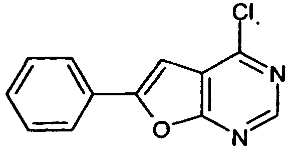

50 g (235.6 mmol) 6-Phenylfuro[2,3-d]pyrimidin-4(3*H*)-on werden bei RT in 375 ml (4023 mmol) Phosphoroxychlorid suspendiert und die Mischung zum Sieden erhitzt (HCl-Entwicklung). Nach 1 h wird die dunkle Lösung auf RT abgekühlt und zu einer kräftig gerührten Mischung von 1.25 Liter Wasser und 2.25 Liter konz. Ammoniak-Lösung (25 Gew.-%) getropft (Erwärmung auf 55-75°C, pH >9). Nach Ende der Zugabe wird auf RT abgekühlt und die Mischung dreimal mit je 1.6 Liter Dichlormethan extrahiert. Die vereinigten organischen Phasen werden getrocknet und im Vakuum eingeengt. Der Rückstand wird mit Diethylether verrührt, abgesaugt und im Hochvakuum getrocknet. Erhalten werden 47.3 g (87% d. Th.) der Zielverbindung.
HPLC (Methode 1): Rₜ = 4.67 min
MS: m/z = 231 (M+H)⁺
¹H-NMR (300 MHz, DMSO-d₆): δ = 8.84 (s, 1H), 8.05 (m, 2H), 7.77 (s, 1H), 7.61-7.50 (m, 3H).

### Beispiel 9A

### 2-Amino-4-phenyl-3-furonitril

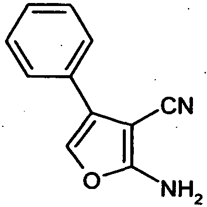

Zu einer Mischung von 10 g (73.4 mmol) Hydroxyacetophenon und 4.852 g (73.4 mmol) Malonsäuredinitril in 24 ml DMF werden unter Kühlung bei RT 3.78 ml (36.7 mmol) Diethylamin getropft. Die dunkle Mischung wird 2 h bei RT gerührt und dann langsam unter Rühren und Kühlung auf Wasser (200 ml) gegeben. Der ausgefallene Niederschlag wird 30 min bei ca. 10°C nachgerührt, abgesaugt, zweimal mit Wasser wieder aufgeschlämmt und erneut abgesaugt. Der Rückstand wird im Hochvakuum bis zur Gewichtskonstanz getrocknet. Erhalten werden 10.99 g (81.2% d. Th.) der Zielverbindung als gelb-brauner Feststoff.
LC-MS (Methode 2): Rₜ =1.81 min.; m/z = 185 (M+H)⁺
¹H-NMR (400 MHz, DMSO-d₆): δ = 7.54 (d, 2H), 7.50 (s, 2H), 7.45-7.32 (m, 4H).

### Beispiel 10A

### 5-Phenylfuro[2,3-d]pyrimidin-4(3H)-on

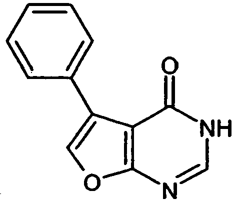

108.5 ml (1154 mmol) Essigsäureanhydrid werden auf 0°C gekühlt und unter Argon mit 52.2 ml (1384 mmol) Ameisensäure versetzt. Die Mischung wird ca. 45 min bei 0°C gerührt und dann 8.5 g (46.2 mmol) 2-Amino-4-phenyl-3-furonitril in Portionen zugegeben. Es entsteht eine dunkle Mischung, die nach 15 min bei 0°C eine violette Farbe annimmt. Die Kühlung wird entfernt und die nun blaue Suspension auf RT erwärmt. Nach 15 min wird die Mischung auf Rückfluss (Badtemperatur 125-130°C) erhitzt, worauf Gasentwicklung einsetzt. Die Mischung wird über Nacht unter Rückfluss gerührt. Nach dem Abkühlen wird die Mischung im Vakuum konzentriert und der Rückstand im Hockvakuum getrocknet. Aus dem Rohprodukt werden durch Säulenfiltration an Kieselgel (Laufmittelgradient: Dichlormethan → Dichlormethan/Methanol 50:1) ca. 3 g eines tiefdunkelroten bis schwarzen Feststoffs erhalten. Dieser wird in ca. 8 ml Dichlormethan gelöst, mit Diisopropylether gefällt, abgesaugt und im Hochvakuum getrocknet. Erhalten werden 1.81 g (Reinheit ca. 84%, Ausbeute ca. 15% d. Th.) der Zielverbindung als dunkelroter Feststoff.
LC-MS (Methode 3): Rₜ = 3.2 min.; m/z = 211 (M-H)⁺
¹H-NMR (400 MHz, DMSO-d₆): δ = 12.7 (s, 1H), 8.26 (s, 1H), 8.19.(s, 1H), 7.98 (d, 2H), 7.50-7.30 (m, 3H).

### Beispiel 11A

### 4-Chlor-5-phenylfuro[2,3-d]pyrimidin

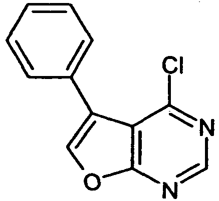

1.8 g (ca. 6.8 mmol) 5-Phenylfuro[2,3-d]pyrimidin-4(3*H*)-on werden bei RT mit 9.5 ml (101.8 mmol) Phosphoroxychlorid versetzt und die Mischung 1h unter Rückfluss erhitzt. Die resultierende schwarze Mischung wird auf RT gekühlt und vorsichtig tropfenweise bei <10°C zu einer gut gerührten, auf 0°C gekühlten Lösung von 70 ml konz. Ammoniak-Lösung und 50 ml Wasser getropft (pH >9). Nach Ende der Zugabe wird die schwarze Suspension auf RT erwärmt und noch 15 min nachgerührt. Der schwarze Feststoff wird abgesaugt, dreimal mit Wasser wieder aufgeschlämmt, erneut abgesaugt und im Hochvakuum getrocknet. Der Feststoff wird in Dichlormethan gelöst und an Kieselgel säulenfiltriert (Laufmittel: Dichlormethan). Es werden 1371 mg (80.6% d. Th.) der Zielverbindung als gelber Feststoff erhalten.
LC-MS (Methode 4): Rₜ = 2.47 min.; m/z = 231 (M+H)⁺
¹H-NMR (400 MHz, DMSO-d₆): δ = 8.90 (s, 1H), 8.49 (s, 1H), 7.64-7.58 (m, 2H), 7.52-7.45 (m, 3H).

### Beispiel 12A

### N-(4,4-Diethoxybutyl)-5-(4-methoxyphenyl)-6-phenylfuro[2,3-d]pyrimidin-4-amin

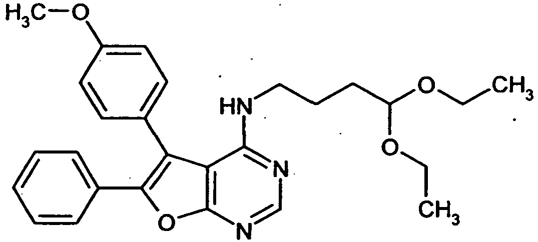

600 mg (1.78 mmol) 4-Chlor-5-(4-methoxyphenyl)-6-phenylfuro[2,3-d]pyrimidin, 344.7 mg (2.14 mmol) 4-Aminobutyraldehyd-Diethylacetal und 0.465 ml (2.67 mmol) DIEA in 5 ml DMF werden über Nacht bei 80°C gerührt. Nach dem Abkühlen wird die Mischung direkt mittels präparativer RP-HPLC (Gradient Acetonitril/Wasser) aufgereinigt. Erhalten werden 746 mg (90.7% d. Th.) der Zielverbindung.
LC-MS (Methode 2): Rₜ = 2.87 min.; m/z = 462 (M+H)⁺
¹H-NMR (400 MHz, DMSO-d₆): δ = 8.34 (s, 1H), 7.48-7.41 (m, 4H), 7.38-7.30 (m, 3H), 7.13 (d, 2H), 5.12 (t, 1H), 4.40 (t, 1H), 3.35 (s, 3H), 3.55-3.47 (m, 2H), 3.42-3.35 (m, 4H), 1.49-1.38 (m, 4H), 1.09 (t, 6H).

### Beispiel 13A

### 4-{[5-(4-Methoxyphenyl)-6-phenylfuro[2,3-d]pyrimidin-4-yl]amino}butanal

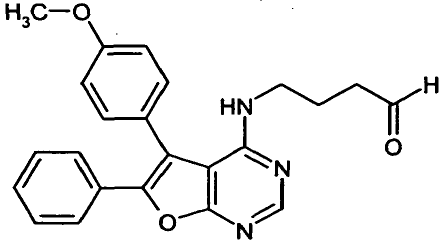

640 mg (1.39 mmol) *N*-(4,4-Diethoxybutyl)-5-(4-methoxyphenyl)-6-phenylfuro[2,3-d]pyrimidin-4-amin werden in 5 ml Aceton gelöst und bei RT mit 1 ml 1 N Salzsäure versetzt. Nach 1 h wird die Reaktionsmischung auf Wasser gegeben und dreimal mit Dichlormethan extrahiert. Die vereinigten organischen Phasen werden mit Pufferlösung (pH 7) und gesättigter Natriumchlorid-Lösung gewaschen, über Magnesiumsulfat getrocknet und im Vakuum konzentriert. Das Rohprodukt wird durch Chromatographie an Kieselgel gereinigt (Laufmittel: Dichlormethan/Ethylacetat 2:1). Erhalten werden 191 mg (35.6% d. Th.) der Zielverbindung.
LC-MS (Methode 5): Rₜ = 2.57 min.; m/z = 388 (M+H)⁺.

### Beispiel 14A

### 6-[(6-Phenylfuro[2,3-d]pyrimidin-4-yl)amino]hexansäuremethylester

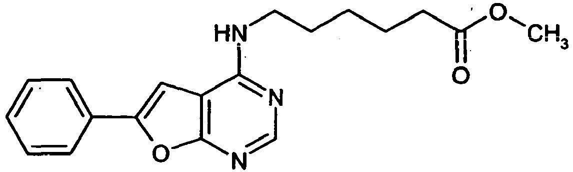

2.0 g (8.67 mmol) 4-Chlor-6-phenylfuro[2,3-d]pyrimidin und 6.04 ml (34.7 mmol) DIEA in 5 ml DMF werden auf 160°C erhitzt. Man gibt 3.15 g (17.34 mmol) 6-Aminohexansäuremethylester-Hydrochlorid hinzu und rührt 4 h bei 160°C. Nach dem Abkühlen wird die Mischung auf Eiswasser gegeben und dreimal mit Ethylacetat extrahiert. Die vereinigten organischen Phasen werden mit gesättigter Ammoniumchlorid-Lösung gewaschen, über Magnesiumsulfat getrocknet und im Vakuum konzentriert. Das zurückbleibende Öl wird mit Methanol versetzt. Man saugt den ausgefallenen Feststoff ab, wäscht mit Methanol nach und trocknet den Feststoff im Hochvakuum. Erhalten werden 1.85 g (57.2% d. Th.) der Zielverbindung.
LC-MS (Methode 5): Rₜ = 2.38 min.; m/z = 340 (M+H)⁺
¹H-NMR (400 MHz, DMSO-d₆): δ = 8.24 (s, 1H), 7.98 (br. s, 1H), 7.79 (d, 2H), 7.51 (t, 2H), 7.43-7.37 (m, 2H), 3.59 (s, 3H), 3.49 (q, 2H), 2.32 (t, 2H), 1.65-1.56 (m, 4H), 1.41-1.35 (m, 2H).

### Beispiel 15A

### 6-[(5-Brom-6-phenylfuro[2,3-d]pyrimidin-4-yl)amino]hexansäuremethylester

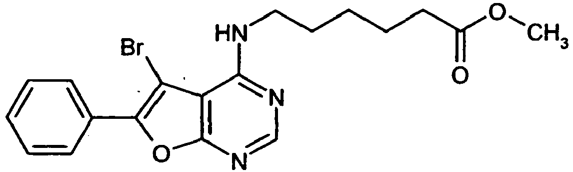

1.75 g (5.15 mmol) 6-[(6-Phenylfuro[2,3-d]pyrimidin-4-yl)amino]hexansäuremethylester werden in 5.2 ml Tetrachlormethan vorgelegt. Bei RT werden 1.054 g (5.92 mmol) *N*-Bromsuccinimid hinzugefügt und die Mischung anschließend ca. 1 h unter Rückfluss erhitzt. Nach dem Abkühlen wird im Vakuum eingeengt und der Rückstand an Kieselgel chromatographiert (Laufmittel: Cyclohexan/Ethylacetat 4:1). Erhalten werden 0.89 g (41.2% d. Th.) der Zielverbindung.
LC-MS (Methode 2): Rₜ = 2.64 min.; m/z = 420 (M+H)⁺
¹H-NMR (400 MHz, DMSO-d₆): δ = 8.33 (s, 1H), 8.02 (d, 2H), 7.61-7.49 (m, 3H), 7.04 (t, 1H), 3.59 (s, 3H), 3.59-3.52 (m, 2H), 2.31 (t, 2H), 1.68-1.54 (m, 4H), 1.40-1.31 (m, 2H).

### Beispiel 16A

### 6-[(5-Phenylfuro[2,3-d]pyrimidin-4-yl)amino]hexansäuremethylester

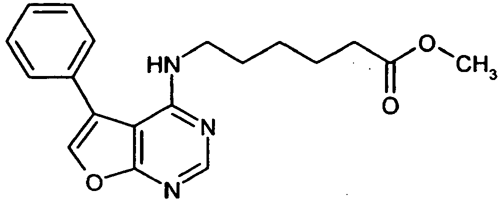

500 mg (2.19 mmol) 4-Chlor-5-phenylfuro[2,3-d]pyrimidin, 1.51 ml (8.67 mmol) DIEA und 1 ml DMF werden auf 160°C erhitzt und mit 787.6 mg (4.34 mmol) 6-Aminohexansäuremethylester-Hydrochlorid versetzt. Nach 4 h bei 160°C wird die Reaktionsmischung abgekühlt, auf Eiswasser gegeben und dreimal mit Ethylacetat extrahiert. Die vereinigten organischen Phasen werden mit gesättigter Ammoniumchlorid-Lösung gewaschen, über Magnesiumsulfat getrocknet und im Vakuum konzentriert. Der Rückstand wird durch Chromatographie an Kieselgel (Laufmittel: Cyclohexan/Ethylacetat 10:1 → 3:1) gereinigt. Erhalten werden 470 mg (63.9% d. Th.) der Zielverbindung.
LC-MS (Methode 5): Rₜ = 2.43 min.; m/z = 340 (M+H)⁺
¹H-NMR (400 MHz, DMSO-d₆): δ = 8.35 (s, 1H), 7.48 (s, 1H), 7.55-7.45 (m, 5H), 5.82 (t, 1H), 3.49 (s, 3H), 3.44 (q, 2H), 2.31 (t, 2H), 1.60-1.50 (m, 4H), 1.33-1.25 (m, 2H).

### Beispiel 17A

### 6-[(6-Brom-5-phenylfuro[2,3-d]pyrimidin-4-yl)amino]hexansäuremethylester

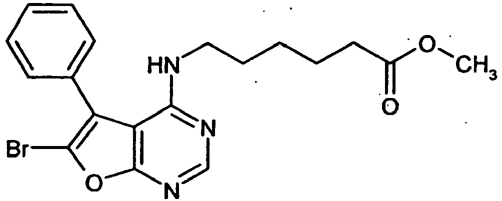

Zu einer Mischung von 100 mg (0.295 mmol) 4-Chlor-5-phenylfuro[2,3-d]pyrimidin und 0.3 ml Tetrachlormethan werden bei RT 57.7 mg (0.324 mmol) *N*-Bromsuccinimid gegeben. Nach 1 h bei RT wird die Reaktionsmischung im Vakuum eingeengt und der Rückstand mittels präparativer RP-HPLC (Gradient Acetonitril/Wasser) aufgereinigt. Erhalten werden 72 mg (58.4% d. Th.) der Zielverbindung.
LC-MS (Methode 2): Rₜ = 2.52 min.; m/z = 418/420 (M+H)⁺
¹H-NMR (400 MHz, DMSO-d₆): δ = 8.32 (s, 1H), 7.61-7.50 (m, 5H), 5.07 (t, 1H), 3.57 (s, 3H), 3.49 (q, 2H), 2.29 (t, 2H), 1.52-1.42 (m, 4H), 1.28-1.20 (m, 2H).

### Beispiel 18A

### 6-{[5-(4-Methoxyphenyl)-6-phenylfuro[2,3-d]pyrimidin-4-yl]amino}hexannitril

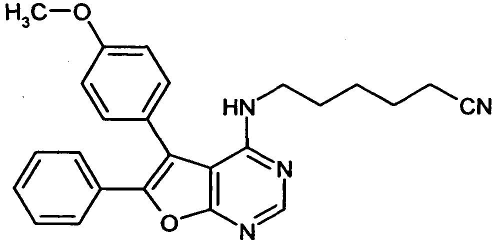

1.0 g (3.0 mmol) 4-Chlor-5-(4-methoxyphenyl)-6-phenylfuro[2,3-d]pyrimidin in 10 ml DMF werden mit 1.15 g (8.9 mmol) DIEA und 0.67 g (5.9 mmol) 6-Aminocapronitril versetzt und 2 h auf 120°C erhitzt. Nach dem Abkühlen wird der Ansatz mit Wasser versetzt und dreimal mit Ethylacetat extrahiert. Die organische Phase wird mit Wasser, verdünnter Salzsäure und gesättigter Natriumchlorid-Lösung gewaschen, getrocknet und eingeengt. Man erhält 1.2 g (98% d. Th.) eines gelben Öls, das als Rohprodukt weiter umgesetzt wird.
LC-MS (Methode 5): Rₜ = 2.87 min.; m/z = 412 (M+H)⁺
¹H-NMR (400 MHz, DMSO-d₆): δ = 8.4 (s, 1H), 7.55 (m, 2H), 7.4 (m, 2H), 7.25 (m, 3H), 7.15 (m, 2H), 4.4 (br. s, 1H), 3.9 (s, 3H), 3.5 (m, 2H), 2.35 (t, 2H), 1.65 (m, 2H), 1.5 (m, 2H), 1.4 (m, 2H).

### Beispiel 19A

### 7-{[5-(4-Methoxyphenyl)-6-phenylfuro[2,3-d]pyrimidin-4-yl]amino}heptannitril

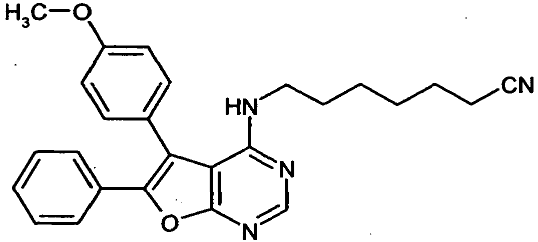

Die Titelverbindung ist in drei Stufen aus 4-Chlor-5-(4-methoxyphenyl)-6-phenylfuro[2,3-d]pyrimidin zugänglich:

### Stufe 1:

1.0 g (3.0 mmol) 4-Chlor-5-(4-methoxyphenyl)-6-phenylfuro[2,3-d]pyrimidin und 0.70 g (5.9 mmol) 6-Aminohexanol in 10 ml DMF werden mit 1.15 g (8.9 mmol) DIEA versetzt und 4 h auf 120°C erhitzt. Der Ansatz wird danach mit Ethylacetat verdünnt, mit Wasser und verdünnter Salzsäure gewaschen, getrocknet und eingeengt. Der Rückstand wird durch RP-HPLC gereinigt (Säule: Gromsil 250 mm x 40 mm, 10 µm; Acetonitril/Wasser-Gradient: 0-3 min 5% Acetonitril, 3-34 min 5% → 98% Acetonitril, 34-38 min 98% Acetonitril). Man erhält 364 mg (29% d. Th.) 6-{[5-(4-Methoxyphenyl)-6-phenylfuro[2,3-d]pyrimidin-4-yl]amino}hexan-1-ol als gelbes Öl, das nach 2 Tagen Stehen erstarrt.

### Stufe 2:

333 mg (0.80 mmol) 6-{[5-(4-Methoxyphenyl)-6-phenylfuro[2,3-d]pyrimidin-4-yl]amino}hexan-1-ol und 122 µl (0.88 mmol) Triethylamin werden in Dichlormethan gelöst und bei 0°C mit 62 µl (0.80 mmol) Methansulfonsäurechlorid, gelöst in Dichlormethan, versetzt (die Gesamtmenge an Dichlormethan beträgt 20 ml). Nach Rühren über Nacht bei RT wird der Ansatz mit Wasser und mit gesättigter Kochsalz-Lösung gewaschen und über Magnesiumsulfat getrocknet. Nach Einengen erhält man 400 mg (quant.) 6-{[5-(4-Methoxyphenyl)-6-phenylfuro[2,3-d]pyrimidin-4-yl]amino}-hexyl-methansulfonat, das als Rohprodukt weiter umgesetzt wird.

### Stufe 3:

Eine Mischung von 400 mg (ca. 0.80 mmol) 6-{[5-(4-Methoxyphenyl)-6-phenylfuro[2,3-d]pyrimidin-4-yl]amino}hexyl-methansulfonat und 526 mg (8.1 mmol) Kaliumcyanid in 20 ml DMF wird über Nacht bei 80°C gerührt. Nach Abkühlen wird mit Essigsäureethylester verdünnt und mit Wasser und gesättigter Kochsalz-Lösung gewaschen. Die organische Phase wird über Magnesiumsulfat getrocknet und eingeengt. Das Rohprodukt wird durch RP-HPLC gereinigt (Säule: Gromsil 250 mm x 40 mm, 10 µm; Acetonitril/Wasser-Gradient: 0-3 min 5% Acetonitril, 3-50 min 5% → 98% Acetonitril, 50-55 min 98% Acetonitril). Man erhält 249 mg (72% d. Th.) der Titelverbindung als gelbliches Öl.
LC-MS (Methode 4): Rₜ = 2.90 min.; m/z = 426 (M+H)⁺
¹H-NMR (400 MHz, DMSO-d₆): δ = 8.35 (s, 1H), 7.55 (m, 4H), 7.35 (m, 3H), 7.15 (m, 2H), 5.0 (t, 1H), 3.85 (s, 3H), 3.45 (m, 2H), 2.45 (t, 2H), 1.55 (m, 2H), 1.4 (m, 2H), 1.3 (m, 2H), 1.15 (m, 2H).

### Beispiel 20A

### 6-{[5-(4-Methoxyphenyl)-6-phenylfuro[2,3-d]pyrimidin-4-yl]oxylhexannitril

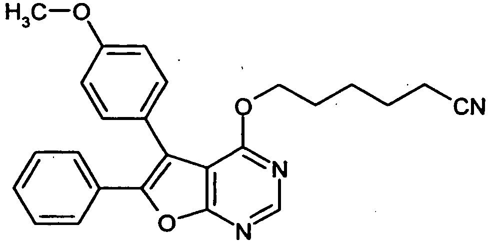

850 mg (3.8 mmol) 6-Hydroxyhexannitril [erhalten nach Eur. J. Med. Chem. 36 (4), 303-311 (2001)] werden in 15 ml DMF gelöst, bei 0°C mit 180 mg 60%-igem Natriumhydrid (Dispersion in Mineralöl; ca. 4.5 mmol) versetzt und das Gemisch 1 h bei Raumtemperatur gerührt. Anschließend werden 1.26 g (3.8 mmol) 4-Chlor-5-(4-methoxyphenyl)-6-phenylfuro[2,3-d]pyrimidin zugesetzt und der Ansatz über Nacht bei 120°C gerührt. Nach dem Abkühlen wird das Gemisch mit Wasser versetzt und mit Ethylacetat extrahiert. Die organische Phase wird getrocknet und eingeengt. Der Rückstand wird durch Flash-Chromatographie an Kieselgel (Laufmittel: Cyclohexan/Ethylacetat 2:1 → Cyclohexan/Ethylacetat 1:2) aufgereinigt. Man erhält 1.05 g (68% d. Th.) eines orangefarbenen Öls, welches als Rohprodukt weiter umgesetzt wird.
LC-MS (Methode 5): Rₜ = 2.97 min.; m/z = 413 (M+H)⁺
¹H-NMR (400 MHz, DMSO-d₆): δ = 8.60 (s, 1H), 7.55 (m, 2H), 7.25-7.45 (m, 5H), 7.0-7.1 (m, 2H), 4.4 (t, 2H), 3.85 (s, 3H), 2.4 (t, 2H), 1.6 (m, 2H), 1.5 (m, 2H), 1.25 (m, 2H).

### Beispiele 21A

### 5-(4-Methoxyphenyl)-6-phenyl-N-{3-[2-cyanoethoxy]propyl}furo[2,3-d]pyrimidin-4-amin

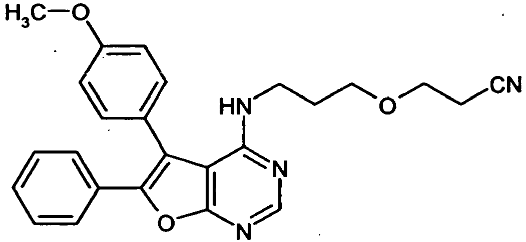

### Stufe 1:

1.00 g (3.0 mmol) 4-Chlor-5-(4-methoxyphenyl)-6-phenylfuro[2,3-d]pyrimidin werden in 10 ml DMF gelöst und mit 1.15 g (8.9 mmol) DIEA versetzt. Es werden-0.45 g (5.9 mmol) 3-Aminopropanol zugegeben und die Mischung dann 2 h lang auf 120°C erhitzt. Nach Abkühlen wird der Ansatz mit Essigsäureethylester verdünnt und nacheinander mit verdünnter Salzsäure und gesättigter Kochsalz-Lösung gewaschen. Die organische Phase wird über Magnesiumsulfat getrocknet und eingeengt. Der Rückstand wird durch RP-HPLC gereinigt (Säule: Gromsil 250 mm x 30 mm, 10 µm; Acetonitril/Wasser-Gradient: 0-3 min 5% Acetonitril, 3-50 min 5% → 98% Acetonitril, 50-55 min 98% Acetonitril). Man erhält 671 mg (60% d. Th.) 3-{[5-(4-Methoxyphenyl)-6-phenylfuro-[2,3-d]pyrimidin-4-yl]amino}propan-1-ol in Form beiger Kristalle.
LC-MS (Methode 2): Rₜ = 2.13 min.; m/z = 376 (M+H)⁺.

### Stufe 2:

300 mg (0.80 mmol) der Verbindung aus Stufe 1 werden mit 47 mg (0.89 mmol) Acrylsäurenitril und 57 mg (0.83 mmol) Natriumethylat versetzt. Das Gemisch wird über Nacht bei 80°C gerührt. Nach Abkühlen wird der Ansatz in DMSO aufgenommen und direkt durch RP-HPLC gereinigt (Säule: Gromsil 250 mm x 30 mm, 10 µm; Acetonitril/Wasser-Gradient: 0-3 min 5% Acetonitril, 3-50 min 5% → 98% Acetonitril, 50-55 min 98% Acetonitril). Man erhält 160 mg (47% d. Th.) der Titelverbindung als gelbes Öl.
LC-MS (Methode 2): Rₜ = 2.43 min.; m/z = 429 (M+H)⁺
¹H-NMR (400 MHz, DMSO-d₆): δ = 8.3 (s, 1H), 7.1-7.45 (m, 9H), 5.2 (m, 1H), 3.85 (s, 3H), 3.5 (m, 4H), ca. 3.3 (m, 2H, teilweise verdeckt durch H₂O) 2.7 (t, 2H), 1.7 (quin, 2H).

### Beispiel 22A

### 5-(4-Methoxyphenyl)-6-phenyl-N-(5-aminopentyl)-furo[2,3-d]pyrimidin-4-amin

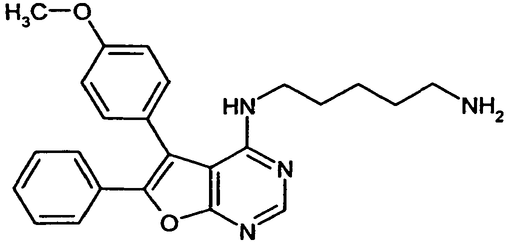

### Stufe 1:

1.00 g (3.0 mmol) 4-Chlor-5-(4-methoxyphenyl)-6-phenylfuro[2,3-d]pyrimidin werden in 5 ml DMF gelöst und mit 1.15 g (8.9 mmol) DIEA versetzt. Es werden 1.20 g (5.9 mmol) 5-[(*tert*.-Butyloxycarbonyl)amino]-1-pentylamin [aus 1,5-Diaminopentan erhältlich gemäß J. Med. Chem. 47 (20), 4933-4940 (2004)] zugegeben und die Mischung dann für 3 h auf 80°C erhitzt. Nach Abkühlen wird der Ansatz mit Dichlormethan verdünnt und nacheinander mit Wasser und gesättigter Kochsalz-Lösung gewaschen. Die organische Phase wird über Magnesiumsulfat getrocknet und eingeengt. Der Rückstand wird durch RP-HPLC gereinigt (Säule: Gromsil 250 mm x 30 mm, 10 µm; Acetonitril/Wasser-Gradient: 0-3 min 5% Acetonitril, 3-50 min 5% → 98% Acetonitril, 50-55 min 98% Acetonitril). Man erhält in zwei Fraktionen insgesamt 1.07 g (67% d. Th.) *tert*.-Butyl-(5-{[5-(4-methoxyphenyl)-6-phenylfuro[2,3-d]pyrimidin-4-yl]amino}pentyl)carbamat in Form beiger Kristalle.
LC-MS (Methode 2): Rₜ = 2.85 min.; m/z = 503 (M+H)⁺.

### Stufe 2:

380 mg (0.76 mmol) der Verbindung aus Stufe 1 werden in 5 ml Methylenchlorid gelöst und nacheinander mit 0.4 ml Anisol sowie 5.5 ml Trifluoressigsäure versetzt. Es wird 2 h bei Raumtemperatur gerührt. Der Ansatz wird mit Dichlormethan verdünnt und mit Natriumhydrogencarbonat-Lösung neutral gewaschen. Nach Waschen der organischen Phase mit gesättigter Natriumchlorid-Lösung wird über Magnesiumsulfat getrocknet. Der nach Einengen verbleibende Rückstand wird in Ethanol gelöst und eingeengt; dieser Vorgang wird dreimal wiederholt. Man erhält 349 mg (82% d. Th.) der Titelverbindung als gelblichen Schaum.
LC-MS (Methode 2): Rₜ = 2.43 min.; m/z = 429 (M+H)⁺
¹H-NMR (400 MHz, DMSO-d₆): δ = 8.3 (s, 1H), 7.1-7.45 (m, 9H), 5.4 (br. m, 1H), 5.1 (m, 1H), 3.85 (s, 3H), ca. 3.3 (m, 2H, teilweise verdeckt durch H₂O), 2.6 (t, 2H), 1.1-1.57 (m, 6H).

### Beispiel 23A

### 4-[3-(2-Cyanoethoxy)ethoxy]-5-(4-methoxyphenyl)-6-phenylfuro[2,3-d]pyrimidin

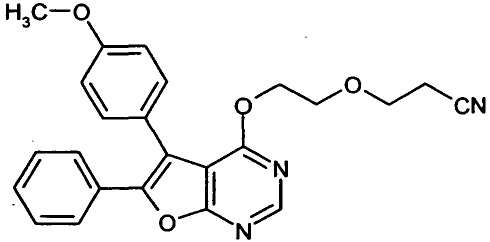

### Stufe 1:

Zu einer Lösung von 461 mg (7.4 mmol) Ethylenglykol in 10 ml DMF werden bei 0°C 59 mg (1.5 mmol) 60%-iges Natriumhydrid gegeben. Nach Aufwärmen auf Raumtemperatur wird 1 h nachgerührt. Dann werden 0.50 g (1.5 mmol) 4-Chlor-5-(4-methoxyphenyl)-6-phenylfuro[2,3-d]pyrimidin zugegeben und der Ansatz 3 h lang bei Raumtemperatur gerührt. Anschließend wird mit Wasser verdünnt und mit Essigsäureethylester extrahiert. Die organische Phase wird mit gesättigter Kochsalz-Lösung gewaschen, über Magnesiumsulfat getrocknet und eingeengt. Das so erhaltene Rohprodukt wird durch RP-HPLC gereinigt (Säule: Gromsil 250 mm x 30 mm, 10 µm; Acetonitril/Wasser-Gradient: 0-3 min 5% Acetonitril, 3-50 min 5% → 98% Acetonitril, 50-55 min 98% Acetonitril). Man erhält auf diese Weise 412 mg (77% d. Th.) 2-{[5-(4-Methoxyphenyl)-6-phenyl-furo[2,3-d]pyrimidin-4-yl]oxy}ethanol.

### Stufe 2:

Analog zu Beispiel 21A, Stufe 2 erhält man aus 350 mg (0.97 mmol) der Verbindung aus Stufe 1 245 mg (61 % d. Th.) der Titelverbindung in Form gelber Kristalle.
Fp.: 103-104°C
LC-MS (Methode 2): Rₜ = 2.84 min.; m/z = 416 (M+H)⁺
¹H-NMR (400 MHz, DMSO-d₆): δ = 8.6 (s, 1H), 7.35-7.55 (m, 7H), 7.05 (m, 2H), 4.55 (m, 2H), 3.85 (s, 3H), 3.7 (m, 2H), 3.45 (t, 2H), 2.7 (t, 2H).

### Beispiel 24A

### 4-[3-(2-Cyanoethoxy)propoxy]-5-(4-methoxyphenyl)-6-phenylfuro[2,3-d]pyrimidin

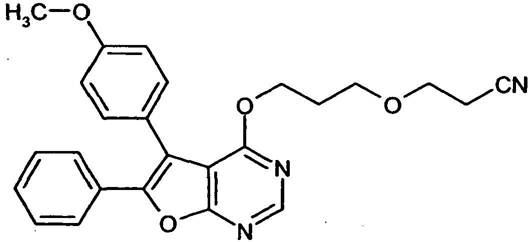

Die Titelverbindung wird analog zu Beispiel 23A in zwei Stufen aus 1,3-Propandiol und 4-Chlor-5-(4-methoxyphenyl)-6-phenylfuro[2,3-d]pyrimidin erhalten.
Ausbeute: 304 mg (89% d. Th.)
Fp.: 88-89°C
LC-MS (Methode 4): Rₜ = 2.94 min.; m/z = 430 (M+H)⁺
¹H-NMR (400 MHz, DMSO-d₆): δ = 8.6 (s, 1H), 7.35-7.55 (m, 7H), 7.05 (m, 2H), 4.45 (m, 2H), 3.85 (s, 3H), 3.7 (m, 2H), 3.5 (t, 2H), 2.7 (t, 2H), 1.7 (quin, 2H).

### Beispiel 25A

### (4-Ethylyphenyl)[(trimethylsilyl)oxy]acetonitril

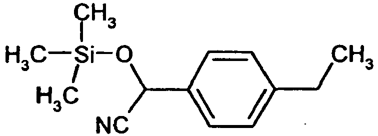

600 g (4.47 mol) 4-Ethylbenzaldehyd werden in 5.3 Litern Toluol mit 2.4 g (7.5 mmol) Zinkiodid vermischt. Bei RT werden unter gelinder Kühlung 587.4 ml (4.7 mol) Trimethylsilylcyanid, gelöst in 3.6 Litern Toluol, über ca. 5 min zugegeben. Die Mischung wird 90 min bei RT gerührt, bevor flüchtige Komponenten im Vakuum entfernt werden und der Rückstand an Kieselgel schnell chromatographiert wird (Eluent: Petrolether/Ethylacetat 9:1). Erhalten werden 990 g (94.9% d. Th.) der Titelverbindung als farbloses Öl.
¹H-NMR (400 MHz, CDCl₃): δ = 7.38 (d, 2H), 7.23 (d, 2H), 4.97 (s, 1H), 2.68 (q, 2H), 1.25 (t, 3H), 0.23 (s, 9H).

### Beispiel 26A

### 1-(4-Ethylphenyl)-2-hydroxy-2-phenylethanon

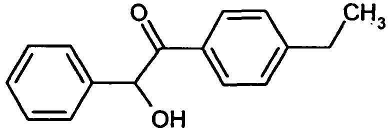

290 ml (2.069 mol) Diisopropylamin werden in 3.6 Litern DME gelöst und auf -78°C vorgekühlt. 820 ml (2.05 mol) n-Butyllithium (2.5 M Lösung in Hexan) werden innerhalb von ca. 20 min zugetropft (Temperatur < -60°C). Nach 15 min bei -60°C wird eine Lösung von 435 g (1.864 mol) (4-Ethylphenyl)[(trimethylsilyl)oxy]acetonitril in 1.4 Litern DME zugetropft (Temperatur < -60°C). Die Mischung wird 30 min bei -60°C nachgerührt, bevor eine Lösung von 189.5 ml (1.864 mol) Benzaldehyd in 1.4 Litern DME zugegeben wird (Dauer ca. 20 min, Temperatur -60°C). Die Mischung wird über 4 h auf RT erwärmt, bevor 7 Liter ges. Ammoniumchlorid-Lösung zugesetzt werden. Die Reaktionsmischung wird mit Ethylacetat extrahiert. Nach Phasentrennung wird die organische Phase mit ges. Ammoniumchlorid-Lösung gewaschen, getrocknet und im Vakuum eingeengt. Der Rückstand wird in 7 Litern Dioxan sowie 5 Litern Methanol gelöst und mit 6 Litern 1 N Salzsäure versetzt. Die Mischung wird über Nacht bei RT gerührt, bevor nach Zugabe von 11 Litern ges. Natriumchlorid-Lösung mit 6.5 Litern Ethylacetat extrahiert wird. Die organische Phase wird mit Wasser und mit ges. Natriumchlorid-Lösung gewaschen, getrocknet und im Vakuum eingeengt. Der Rückstand wird in 2 Litern Diisopropylether gelöst, mit Impfkristallen versetzt und 2 h gerührt. Der ausgefallene Feststoff wird abgesaugt, mit 300 ml Diisopropylether und Petrolether gewaschen und im Vakuum getrocknet. Die Mutterlauge wird aufkonzentriert, und nach Lagern über 2 Tage bei 4°C wird erneut vom ausgefallenen Feststoff abgesaugt, mit ca. 100 ml Diisopropylether und Petrolether gewaschen und im Vakuum getrocknet. Beide Feststoffe werden vereinigt, erhalten werden 154.9 g (34% d. Th.) des Zielprodukts.
HPLC (Methode 1): Rₜ = 4.55 min.
MS (DCI): m/z = 258 (M+NH₄)⁺
¹H-NMR (400 MHz, CDCl₃): δ = 7.85 (d, 2H), 7.48-7.35 (m, 5H), 7.21 (d, 2H), 5.92 (d, 1H), 4.59 (d, 1H), 2.65 (q, 2H), 1.20 (t, 3H).

### Beispiel 27A

### 2-Amino-4-(4-ethylphenyl)-5-phenyl-3-furonitril

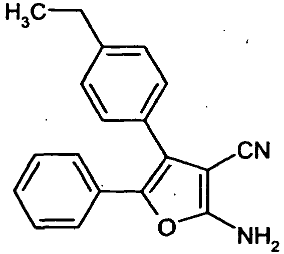

Eine Mischung aus 145 g (603 mmol) 1-(4-Ethylphenyl)-2-hydroxy-2-phenylethanon und 51.8 g (784.4 mmol) Malonsäuredinitril in 2.23 Litern DMF wird auf 0°C gekühlt und unter Kühlung mit 53.7 ml (518 mmol) Diethylamin versetzt. Nach 1 h wird die Reaktionsmischung auf RT erwärmt und weitere 4 h gerührt, bevor 1.5 Liter Wasser zugesetzt werden. Nach 30 min wird ein Großteil des Wassers abdekantiert und durch 750 ml frisches Wasser ersetzt. Die Mischung wird kräftig gerührt, bevor vom klebrigen organischen Rückstand abdekantiert wird. Man löst den Rückstand in Ethylacetat, trocknet und konzentriert im Vakuum, bis das Produkt auszukristallisieren beginnt. Es werden 450 ml Diisopropylether zugesetzt, verrührt und dann über Nacht stehengelassen. Der kristalline Niederschlag wird abgesaugt, zweimal mit 50 ml Diisopropylether gewaschen und im Vakuum getrocknet. Es werden 98.5 g (56.6% d. Th.) des Zielprodukts erhalten.
HPLC (Methode 1): Rₜ = 5.10 min.
MS (DCI): m/z = 306 (M+NH₄)⁺
¹H-NMR (400 MHz, CDCI₃): δ = 7.90-7.82 (m, 4H), 7.28-7.18 (m, 5H), 4.98 (s, 2H), 2.69 (q, 2H), 1.28 (t, 3H).

### Beispiel 28A

### 5-(4-Ethylphenyl)-6-phenylfuro[2,3-d]pyrimidin-4(3H)-on

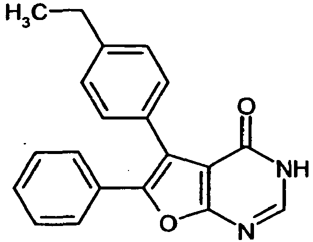

770 ml (8.16 mol) Essigsäureanhydrid werden auf 0°C gekühlt und unter Kühlung mit 372 ml (10.4 mol) Ameisensäure versetzt. Die Mischung wird 30 min bei 0°C gerührt, bevor 98 g (340 mmol) 2-Amino-4-(4-ethylphenyl)-5-phenyl-3-furonitril eingetragen werden. Die Mischung wird auf Rückfluss erhitzt (dabei zunehmend starke Gasentwicklung) und 24 h bei Rückfluss gerührt. Nach Abkühlen wird etwa 2 h bei 10°C gerührt und dann der ausgefallene Feststoff abgesaugt, mit Diisopropylether gewaschen und im Hochvakuum getrocknet. Erhalten werden 69.3 g (64.5% d. Th.) des Zielprodukts.
HPLC (Methode 1): Rₜ = 4.77 min.
MS (DCI): m/z = 334 (M+NH₄)⁺, 317 (M+H)⁺
¹H-NMR (400 MHz, DMSO-d₆): δ = 12.63 (br. s, 1H), 8.19 (s, 1H), 7.43 (d, 2H), 7.40-7.30 (m, 5H), 7.25 (m, 2H), 3.35 (s, 2H), 2.68 (d, 2H), 1.25 (t, 3H).

### Beispiel 29A

### 4-Chlor-5-(4-ethylphenyl)-6-phenylfuro[2,3-d]pyrimidin

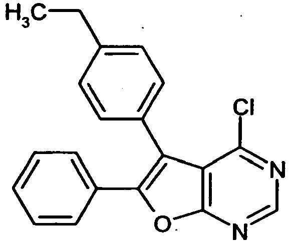

72 g (227.6 mmol) 5-(4-Ethylphenyl)-6-phenylfuro[2,3-d]pyrimidin-4(3*H*)-on werden in 360 ml (4.6 mol) Phosphoroxychlorid vorgelegt und auf Rückfluss erhitzt. Die Mischung wird ca. 1 h bei 120°C gerührt, bevor das Reaktionsgemisch nach Abkühlen auf RT dosier-kontrolliert unter kräftigem Rühren zu einer Mischung von 2.2 Litern 25%-iger Ammoniaklösung und 1.2 Litern Wasser getropft wird (pH >9, Temperatur 55-75°C). Die wässrige Mischung wird dreimal mit Dichlormethan extrahiert, und die vereinigten organischen Phasen werden über Natriumsulfat getrocknet und im Vakuum eingeengt. Der Rückstand wird mit wenig Diisopropylether gewaschen, und nach Abfiltrieren und Trocknen im Hochvakuum erhält man 66.1 g (85.2% d. Th.) des Zielprodukts.
HPLC) (Methode 6): Rₜ = 5.68 min.
MS (DCI): m/z = 335 (M+H)⁺
¹H-NMR (400 MHz, CDCl₃): δ = 8.76 (s, 1H), 7.61 (d, 2H), 7.48-7.30 (m, 7H), 2.78 (q, 2H), 1.36 (t, 3H).

### Beispiel 30A

### 6-Phenylfuro[2,3-d]pyrimidin-4-amin

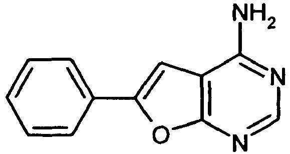

110 g (597 mmol) 2-Amino-5-phenyl-3-furonitril werden in 355 ml (9 mol) Formamid suspendiert und 1.5 h lang erhitzt (Badtemperatur ca. 210°C). Die Mischung wird danach auf RT abgekühlt und in Wasser eingerührt. Der ausgefallene Feststoff wird abgesaugt und mit Wasser gewaschen. Das noch feuchte Produkt wird in Dichlormethan verrührt, erneut abgesaugt und im Vakuum getrocknet. Erhalten werden 106 g (80% d. Th.) der Zielverbindung.
LC-MS (Methode 3): Rₜ = 3.1 min.; m/z = 212 (M+H)⁺
HPLC (Methode 1): Rₜ = 3.63 min.
¹H-NMR (400 MHz, DMSO-d₆): δ = 8.20 (s, 1H), 7.8 (d, 2H), 7.55-7.32 (m, 6H).

### Beispiel 31A

### 5-Brom-6-phenylfuro[2,3-d]pyrimidin-4-amin

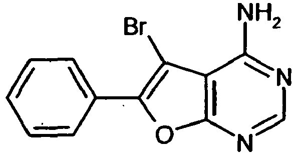

80 g (378.7 mmol) 6-Phenylfuro[2,3-d]pyrimidin-4-amin werden in 770 ml Tetrachlorkohlenstoff auf 60°C erhitzt. 84.3 g (473.4 mmol) *N*-Bromsuccinimid werden hinzugefügt, und die Mischung wird über Nacht unter Rückfluss gerührt. Nach dem Abkühlen wird abfiltriert, der Filterkuchen nacheinander mit Dichlormethan und Acetonitril verrührt und erneut abfiltriert. Der Filterkuchen wird dann im Vakuum getrocknet. Man erhält 86 g des Zielprodukts (78.2% d. Th.).
MS (DCI): m/z = 290/292 (M+H)⁺
¹H-NMR (400 MHz, DMSO-d₆): δ = 8.28 (s, 1H), 8.03 (d, 2H), 7.60-7.50 (m, 5H).

### Beispiel 32A

### 5-Brom-4-chlor-6-phenylfuro[2,3-d]pyrimidin

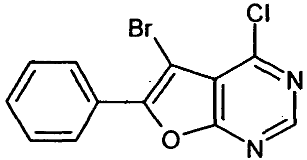

54 g (186 mmol) 5-Brom-6-phenylfuro[2,3-d]pyrimidin-4-amin werden in 135 ml Chloroform vorgelegt, mit 70 ml 4 N Chlorwasserstoff in Dioxan (280 mmol) versetzt und auf Rückfluss erhitzt. tropfenweise werden unter Gasentwicklung 50 ml (372 mmol) Isoamylnitrit zugegeben. Nach Ende der Zugabe wird 3 h bei Rückfluss gerührt, bevor die abgekühlte Reaktionsmischung in Wasser gegeben und mit Dichlormethan extrahiert wird. Die organische Phase wird mit ges. Natriumhydrogencarbonat-Lösung gewaschen, über Natriumsulfat getrocknet und im Vakuum eingeengt. Das Rohprodukt wird durch Chromatographie an Silicagel (Eluent: Dichlormethan) gereinigt. Das Produkt wird zur weiteren Reinigung in Methanol verrührt, abgesaugt und im Hochvakuum getrocknet. Erhalten werden 32 g des Zielprodukts (55.5% d. Th.).
LC-MS (Methode 2): Rₜ = 2.54 min.; m/z = 309/310 (M+H)⁺
HPLC (Methode 1): Rₜ = 5.08 min.
¹H-NMR (400 MHz, CDCl₃): δ = 8.79 (s, 1H), 8.23-8.20 (m, 2H), 7.58-7.51 (m, 3H).

### Beispiel 33A

### [(5-Brom-6-phenylfuro[2,3-d]pyrimidin-4-yl)(methyl)amino]hexansäuremethylester

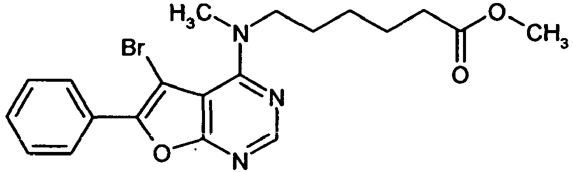

Zu einer Mischung von 500 mg (1.2 mmol) 6-[(5-Brom-6-phenylfuro[2,3-d]pyrimidin-4-yl)amino]-hexansäuremethylester und 112 µl (1.79 mmol) Methyliodid in 1 ml DMF werden bei 0°C portionsweise 52.5 mg (1.32 mmol) 60%-iges Natriumhydrid eingetragen. Die Eiskühlung wird entfernt und die Mischung auf RT erwärmt. Nach 1 h wird mit Wasser und Dichlormethan verdünnt und die abgetrennte wässrige Phase mit Dichlormethan extrahiert. Die vereinigten organischen Phasen werden mit ges. Natriumchlorid-Lösung gewaschen, über Natriumsulfat getrocknet und im Vakuum eingeengt. Erhalten werden 472.6 mg (91.2% d. Th.) eines Öls.
LC-MS (Methode 7): Rₜ = 4.24 min.; m/z = 432/434 (M+H)⁺
¹H-NMR (400 MHz, DMSO-d₆): δ = 8.38 (s, 1H), 8.0 (d, 2H), 7.60-7.50 (m, 3H), 3.70 (t, 2H), 3.29 (s, 3H), 2.29 (t, 2H), 1.72-1.65 (m, 2H), 1.60-1.52 (m, 2H), 1.31-1.26 (m, 2H).

### Beispiel 34A

### (-)-(2R)-2-Methyl-3-(trityloxy)propansäuremethylester

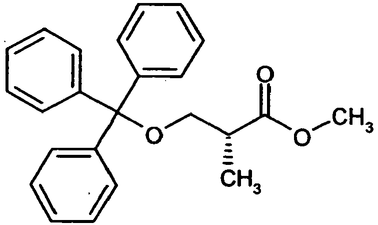

1.5 g (12.7 mmol) (-)-Methyl-D-β-hydroxyisobutyrat werden in 13 ml Dichlormethan und 2.5 ml (17.8 mmol) Triethylamin vorgelegt, auf 0°C gekühlt und mit 4.43 g (15.9 mmol) Triphenylmethylchlorid, gelöst in Dichlormethan, versetzt. Die Kühlung wird entfernt und die Mischung für 2 h gerührt, bevor nach Verdünnen mit Dichlormethan mehrmals mit Wasser und mit ges. Natriumchlorid-Lösung gewaschen wird. Die organische Phase wird über Magnesiumsulfat getrocknet und im Vakuum eingeengt. Das Produkt wird durch Chromatographie an Silicagel (Eluent: Cyclohexan/Ethylacetat 20:1) gereinigt. Erhalten werden 2.81 g des Zielprodukts (61.4% d. Th.).
LC-MS (Methode 2): Rₜ = 2.98 min.; m/z = 243
¹H-NMR (400 MHz, DMSO-d₆): δ = 7.38-7.20 (m, ca. 15H), 3.63 (s, 3H), 3.17-3.09 (m, 2H), 2.72 (q, 1H), 1.05 (d, 3H).
[α]_{D}²⁰ = -15.5°, c = 0.545, Chloroform.

### Beispiel 35A

### (-)-(2S)-2-Methyl-3-(trityloxy)propan-1-ol

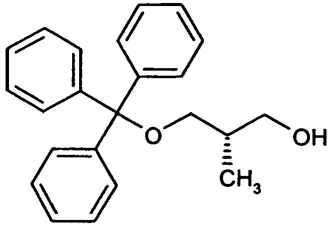

1.4 g (3.88 mmol) (-)-(2*R*)-2-Methyl-3-(trityloxy)propansäuremethylester werden in 5 ml abs. THF gelöst, auf -20°C gekühlt und tropfenweise mit 1.94 ml (1.94 mmol) einer 1 M Lösung von Lithiumaluminiumhydrid in THF versetzt. Die Mischung wird 1 h bei -10°C gerührt, bevor mit einem Gemisch von Aceton und Dichlormethan verdünnt und mit Wasser versetzt wird. Die wässrige Phase wird zweimal mit Dichlormethan extrahiert. Man vereinigt die organischen Phasen, wäscht mit ges. Natriumchlorid-Lösung, trocknet über Magnesiumsulfat und konzentriert im Vakuum. Erhalten werden nach Chromatographie an Silicagel (Eluent: Cyclohexan/Ethylacetat 8:1 1 → 4:1) 0.98 g (75.5% d. Th.) des Zielprodukts.
LC-MS (Methode 8): Rₜ = 2.85 min.; m/z = 243, 355 (M+Na)⁺
MS (DCI): m/z = 243, 350 (M+NH₄)⁺
¹H-NMR (400 MHz, DMSO-d₆): δ = 7.40-7.24 (m, ca. 15H), 4.38 (t, 1H), 3.43-3.37 (m, 1H), 3.32-3.28 (m, 1H), 3.01 (dd, 1H), 2.83 (dd, 1H), 1.84 (m, 1H), 0.88 (d, 3H).
[α]_{D}²⁰ = -30°, c = 0.49, Chloroform.

### Beispiel 36A

### (-)-{[(2S)-2-Methyl-3-(trityloxy)propyl]oxy}essigsäure-tert.-butylester

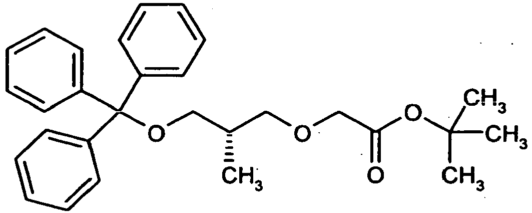

Zu einer Lösung von 800 mg (2.41 mmol) (-)-(2*S*)-2-Methyl-3-(trityloxy)propan-1-ol in 2 ml Dichlormethan werden 53.2 mg (0.12 mmol) Rhodiumdiacetat [als Dimer Rh₂(OAc)₄] gegeben. In die kräftig gerührte Suspension wird unter N₂-Entwicklung langsam ein Überschuss von Diazoessigsäure-*tert*.-butylester (ca. 2 Äquivalente) getropft (Dauer ca. 1 h). Die Reaktionsmischung wird dann mit Dichlormethan verdünnt, dreimal mit Wasser und einmal mit ges. Natriumchlorid-Lösung gewaschen, über Magnesiumsulfat getrocknet und im Vakuum eingeengt. Das Produkt wird durch Chromatographie an Silicagel (Eluent: Cyclohexan/Ethylacetat 10:1) gereinigt. Erhalten werden ca. 1000 mg leicht verunreinigtes Zielprodukt.
MS (DCI): m/z = 464 (M+NH₄)⁺
¹H-NMR (400 MHz, DMSO-d₆): δ = 7.40-7.25 (m, 15H), 3.91 (s, 2H), 3.48 (dd, 1H), 3.35 (dd, 1H), 2.98 (dd, 1H), 2.88 (dd, 1H), 1.98 (m, 1H), 1.41 (s, 9H), 0.89 (d, 3H).
[a]_{D}²⁰ = -6.6°, c = 0.505, Chloroform.

### Beispiel 37A

### (+)-{[(2R)-3-Hydroxy-2-methylpropyl]oxy}essigsäure-tert.-butylester

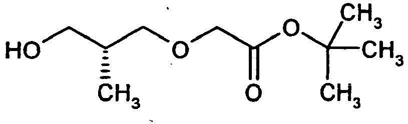

900 mg (ca. 2.02 mmol) (-)-{[(2*S*)-2-Methyl-3-(trityloxy)propyl]oxy}essigsäure-*tert*.-butylester werden in 2 ml Dichlormethan und 0.5 ml Methanol gelöst und zunächst bei 0°C, dann bei RT portionsweise mit einem Überschuss (ca. 3 Äquivalente) wasserfreiem Zinkbromid versetzt. Die Mischung wird 2-3 h bei RT gerührt, bevor nach Verdünnen mit Dichlormethan zweimal mit Wasser und mit ges. Natriumchlorid-Lösung gewaschen wird. Man trocknet über Magnesiumsulfat und engt im Vakuum ein. Mittels Chromatographie an Silicagel (Eluent: Cyclohexan/Ethylacetat 10:1 → 2:1) werden 257 mg des Zielprodukts isoliert (ca. 62% d. Th.).
MS (DCI): m/z = 222 (M+NH₄)⁺
¹H-NMR (400 MHz, DMSO-d₆): δ = 4.40 (t, 1H), 3.95 (s, 2H), 3.42-3.36 (m, 2H), 3.28-3.22 (m, 2H), 1.77 (m, 1H), 1.44 (s, 9H), 0.85 (d, 3H).
[α]_{D}²⁰=+10.5°, c = 0.525, Chloroform.

### Beispiel 38A

### (+)-(2S)-2-Methyl-3-(trityloxy)propansäuremethylester

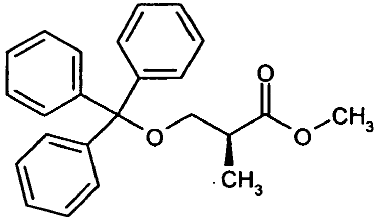

10.33 g (87.5 mmol) (+)-Methyl-L-β-hydroxyisobutyrat werden in 10 ml Dichlormethan und 14.2 ml (174.9 mmol) Pyridin vorgelegt, auf 0°C gekühlt und mit 1.07 g (8.7 mmol) DMAP und dann unter Eiskühlung mit 30.5 g (109 mmol) Triphenylmethylchlorid, gelöst in Dichlormethan, versetzt. Die Kühlung wird entfernt und die Mischung 5 h gerührt, bevor nach Verdünnen mit reichlich Dichlormethan nacheinander mit Wasser, 1 N Salzsäure, ges. Natriumhydrogencarbonat-Lösung und ges. Natriumchlorid-Lösung gewaschen wird. Die organische Phase wird über Natriumsulfat getrocknet und im Vakuum eingeengt. Die ausgefallenen Kristalle werden in Methanol verrührt und nach Abfiltrieren im Vakuum getrocknet. Erhalten werden 25.36 g des Zielprodukts (41.4% d. Th.).
MS (DCI): m/z = 378 (M+NH₄)⁺
[α]_{D}²⁰ = +6.4°, c = 0.555, Chloroform.

### Beispiel 39A

### (+)-(2R)-2-Methyl-3-(trityloxy)propan-1-ol

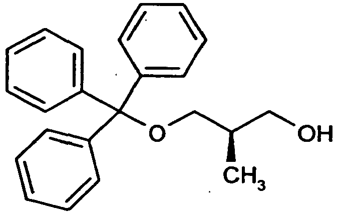

23 g (63.8 mmol) (+)-(2*S*)-2-Methyl-3-(trityloxy)propansäuremethylester werden in 100 ml abs. THF gelöst, auf -20°C abgekühlt und tropfenweise mit 31.9 ml (31.9 mmol) einer 1 M Lösung von Lithiumaluminumhydrid in THF versetzt. Nach Ende der Zugabe wird 10 min bei -10°C nachgerührt, bevor mit Dichlormethan verdünnt und vorsichtig bei ca. 0°C mit ges. Ammoniumchlorid-Lösung versetzt wird. Die organische Phase wird mit ges. Natriumchlorid-Lösung gewaschen, über Natriumsulfat getrocknet und im Vakuum eingeengt. Das Produkt wird durch Chromatographie an Silicagel (Eluent: Cyclohexan/Ethylacetat 5:1) gereinigt. Erhalten werden 11.16 g der Zielverbindung (52.6% d. Th.).
¹H-NMR (400 MHz, DMSO-d₆): δ = 7.40-7.25 (m, ca. 15H), 4.39 (t, 1H), 3.43-3.38 (m, 1H), 3.32-3.28 (m, 1H), 3.02 (dd, 1H), 2.82 (dd, 1H), 1.84 (m, 1H), 0.88 (d, 3H).
[α]_{D}²⁰ = +25.1°, c = 0.575, Chloroform.

### Beispiel 40A

### (-)- {[(2R)-2-Methyl-3-(trityloxy)propyl]oxy}essigsäureethylester

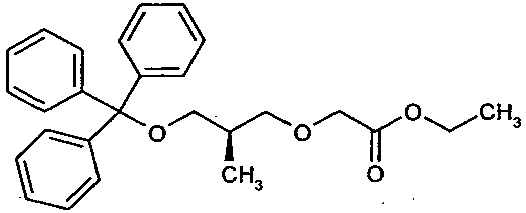

Zu einer Suspension von 5.0 g (15.0 mmol) (+)-(2*R*)-2-Methyl-3-(trityloxy)propan-1-ol und 0.332 g (0.75 mmol) Rhodium(II)acetat-Dimer in 25 ml trockenem Dichlormethan werden unter kräftigem Rühren bei 0°C 3.4 ml (33.1 mmol) Diazoessigsäureethylester getropft. Nach Ende der Zugabe wird noch 5 min bei 0°C gerührt, dann auf RT erwärmt und weitere 2.5 h bei RT nachgerührt. Nach Verdünnen mit Dichlormethan wird mit Wasser und ges. Natriumchlorid-Lösung gewaschen, über Natriumsulfat getrocknet und im Vakuum eingeengt. Das Rohprodukt wird durch Chromatographie an Silicagel (Eluent: Cylohexan/Ethylacetat 20:1) gereinigt. Erhalten werden 5.18 g der Zielverbindung (79.7% d. Th.).
MS (DCI): m/z = 436 (M+NH₄)⁺
¹H-NMR (400 MHz, DMSO-d₆): δ = 7.40-7.25 (m, 15H), 4.10 (q, 2H), 4.03 (s, 2H), 3.48 (dd, 1H), 3.38 (dd, 1H), 2.98 (dd, 1H), 2.40 (dd, 1H), 1.98 (m, 1H), 1.18 (t, 3H), 0.90 (d, 3H).
[α]_{D}²⁰ = -0.9°, c = 0_{.}47, Chloroform.

### Beispiel 41A

### (-)-{[(2S)-3-Hydroxy-2-methylpropyl]oxy}essigsäureethylester

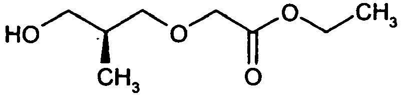

2.75 g (6.58 mmol) (-)-{[(2*R*)-2-Methyl-3-(trityloxy)propyl]oxy}essigsäureethylester werden in 25 ml Ethanol gelöst, mit 300 mg 10%-igem Pd/C versetzt und bei RT 3 h unter einer WasserstoffAtmosphäre (Normaldruck) gerührt. Man filtriert über Celite und engt das aufgefangene Filtrat im Vakuum ein. Das Rohprodukt wird durch Filtration an Silicagel (Eluent: Cylohexan/Ethylacetat 7:1 → 4:1) gereinigt. Erhalten werden 1.05 g der Zielverbindung (90.6% d. Th.).
¹H-NMR (400 MHz, DMSO-d₆): δ = 4.40 (t, 1H), 4.12 (q, 2H), 4.05 (s, 2H), 3.41 (dd, 1H), 3.38-3.32 (m, 1H), 3.30-3.23 (m, 2H), 1.78 (m, 1H), 1.20 (t, 3H), 0.85 (d, 3H).
[α]_{D}²⁰ =-12.4°, c = 0.50, Chloroform.

### Beispiel 42A

### 3-[(1S)-2-Benzyloxy-1-methylethoxy]propansäure-tert.-butylester

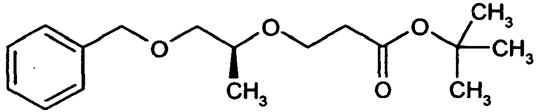

Eine Mischung von 20 g (120.3 mmol) (+)(*S*)-1-Benzyloxy-2-propanol und 123 g (962 mmol) *tert*.-Butylacrylat wird auf 0°C gekühlt und in mehreren Portionen mit 962 mg (24 mmol, 60%-ig) Natriumhydrid versetzt. Die Mischung wird 10 min bei 0°C gerührt, bevor vorsichtig mit ges. Ammoniumchlorid-Lösung versetzt wird. Nach Phasentrennung wird die wässrige Phase zweimal mit Dichlormethan extrahiert. Man vereinigt die organischen Phasen, trocknet über Magnesiumsulfat und engt im Vakuum, dann im Hochvakuum ein. Das Rohprodukt wird durch Chromatographie an Silicagel (Eluent: Cyclohexan/Ethylacetat 30:1) gereinigt. Erhalten werden 18.4 g der Zielverbindung (51.9% d. Th.).
¹H-NMR (400 MHz, DMSO-d₆): δ = 7.38-7.25 (m, 5H), 4.49 (s, 2H), 3.64 (t, 2H), 3.61-3.58 (m, 1H), 3.40 (dd, 1H), 3.32 (dd, 1H), 2.39 (t, 2H), 1.39 (s, 9H), 1.05 (d, 3H).

### Beispiel 43A

### (+)-3-[(1S)-2-Hydroxy-1-methylethoxy]propansäure-tert.-butylester

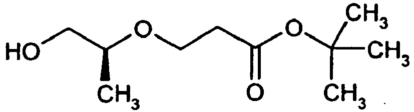

18.1 g (61.5 mmol) 3-[(1*S)*-2-Benzyloxy-1-methylethoxy]propansäure-*tert*.-butylester werden in 100 ml Ethanol gelöst, mit 1.96 g 10%-igem Pd/C versetzt und bei RT 2 h unter einer WasserstoffAtmosphäre (Normaldruck) gerührt. Man filtriert über Celite und engt das aufgefangene Filtrat im Vakuum ein. Erhalten werden 13.8 g der Zielverbindung als Rohprodukt, das nicht weiter gereinigt wird (ca. 92% d. Th.).
MS (DCI): m/z = 222 (M+NH₄)⁺
¹H-NMR (400 MHz, DMSO-d₆): δ = 4.50 (t, 1H), 3.67-3.60 (m, 2H), 3.40-3.34 (m, ca. 2HY); 3.27-3.21 (m, 1H), 2.39 (t, 2H), 1.39 (s, 9H), 1.02 (d, 3H).
[α]_{D}²⁰ = +15.0°, c = 0.49, Chloroform.

### Beispiel 44A

### 6-Oxo-heptansäuremethylester

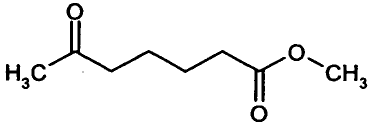

10 g 6-Oxoheptansäure (69.4 mmol) werden in 100 ml Methanol gelöst. Man gibt einige Tropfen konzentrierte Schwefelsäure hinzu und rührt 1.5 h unter Rückfluss. Danach wird eingeengt. Man nimmt in Dichlormethan auf und wäscht einmal mit ges. Natriumhydrogencarbonat-Lösung. Man trennt die Phasen, trocknet die organische Phase und engt ein. Man erhält 10.1 g (91.1% d. Th.) der Zielverbindung.
¹H-NMR (400 MHz, CDCl₃): δ = 3.67 (s, 3H), 2.44 (t, 2H), 2.32 (t, 2H), 2.13 (s, 3H), 1.67-1.55 (m, 4H).

### Beispiel 45A

### (+/-)-6-Hydroxy-heptansäuremethylester

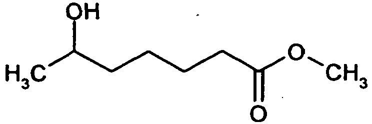

10 g (63.2 mmol) 6-Oxo-heptansäuremethylester werden in 50 ml Methanol vorgelegt. Dazu gibt man portionsweise 1.196 g (31.6 mmol) Natriumborhydrid. Nachdem die exotherme Reaktion abgeklungen ist, wird noch für 30 min unter Rückfluss nachgerührt. Danach wird eingeengt. Der Rückstand wird in Wasser aufgenommen, mit 1 M Salzsäure sauer gestellt und zweimal mit Dichlormethan extrahiert. Man trocknet die organische Phasen und engt ein. Man erhält 7.9 g (78.0% d. Th.) der Zielverbindung.
¹H-NMR (400 MHz, CDCl₃): δ = 3.83-3.75 (m, 1H), 3.67 (s, 3H), 2.32 (t, 2H), 1.69-1.58 (m, 2H), 1.53-1.30 (m, 4H), 1.19 (d, 3H).

### Beispiel 46A

### [(3R)-3-Hydroxybutyl]oxy-essigsäure-tert.-butylester

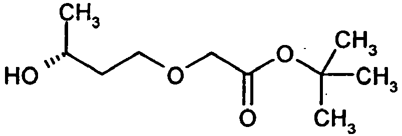

1.0 g (11.1 mmol) (3*R*)-Butan-1,3-diol wird in 20 ml THF bei 0°C vorgelegt. Man tropft 5.55 ml (11.1 mmol) einer 2 M Lösung der Phosphazenbase P2-*tert*.-Butyl in THF hinzu und rührt 30 min bei 0°C. Dann werden 2.27 g (11.65 mmol) Bromessigsäure-*tert*.-butylester zugegeben. Man rührt 30 min bei 0°C, läßt dann auf RT kommen und rührt noch 1 h nach. Danach wird mit Ethylacetat verdünnt, mit Wasser versetzt und mit 10%-iger Zitronensäure-Lösung sauer gestellt. Man extrahiert noch einmal mit Ethylacetat, vereinigt die organischen Phasen, wäscht einmal mit ges. Natriumchlorid-Lösung, trocknet über Magnesiumsulfat und engt ein. Man reinigt durch Chromatographie an Silicagel (Laufmittel: Cyclohexan/Ethylacetat 8:2). Man erhält 730 mg (32.2% d. Th.) der Zielverbindung, welche laut ¹H-NMR (Doublett bei 1.18 ppm) ca. 10% des Regioisomers [(1*R*)-3-Hydroxy-1-methylpropyl]oxy}essigsäure-*tert*.-butylester enthält.
¹H-NMR (400 MHz, CDCl₃): δ = 4.11-4.02 (m, 1H), 3.96 (d, 2H), 3.76-3.62 (m, 2H), 1.79-1.62 (m, 2H), 1.48 (s, 9H), 1.21 (d, 3H).

### Beispiel 47A

### (2R)-1-{[5-(4-Methoxyphenyl)-6-phenylfuro[2,3-d)pyrimidin-4-yl]oxy}propan-2-ol

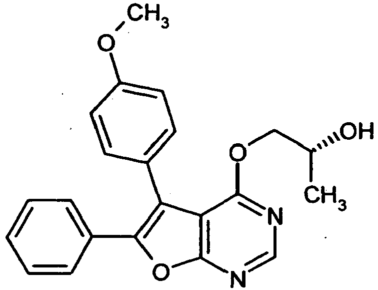

*und*

### (2R)-2-{[5-(4-Methoxyphenyl)-6-phenylfuro[2,3-d]pyrimidin-4-yl]oxy}propan-1-ol

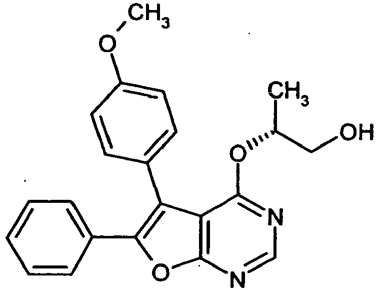

5.648 g (74.23 mmol) (2*R*)-Propan-1,2-diol werden in 30 ml THF vorgelegt. Man gibt 4.165 g (37.11 mmol) Kalium-*tert*.-butylat hinzu und rührt 15 min bei RT nach. Dann wird auf 0°C abgekühlt und eine Lösung von 5.00 g (14.85 mmol) 4-Chlor-5-(4-methoxyphenyl)-6-phenylfuro[2,3-d]pyrimidin in 15 ml THF während 30 min zugetropft. Danach läßt man auf RT kommen und rührt noch 3 h nach. Dann wird mit Dichlormethan verdünnt, mit Wasser versetzt und mit 10%-iger Zitronensäure-Lösung sauer gestellt. Man trennt die Phasen, extrahiert die wässrige Phase einmal mit Dichlormethan, vereinigt die organischen Phasen, wäscht einmal mit ges. Natriumchlorid-Lösung, trocknet über Magnesiumsulfat und engt ein. Man reinigt durch Chromatographie an Silicagel (Laufmittel: Cyclohexan/Ethylacetat 7:3). Bei dem Isolat handelt es sich laut ¹H-NMR um ein Gemisch der beiden Titelverbindungen. Man erhält zusammen 3.56 g (63.7% d. Th.).
LC-MS (Methode 8): Rₜ = 2.71 min (single peak); m/z = 377 (M+H)⁺
¹H-NMR (400 MHz, CDCl₃): δ = 8.50 (2x s, 2x 1H), 7.62 (m, 2x 2H), 7.42 (m, 2x 2H), 7.31 (m, 2x 3H), 6.97 (m, 2x 2H), 5.31 (m, 1x 1H), 4.48 (dd, 1x 1H), 4.14 (dd, 1x 1H), 4.01 (m, 1x 1H), 3.39 (2x s, 2x 3H), 3.72 (m, 1x 1H), 3.55 (m, 1x 1H), 1.31 (d, 1x 3H), 1.15 (d, 1x 3H).

Die in der folgenden Tabelle aufgeführten Verbindungen werden analog zur oben aufgezeigten Synthese hergestellt. Man,geht entsprechend von 4-Chlor-5-(4-methoxyphenyl)-6-phenylfuro[2,3-d]pyrimidin bzw. von 4-Chlor-5-(4-ethylphenyl)-6-phenylfuro[2,3-d]pyrimidin aus und verwendet (2*S*)-Propan-1,2-diol bzw. (2*R*)-Propan-1,2-diol:

| **Beispiel** | **Struktur** | **Analytische Daten** |
|---|---|---|
| **48A** | 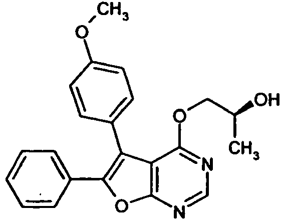 | LC-MS (Methode 7): Rₜ = 3.76 min (single peak); m/z = 377 (M+H)⁺ |
| | *und* | ¹H-NMR (400 MHz, DMSO-d₆):δ = 8.58 (2x s, 2x 1H), 7.52 (m,2x 7.40 2x 7.002H), (m, 5H), (m, 2x 2H), 5.36 (m, 1 x 1H), 4.80-4.70 (m, 1x 1H), 4.37 (dd, 1x 1H), 4.14 (dd, 1x 1H), 3.81 (2x s, 2x 3H), 3.46 (m, 1x 2H), 1.20 (d, 1x 3H). |
| | 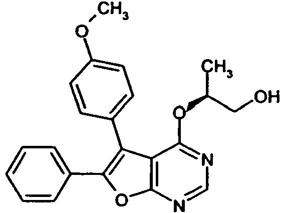 | |
| **49A** | 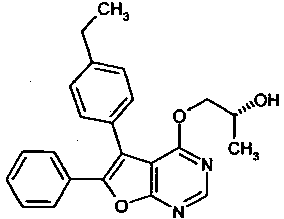 | LC-MS (Methode 8): Rₜ = 3.06 min (single peak); m/z = 375 (M+H)⁺ |
| | *und* | ¹H-NMR (400 MHz, DMSO-d₆):δ = 8.57 (2x s, 2x 1H), 7.55 (m,2x 2H), 7.41 (m, 2x 5H), 7.29 (m, 2x 2H), 5.33 (m, 1x 1H), 4.78 (t, 1x 1H), 4.72 (d, 1x 1H), 4.35 (dd, 1x 1H), 4.11 (dd, 1x 1H), 3.79 (m, 1x 1H), 3.42 (m, 1x 2H), 2.69 (2x q, 2x 2H), 1.22 (2x t, 2x 3H), 1.18 (d, 1x 3H), 0.90 (d, 1x 3H). |
| | 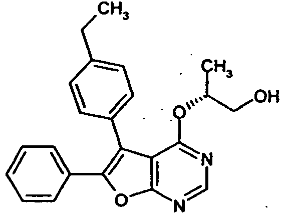 | |
| **50A** | 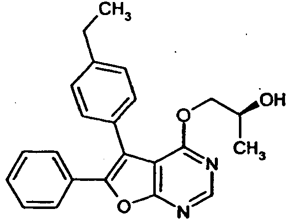 | LC-MS (Methode 2): Rₜ = 2.75 min (single peak); m/z = 375 (M+H)⁺ |
| | *und* | ¹H-NMR (400 MHz, DMSO-d₆):δ = 8.57 (2x s, 2x 1H), 7.53 (m,(m, 2x 2H), 5.32(m, 1x 1H), 4.78 (t, 1x 1H), 4.72 (d, 1x 1H), 4.35 (dd, 1x 1H), 4.11 (dd, 1x 1H), 3.79 (m, 1x 1H), 3.43 (m, 1x 2H), 2.69 (2x q, 2x 2H), 1.22 (2x t, 2x 3H), 1.18 (d, 1x 3H), 0.90 (d, 1x 3H). |
| | 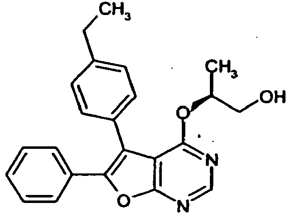 | |

### Beispiel 51A

### 1-[Benzyl(methyl)amino]aceton

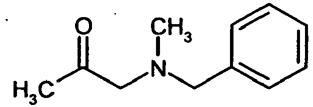

12.118 g (100 mmol) *N*-Methylbenzylamin werden mit 16.584 g (120 mmol) Kaliumcarbonat in 100 ml Toluol vorgelegt. Man tropft 11.103 g (120 mmol) Chloraceton hinzu und rührt über Nacht unter Rückfluss. Danach wird auf RT abgekühlt, vom Salz abfiltriert und eingeengt. Man reinigt den Rückstand durch Chromatographie an Silicagel (Laufmittel: Cyclohexan/Ethylacetat 8:2) und erhält 9.0 g (50.8% d. Th.) der Zielverbindung.
¹H-NMR (400 MHz, CDCl₃): δ = 7.36-7.22 (m, 5H), 3.57 (s, 2H), 3.13 (s, 2H), 2.29 (s, 3H), 2.13 (s, 3H).

### Beispiel 52A

### (+/-)-1-[Benzyl(methyl)amino]propan-2-ol

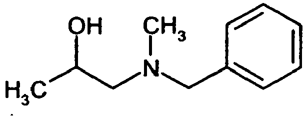

8.00 g (45.13 mmol) 1-[Benzyl(methyl)amino]aceton werden in 40 ml Methanol vorgelegt. Dazu gibt man bei RT portionsweise unter Rühren 854 mg (22.57 mmol) Natriumborhydrid. Man rührt 30 min bei RT und anschließend weitere 30 min unter Rückfluss nach. Man engt ein und nimmt den Rückstand in Wasser auf. Man extrahiert zweimal mit Ethylacetat, wäscht die vereinigten organischen Phasen einmal mit ges. Natriumhydrogencarbonat-Lösung, trocknet über Magnesiumsulfat und engt ein. Man erhält 7.80 g (81.9% d. Th.) der Zielverbindung ohne weitere Aufreinigung.
¹H-NMR (400 MHz, CDCl₃): δ = 7.36-7.22 (m, 5H), 3.90-3.80 (m, 1H), 3.66 (d, 1H), 3.43 (d, 1H), 2.33 (dd, 1H), 2.31 (dd, 1H), 2.21 (s, 3H), 1.11 (d, 3H).

### Beispiel 53A

### (+/-)-N-Benzyl-2-{[5-(4-methoxyphenyl)-6-phenylfuro[2,3-d]pyrimidin-4-yl]oxy}-N-methyl-propan-1-amin

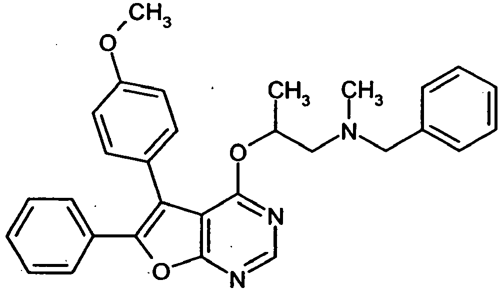

1.878 g (8.91 mmol) (+/-)-1-[Benzyl(methyl)amino]propan-2-ol werden unter Argon in 20 ml THF vorgelegt und auf 0°C abgekühlt. Man gibt 4.5 ml (8.91 mmol) einer 2 M Lösung der Phosphazen-base P2-*tert*.-Butyl in THF hinzu und rührt 10 min bei RT nach. Dann wird wieder auf 0°C abgekühlt. Man gibt 2.00 g (5.94 mmol) 4-Chlor-5-(4-methoxyphenyl)-6-phenylfuro[2,3-d]pyrimidin hinzu und rührt 1 h bei RT. Man verdünnt danach mit Ethylacetat und wäscht mit Wasser. Die wässrige Phase wird noch einmal mit Ethylacetat extrahiert. Die vereinigten organischen Phasen werden mit ges. Natriumchlorid-Lösung gewaschen, über Magnesiumsulfat getrocknet und eingeengt. Man reinigt durch Chromatographie an Silicagel (Laufmittel: Cyclohexan/Ethylacetat 85:15) und erhält 1.71 g (60.0% d. Th.) der Zielverbindung.
LC-MS (Methode 8): Rₜ = 1.88 min.; m/z = 480 (M+H)⁺
¹H-NMR (400 MHz, DMSO-d₆): δ = 8.50 (s, 1H), 7.62 (m, 2H), 7.36 (d, 2H), 7.32-7.15 (m, 8H), 6.86 (d, 2H), 5.65-5.57 (m, 1H), 3.81 (s, 3H), 3.41 (dd, 2H), 2.62 (dd, 1H), 2.43 (dd, 1H), 2.10 (s, 3H), 1.29 (d, 3H).

### Beispiel 54A

### (+/-)-2-{[5-(4-Methoxyphenyl)-6-phenylfuro[2,3-d]pyrimidin-4-yl]oxy}-N-methylpropan-1-amin

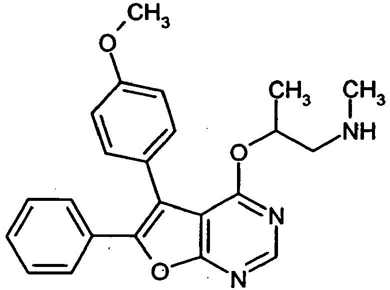

500 mg Palladium auf Kohle (10%-ig) werden unter Argon in 100 ml Methanol vorgelegt. Man gibt 1.7 g (3.55 mmol) (+/-)-*N*-Benzyl-2-{[5-(4-methoxyphenyl)-6-phenylfuro[2,3-d]pyrimidin-4-yl]oxy}-*N*-methylpropan-1-amin sowie 2.5 ml Essigsäure hinzu und hydriert bei RT unter Normaldruck. Nach 2 h filtriert man über Kieselgur und engt ein. Der Rückstand wird in Wasser gelöst und zweimal mit Ethylacetat gewaschen. Die Ethylacetat-Phasen werden verworfen. Man stellt die wässrige Phase mit festem Natriumhydrogencarbonat basisch und extrahiert zweimal mit Ethylacetat. Die vereinigten Ethylacetat-Phasen werden einmal mit ges. Natriumchlorid-Lösung gewaschen, über Magnesiumsulfat getrocknet und eingeengt. Man erhält 900 mg (65.2% d. Th.) der Zielverbindung.
LC-MS (Methode 2): Rₜ = 1.57 min.; m/z = 390 (M+H)⁺
¹H-NMR (400 MHz, CDCl₃): δ = 8.50 (s, 1H), 7.63 (m, 2H), 7.38 (d, 2H), 7.34-7.25 (m, 3H), 6.96 (d, 2H), 5.45-5.35 (m, 1H), 3.88 (s, 3H), 2.64 (m, 2H), 2.30 (s, 3H), 1.35 (d, 3H).

### Beispiel 55A

### (+)-tert.-Butyl{[1,5-dimethylhex-4-en-1-yl]oxy}diphenylsilan

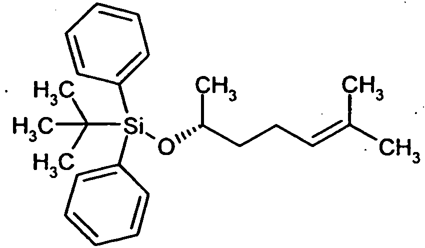

50 g (390.0 mmol) (6*R*)-6-Methyl-5-hepten-2-ol werden in 500 ml Dichlormethan vorgelegt. Dazu gibt man 53.10 g (779.9 mmol) Imidazol und 2.382 g (19.50 mmol) 4-Dimethylaminopyridin. Man kühlt auf 0°C und tropft 117.91 g (429.0 mmol) *tert.*-Butyldiphenylchlorsilan hinzu. Man entfernt die Kühlung, lässt auf RT kommen und rührt eine Stunde bei RT nach. Man gibt 250 ml Dichlormethan hinzu und wäscht zweimal mit jeweils 500 ml Wasser. Die organische Phase wird über Magnesiumsulfat getrocknet und eingeengt. Der Rückstand wird durch Chromatographie an Silicagel (Laufmittel: Petrolether/Ethylacetat 95:5) gereinigt. Man erhält 135.0 g (94.4% d. Th.) der Zielverbindung.
¹H-NMR (400 MHz, CDCl₃): δ = 7.69 (m, 4H), 7.42-7.32 (m, 6H), 5.00-4.95 (t, 1H), 3.88-3.80 (m, 1H), 2.02-1.88 (m, 2H), 1.62 (s, 3H), 1.52 (s, 3H), 1.52-1.48 (m, 2H), 1.06 (s, 3H), 1.05 (s, 9H).
[α]_{D}²⁰ = +20.2°, c = 0.689, Methanol.

### Beispiel 56A

### (+)-(2E)-6-{[tert.-Butyl(diphenyl)silyl]oxy}hept-2-ensäure-tert.-butylester

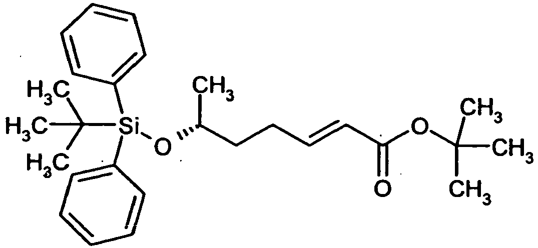

22.20 g (60.55 mmol) (+)-*tert*.-Butyl{[1,5-dimethylhex-4-en-1-yl]oxy}diphenylsilan werden mit 165 mg (1.96 mmol) Natriumhydrogencarbonat in 240 ml Dichlormethan vorgelegt und auf -78°C abgekühlt. Man leitet bei -78°C Ozon-Gas ein, bis eine schwache bläuliche Färbung der Lösung vorliegt. Man gibt dann 47.376 g (762 mmol) Dimethylsulfid hinzu, lässt auf RT kommen und rührt noch eine Stunde bei RT nach. Danach werden 27.352 g (72.66 mmol) Triphenylphosphoranyliden-essigsäure-*tert.*-butylester zugegeben und die Mischung über Nacht bei RT nachgerührt. Das Gemisch wird dann eingeengt. Man reinigt den Rückstand durch Chromatographie an Silicagel (Laufmittel: Cyclohexan/Ethylacetat 95:5) und erhält 25.1 g (95.4% d. Th.) der Zielverbindung. Laut ¹H-NMR liegt ein *E*/*Z-*Verhältnis von >10:1 vor.
MS (DCI): m/z = 456 (M+NH₄)⁺
¹H-NMR (400 MHz, CDCl₃): δ = 7.67 (d, 4H), 7.43-7.33 (m, 6H), 6.82-6.73 (dt, 1H), 5.65 (d, 1H), 3.91-3.82 (m, 1H), 2.28-2.10 (m, 2H), 1.65-1.42 (m, 2H), 1.48 (s, 9H), 1.06 (d, 3H), 1.05 (s, 9H).
[α]_{D}²⁰ = +22.5°, c = 0.520, Methanol.

### Beispiel 57A

### (2E, 6R)-6-Hydroxyhept-2-ensäure-tert.-butylester

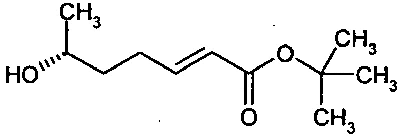

Lösung A: 10.71 g (267.7 mmol) 60%-iges Natriumhydrid werden in 150 ml abs. THF suspendiert und tropfenweise unter Kühlung mit 43.3 ml (276.7 mmol) *P,P*-Dimethylphosphonoessigsäure-tert.-butylester versetzt. Die Mischung wird bei RT gerührt, wobei nach ca. 30 min eine Lösung entsteht.

Zu einer auf -78°C gekühlten Lösung von 17.87 g (178.5 mmol) (*R*)-γ-Valerolacton [(5*R*)-5-methyldihydrofuran-2(3*H*)-on] in 200 ml abs. THF werden 187.4 ml (187.4 mmol) einer 1 M Lösung von DIBAH in THF getropft. Die Lösung wird 1 h bei -78°C nachgerührt und dann die oben hergestellte Lösung A zugegeben. Nach Ende der Zugabe wird die Mischung langsam auf RT erwärmt und über Nacht bei RT gerührt. Man gibt die Reaktionsmischung zu 300 ml Ethylacetat und rührt mit 50 ml konzentrierter Kalium-Natrium-Tartratlösung aus. Nach Phasentrennung wird die wässrige Phase mit Ethylacetat re-extrahiert. Man vereinigt die organischen Phasen, wäscht mit ges. Natriumchlorid-Lösung, trocknet über Magnesiumsulfat und konzentriert im Vakuum. Der Rückstand wird mittels Chromatographie an Silicagel (Eluent: Cyclohexan/Ethylacetat-5:1) gereinigt. Erhalten werden 32.2 g (90.1% d. Th.) des Zielprodukts, welches geringe Mengen des cis-Isomeren enthält.
MS (DCI): m/z = 218 (M+NH₄)⁺
¹H-NMR (400 MHz, DMSO-d₆): δ = 6.70 (dt, 1H), 5.73 (d, 1H), 4.44 (d, 1H), 3.58 (m, 1H), 2.28-2.13 (m, 2H), 1.47-1.40 (m, 2H), 1.45 (s, 9H), 1.04 (d, 3H).

### Beispiel 58A

### (+)-6-{[tert.-Butyl(diphenyl)silyl]oxy}heptansäure-tert.-butylester

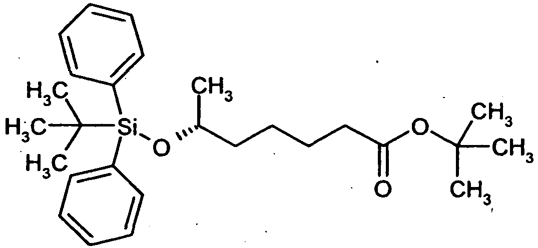

149.0 g (339.64 mmol) (+)-(2*E*)-6-{[*tert*.-Butyl(diphenyl)silyl]oxy}hept-2-ensäure-*tert*.-butylester werden in 1000 ml Ethanol bei RT unter Argon vorgelegt. Man gibt 15.0 g Palladium/Kohle (20%-ig, wasserfeucht) hinzu und hydriert unter Normaldruck bei RT. Nach Beendigung der Wasserstoffaufnahme filtriert man den Ansatz über Kieselgur und engt ein. Man erhält 142.0 g (95.0% d. Th.) der Zielverbindung.
MS (DCI): m/z = 458 (M+NH₄)⁺
¹H-NMR (400 MHz, CDCl₃): δ = 7.68 (d, 4H), 7.43-7.33 (m, 6H), 3.87-3.80 (m, 1H), 2.12 (t, 2H), 1.53-1.20 (m, 6H), 1.45 (s, 9H), 1.05 (d, 3H), 1.05 (s, 9H).
[α]_{D}²⁰ = +14.7°, c = 0.7925, Methanol.

### Beispiel 59A

### (-)-6-Hydroxyheptansäure-tert.-butylester

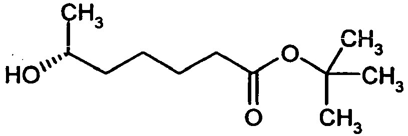

### Methode 1:

141.0 g (319.94 mmol) (+)-6-{[*tert*.-Butyl(diphenyl)silyl]oxy}heptansäure-*tert*.-butylester werden in 280 ml THF vorgelegt. Man tropft 479.90 ml (479.90 mmol) einer 1 M Lösung von Tetrabutylammoniumfluorid in THF hinzu und rührt über Nacht bei RT. Man versetzt dann mit 4000 ml einer 10%-igen wässrigen Natriumchlorid-Lösung und stellt mit Zitronensäure auf einen pH-Wert von etwa 3-4 ein. Man extrahiert zweimal mit jeweils 2000 ml Ethylacetat und wäscht die vereinigten Ethylacetat-Phasen einmal mit 2000 ml ges. Natriumchlorid-Lösung. Man trocknet über Magnesiumsulfat, engt ein und reinigt durch Chromatographie an Silicagel (Laufmittel: Cyclohexan/Ethylacetat 7:3). Es werden 50.2 g (77.5% d. Th.) des Zielprodukts erhalten.
MS (DCI): m/z = 220 (M+NH₄)⁺
¹H-NMR (400 MHz, CDCl₃): δ = 3.85-3.75 (m, 1H), 2.22 (t, 2H), 1.68-1.54 (m, 2H), 1.53-1.30 (m, 4H), 1.45 (s, 9H), 1.18 (d, 3H).
[α]_{D}²⁰= -6.8°, c = 1.073, Methanol.

### Methode 2:

32.2 g (160.8 mmol) (2*E*,6*R*)-6-Hydroxyhept-2-ensäure-*tert*.-butylester werden in 200 ml Ethanol gelöst und mit 1.7 g 10% Palladium auf Kohle versetzt. Die Mischung wird bei RT unter einer Wasserstoffatmosphäre (Normaldruck) 2 h lang gerührt und dann über Celite abfiltriert. Das Filtrat wird im Vakuum eingeengt. Aus dem Rückstand erhält man nach Chromatographie an Silicagel (Eluent: Cyclohexan/Ethylacetat 10:1 → 6:1) 15.66 g des Zielprodukts (48.1% d. Th.).
[α]_{D}²⁰ = -21°, c = 0.118, Chloroform.

### Beispiel 60A

### (+)1-{[5-(4-Methoxyphenyl)-6-phenylfuro[2,3-d]pyrimidin-4-yl]amino}propan-2-ol

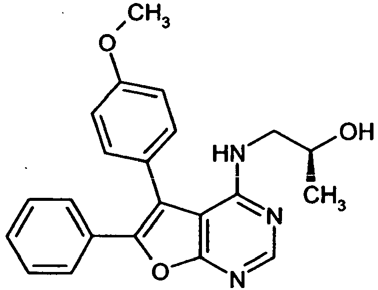

500 mg (1.49 mmol) 4-Chlor-5-(4-methoxyphenyl)-6-phenylfuro[2,3-d]pyrimidin werden mit 2 ml DMF, 223 mg (2.97 mmol) (*S*)-(+)-1-Amino-2-propanol und 768 mg (5.94 mmol) *N,N-*Diisopropylethylamin versetzt. Man erhitzt 2 h auf 100°C und lässt anschließend auf RT abkühlen. Man trennt das Gemisch ohne weitere Aufarbeitung direkt mittels präparativer RP-HPLC (Laufmittel: Acetonitril/Wasser-Gradient) und erhält 230 mg (41.3% d. Th.) der Zielverbindung.
LC-MS (Methode 2): Rₜ = 2.25 min.; m/z = 376 (M+H)⁺
¹H-NMR (400 MHz, CDCl₃): δ = 8.36 (s, 1H), 7.53 (m, 2H), 7.40 (d, 2H), 7.27 (m, 3H), 7.07 (d, 2H), 5.13 (m, 1H), 3.92 (m, 4H), 3.56 (m, 1H), 3.32 (m, 1H), 1.15 (d, 2H).
[α]_{D}²⁰ = +3.0°, c = 0.298, Methanol.

### Beispiel 61A

### (-)-1-{[5-(4-Methoxyphenyl)-6-phenylfuro[2,3-d]pyrimidin-4-yl]amino}propan-2-ol

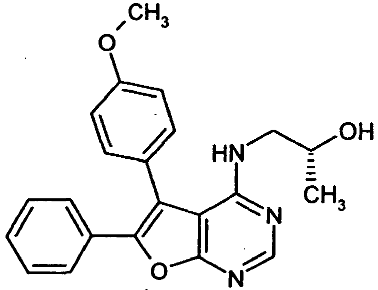

1.00 g (2.97 mmol) 4-Chlor-5-(4-methoxyphenyl)-6-phenylfuro[2,3-d]pyrimidin werden mit 5 ml DMSO, 446 mg (5.94 mmol) (*R*)-(-)-1-Amino-2-propanol und 2.07 ml (11.88 mmol) *N,N-*Diisopropylethylamin versetzt. Man erhitzt 2 h auf 100°C und lässt anschließend auf RT abkühlen. Man gießt danach auf ein Eis-Wasser-Gemisch und lässt das Eis auftauen. Man dekantiert die wässrige Phase ab, verdünnt die organische Phase mit Dichlormethan und wäscht einmal mit Wasser. Die wässrige Phase wird einmal mit Dichlormethan rückextrahiert. Die vereinigten organischen Phasen werden einmal mit ges. Natriumchlorid-Lösung gewaschen, über Magnesiumsulfat getrocknet und eingeengt. Man erhält 1.10 g (98.7% d. Th.) der Zielverbindung.
LC-MS (Methode 2): Rₜ = 2.25 min.; m/z = 376 (M+H)⁺
¹H-NMR (400 MHz, CDCl₃): δ = 8.36 (s, 1H), 7.53 (m, 2H), 7.40 (d, 2H), 7.27 (m, 3H), 7.07 (d, 2H), 5.13 (t, 1H), 3.92 (m, 4H), 3.56 (m, 1H), 3.32 (m, 1H), 1.15 (d, 2H).
[α]_{D}²⁰ = -3.1°, c = 0.455, Methanol.

### Beispiel 62A

### 3-{[5-(4-Methoxyphenyl)-6-phenylfuro[2,3-d]pyrimidin-4-yl]oxy}-2,2-dimethylpropan-1-ol

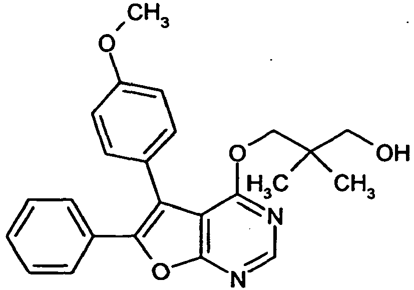

1.546 g (14.85 mmol) 2,2-Dimethylpropan-1,3-diol werden in 30 ml THF vorgelegt. Man gibt 833 mg (7.42 mmol) Kalium-*tert.*-butylat hinzu und rührt 15 min bei RT nach. Danach wird auf 0°C abgekühlt und eine Lösung von 1.00 g (2.97 mmol) 4-Chlor-5-(4-methoxyphenyl)-6-phenylfuro-[2,3-d]pyrimidin in 15 ml THF während 30 min zugetropft. Man läßt auf RT kommen und rührt 30 min bei RT nach. Danach versetzt man mit Dichlormethan und Wasser, stellt mit 10%-iger Zitronensäure-Lösung sauer und trennt die Phasen. Die wässrige Phase wird einmal mit Dichlormethan extrahiert. Die vereinigten organischen Phasen werden einmal mit ges. Natriumchlorid-Lösung gewaschen, über Magnesiumsulfat getrocknet und eingeengt. Man erhält 1.20 g (99.9% d. Th.) der Zielverbindung.
LC-MS (Methode 7): Rₜ = 3.99 min.; m/z = 405 (M+H)⁺
¹H-NMR (400 MHz, DMSO-d₆): δ = 8.57 (s, 1H), 7.53 (d, 2H), 7.42-7.33 (m, 5H), 7.02 (d, 2H), 4.52 (t, 1H), 4.11 (s, 2H), 3.81 (s, 3H), 3.01 (d, 2H), 0.69 (s, 6H).

### Beispiel 63A

### (+)-4-(Trityloxy)butan-2-ol

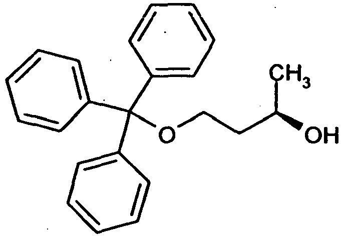

18.560 g (205.94 mmol) (*R*)-(-)-1,3-Butandiol werden in 260 ml Dichlormethan vorgelegt und mit 27.092 g (267.72 mmol) Triethylamin versetzt. Man kühlt auf 0°C und gibt langsam 57.987 g (208.00 mmol) Chlortriphenylmethan hinzu. Man lässt auf RT kommen und rührt über Nacht bei RT nach. Man versetzt dann mit 12.9 ml Methanol und rührt für 30 min. Man wäscht zweimal mit Wasser, zweimal mit ges. Ammoniumchlorid-Lösung und einmal mit ges. Natriumchlorid-Lösung, trocknet über Magnesiumsulfat und engt ein. Der Rückstand wird durch Chromatographie an Silicagel (Laufmittel: Cyclohexan/Ethylacetat 10:1 → 2:1) gereinigt. Man erhält 60.990 g (89.1% d. Th.) der Zielverbindung.
MS (DCI): m/z = 350 (M+NH₄)⁺
¹H-NMR (400 MHz, DMSO-d₆): δ = 7.40-7.30 (m, 12H), 7.38-7.23 (m, 3H), 4.32 (d, 1H), 3.80-3.70 (m, 1H), 3.10-2.97 (m, 2H), 1.70-1.55 (m, 2H), 1.00 (d, 3H).
[α]_{D}²⁰ = +24.2°, c = 0.520, Chloroform.

### Beispiel 64A

### (-)-[{[3-(Benzyloxy)butyl]oxy}(diphenyl)methyl]benzol

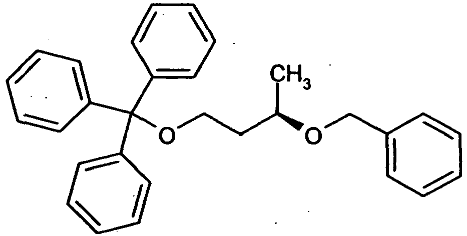

10.997 g (274.95 mmol) Natriumhydrid werden in 150 ml DMF bei RT vorgelegt. Man gibt 60.937 g (183.29 mmol) (+)-4-(Trityloxy)butan-2-ol hinzu und rührt 15 min bei RT nach. Man kühlt auf 0°C und versetzt mit 62.704 g (366.59 mmol) Benzylbromid. Man gibt danach weitere 50 ml DMF hinzu, lässt auf RT kommen und rührt über Nacht. Man versetzt vorsichtig mit Wasser und extrahiert zweimal mit Ethylacetat. Die vereinigten organischen Phasen werden zweimal mit ges. Natriumchlorid-Lösung gewaschen, über Magnesiumsulfat getrocknet und eingeengt. Der Rückstand wird durch Chromatographie an Silicagel (Laufmittel: Cyclohexan/Dichlormethan 5:1 → 1:1) gereinigt. Man erhält 71.750 g (92.6% d. Th.) der Zielverbindung.
MS (DCI): m/z = 440 (M+NH₄)⁺
¹H-NMR (400 MHz, DMSO-d₆): δ = 7.40-7.23 (m, 18H), 7.16 (d, 2H), 4.48 (d, 1H), 4.31 (d, 1H), 3.73-3.66 (m, 1H), 3.13-3.02 (m, 2H), 1.81-1.68 (m, 2H), 1.10 (d, 3H).
[α]_{D}²⁰ = -10.8°, c = 0.500, Chloroform.

### Beispiel 65A

### (-)-3-(Benzyloxy)butan-1-ol

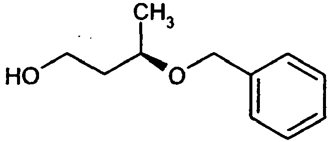

71.750 g (169.79 mmol) (-)-[{[3-(Benzyloxy)butyl]oxy}(diphenyl)methyl]benzol werden mit einem Gemisch aus Wasser/Essigsäure/Methanol (3:4:3) versetzt und über Nacht bei 50°C gerührt. Man gibt dann Wasser hinzu und extrahiert mit Dichlormethan. Die organische Phase wird über Magnesiumsulfat getrocknet und eingeengt. Der Rückstand wird mit Cyclohexan verrührt. Man filtriert den Feststoff über eine Fritte ab, wäscht dreimal mit Cyclohexan, verwirft den Feststoff und engt das Filtrat ein. Der Rückstand wird durch Chromatographie an Silicagel (Laufmittel: Cylohexan/Ethylacetat 10:1 → 4:1) gereinigt. Man erhält 21.97 g (71.8% d. Th.) der Zielverbindung.
MS (DCI): m/z = 190 (M+NH₄)⁺
¹H-NMR (400 MHz, DMSO-d₆): δ = 7.35-7.24 (m, 5H), 4.52 (d, 1H), 4.39 (d, 1H), 4.33 (t, 1H), 3.65-3.58 (m, 1H), 3.52-3.45 (m, 2H), 1.74-1.65 (m, 1H), 1.57-1.48 (m, 1H), 1.13 (d, 3H).
[α]_{D}²⁰ = -65.7°, c = 0.530, Chloroform.

### Beispiel 66A

### (-)-4-{[3-(Benzyloxy)butyl]oxy}-5-(4-methoxyphenyl)-6-phenylfuro[2,3-d]pyrimidin

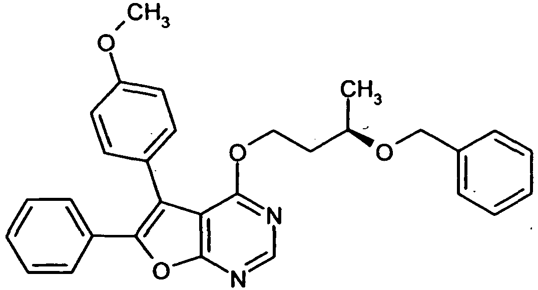

802 mg (4.45 mmol) *(3R)*-3-(Benzyloxy)butan-1-ol werden unter Argon in 10 ml THF vorgelegt und auf 0°C abgekühlt. Man gibt 2.30 ml (4.45 mmol) einer 2 M Lösung der Phosphazenbase P2-*tert*.-Butyl in THF hinzu und rührt 10 min bei RT. Danach wird wieder auf 0°C abgekühlt. Man gibt 1.0 g (2.97 mmol) 4-Chlor-5-(4-methoxyphenyl)-6-phenylfuro[2,3-d]pyrimidin hinzu und rührt über Nacht bei RT. Dann wird mit Ethylacetat verdünnt, mit Wasser versetzt und mit 10%-iger Zitronensäure-Lösung sauer gestellt. Die wässrige Phase wird einmal mit Ethylacetat extrahiert. Die vereinigten Ethylacetat-Phasen werden einmal mit ges. Natriumchlorid-Lösung gewaschen, über Magnesiumsulfat getrocknet und eingeengt. Man reinigt durch Chromatographie an Silicagel (Laufmittel: Cyclohexan/Ethylacetat 9:1) und erhält 1.16 g (81.3% d. Th.) der Zielverbindung.
LC-MS (Methode 7): Rₜ = 4.70 min.; m/z = 481 (M+H)⁺
¹H-NMR (400 MHz, DMSO-d₆): δ = 8.52 (s, 1H), 7.62 (m, 2H), 7.31 (m, 5H), 7.25-7.12 (m, 5H), 6.91 (d, 2H), 4.55-4.44 (m, 3H), 4.22 (d, 1H), 3.82 (s, 3H), 3.40-3.31 (m, 1H), 1.88-1.82 (m, 1H), 1.79-1.71 (m, 1H), 1.12 (d, 3H).
[α]_{D}²⁰ = -79.0°, c = 0.455, Methanol.

### Beispiel 67A

### (-)-4-{[5-(4-Methoxyphenyl)-6-phenylfuro[2,3-d]pyrimidin-4-yl]oxy}butan-2-ol

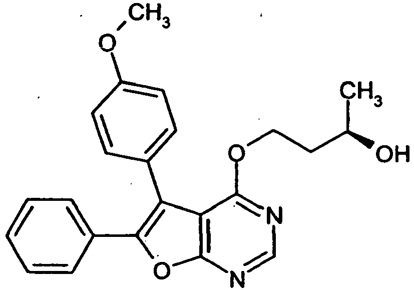

1.0 g (2.08 mmol) (-)-4-{[3-(Benzyloxy)butyl]oxy}-5-(4-methoxyphenyl)-6-phenylfuro[2,3-d]pyrimidin werden in 20 ml Dioxan gelöst und mit 100 mg Palladium/Kohle (10%-ig) versetzt. Man hydriert bei Normaldruck und RT ca. 5 h bis zur Beendigung der Wasserstoffaufnahme. Der Katalysator wird anschließend über Celite abfiltriert und das Filtrat eingeengt. Man reinigt den Rückstand durch Chromatographie an Silicagel (Laufmittel: Cyclohexan/Ethylacetat 7:3 → 1:1) und erhält 675 mg (83.1% d. Th.) der Zielverbindung.
LC-MS (Methode 7): Rₜ = 3.78 min.; m/z = 391 (M+H)⁺
¹H-NMR (400 MHz, CDCl₃): δ = 8.51 (s, 1H), 7.62 (m, 2H), 7.38 (d, 2H), 7.31 (m, 3H), 6.95 (d, 2H), 4.73-4.67 (m, 1H), 4.46-4.40 (m, 1H), 3.88 (s, 3H), 3.75-3.65 (m, 1H), 2.20 (br. s, 1H), 1.83-1.76 (m, 1H), 1.75-1.68 (m, 1H), 1.16 (d, 3H).
[α]_{D}²⁰ = -60.0°, c = 0.5305, Methanol.

### Beispiel 68A

### 3-{[5-(4-Methoxyphenyl)-6-phenylfuro[2,3-d]pyrimidin-4-yl]oxy}propan-1-ol

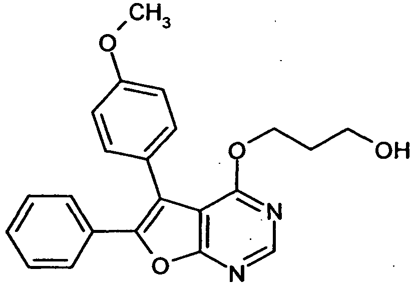

1.13 g (14.85 mmol) 1,3-Propandiol werden in 30 ml THF vorgelegt. Man gibt 833 mg (7.42 mmol) Kalium-*tert*.-butylat hinzu und rührt 15 min bei RT. Danach wird auf 0°C abgekühlt und eine Lösung von 1.0 g (2.97 mmol) 4-Chlor-5-(4-methoxyphenyl)-6-phenylfuro[2,3-d]pyrimidin in 15 ml THF während 30 min zugetropft. Dann läßt man auf RT kommen und rührt noch 2 h nach. Man verdünnt mit Dichlormethan und Wasser, stellt mit 10%-iger Zitronensäure-Lösung sauer und trennt die Phasen. Die wässrige Phase wird einmal mit Dichlormethan extrahiert. Die organischen Phasen werden vereinigt und einmal mit ges. Natriumchlorid-Lösung gewaschen. Man trocknet über Magnesiumsulfat, engt ein und reinigt den Rückstand durch Chromatographie an Silicagel (Laufmittel: Cyclohexan/Ethylacetat 7:3 → 1:1). Es werden 772 mg (69.0% d. Th.) der Zielverbindung erhalten.
LC-MS (Methode 9): Rₜ = 3.47 min.; m/z = 377 (M+H)⁺
¹H-NMR (400 MHz, CDCl₃): δ = 8.52 (s, 1H), 7.62 (m, 2H), 7.39 (d, 2H), 7.31 (m, 3H), 6.96 (d, 2H), 4.58 (t; 2H), 3.89 (s, 3H), 3.58 (t, 2H), 1.90 (quin, 2H).

### Beispiel 69A

### 1-[(Z)-2-Chlor-2-nitrovinyl]-4-methoxybenzol

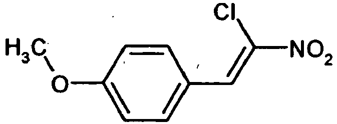

Analog einer Literaturvorschrift [D. Dauzonne, *Synthesis,* 1990, 66-70] wird eine Mischung von 10.0 g (73.5 mmol) 4-Methoxybenzaldehyd, 9.0 ml (13.5 g, 96.2 mmol) Bromnitromethan, 53.9 g (661.0 mmol) Dimethylammoniumchlorid und 0.6 g (11.0 mmol) Kaliumfluorid in 150 ml Xylol am Wasserabscheider bei 160°C für 15 Stunden gerührt. Nach Zugabe von 25 ml Wasser und 100 ml Dichlormethan wird die organische Phase abgetrennt und die wässrige Phase dreimal mit je 100 ml Dichlormethan extrahiert. Die vereinigten organischen Extrakte werden über Natriumsulfat getrocknet, filtriert und im Vakuum eingeengt. Der Rückstand wird an Kieselgel (Laufmittel: Cyclohexan/Dichlormethan 1:1) chromatographiert. Es werden 9.6 g (59% d. Th.) der Zielverbindung erhalten.
LC-MS (Methode 8): Rₜ = 2.52 min.
¹H-NMR (400 MHz, CDCl₃): δ = 8.60 (s, 1H), 8.03 (d, 2H), 7.15 (d, 2H), 3.86 (s, 3H).

### Beispiel 70A

### 5-(4-Methoxyphenyl)furo[2,3-d]pyrimidin-4(3H)-on

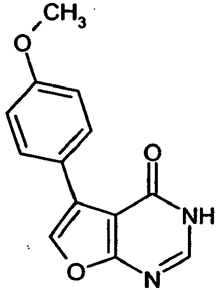

Analog Literaturvorschrift [D. Dauzonne, *Tetrahedron,* 1992, 3069-3080] wird eine Suspension von 10.1 g (47.4 mmol) 1-[(*Z*)-2-Chlor-2-nitrovinyl]-4-methoxybenzol und 5.8 g (52.2 mmol) 4,6-Dihydroxypyrimidin in 200 ml Ethanol zehn Minuten bei 85°C gerührt. Anschließend werden 15.6 ml (15.9 g, 104.3 mmol) 1,8-Diazabicyclo[5.4.0]undec-7-en langsam zugegeben. Es wird 15 h bei dieser Temperatur gerührt und dann im Vakuum eingeengt. Der Rückstand wird in Dichlormethan aufgenommen und an Kieselgel (Laufmittel: Dichlormethan/Methanol 95:5) chromatographiert. Der erhaltene Feststoff wird in Acetonitril verrührt und filtriert. Es werden 2.3 g (20% d. Th.) des Zielprodukts erhalten.
LC-MS (Methode 2): Rₜ = 1.57 min.; m/z = 290 (M+H)⁺
¹H-NMR (400 MHz, CDCl₃): δ = 12.66 (s, NH), 8.15 (s, 1H), 8.14 (s, 1H), 7.92 (d, 2H), 6.98 (d, 2H), 3.79 (s, 3H).

### Beispiel 71A

### 4-Chlor-5-(4-methoxyphenyl)furo[2,3-d]pyrimidin

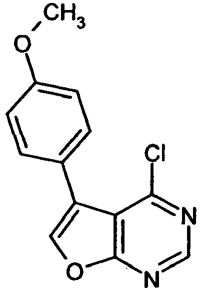

Eine Suspension von 10.0 g (41.3 mmol) 5-(4-Methoxyphenyl)furo[2,3-d]pyrimidin-4(3*H*)-on in 250 ml Toluol wird mit 14:5 ml (13.6 g, 90.8 mmol) *N,N-*Diethylanilin versetzt und auf 100°C erwärmt. Bei dieser Temperatur werden 4.2 ml (7.0 g, 45.4 mmol) Phosphorylchlorid zugetropft, und das Reaktionsgemisch wird 15 h bei 100°C gerührt. Danach werden weitere 1.2 ml (2.0 g, 13 mmol) Phosphorylchlorid zugesetzt und das Reaktionsgemisch erneut für 22 h bei 100°C gerührt. Nach Abkühlen auf Raumtemperatur wird die Reaktionslösung nacheinander schnell mit 250 ml Eiswasser, zweimal je 250 ml kalter 20%-iger Natronlauge, erneut 250 ml Eiswasser, 250 ml ges. Natriumchlorid-Lösung, 1 N Salzsäure sowie 250 ml Eiswasser gewaschen. Die organische Phase wird über Natriumsulfat getrocknet, filtriert und im Vakuum eingeengt. Es werden 6.3 g (59% d. Th.) der Titelverbindung erhalten.
LC-MS (Methode 10): Rₜ = 2.28 min.; m/z = 261 (M+H)⁺
¹H-NMR (400 MHz, CDCl₃): δ = 8.86 (s, 1H), 8.40 (s, 1H), 7.52 (d, 2H), 7.08 (d, 2H), 3.82 (s, 3H).

### Beispiel 72A

### 6-{[5-(4-Methoxyphenyl)furo[2,3-d]pyrimidin-4-yl]amino}hexansäuremethylester

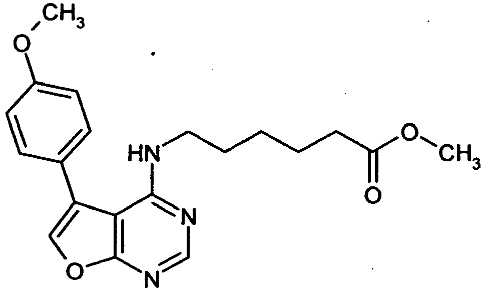

7.1 g (27.2 mol) 4-Chlor-5-(4-methoxyphenyl)furo[2,3-d]pyrimidin werden in 250 ml Acetonitril gelöst und mit 5.9 g (32.7 mmol) 6-Aminohexansäuremethylester-Hydrochlorid sowie 9.4 g (68.1 mmol) Kaliumcarbonat versetzt. Das Gemisch wird für 18 Stunden unter Rückfluss erhitzt und dann nach Abkühlen auf Raumtemperatur filtriert. Der Rückstand wird dreimal in je 50 ml Wasser verrührt; filtriert und im Vakuum getrocknet. Es werden 4.1 g (41% d. Th.) der Titelverbindung erhalten.
LC-MS (Methode 8): Rₜ = 2.47 min.; m/z = 370 (M+H)⁺
¹H-NMR (400 MHz, CDCl₃): δ = 8.31 (s, 1H), 7.88 (s, 1H), 7.42 (d, 2H), 7.10 (d; 2H), 5.79 (t, NH), 3.82 (s, 3H), 3.57 (s, 3H), 3.43 (q, 2H), 2.30 (t, 2H), 1.57-1.48 (m, 4H), 1.31-1.24 (m, 2H).

### Beispiel 73A

### 6-{[6-Brom-5-(4-methoxyphenyl)furo[2,3-d]pyrimidin-4-yl]amino}hexansäuremethylester

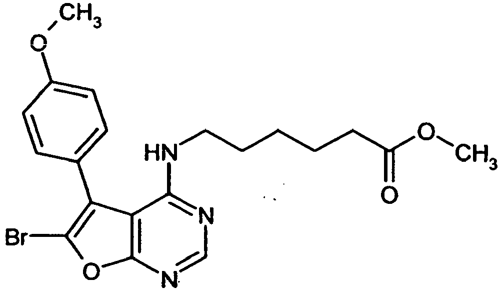

4.1 g (11.1 mmol) 6-{[5-(4-Methoxyphenyl)furo[2,3-d]pyrimidin-4-yl]amino}hexansäuremethylester werden bei Raumtemperatur in 150 ml Tetrachlorkohlenstoff gelöst und mit 2.2 g (12.2 mmol) *N*-Bromsuccinimid versetzt. Die Mischung wird drei Stunden unter Rückfluss gerührt, dann nach Abkühlen auf Raumtemperatur filtriert und das Filtrat im Vakuum eingeengt. Es werden 4.8 g (96% d. Th.) der Titelverbindung erhalten.
LC-MS (Methode 10): Rₜ = 2.65 min.; m/z = 448 (M+H)⁺
¹H-NMR (400 MHz, CDCl₃): δ = 8.29 (s, 1H), 7.41 (d, 2H), 7.12 (d, 2H), 5.61 (t, NH), 3.82 (s, 3H), 3.57 (s, 3H), 3.38 (q, 2H), 2.28 (t, 2H), 1.54-1.42 (m, 4H), 1.26-1.18 (m, 2H).

### Beispiel 74A

### 2-(2-Fluorphenyl)-2-hydroxy-1-(4-methoxyphenyl)ethanon

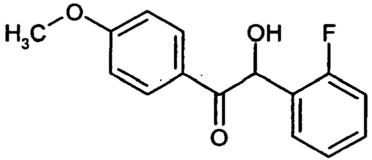

441 ml (1.10 mol) einer 2.5 M n-Butyllithium-Lösung in n-Hexan werden bei -78°C zu einer Lösung von 156 ml (1.11 mol) *N,N*-Diisopropylamin in 1937 ml 1,2-Dimethoxyethan in dem Maße zugetropft, dass die Temperatur -60°C nicht überschreitet. Nach 1 min Rühren bei dieser Temperatur wird innerhalb von 30 min eine Lösung von 236 g (1.00 mol) (4-Methoxyphenyl)[(tri-methylsilyl)oxy]acetonitril [N. Kurono, J. Org. Chem. 2005, 16, 6530-6532] in 753 ml 1,2-Dimethoxyethan zugetropft. Anschließend wird nach 30 min Rühren bei dieser Temperatur eine Lösung von 128 g (1.00 mol) 2-Fluorbenzaldehyd in 753 ml 1,2-Dimethoxyethan innerhalb von 20 min zugetropft. Das Reaktionsgemisch lässt man innerhalb von 4 h auf Raumtemperatur erwärmen. Nach Zugabe von 3800 ml ges. wässriger Ammoniumchlorid-Lösung wird mit Essigsäureethylester extrahiert. Die organische Phase wird mit ges. Ammoniumchlorid-Lösung gewaschen, über Natriumsulfat getrocknet, filtriert und das Filtrat im Vakuum eingeengt. Der Rückstand wird mit 3800 ml Dioxan, 2700 ml Methanol sowie 3120 ml 1 M Salzsäure versetzt und 16 h bei Raumtemperatur gerührt. Nach Zugabe von 8000 ml ges. wässriger Natriumchlorid-Lösung wird mit 4000 ml Essigsäureethylester extrahiert. Die wässrige Phase wird mit 2000 ml Essigsäureethylester nachextrahiert. Die vereinigten organischen Phasen werden mit 2000 ml Wasser und 2000 ml ges. Natriumchlorid-Lösung gewaschen, über Natriumsulfat getrocknet, filtriert und das Filtrat im Vakuum eingeengt. Der Rückstand wird in 600 ml Diisopropylether verrührt und filtriert. Die Mutterlauge wird im Vakuum eingeengt. Der Rückstand wird in Dichlormethan aufgenommen und mittels Flash-Chromatographie an Kieselgel (Laufmittel: Cyclohexan/Essigsäureethylester 4:1) gereinigt. Die so erhaltene Produktfraktion wird in Diisopropylether/Petrolether (1:1) verrührt, filtriert und im Vakuum getrocknet. Es werden 94 g (80% Reinheit, 29% d. Th.) der Titelverbindung erhalten.
LC-MS (Methode 11): Rₜ = 4.59 min.; m/z = 261 (M+H)⁺
¹H-NMR (400 MHz, CDCl₃): δ = 7.93-7.91 (m, 2H), 7.28-7.18 (m, 2H), 7.10-7.04 (m, 2H), 6.89-6.86 (m, 2H), 6.19 (d, 1H), 4.69 (s, 1H), 3.82 (s, 3H).

### Beispiel 75A

### 2-Amino-5-(2-fluorphenyl)-4-(4-methoxyphenyl)-3-furonitril

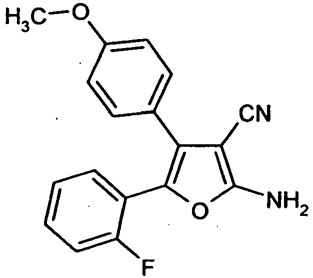

84 g (0.32 mol) 2-(2-Fluorphenyl)-2-hydroxy-1-(4-methoxyphenyl)ethanon und 32 g (0.48 mol) Malonsäuredinitril werden in 153 ml THF vorgelegt. Nach fünf Minuten Rühren werden unter Eiskühlung 49 ml (36 g, 0.36 mol) Triethylamin zugesetzt. Das Reaktionsgemisch wird 1 h unter Eiskühlung gerührt. Dann lässt man das Reaktionsgemisch auf Raumtemperatur erwärmen und 4 h bei dieser Temperatur rühren. Nach Zugabe von 1000 ml Essigsäureethylester wird die organische Phase fünfmal mit 300 ml Wasser gewaschen, über Natriumsulfat getrocknet und filtriert. Das Filtrat wird im Vakuum eingeengt. Der Rückstand wird in Dichlormethan aufgenommen und mittels Flash-Chromatographie an Kieselgel (Laufmittel: Dichlormethan/Methanol 70:1, dann Cyclohexan/Essigsäureethylester 2:1) gereinigt. 37 g (0.11 mol) an dabei zurückgewonnenem 2-(2-Fluorphenyl)-2-hydroxy-1-(4-methoxyphenyl)ethanon werden nach obiger Vorschrift erneut mit 14 g (0.03 mol) Malonsäuredinitril und 21 ml (15 g, 0.15 mol) Triethylamin in 67 ml THF umgesetzt. Es werden insgesamt 70 g (52% Reinheit, 36% d. Th.) der Zielverbindung erhalten.
¹H-NMR (400 MHz, CDCl₃): δ = 7.23-7.11 (m, 4H), 7.03-6.95 (m, 2H), 6.82-6.79 (m, 2H), 4.86 (s, NH₂), 3.74 (s, 3H).

### Beispiel 76A

### 6-(2-Fluorphenyl)-5-(4-methoxyphenyl)furo[2,3-d]pyrimidin-4(3H)-on

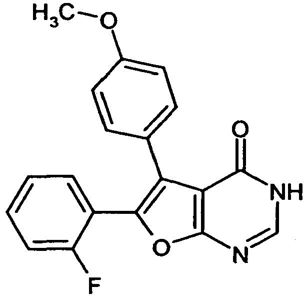

436 ml Essigsäureanhydrid werden bei 0°C tropfenweise mit 268 ml Ameisensäure versetzt und 30 min bei dieser Temperatur gerührt. Dann wird eine Lösung von 70 g (0.12 mol) 2-Amino-5-(2-fluorphenyl)-4-(4-methoxyphenyl)-3-furonitril in 100 ml Essigsäureanhydrid hinzugefügt und das Gemisch 24 h bei 130°C gerührt. Nach Abkühlen auf Raumtemperatur wird der Ansatz im Ölpumpenvakuum bei 50°C eingeengt. Der Rückstand wird in 250 ml Diisopropylether unter Eiskühlung 30 min verrührt, filtriert, mit 70 ml Diisopropylether gewaschen und im Vakuum getrocknet. Es werden 23.7 g (60% d. Th.) der Titelverbindung erhalten.
HPLC (Methode 1): Rₜ = 4.27 min.
MS (DCI): m/z = 354 (M+NH₄)⁺
¹H-NMR (400 MHz, CDCl₃): δ = 12.68 (br. s, NH), 8.19 (d, 1H), 7.53-7.45 (m, 2H), 7.34-7.25 (m, 4H), 6.91-6.88 (m, 2H), 3.76 (s, 3H).

### Beispiel 77A

### 4-Chlor-6-(2-fluorphenyl)-5-(4-methoxyphenyl)furo[2,3-d]pyrimidin

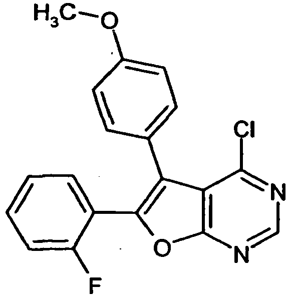

Ein Gemisch aus 20 g (0.06 mol) 6-(2-Fluorphenyl)-5-(4-methoxyphenyl)furo[2,3-d]pyrimidin-4(3*H*)-on in 78 ml Sulfolan und 11 ml (18 g, 0.12 mol) Phosphorylchlorid wird 1 h bei 120°C gerührt. Nach Abkühlen auf Raumtemperatur wird die Reaktionslösung unter kräftigem Rühren und Eiskühlung in ein Gemisch aus 1000 ml Wasser und 100 ml 25%-ige wässrige Ammoniak-Lösung getropft. Der bei 10°C ausfallende Feststoff wird abfiltriert und mehrmals mit Wasser gewaschen. Der Feststoff wird erneut in 700 ml Essigsäureethylester gelöst und die Lösung zweimal mit je 500 ml Wasser gewaschen. Die organische Phase wird über Natriumsulfat getrocknet, filtriert und das Filtrat im Vakuum eingeengt. Der Rückstand wird in 60 ml Diisopropylether verrührt, filtriert und im Vakuum getrocknet. Es werden 18 g (81% d. Th.) der Titelverbindung erhalten.
HPLC (Methode 1): Rₜ = 5.03 min.
¹H-NMR (400 MHz, DMSO-d₆): δ = 8.90 (s, 1H), 7.58-7.50 (m, 2H), 7.36-7.27 (m, 4H), 7.01-6.97 (m, 2H), 3.79 (s, 3H).

### Beispiel 78A

### 1-(4-Ethylphenyl)-2-(2-fluorphenyl)-2-hydroxyethanon

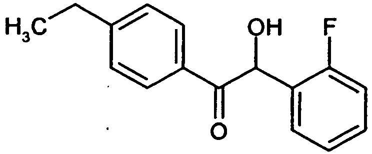

217 ml (0.54 mol) einer 2.5 M n-Butyllithium-Lösung in Hexan werden bei -78°C zu einer Lösung von 77 ml (56 g, 0.55 mol) *N,N*-Diisopropylamin in 960 ml 1,2-Dimethoxyethan so zugetropft, dass die Temperatur -60°C nicht überschreitet. Nach 15 min Rühren bei dieser Temperatur wird innerhalb von 30 min eine Lösung von 116 g (0.50 mol) (4-Ethylphenyl)[(trimethylsilyl)oxy]acetonitril [D.S. Dhanoa, J. Med. Chem. 1993, 36 (23), 3738-3742] in 373 ml 1,2-Dimethoxyethan zugetropft. Anschließend wird nach 30 min Rühren bei dieser Temperatur eine Lösung von 64 g (0.50 mol) 2-Fluorbenzaldehyd in 373 ml 1,2-Dimethoxyethan innerhalb von 20 min zugetropft. Das Reaktionsgemisch lässt man innerhalb von 4 h auf Raumtemperatur erwärmen. Nach Zugabe von 1900 ml ges. wässriger Ammoniumchlorid-Lösung wird mit Essigsäureethylester extrahiert. Die organische Phase wird mit ges. Ammoniumchlorid-Lösung gewaschen, über Natriumsulfat getrocknet und filtriert. Das Filtrat wird im Vakuum eingeengt. Der Rückstand wird mit 1900 ml Dioxan, 1350 ml Methanol sowie 1560 ml 1 M Salzsäure versetzt und 16 h bei Raumtemperatur gerührt. Nach Zugabe von 4000 ml ges. wässriger Natriumchlorid-Lösung wird mit 2000 ml Essigsäureethylester extrahiert. Die organische Phase wird mit 1000 ml Wasser und 1000 ml ges. Natriumchlorid-Lösung gewaschen, über Natriumsulfat getrocknet und filtriert. Das Filtrat wird im Vakuum eingeengt. Der Rückstand wird mittels Flash-Chromatographie an Kieselgel (Laufmittel: Cyclohexan/Essigsäureethylester 5: 1) gereinigt. Die so erhaltene Produktfraktion wird in 80 ml Diisopropylether und 240 ml Petrolether verrührt, filtriert, mit Petrolether gewaschen und im Vakuum getrocknet. Es werden 50 g (85% Reinheit, 33% d. Th.) der Titelverbindung erhalten.
HPLC (Methode 1): Rₜ = 4.50 min.
MS (DCI): m/z = 276 (M+NH₄)⁺
¹H-NMR (400 MHz, CDCl₃): δ = 7.87-7.85 (m, 2H), 7.28-7.19 (m, 4H), 7.11-7.04 (m, 2H), 6.22 (d, 1H), 4.64 (d, 1H), 2.65 (q, 2H), 1.21 (t, 3H).

### Beispiel 79A

### 2-Amino-4-(4-ethylphenyl)-5-(2-fluorphenyl)-3-furonitril

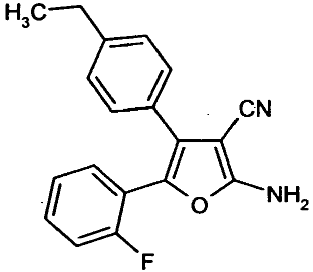

50 g (0.19 mol) 1-(4-Ethylphenyl)-2-(2-fluorphenyl)-2-hydroxyethanon und 17 g (0.25 mol) Malonsäuredinitril werden in 93 ml DMF vorgelegt. Nach fünf Minuten Rühren werden unter Eiskühlung 17 ml (12 g, 0.12 mol) Diethylamin hinzugefügt. Das Reaktionsgemisch wird 1 h unter Eiskühlung gerührt. Dann lässt man auf Raumtemperatur erwärmen und 4 h bei dieser Temperatur rühren. Nach Zugabe von 500 ml Wasser wird nach 30 min Rühren die wässrige Phase abdekantiert. Durch erneute Zugabe von 500 ml Wasser und erneutes Dekantieren erhält man einen öligen Rückstand, der in Essigsäureethylester gelöst, über Natriumsulfat getrocknet und filtriert wird. Das Filtrat wird im Vakuum eingeengt. Der Rückstand enthält laut DC-Analyse (Laufmittel: Cyclohexan/Essigsäureethylester 4:1) noch 1-(4-Ethylphenyl)-2-(2-fluorphenyl)-2-hydroxyethanon. Der Rückstand wird daher nach obiger Vorschrift in 90 ml DMF erneut mit 5.5 g (0.08 mol) Malonsäuredinitril und 10 ml (7 g, 0.10 mol) Diethylamin umgesetzt. Das Reaktionsgemisch wird in 500 ml Essigsäureethylester gegeben und dreimal mit je 300 ml Wasser und einmal mit 300 ml ges. Natriumchlorid-Lösung gewaschen. Die organische Phase wird über Natriumsulfat getrocknet und filtriert. Das Filtrat wird im Vakuum eingeengt. Der Rückstand wird mittels Flash-Chromatographie an Kieselgel (Laufmittel: Cyclohexan/Essigsäureethylester 3:1) gereinigt. Es werden 36 g (61% d. Th.) der Titelverbindung erhalten, welche ohne weitere Charakterisierung umgesetzt werden.

### Beispiel 80A

### 5-(4-Ethylphenyl)-6-(2-fluorphenyl)furo[2,3-d]pyrimidin-4(3H)-on

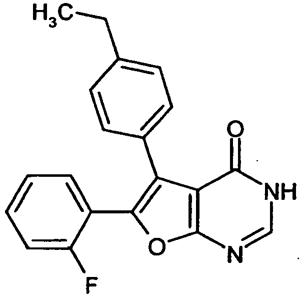

280 ml (2.97 mol) Essigsäureanhydrid werden bei 0°C tropfenweise mit 140 ml (3.71 mol) Ameisensäure versetzt und 30 min bei dieser Temperatur gerührt. Dann werden 36.0 g (0.12 mol) 2-Amino-4-(4-ethylphenyl)-5-(2-fluorphenyl)-3-furonitril hinzugefügt und das Gemisch 24 h bei 130°C gerührt. Nach Abkühlen auf Raumtemperatur wird der Ansatz im Ölpumpenvakuüm bei 50°C eingeengt. Der Rückstand wird in 150 ml Diisopropylether bei -10°C 30 min lang verrührt, abfiltriert, mit 50 ml eisgekühltem Diisopropylether gewaschen und im Vakuum getrocknet. Es werden 20.6 g (86% Reinheit, 45% d. Th.) der Titelverbindung erhalten.
HPLC (Methode 1): Rₜ = 4.65 min.
MS (ESIpos): m/z = 335 (M+H)⁺
¹H-NMR (400 MHz, DMSO-d₆): δ = 12.68 (br. s, NH), 8.20 (s, 1H), 7.53-7.45 (m, 2H), 7.36-7.25 (m, 4H), 7.21-7.16 (m, 2H), 2.61 (q, 2H), 1.19 (t, 3H).

### Beispiel 81A

### 4-Chlor-5-(4-ethylphenyl)-6-(2-fluorphenyl)furo[2,3-d]pyrimidin

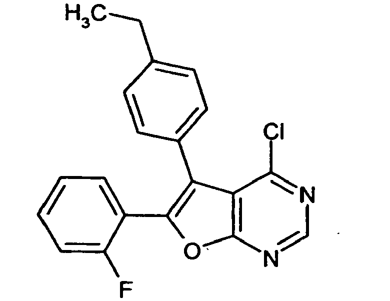

Eine Suspension von 20.0 g (0.06 mol) 5-(4-Ethylphenyl)-6-(2-fluorphenyl)furo[2,3-d]pyrimidin-4(3*H*)-on in 100 ml (165 g, 1.07 mol) Phosphorylchlorid wird 1 h bei 120°C gerührt. Nach Abkühlen auf Raumtemperatur wird die Reaktionslösung unter kräftigem Rühren in ein Gemisch aus 330 ml Wasser und 610 ml 25%-iger wässriger Ammoniak-Lösung getropft, wobei ein Temperaturanstieg auf 55-65°C beobachtet wird. Man lässt das Reaktionsgemisch auf Raumtemperatur abkühlen. Nach zweimaliger Extraktion mit je 500 ml Dichlormethan wird die organische Phase mit ges. wässriger Natriumchlorid-Lösung gewaschen, über Natriumsulfat getrocknet und filtriert. Das Filtrat wird im Vakuum eingeengt. Der Rückstand wird in 150 ml Petrolether verrührt, abfiltriert, mit eisgekühltem Petrolether gewaschen und im Vakuum getrocknet. Es werden 18.7 g (90% Reinheit, 80% d. Th.) der Titelverbindung erhalten.
LC-MS (Methode 5): Rₜ = 3.14 min.; m/z = 353 (M+H)⁺
¹H-NMR (400 MHz, DMSO-d₆): δ = 8.91 (s, 1H), 7.58-7.49 (m, 2H), 7.36-7.24 (m, 6H), 2.66 (q, 2H), 1.21 (t, 3H).

### Beispiel 82A

### 3-{[5(-4-Methoxyphenyl)-6-phenylfuro[2,3-d]pyrimidin-4-yl]oxy}-2-methylpropan-1-ol

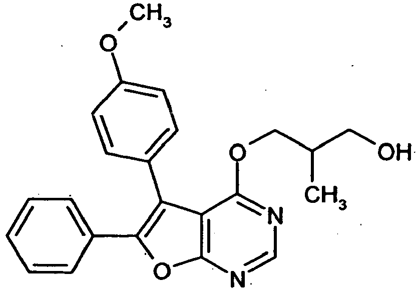

Eine Lösung von 2.68 g (29.7 mmol) 2-Methylpropan-1,3-diol in 45 ml Toluol, 15 ml 1,2-Dimethoxyethan und 15 ml Wasser wird bei 70°C mit 4.8 ml einer 12.5 N Natronlauge versetzt. Nach Zugabe von 202 mg (0.59 mmol) Tetra-*n*-butylammoniumhydrogensulfat und 2.0 g (5.94 mmol) 4-Chlor-5-(4-methoxyphenyl)-6-phenylfuro[2,3-d]pyrimidin wird das Reaktionsgemisch 17 h bei 70°C gerührt. Nach Abkühlen auf Raumtemperatur wird mit konzentrierter Salzsäure auf pH 7 eingestellt. Es wird dreimal mit je 50 ml Dichlormethan extrahiert. Die vereinigten organischen Extrakte werden mit ges. Natriumchlorid-Lösung gewaschen, über Natriumsulfat getrocknet, filtriert und im Vakuum eingeengt. Das Rohprodukt wird in Methanol verrührt, filtriert und das Filtrat mittels präparativer RP-HPLC (Gradient: Wasser/Acetonitril) aufgereinigt. Es werden 1.26 g (54% d. Th.) des gewünschten Produkts (Racemat) erhalten.
LC-MS (Methode 8): Rₜ = 2.73 min.; m/z = 391 (M+H)⁺
¹H-NMR (400 MHz, CDCl₃): δ = 8.58 (s, 1H), 7.55 (d, 2H), 7.48-7.35 (m, 5H), 7.00 (d, 2H), 4.48 (t, OH), 4.34 (dd, 1H), 4.24 (dd, 1H), 3.81 (s, 3H), 3.23-3.14 (m, 2H), 1.86-1.78 (m, 1H), 0.72 (d, 3H).

### Beispiel 83A

### (2R,3R)3-{[5-(4-Methoxyphenyl)-6-phenylfuro[2,3-d]pyrimidin-4-yl]oxy}butan-2-ol

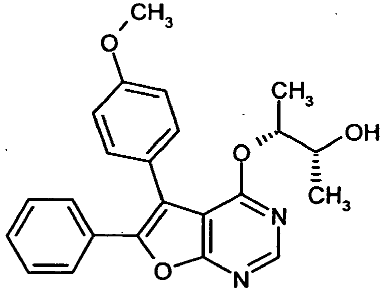

Eine Lösung von 1.34 g (14.8 mmol) (*2R,3R*)-Butan-2,3-diol in 20 ml Toluol, 7 ml 1,2-Dimethoxyethan und 7 ml Wasser wird bei 70°C mit 2.4 ml einer 12.5 N Natronlauge versetzt. Nach Zugabe von 101 mg (0.30 mmol) Tetra-*n*-butylammoniumhydrogensulfat und 1.00 g (2.97 mmol) 4-Chlor-5-(4-methoxyphenyl)-6-phenylfuro[2,3-d]pyrimidin wird das Reaktionsgemisch 17 h bei 70°C gerührt. Nach Abkühlen auf Raumtemperatur wird mit konzentrierter Salzsäure auf pH 7 eingestellt. Es wird dreimal mit je 50 ml Dichlormethan extrahiert. Die vereinigten organischen Extrakte werden mit ges. Natriumchlorid-Lösung gewaschen, über Natriumsulfat getrocknet, filtriert und im Vakuum eingeengt. Das Rohprodukt wird in Methanol verrührt, filtriert und das Filtrat mittels präparativer RP-HPLC (Gradient: Wasser/Acetonitril) gereinigt. Es werden 0.60 g (50% d. Th.) des gewünschten Produkts erhalten.
LC-MS (Methode 7): Rₜ = 3.95 min.; m/z = 391 (M+H)⁺
¹H-NMR (400 MHz, CDCl₃): δ = 8.56 (s, 1H), 7.55 (d, 2H), 7.48-7.35 (m, 5H), 7.00 (d, 2H), 5.29 (dt, 1H), 4.71 (d, OH), 3.81 (s, 3H), 3.73-3.62 (m, 1H), 1.13 (d, 3H), 0.85 (d, 3H).

### Beispiel 84A

### (2R)-1-[Benzyl(methyl)amino]propan-2-ol

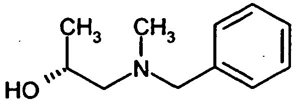

Ein Gemisch aus 3.5 g (21.2 mmol) (*2R*)-1-(Benzylamino)propan-2-ol [F.L. Delft, Synthesis 1997, 4, 450-454], 1.85 ml (2.0 g, 23.3 mmol) einer 35%-igen wässrigen Formaldehyd-Lösung und 3.6 ml (4.4 g, 95.3 mmol) Ameisensäure wird 16 h unter Rückfluss gerührt. Nach dem Abkühlen auf Raumtemperatur wird mit 45%-iger Natronlauge zunächst neutralisiert und dann ein pH-Wert von 9 eingestellt. Es wird mit Essigsäureethylester extrahiert. Die organische Phase wird dreimal mit je 10 ml Wasser gewaschen, über Natriumsulfat getrocknet und filtriert. Das Filtrat wird im Vakuum eingeengt und getrocknet. Man erhält 3.08 g (78% d. Th.) des gewünschten Produkts.
LC-MS (Methode 3): Rₜ = 1.85 min.; m/z = 180 (M+H)⁺
¹H-NMR (400 MHz, DMSO-d₆): δ = 7.31-7.21 (m, 5H), 4.28 (d, 1H), 3.81-3.72 (m, 1H), 3.48 (q, 2H), 2.24 (dq, 2H), 2.13 (s, 3H), 1.04 (d, 3H).

### Beispiel 85A

### (2R)-N-Benzyl-2-{[5-(4-ethylphenyl)-6-phenylfuro[2,3-d]pyrimidin-4-yl]oxy}-N-methylpropan-1-amin

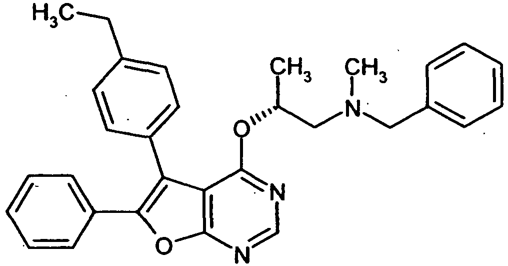

Eine Lösung von 600 mg (3.35 mmol) (*2R*)-1-[Benzyl(methyl)amino]propan-2-ol in 7 ml THF wird bei Raumtemperatur mit 167 mg (4.18 mmol) Natriumhydrid (als 60%-ige Dispersion in Mineralöl) versetzt. Nach zehn Minuten Rühren werden eine Lösung von 1177 mg (3.51 mmol) 4-Chlor-5-(4-ethylphenyl)-6-phenylfuro[2,3-d]pyrimidin in 8 ml THF sowie 62 mg (0.17 mmol) Tetra-n-butylammoniumiodid zugegeben. Das Reaktionsgemisch wird 16 h unter Rückfluss gerührt. Nach Zugabe von Wasser und Essigsäureethylester wird die abgetrennte organische Phase mit 1 N Salzsäure und ges. Natriumchlorid-Lösung gewaschen. Die wässrige Phase wird mit Essigsäureethylester rückextrahiert. Die vereinigten organischen Phasen werden über Natriumsulfat getrocknet und filtriert. Das Filtrat wird im Vakuum eingeengt. Der Rückstand wird in Cyclohexan/ Essigsäureethylester/Dichlormethan aufgenommen und an Kieselgel chromatographiert (Laufmittel: Cyclohexan/Essigsäureethylester 5:1, 2:1, 1:1, dann Essigsäureethylester). Man erhält 1366 mg (83% d. Th.) des gewünschten Produkts.
LC-MS (Methode 8): Rₜ = 2.08 min.; m/z = 478 (M+H)⁺
¹H-NMR (400 MHz, DMSO-d₆): δ = 8.57 (s, 1H), 7.55-7.53 (m, 2H), 7.42-7.34 (m, 5H), 7.26-7.17 (m, 5H), 7.13-7.11 (m, 2H), 5.57-5.49 (m, 1H), 3.36 (d, 2H), 2.63 (q, 2H), 2.48-2.39 (m, 2H), 1.98 (s, 3H), 1.23-1.17 (m, 6H).

### Beispiel 86A

### (2R)-2-{[5-(4-Ethylphenyl)-6-phenylfuro[2,3-d]pyritnidin-4-yl]oxy}-N-methylpropan-1-ammoniumformiat

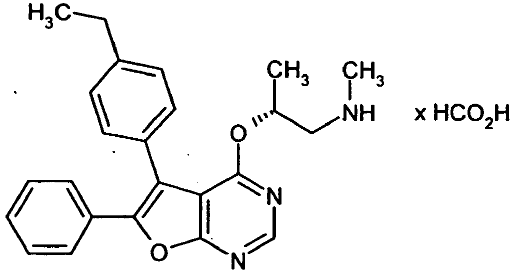

Eine mit Argon überschichtete Lösung von 1.45 g (3.04 mmol) (2*R*)-*N-*Benzyl-2-{[5-(4-ethylphenyl)-6-phenylfuro[2,3-d]pyrimidin-4-yl]oxy}-*N*-methylpropan-1-amin in 20 ml 4.4%-iger methanolischer Ameisensäure wird mit 0.50 g (4.70 mmol) Palladium Black versetzt und 14 h bei Raumtemperatur gerührt. Nach dem Filtrieren über einen Millipore-Filter und mehrmaligem Waschen mit Methanol/Wasser wird das Filtrat im Vakuum eingeengt. Der Rückstand wird in Acetonitril/Methanol aufgenommen und mittels präparativer RP-HPLC (Gradient: Wasser/Acetonitril) gereinigt. Man erhält 1.03 g (77% d. Th.) des gewünschten Produkts.
LC-MS (Methode 8): Rₜ = 1.79 min.; m/z = 388 (M-HCO₂H+H)⁺
¹H-NMR (400 MHz, DMSO-d₆): δ = 8.60 (s, 1H), 8.18 (s, HCOO⁻), 7.56-7.52 (m, 2H), 7.46-7.35 (m, 5H), 7.34-7.28 (m, 2H), 5.48-5.40 (m, 1H), 4.22-2.93 (br. s, H₂N⁺), 2.86-2.82 (m, 1H), 2.72-2.62 (m, 3H), 2.24 (s, 3H), 1.27-1.23 (m, 6H).

### Beispiel 87A

### 4-{[(2S)-2-Hydroxypropyl]amino}buttersäure-tert.-butylester

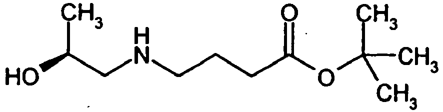

Eine Lösung von 936 mg (12.46 mmol) (*2S*)-1-Aminopropan-2-ol in 10 ml THF wird mit 2583 mg (18.69 mmol) Kaliumcarbonat, 2780 mg (12.46 mmol) 4-Brombuttersäure-*tert*.-butylester sowie 184 mg (0.50 mmol) Tetra-*n*-butylammoniumiodid versetzt. Das Reaktionsgemisch wird 48 h bei Raumtemperatur gerührt. Nach Abfiltrieren der anorganischen Salze wird das Filtrat im Vakuum eingeengt. Der Rückstand wird in Dichlormethan aufgenommen und an Kieselgel (Laufmittel: Dichlormethan/Methanol/35%-ige wässrige Ammoniak-Lösung 9:1:0.1) chromatographiert. Es werden 810 mg (30% d. Th.) des gewünschten Produkts erhalten.
GC-MS (Methode 12): Rₜ = 4.73 min.; m/z = 172 (M-CH₃CHOH)⁺
¹H-NMR (400 MHz, DMSO-d₆): δ = 4.39 (br. s, OH), 3.67-3.59 (m, 1H), 3.38-3.20 (br. s, NH), 2.51-2.47 (m, 2H), 2.39-2.37 (m, 2H), 2.21 (t, 2H), 1.60 (quin, 2H), 1.39 (s, 9H), 1.02 (d, 3H).

### Beispiel 88A

### 4-{[(2S)-2-Hydroxypropyl](methyl)amino}buttersäure-tert.-butylester

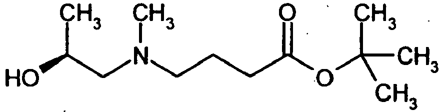

Eine Lösung von 350 mg (1.61 mmol) 4-{[(*2S*)-2-Hydroxypropyl]amino}buttersäure-*tert*.-butylester in 10 ml Methanol wird mit 0.62 ml (670 mg, 7.81 mmol) 35%-iger wässriger Formaldehyd-Lösung und 101 mg (1.61 mmol) Natriumcyanoborhydrid versetzt. Das Reaktionsgemisch wird 16 h bei Raumtemperatur gerührt. Nach Zugabe von 30 ml Wasser und 40 ml Dichlormethan wird die organische Phase über Natriumsulfat getrocknet und filtriert. Das Filtrat wird im Vakuum eingeengt und getrocknet. Es werden 323 mg (81% d. Th.) des gewünschten Produkts erhalten.
GC-MS (Methode 12): Rₜ = 4.57 min.; m/z = 186 (M-CH₃CHOH)⁺
¹H-NMR (400 MHz, DMSO-d₆): δ = 4.16 (d, 1H), 3.71-3.62 (m, 1H), 2.29 (t, 2H), 2.23-2.18 (m, 3H), 2.16-2.10 (m, 4H), 1.63-1.55 (m, 2H), 1.39 (s, 9H), 1.02 (d, 3H).

### Beispiel 89A

### 4-{[5-(4-Methoxyphenyl)-6-phenylfuro[2,3-d]pyrimidin-4-yl]oxy}pentan-2-ol

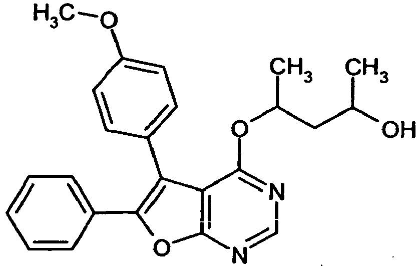

Eine Lösung von 4.64 g (44.54 mmol) Pentan-2,4-diol in 75 ml Toluol, 27 ml 1,2-Dimethoxyethan und 25 ml Wasser wird bei 70°C mit 7.9 ml einer 11.25 N Natronlauge versetzt. Nach Zugabe von 302 mg (0.89 mmol) Tetra-*n*-butylammoniumhydrogensulfat und 3.00 g (8.91 mmol) 4-Chlor-5-(4-methoxyphenyl)-6-phenylfuro[2,3-d]pyrimidin wird das Reaktionsgemisch 17 h bei 70°C gerührt. Nach Abkühlen auf Raumtemperatur wird mit konzentrierter Salzsäure auf pH 7 eingestellt. Es wird dreimal mit je 150 ml Dichlormethan extrahiert. Die vereinigten organischen Extrakte werden mit ges. Natriumchlorid-Lösung gewaschen, über Natriumsulfat getrocknet und filtriert. Das Filtrat wird im Vakuum eingeengt. Der Rückstand wird in Acetonitril verrührt, filtriert und das Filtrat an Kieselgel (Laufmittel: Dichlonmethan/Methanol) chromatographiert. Es werden 2.37 g (65% d. Th.) des gewünschten Produkts als racemisches Diastereomerengemisch erhalten.
LC-MS (Methode 8): Rₜ = 2.81 min.; m/z = 405 (M+H)⁺
¹H-NMR (400 MHz, DMSO-d₆): [Minder-Stereoisomer in Klammern] δ = [8.57, s, 1H], 8.56 (s, 1H), 7.56-7.52 (m, 2H), 7.42-7.37 (m, 5H), 7.02-6.99 (m, 2H), 5.54-5.46 (m, 1H), [5.38-5.30, m, 1H], 4.46 (d, OH), [4.39, d, OH], 3.82 (s, 3H), [3.81, s, 3H], 3.69-3.60 (m, 1H), [3.46-3.37, m, 1H], 1.77-1.70 (m, 1H), 1.47-1.41 (m, 1H), [1.28, d, 3H], 1.26 (d, 3H), 1.00 (d, 3H), [0.93, d, 3H].

### Beispiel 90A

### 2-({[5-(4-Ethylphenyl)-6-phenylfuro[2,3-d]pyrimidin-4-yl]oxy}methyl)-3,3-dimethylbutan-1-ol

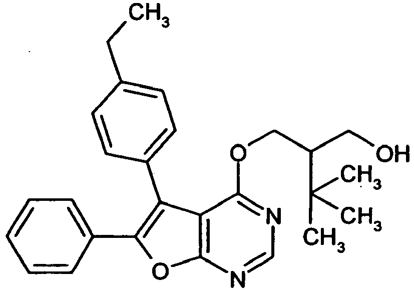

Eine Lösung von 1974 mg (14.93 mmol) 2-*tert*.-Butylpropan-1,3-diol in 25 ml Toluol, 8 ml 1,2-Dimethoxyethan und 8 ml Wasser wird bei 70°C mit 2.7 ml einer 11.25 N Natronlauge versetzt. Nach Zugabe von 101 mg (0.30 mmol) Tetra-*n*-butylammoniumhydrogensulfat und 1000 mg (2.99 mmol) 4-Chlor-5-(4-ethylphenyl)-6-phenylfuro[2,3-d]pyrimidin wird das Reaktionsgemisch 17 h bei 70°C gerührt. Nach Abkühlen auf Raumtemperatur wird mit konzentrierter Salzsäure auf pH 7 eingestellt. Es wird mit Dichlormethan extrahiert. Die organische Phase wird mit ges. Natriumchlorid-Lösung gewaschen, über Natriumsulfat getrocknet und filtriert. Das Filtrat wird im Vakuum eingeengt. Der Rückstand wird in Methanol verrührt, filtriert und mit Diethylether gewaschen. Das Filtrat wird mittels präparativer RP-HPLC (Gradient: Wasser/Acetonitril) gereinigt. Es werden 275 mg (21 % d. Th.) des gewünschten Produkts (Racemat) erhalten.
LC-MS (Methode 9): Rₜ = 4.55 min.; m/z = 431 (M+H)⁺
¹H-NMR (400 MHz, DMSO-d₆): δ = 8.60 (s, 1H), 7.53-7.51 (m, 2H), 7.39-7.36 (m, 5H), 7.30-7.28 (m, 2H), 4.53 (dd, 1H), 4.45 (dd, 1H), 4.40 (t, 1H), 3.45-3.41 (m, 1H), 3.30-3.25 (m, 1H), 2.68 (q, 2H), 1.39-1.34 (m, 1H), 1.29 (t, 3H), 0.67 (s, 9H).

### Beispiel 91A

### 3-{[5-(4-Methoxyphenyl)-6-phenylfuro[2,3-d]pyrimidin-4-yl]oxy}butan-2-ol

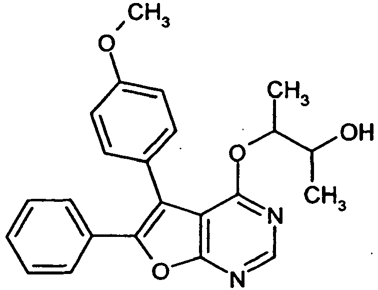

Ein Gemisch aus 2.68 g (29.70 mmol) (*2R*,*3S*)-Butan-2,3-diol in 45 ml Toluol, 15 ml 1,2-Dimethoxyethan und 15 ml Wasser wird bei 70°C mit 4.8 ml einer 12.5 N Natronlauge versetzt. Nach Zugabe von 0.20 g (0.60 mmol) Tetra-*n*-butylammoniumhydrogensulfat und 2.00 g (5.94 mmol) 4-Chlor-5-(4-methoxyphenyl)-6-phenylfuro[2,3-d]pyrimidin wird das Reaktionsgemisch 17 h bei 70°C gerührt. Nach Abkühlen auf Raumtemperatur wird mit konzentrierter Salzsäure auf pH 7 eingestellt. Es wird dreimal mit je 100 ml Dichlormethan extrahiert. Die vereinigten organischen Extrakte werden mit ges. Natriumchlorid-Lösung gewaschen, über Natriumsulfat getrocknet, filtriert und im Vakuum eingeengt. Das Rohprodukt wird mittels präparativer RP-HPLC (Gradient: Wasser/Acetonitril) gereinigt. Es werden 1.26 g (54% d. Th.) des gewünschten Produkts als (*R,Sl S*,*R*)-Racemat erhalten.
LC-MS (Methode 8): Rₜ = 2.81 min.; m/z = 391 (M+H)⁺
¹H-NMR (400 MHz, DMSO-d₆): δ = 8.57 (s, 1H), 7.55-7.53 (m, 2H), 7.43-7.36 (m, 5H), 7.01-7.00 (m, 2H), 5.21-5.16 (m, 1H), 4.68 (d, OH), 3.81 (s, 3H), 3.61-3.55 (m, 1H), 1.19 (d, 3H), 0.86 (d, 3H).

### Beispiel 92A

### (+/-)-2-Methoxy-3-{[5-(4-methoxyphenyl)-6-phenylfuro[2,3-d]pyrimidin-4-yl]oxy}propan-1-ol

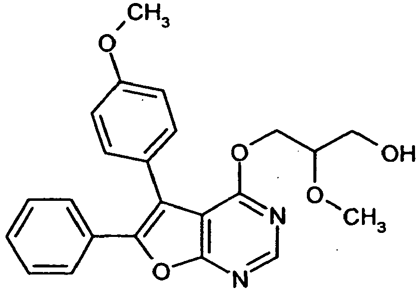

1.014 g (9.65 mmol) 2-Methoxypropan-1,3-diol werden in 20 ml THF vorgelegt. Man gibt 542 mg (4.825 mmol) Kalium-*tert*.-butylat hinzu und rührt 15 min bei RT. Dann wird auf 0°C abgekühlt und eine Lösung von 650 mg (1.93 mmol) 4-Chlor-5-(4-methoxyphenyl)-6-phenylfuro[2,3-d]pyrimidin in 10 ml THF innerhalb von 30 min zugetropft. Man lässt man auf RT kommen und rührt über Nacht bei RT. Anschließend wird mit *tert*.-Butylmethylether und Wasser verdünnt. Man stellt mit 10%-iger Zitronensäure-Lösung sauer und trennt die Phasen. Die wässrige Phase wird einmal mit *tert*.-Butylmethylether nachextrahiert. Die organischen Phasen werden vereinigt und einmal mit ges. Natriumchlorid-Lösung gewaschen. Dann wird über Magnesiumsulfat getrocknet und eingeengt. Man reinigt den Rückstand durch Chromatographie an Silicagel (Laufmittel: Cyclohexan/ Ethylacetat 7:3) und erhält so 587 mg (74.8% d. Th.) der Zielverbindung.
LC-MS (Methode 8): Rₜ = 2.53 min.; m/z = 407 (M+H)⁺
¹H-NMR (400 MHz, CDCl₃): δ = 8.53 (s, 1H), 7.62 (m, 2H), 7.39 (m, 2H), 7.32 (m, 3H), 6.97 (d, 2H), 4.53 (d, 2H), 3.88 (s, 3H), 3.62-3.52 (m, 1H), 3.52-3.43 (m, 2H), 3.32 (s, 3H).

### Beispiel 93A

### 6-{[(1R)-1-Phenylethyl]amino}heptansäuremethylester

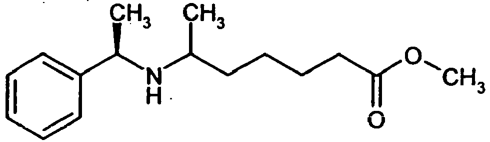

8.80 g (55.63 mmol) 6-Oxoheptansäure und 6.741 g (55.63 mmol) *(R)*-(+)-1-Phenylethylamin werden zusammen bei RT in 100 ml Toluol vorgelegt. Man gibt eine katalytische Menge (ca. 50 mg) p-Toluolsulfonsäure hinzu und erhitzt über Nacht unter Rühren am Wasserabscheider. Man engt danach teilweise ein, filtriert einige feste Bestandteile mit Hilfe eines Papierfilters ab und engt das Filtrat dann vollständig ein. Der Rückstand wird in 170 ml Methanol gelöst. Man gibt ca. 1.7 g Raney-Nickel (wasserfeucht) hinzu und hydriert 48 h bei 4 bar. Anschließend wird über Kieselgur filtriert und das Filtrat eingeengt. Man reinigt den Rückstand durch Säulenchromatographie an Silicagel (Laufmittel: Dichlormethan/Methanol 95:5) und erhält so 4.15 g (48.5% d. Th.) der Zielverbindung, welche ohne weitere Charakterisierung umgesetzt wird.

### Ausführungsbeispiele:

### Beispiel 1

### 6-{[5-(4-Methoxyphenyl)-6-phenylfuro[2,3-d]pyrimidin-4-yl]amino}hexansäure

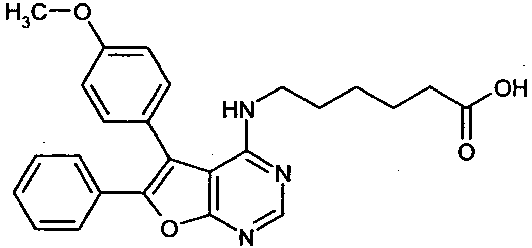

1.0 g (3.0 mmol) 4-Chlor-5-(4-methoxyphenyl)-6-phenylfuro[2,3-d]pyrimidin, 0.78 g (5.94 mmol) 6-Aminohexansäure und 1.5 ml DIEA in 10 ml DMF werden über Nacht bei 120°C gerührt. Die Reaktionsmischung wird auf Wasser gegeben und dreimal mit Ethylacetat extrahiert. Die vereinigten organischen Phasen werden mit gesättigter Natriumchlorid-Lösung gewaschen, über Magnesiumsulfat getrocknet und im Vakuum eingeengt. Der Rückstand wird an Kieselgel chromatographiert (Laufmittel: Cyclohexan/Ethylacetat 2:1 → 1:2). Es werden 560 mg (43.7% d. Th.) der Zielverbindung erhalten.
LC-MS (Methode 4): Rₜ = 2.62 min.; m/z = 432 (M+H)⁺
¹H-NMR (400 MHz, DMSO-d₆): δ = 12.0 (br. s, 1H), 8.31 (s, 1H), 7.47-7.42 (m, 4H), 7.40-7.30 (m, 3H), 7.15 (d, 2H), 5.08 (t, 1H), 3.87 (s, 3H), 2.08 (t, 2H), 1.50-1.35 (m, 4H), 1.25-1.10 (m, 4H).

### Beispiel 2

### 7-{[5-(4-Methoxyphenyl)-6-phenylfuro[2,3-d]pyrimidin-4-yl]amino}heptansäure

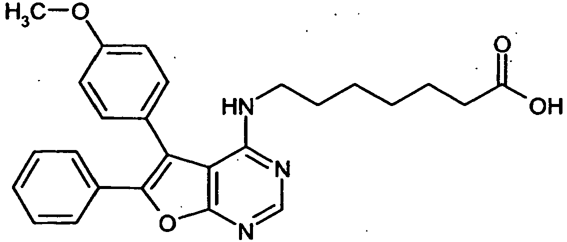

Die Titelverbindung wird auf analoge Weise zu Beispiel 1 in einer Ausbeute von 55.1% d. Th. erhalten.
LC-MS (Methode 4): Rₜ = 2.72 min.; m/z = 446 (M+H)⁺
¹H-NMR (400 MHz, DMSO-d₆): δ = 8.33 (s, 1H), 7.48-7.43 (m, 4H), 7.40-7.30 (m, 3H), 7.15 (d, 2H), 5.04 (t, 1H), 3.85 (s, 3H), 2.08 (t, 2H), 1.50-1.38 (m, 4H), 1.20-1.11 (m, 2H).

### Beispiel 3

### 6-{[5-(4-Methoxyphenyl)-6-phenylfuro[2,3-d]pyrimidin-4-yl]amino}hexansäure Natriumsalz

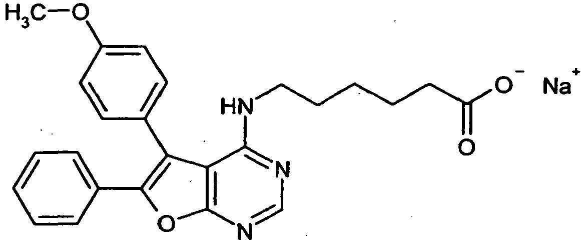

200 mg (0.464 mmol) 6-{[5-(4-Methoxyphenyl)-6-phenylfuro[2,3-d]pyrimidin-4-yl]amino}hexansäure werden bei RT in 0.75 ml Methanol, 0.5 ml THF und einigen Tropfen Wasser gelöst und mit 0.464 ml 1 N Natronlauge versetzt. Die Mischung wird 5 min gerührt, dann im Vakuum eingeengt und der Rückstand im Hochvakuum getrocknet. Es werden 221 mg der Zielverbindung erhalten.
LC-MS (Methode 2): Rₜ = 2.34 min.; m/z = 432 (M-Na+2H)⁺
¹H-NMR (300 MHz, DMSO-d₆): δ = 8.32 (s, 1H), 7.48-7.43 (m, 4H), 7.39-7.30 (m, 3H), 7.17 (d, 2H), 5.04 (t, 1H), 3.88 (s, 3H), 1.72 (t, 2H), 1.40-1.32 (m, 4H), 1.15-1.08 (m, 2H).

### Beispiel 4

### 5-(4-Methoxyphenyl)-6-phenyl-N-[5-(1H-tetrazol-5-yl)pentyl]furo[2,3-d]pyrimidin-4-amin

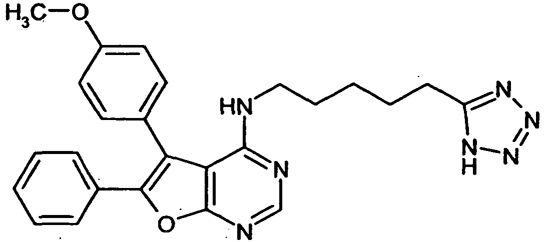

1.00 g (2.4 mmol) 6-{[5-(4-Methoxyphenyl)-6-phenylfuro[2,3-d]pyrimidin-4-yl]amino}hexannitril, 4.19 g (26.3 mmol) Trimethylsilylazid und 0.91 g (3.6 mmol) Di-n-butylzinnoxid in 50 ml Toluol werden über Nacht bei 80°C gerührt. Nach Einengen wird der Rückstand in Wasser aufgenommen, mit verdünnter Salzsäure angesäuert und mit Methylenchlorid extrahiert. Die organische Phase wird mit Natriumchlorid-Lösung gewaschen, über Magnesiumsulfat getrocknet und eingeengt. Das Rohprodukt wird durch RP-HPLC gereinigt (Säule: Gromsil 250 mm x 40 mm, 10 µm; Acetonitril/Wasser-Gradient: 0-3 min 10% Acetonitril, 3-50 min 10% → 98% Acetonitril, 50-55 min 98% Acetonitril). Die vereinigten Produktfraktionen werden aus Diethylether kristallisiert und im Vakuumtrockenschrank über Nacht bei 50°C getrocknet. Es werden 598 mg (54% d. Th.) der Titelverbindung als nahezu weiße Kristalle erhalten.
LC-MS (Methode 2): Rₜ = 2.22 min.; m/z = 455 (M+H)⁺
¹H-NMR (400 MHz, DMSO-d₆): δ = 16 (br. s, 1H), 8.3 (s, 1H), 7.5-7.25 (m, 7H), 7.1 (m, 2H), 5.1 (m, 1H), 3.85 (s, 3H), ca. 3.5 (m, verdeckt durch DMSO-Signal), 2.75 (t, 2H), 1.65 (m, 2H), 1.45 (m, 2H), 1.2 (m, 2H).

### Beispiel 5

### (2E)-6-{[5-(4-Methoxyphenyl)-6-phenylfuro[2,3-d]pyrimidin-4-yl]amino}-hex-2-ensäuremethyl-ester

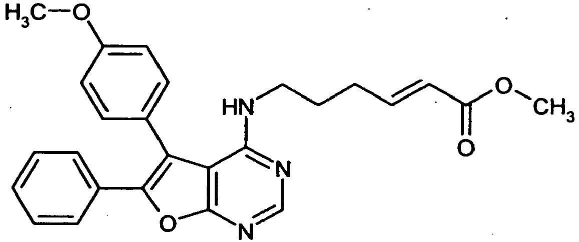

Zu einer Suspension von 21.6 mg Natriumhydrid (60% Dispersion in Öl, ca. 0.539 mmol) in 2 ml THF werden bei RT 0.095 ml (0.588 mmol) Phosphonoessigsäuretrimethylester getropft. Die Mischung wird 1 h nachgerührt und anschließend werden 190 mg (0.49 mmol) 4-{[5-(4-Methoxyphenyl}-6-phenylfuro[2,3-d]pyrimidin-4-yl]amino}butanal eingetragen. Man rührt über Nacht bei RT und verdünnt die Mischung dann mit Dichlormethan und Wasser. Die organische Phase wird mit gesättigter Natriumchlorid-Lösung gewaschen, getrocknet und im Vakuum konzentriert. Aus dem Rohprodukt wird die Zielverbindung mittels zweifacher präparativer RP-HPLC (Gradient: Wasser/Acetonitril) isoliert. Es werden 23.4 mg (10.8% d. Th.) des gewünschten Produkts erhalten.
LC-MS (Methode 2): Rₜ = 2.65 min.; m/z = 444 (M+H)⁺
¹H-NMR (400 MHz, DMSO-d₆): δ = 8.33 (s, 1H), 7.47-7.43 (m, 4H), 7.40-7.31 (m, 3H), 7.14 (d, 2H), 6.85 (dd, 1H), 5.84-5.78 (m, 1H), 5.68 (t, 1H), 3.85 (s, 3H), 3.63 (s, 3H), 3.49 (q, 2H), 2.17-2.10 (m, 2H), 1.63-1.57 (m, 2H).

### Beispiel 6

### (2E)-6-{[5-(4-Methoxyphenyl)-6-phenylfuro[2,3-d]pyrimidin-4-yl]amino}-hex-2-ensäure Natriumsalz

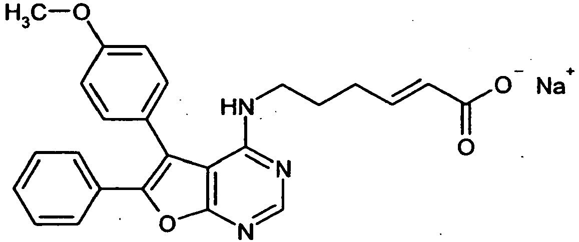

19 mg (0.043 mmol) (2*E*)-6-{[5-(4-Methoxyphenyl)-6-phenylfuro[2,3-d]pyrimidin-4-yl]amino}-hex-2-ensäuremethylester werden in 0.5 ml THF vorgelegt und bei RT mit 0.43 ml 1 N Natronlauge versetzt. Die Mischung wird 24 h bei RT gerührt, dann mit 1 N Salzsäure neutralisiert und im Vakuum eingeengt. Der Rückstand wird mit etwas 1 N Natronlauge versetzt und direkt mittels präparativer RP-HPLC (Gradient: Wasser/ Acetonitril) aufgereinigt. Es werden 9 mg (46.5% d. Th.) der Zielverbindung isoliert.
LC-MS (Methode 5): Rₜ = 2.51 min.; m/z = 429 (M-Na+2H)⁺
¹H-NMR (400 MHz, DMSO-d₆): δ = 8.3 (s, 1H), 7.48-7.3 (m, 7H), 7.12 (d, 2H), 6.20 (dd, 1H), 5.58 (d, 2H), 5.60 (t, 1H), 3.85 (s, 3H), 1.95.(q, 2H), 1.52 (m, 2H).

### Beispiel 7

### 5-(4-Methoxyphenyl)-6-phenyl-N-[6-(1H-tetrazol-5-yl)hexyl]furo[2,3-d]pyrimidin-4-amin

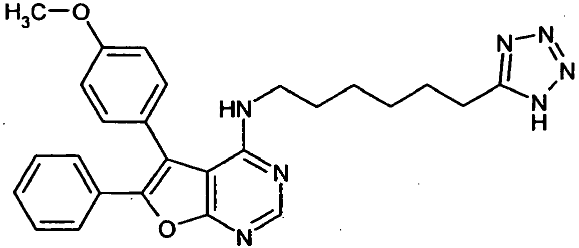

0.098 g (0.23 mmol) 7-{[5-(4-Methoxyphenyl)-6-phenylfuro[2,3-d]pyrimidin-4-yl]amino}heptan-nitril, 0.41 g (3.5 mmol) Trimethylsilylazid und 86 mg (0.25 mmol) Di-n-butylzinnoxid in 5 ml Toluol werden über Nacht bei 80°C gerührt. Nach Einengen wird der Rückstand in Wasser aufgenommen, mit verdünnter Salzsäure angesäuert und mit Methylenchlorid extrahiert. Die organische Phase wird mit Natriumchlorid-Lösung gewaschen, über Magnesiumsulfat getrocknet und eingeengt. Das Rohprodukt wird durch RP-HPLC gereinigt (Säule: Gromsil 250 mm x 30 mm, 10 µm; Acetonitril/Wasser-Gradient: 0-3 min 5% Acetonitril, 3-34 min 5% → 98% Acetonitril, 34-38 min 98% Acetonitril). Es werden 61 mg (55% d. Th.) der Titelverbindung als weiße Kristalle erhalten.
LC-MS (Methode 2): Rₜ = 2.30 min.; m/z = 469 (M+H)⁺
¹H-NMR (400 MHz, DMSO-d₆): δ=16 (br. s, 1H), 8.3 (s, 1H), 7.5-7.25 (m, 7H), 7.15 (m, 2H), 5.1 (m, 1H), 3.85 (s, 3H), ca. 3.5 (m, verdeckt durch DMSO-Signal), 2.85 (t, 2H), 1.65 (m, 2H), 1.35 (m, 2H), 1.1-1.3 (m, 4H).

### Beispiel 8

### 6-[(5,6-Diphenylfuro[2,3-d]pyrimidin-4-yl)amino]hexansäuremethylester

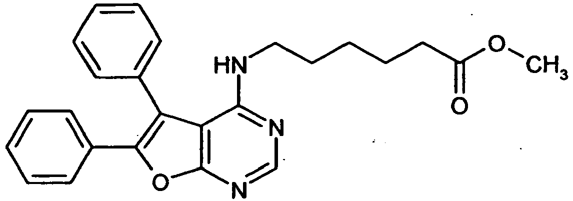

55 mg (0.131 mmol) 6-[(6-Brom-5-phenylfuro[2,3-d]pyrimidin-4-yl)amino]hexansäuremethylester, 0.131 ml einer 2 M wässrigen Natriumcarbonat-Lösung (0.26 mmol), 4.6 mg (0.006 mmol) Bis(triphenylphosphin)palladium(II)chlorid und 20 mg (0.164 mmol) Phenylboronsäure werden unter Argon in 0.4 ml DMSO 1 h lang bei 80°C gerührt. Nach dem Abkühlen wird die Mischung direkt mittels präparativer RP-HPLC (Gradient: Wasser/Acetonitril) aufgereinigt. Es werden 35 mg (64.1% d. Th.) der Zielverbindung erhalten.
LC-MS (Methode 4): Rₜ = 3.01 min.; m/z = 416 (M+H)⁺
¹H-NMR (400 MHz, DMSO-d₆): δ = 8.34 (s, 1H), 7.62-7.53 (m, 5H), 7.47-7.42 (m, 2H), 7.39-7.30 (m, 3H), 4.97 (t, 1H), 3.60 (s, 3H), 3.35 (q, 2H), 2.17 (t, 2H), 1.50-1.35 (m, 4H), 1.18-1.10 (m, 2H).

### Beispiel 9

### 6-{[5-(4-Methylphenyl)-6-phenylfuro[2,3-d]pyrimidin-4-yl]amino}hexansäuremethylester

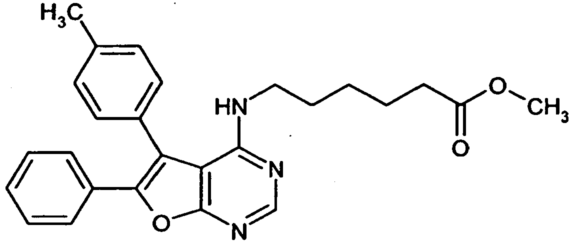

50 mg (0.12 mmol) 6-[(5-Brom-6-phenylfuro[2,3-d]pyrimidin-4-yl)amino]hexansäuremethylester, 0.12 ml einer 2 M wässrigen Natriumcarbonat-Lösung (0.24 mmol), 4.2 mg (0.006 mmol) Bis(triphenylphosphin)palladium(II)chlorid und 20.3 mg (0.149 mmol) p-Toluolboronsäure werden unter Argon in 0.4 ml DMSO 1 h lang bei 80°C gerührt. Nach dem Abkühlen wird die Mischung direkt mittels präparativer RP-HPLC (Gradient: Wasser/Aetonitril) und nachfolgender Chromatographie an Kieselgel (Laufmittel: Cyclohexan/Ethylacetat) aufgereinigt. Es werden 38.1 mg (74.2% d. Th.) der Zielverbindung erhalten.
LC-MS (Methode 4): Rₜ = 3.15 min.; m/z = 430 (M+H)⁺
¹H-NMR (400 MHz, DMSO-d₆): δ = 8.32 (s, 1H), 7.48-7.30 (m, 10H), 5.01 (t, 1H), 3.58 (s, 3H), 3.38 (q, 2H), 2.43 (s, 3H), 2.27 (t, 2H), 1.50-1.35 (m, 4H), 1.17-1.10 (m, 2H).

### Beispiel 10

### 6-[(5,6-Diphenylfuro[2,3-d]pyrimidin-4-yl)amino]hexansäure

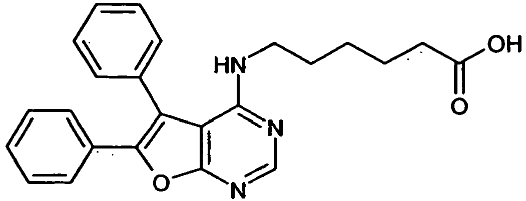

27.5 mg (0.066 mmol) 6-[(5,6-Diphenylfuro[2,3-d]pyrimidin-4-yl)amino]hexansäuremethylester werden in 0.1 ml Methanol, 0.05 ml THF und einem Tropfen Wasser gelöst und mit 0.09 ml 2.5 M Natronlauge versetzt. Die Mischung wird 1 h bei RT gerührt und dann mit 1 N Salzsäure schwach angesäuert. Die wässrige Phase wird dreimal mit Dichlormethan extrahiert. Die vereinigten organischen Phasen werden über Magnesiumsulfat getrocknet, im Vakuum eingeengt und der Rückstand im Hochvakuum getrocknet. Erhalten werden 25 mg (96.1 % d. Th.) der Zielverbindung.
LC-MS (Methode 5): Rₜ = 2.51 min.; m/z = 402 (M+H)⁺
¹H-NMR (300 MHz, DMSO-d₆): δ = 12.0 (br. s, 1H), 8.35 (s; 1H), 7.62-7.51 (m, 5H), 7.46-7.41 (m, 2H), 7.39-7.30 (m, 3H), 4.97 (t, 1H), 3.39 (q, 2H), 2.18 (t, 2H), 1.45-1.35 (m, 4H), 1.18-1.10 (m, 2H).

### Beispiel 11

### 6-{[5-(4-Methylphenyl)-6-phenylfuro[2,3-d]pyrimidin-4-yl]amino}hexansäure

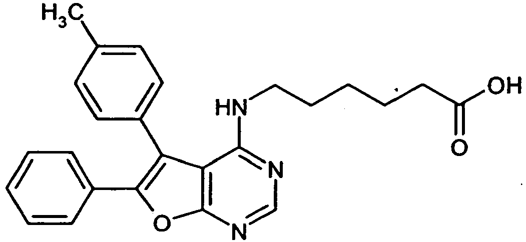

30 mg (0.07 mmol) 6-{[5-(4-Methylphenyl)-6-phenylfuro[2,3-d]pyrimidin-4-yl]amino}hexansäuremethylester werden in 0.1 ml Methanol, 0.05 ml THF und einem Tropfen Wasser gelöst und mit 0.1 ml 2.5 M Natronlauge versetzt. Die Mischung wird 1 h bei RT gerührt und dann mit 1 N Salzsäure schwach angesäuert. Der ausgefallene Niederschlag wird abgesaugt, mehrfach mit Wasser gewaschen und im Hochvakuum getrocknet. Erhalten werden 28 mg (96.5% d. Th.) der Zielverbindung.
LC-MS (Methode 4): Rₜ = 2.70 min.; m/z = 416 (M+H)⁺
¹H-NMR (400 MHz, DMSO-d₆): δ = 12.0 (br. s, 1H), 8.33 (s, 1H), 7.48-7.30 (m, 10H), 4.99 (t, 1H), 3.38 (q, 2H), 2.45 (s, 3H), 2.18 (t, 2H), 1.46-1.37 (m, 4H), 1.18-1.10 (m, 2H).

### Beispiel 12

### 6- {[5-(4-Methoxyphenyl)-6-phenylfuro[2,3-d]pyrimidin-4-yl]oxy}hexansäureethylester

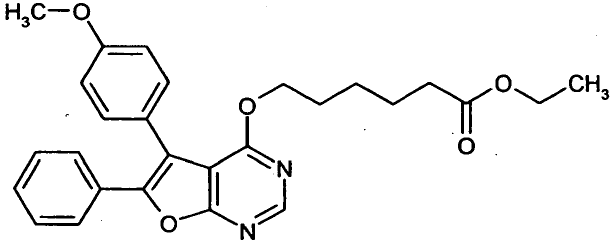

Zu einer Mischung von 300 mg (0.89 mmol) 4-Chlor-5-(4-methoxyphenyl)-6-phenylfuro[2,3-d]-pyrimidin und 214 mg (1.34 mmol) 6-Hydroxyhexansäureethylester in 1.0 ml THF und 0.7 ml DMF werden bei RT in Portionen 42.8 mg Natriumhydrid (60%-ige Dispersion in Öl, ca. 1.07 mmol) eingetragen. Die Mischung wird 1 h bei RT gerührt, bevor Dichlormethan und Wasser zugesetzt werden. Die organische Phase wird mit gesättigter Natriumchlorid-Lösung gewaschen, über Natriumsulfat getrocknet und im Vakuum eingeengt. Aus dem Rückstand werden nach präparativer RP-HPLC 120.9 mg (29.5% d. Th.) der Zielverbindung isoliert.
LC-MS (Methode 5): Rₜ = 3.25 min.; m/z = 461 (M+H)⁺
¹H-NMR (400 MHz, DMSO-d₆): δ = 8.57 (s, 1H), 7.56 (m, 2H), 7.44-7.39 (m, 5H), 7.03 (d, 2H), 4.37 (t, 2H), 4.04 (q, 2H), 3.81 (s, 3H), 2.21 (t, 2H), 1.61-1.55 (m, 2H), 1.50-1.42 (m, 2H), 1.18 (m, 5H).

### Beispiel 13

### 6-{[5-(4-Methoxyphenyl)-6-phenylfuro[2,3-d]pyrimidin-4-yl]oxy}hexansäure

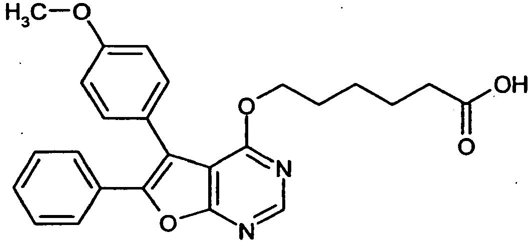

103 mg (0.224 mmol) 6-{[5-(4-Methoxyphenyl)-6-phenylfuro[2,3-d]pyrimidin-4-yl]oxy}hexansäureethylester werden in 2 ml THF gelöst und bei RT mit 2.2 ml 1 N Natronlauge versetzt. Die Mischung wird über Nacht gerührt, dann mit 1 N Salzsäure neutralisiert und im Vakuum eingeengt. Der Rückstand wird mittels präparativer RP-HPLC gereinigt. Es werden 23.2 mg (24% d. Th.) der Zielverbindung erhalten.
LC-MS (Methode 5): Rₜ = 2.73 min.; m/z = 433 (M+H)⁺.

### Beispiel 14

### 5-(4-Methoxyphenyl)-6-phenyl-4-{[5-(1H-tetrazol-5-yl)pentyl]oxy}-furo[2,3-d]pyrimidin

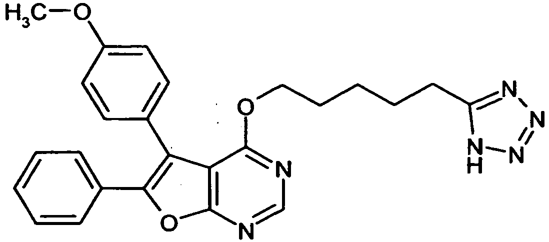

1.00 g (2.1 mmol) 6-{[5-(4-Methoxyphenyl)-6-phenylfuro[2,3-d]pyrimidin-4-yl]oxy}hexannitril, 0.79 g (3.2 mmol) Di-n-butylzinnoxid und 3.68 g (32 mmol) Trimethylsilylazid in 44 ml Toluol werden über Nacht bei 80°C gerührt. Danach wird 1 ml Ethylenglykol zugesetzt, 1 h unter Rückfluss gerührt und anschließend eingeengt. Der Rückstand wird in Ethylacetat aufgenommen, mit verdünnter Salzsäure und mit Natriumchlorid-Lösung gewaschen, getrocknet und eingeengt. Die Reinigung des Rohprodukts erfolgt mittels RP-HPLC (Säule: Gromsil 250 mm x 40 mm, 10 µm; Acetonitril/Wasser-Gradient: 0-3 min 5% Acetonitril, 3-50 min 5% → 98% Acetonitril, 50-55 min 98% Acetonitril). Die vereinigten Produktfraktionen werden aus Diethylether kristallisiert. Man erhält 372 mg (38% d. Th.) der Titelverbindung als beige Kristalle.
LC-MS (Methode 5): Rₜ = 2.39 min.; m/z = 456 (M+H)⁺
¹H-NMR (400 MHz, DMSO-d₆): δ = ca. 16-14 (breit, 1H), 8.6 (s, 1H), 7.55 (m, 2H), 7.4 (m, 5H), 7.0 (m, 2H), 4.35 (t, 2H), 3.8 (s, 3H), 2.3 (t, 2H), 1.6 (m, 4H), 1.2 (m, 2H).

### Beispiel 15

### 5-(4-Methoxyphenyl)-6-phenyl-N-{3-[2-(1H-tetrazol-5-yl)ethoxy]propyl}furo[2,3-d]pyrimidin-4-amin

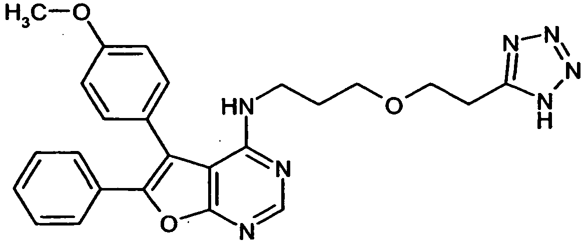

137 mg (0.32 mmol) 5-(4-Methoxyphenyl)-6-phenyl-*N*-{3-[2-cyanoethoxy]propyl}furo[2,3-d]-pyrimidin-4-amin, 552 mg (4.8 mmol) Trimethylsilylazid und 119 mg (0.48 mmol) Di-n-butylzinnoxid werden in 10 ml Toluol über Nacht bei 80°C gerührt. Der Ansatz wird danach abgekühlt und eingeengt. Der verbleibende Rückstand wird in Methylenchlorid gelöst und mit Wasser und Natriumchlorid-Lösung gewaschen. Die organische Phase wird über Magnesiumsulfat getrocknet und am Rotationsverdampfer bei vermindertem Druck eingeengt. Der Rückstand wird durch RP-HPLC (Säule: Gromsil 250 mm x 30 mm, 10 µm; Acetonitril/Wasser-Gradient: 0-3 min 5% Acetonitril, 3-34 min 5% → 98% Acetonitril, 34-38 min 98% Acetonitril) gereinigt. Man erhält 48.6 mg (32% d. Th.) der Titelverbindung als farblosen Feststoff.
LC-MS (Methode 4): Rₜ = 2.44 min.; m/z = 472 (M+H)⁺
¹H-NMR (400 MHz, DMSO-d₆): δ = 16.0 (br. s, 1H), 8.35 (s, 1H), 7.1-7.45 (m, 9H), 5.2 (m, 1H), 3.85 (s, 3H), 3.65 (t, 2H), ca. 3.3 (m, 2H, teilweise verdeckt durch H₂O), 3.05 (t, 2H), 1.65 (quin, 2H).

### Beispiel 16

### 3-(3-{[5-(4-Methoxyphenyl)-6-phenylfüro[2,3-d]pyrimidin-4-yl]amino}propoxy)propansäure

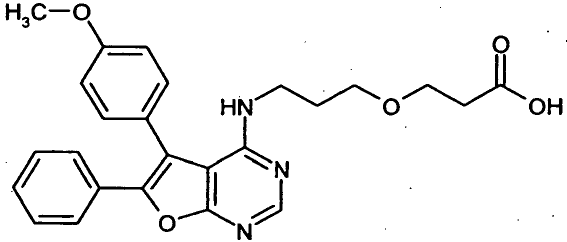

160 mg (0.37 mmol) 5-(4-Methoxyphenyl)-6-phenyl-*N*-{3-[2-cyanoethoxy]propyl}furo[2,3-d]-pyrimidin-4-amin werden in 6 ml konz. Salzsäure zum Sieden erhitzt und 7 h lang gerührt. Nach Abkühlen auf Raumtemperatur wird mit Wasser verdünnt und mit Essigsäureethylester extrahiert. Die organischen Phasen werden über Magnesiumsulfat getrocknet und eingeengt. Mehrfache Reinigung des Rückstands durch RP-HPLC (Säule: Gromsil 250 mm x 30 mm, 10 µm; Acetonitril/Wasser-Gradient: 0-3 min 5% Acetonitril, 3-34 min 5% → 98% Acetonitril, 34-38 min 98% Acetonitril) sowie Flash-Chromatographie an Kieselgel (Laufmittelgradient: Ethylacetat → Ethylacetat/Methanol 5:1) ergibt ein Öl, welches in heissem Ethylacetat gelöst wird. In der Hitze wird bis zur Trübung mit Diisopropylether versetzt. Nach Stehenlassen über Nacht im Kühlschrank erhält man einen gelblichen Feststoff, der in Dichlormethan aufgenommen wird: Nach erneutem Einengen wird der Rückstand mit Diethylether verrührt. Auf diese Weise werden 7.1 mg (4.3% d. Th.) der Titelverbindung als graues Pulver erhalten, welches nach einiger Zeit wieder ölig wird.
LC-MS (Methode 4): Rₜ = 2.42 min.; m/z = 448 (M+H)⁺
¹H-NMR (400 MHz, DMSO-d₆): δ = 8.35 (s, 1H), 7.1-7.45 (m, 9H), 5.2 (m, 1H), 3.85 (s, 3H), 3.65 (t, 2H), ca. 3.3 (m, 4H, teilweise verdeckt durch H₂O), 2.1 (t, 2H), 1.6 (m, 2H).

### Beispiel 17

### (5-{[5-(4-Methoxyphenyl)-6-phenylfuro[2,3-d]pyrimidin-4-yl]amino}pentyl)amino(oxo)methylacetat

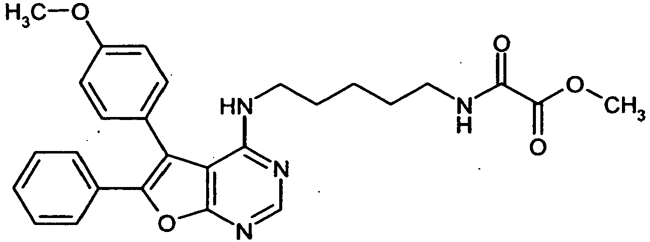

150 mg (0.37 mmol) 5-(4-Methoxyphenyl)-6-phenyl-*N*-(5-aminopentyl)furo[2,3-d]pyrimidin-4-amin werden in 10 ml Methylenchlorid gelöst. Bei 0°C werden 50 mg (0.41 mmol) Oxalsäuremethylesterchlorid und 72 mg (0.56 mmol) DIEA gleichzeitig langsam hinzugetropft. Es wird 1 h bei Raumtemperatur nachgerührt. Der Ansatz wird mit Methylenchlorid verdünnt und mit Wasser und Natriumchlorid-Lösung gewaschen. Die vereinigten organischen Phasen werden über Magnesiumsulfat getrocknet und am Rotationsverdampfer eingeengt. Der Rückstand wird durch RP-HPLC aufgereinigt (Säule: Gromsil 250 mm x 30 mm, 10 µm; Acetonitril/Wasser-Gradient: 0-3 min 5% Acetonitril, 3-34 min 5% → 98% Acetonitril, 34-38 min 98% Acetonitril). Nach Eindampfen der Produktfraktionen wird der Rückstand über Kieselgel chromatographiert (Analogix-Kartusche F12M, Eluent: Cyclohexan/Ethylacetat 1:1). Man erhält 47.8 mg (26% d. Th.) der Titelverbindung als farblosen Schaum.
LC-MS (Methode 4): Rₜ = 2.60 min.; m/z = 489 (M+H)⁺
¹H-NMR (400 MHz, DMSO-d₆): δ = 8.9 (m, 1H), 8.30 (s, 1H), 7.1-7.45 (m, 9H), 5.1 (m, 1H), 3.85 (s, 3H), 3.75 (s, 3H), ca. 3.4 (m, 2H, teilweise verdeckt durch H₂O), 3.1 (q, 2H), 1.6-1.1 (m, 6H).

### Beipiel 18

### (5-{[5-(4-Methoxyphenyl)-6-phenylfuro[2,3-d]pyrimidin-4-yl]amino}pentyl)amino(oxo)essigsäure

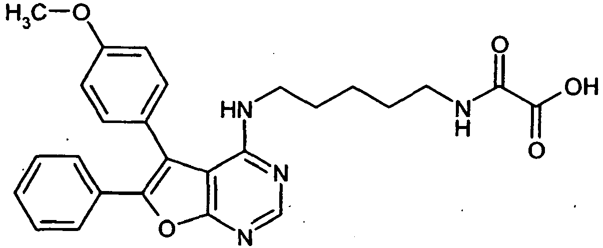

30 mg (0.06 mmol) (5-{[5-(4-Methoxyphenyl)-6-phenylfuro[2,3-d]pyrimidin-4-yl]amino}pentyl)-amino(oxo)methylacetat werden in 3 ml Dioxan gelöst und mit 0.12 ml 1 N Natronlauge versetzt. Anschließend wird 1 h lang bei 50°C gerührt. Der Ansatz wird eingeengt, der Rückstand in Wasser aufgenommen und mit Methylenchlorid gewaschen. Die wässrige Phase wird mit 1 N Salzsäure angesäuert und mit Essigsäureethylester extrahiert. Die organische Phase wird abgetrennt, über Magnesiumsulfat getrocknet und eingeengt. Man erhält 26.5 mg (91% d. Th.) der Titelverbindung als weißen Schaum.
LC-MS (Methode 4): Rₜ = 2.10 min.; m/z = 475 (M+H)⁺
¹H-NMR (400 MHz, DMSO-d₆): δ = 8.75 (m, 1H), 8.30 (s, 1H), 7.1-7.45 (m, 9H), 5.05 (m, 1H), 3.85 (s, 3H), ca. 3.4 (m, 2H, teilweise verdeckt durch H₂O), 3.05 (q, 2H), 1.6-1.1 (m, 6H).

### Beispiel 19

### 5-(4-Methoxyphenyl)-6-phenyl-4-{2-[2-(1H-tetrazol-5-yl)ethoxy]ethoxy}furo[2,3-d]pyrimidin

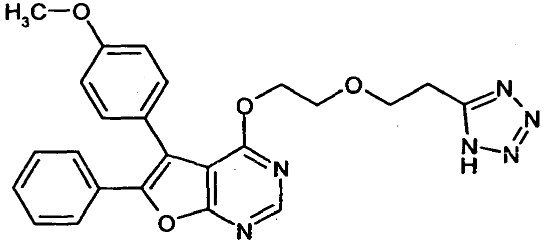

180 mg (0.43 mmol) 4-[3-(2-Cyanoethoxy)ethoxy]-5-(4-methoxyphenyl)-6-phenylfuro[2,3-d]-pyrimidin, 749 mg (6.5 mmol) Trimethylsilylazid und 161 mg (0.65 mmol) Di-n-butylzinnoxid werden in 10 ml Toluol über Nacht bei 80°C gerührt. Der Ansatz wird eingeengt, der Rückstand in Wasser aufgenommen und mit verdünnter Salzsäure angesäuert. Anschließend wird mit Methylenchlorid extrahiert. Die organischen Extrakte werden mit Natriumchlorid-Lösung gewaschen, über Magnesiumsulfat getrocknet und eingeengt. Der verbleibende Rückstand wird mittels RP-HPLC gereinigt (Säule: Gromsil 250 mm x 30 mm, 10 µm; Acetonitril/Wasser-Gradient: 0-3 min 5% Acetonitril, 3-50 min 5% → 98% Acetonitril, 50-55 min 98% Acetonitril). Man erhält einen beigen Schaum, der zur Entfernung von organischen Zinnverbindungen in 10 ml Toluol gelöst und mit 1 ml Ethylenglykol versetzt wird. Nach 1 h Erhitzen unter Rückfluss wird das Lösungsmittel im Vakuum abgezogen. Der Rückstand wird in Methylenchlorid gelöst, mit verdünnter Salzsäure und mit Natriumchlorid-Lösung gewaschen, getrocknet und eingeengt. Reinigung des Rückstands mittels RP-HPLC (Säule: Gromsil 250 mm x 30 mm, 10 µm; Acetonitril/Wasser-Gradient: 0-3 min 5% Acetonitril, 3-34 min 5% → 98% Acetonitril, 34-38 min 98% Acetonitril) ergibt 82.6 mg (42% d. Th.) der Titelverbindung als beigen Schaum.
LC-MS (Methode 5): Rₜ = 2.38 min.; m/z = 459 (M+H)⁺
¹H-NMR (400 MHz, DMSO-d₆): δ = 8.55 (s, 1H), 7.3-7.55 (m, 7H), 6.9 (m, 2H), 4.5 (m, 2H), 3.8 (s, 3H), 3.6 (m, 4H), 3.0 (t, 2H).

### Beispiel 20

### 5-(4-Methoxyphenyl)-6-phenyl-4-{3-[2-(1H-tetrazol-5-yl)ethoxy]propoxy}furo[2,3-d]pyrimidin

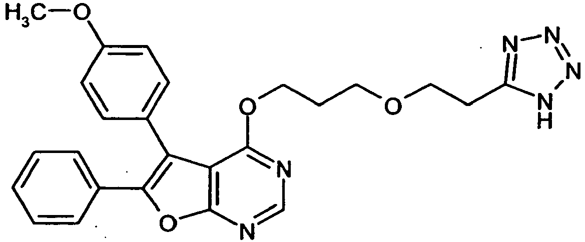

200 mg (0.47 mmol) 4-[3-(2-Cyanoethoxy)propoxy]-5-(4-methoxyphenyl)-6-phenylfuro[2,3-d]-pyrimidin, 804 mg (7.0 mmol) Trimethylsilylazid und 174 mg (0.70 mmol) Di-n-butylzinnoxid in 10 ml Toluol werden über Nacht bei 80°C gerührt. Der Ansatz wird eingeengt, der Rückstand in Wasser aufgenommen und mit verdünnter Salzsäure angesäuert. Anschließend wird mit Methylenchlorid extrahiert. Die organischen Extrakte werden mit Natriumchlorid-Lösung gewaschen, über Magnesiumsulfat getrocknet und eingeengt. Der verbleibende Rückstand wird mittels RP-HPLC gereinigt (Säule: Gromsil 250 mm x 30 mm, 10 µm; Acetonitril/Wasser-Gradient: 0-3 min 5% Acetonitril, 3-50 min 5% → 98% Acetonitril, 50-55 min 98% Acetonitril). Man erhält einen beigen Schaum, der zur Entfernung von organischen Zinnverbindungen in 10 ml Toluol gelöst und mit 1 ml Ethylenglykol versetzt wird. Nach 1 h Erhitzen unter Rückfluss wird das Lösungsmittel im Vakuum abgezogen. Der Rückstand wird in Methylenchlorid gelöst, mit verdünnter Salzsäure und mit Natriumchlorid-Lösung gewaschen, getrocknet und eingeengt. Reinigung des Rückstands mittels RP-HPLC (Säule: Gromsil 250 mm x 30 mm, 10 µm; Acetonitril/Wasser-Gradient: 0-3 min 5% Acetonitril, 3-34 min 5% → 98% Acetonitril, 34-38 min 98% Acetonitril) ergibt 46 mg (21% d. Th.) der Titelverbindung als beigen Schaum.
LC-MS (Methode 4): Rₜ = 2.62 min.; m/z = 473 (M+H)⁺
¹H-NMR (400 MHz, DMSO-d₆): δ = 16.0 (br. s, 1H), 8.55 (s, 1H), 7.3-7.55 (m, 7H), 6.9 (m, 2H), 4.35 (m, 2H), 3.8 (s, 3H), 3.65 (t, 2H), ca. 3.4 (m, 2H, teilweise verdeckt durch H₂O), 3.05 (t, 2H), 1.65 (quin, 2H).

### Allgemeine Vorschrift A: Palladium-katalysierte Arylierung von 5-Brom-6-phenylfuro[2,3-d]pyrimidin-Derivaten

Zu einer Lösung von 1.0 eq. 6-[(5-Brom-6-phenylfuro[2,3-d]pyrimidin-4-yl)amino]-hexansäuremethylester in DMSO (ca. 3 mol/l) werden bei RT nacheinander 1.2 bis 1.5 eq. der entsprechenden Arylboronsäure, ca. 2.0 eq. Natriumcarbonat (als 2 M wässrige Lösung) sowie ca. 5 mol-% Bis(triphenylphosphin)palladium(II)chlorid gegeben. Die Mischung wird für einen Zeitraum von 3-18 h bei Temperaturen von 70-90°C gerührt. Nach dem Abkühlen wird das Zielprodukt direkt aus der Reaktionslösung durch RP-HPLC (Eluent: Acetonitril/Wasser-Gradient) isoliert. Falls erforderlich, kann eine weitere Aufreinigung durch Chromatographie an Silicagel erfolgen (Laufmittel: Dichlormethan/Methanol- oder Cyclohexan/Ethylacetat-Gemische).

Die folgenden Beispiele werden gemäß der Allgemeinen Vorschrift A hergestellt:

| **Beispiel** | **Struktur** | **Analytische Daten** |
|---|---|---|
| 21 | 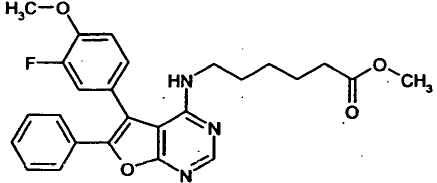 | LC-MS (Methode 5): Rₜ = 2.95 min.; m/z = 464 (M+H)⁺ |
| | | ¹H-NMR (400 MHz, DMSO-d₆): δ = 8.33 (s, 1H), 7.45-7.25 (m, 8H), 5.35 (t, 1H), 3.94 (s, 3H), 3.59 (s, 3H), 3.39 (q, 2H), 2.29 (t, 2H), 1.54-1.40 (m, 4H), 1.26-1.17 (m, 2H). |
| **22** | 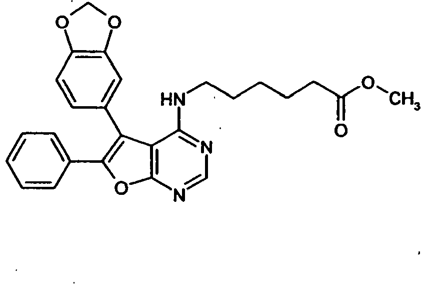 | LC-MS (Methode 5): Rₜ = 2.89 min.; m/z = 460 (M+H)⁺ |
| | | ¹H-NMR (400 MHz, DMSO-d₆): δ = 8.32 (s, 1H), 7.46 (d, 2H), 7.41-7.32 (m, 3H), 7.13-7.08 (m, 2H), 6.97 (d, 1H), 5.30 (t, 1H), 3.59 (s, 3H), 3.42-3.35 (m, 2H), 2.29 (t, 2H), 1.54-1.40 (m, 4H), 1.24-1.15 (m, 2H). |
| **23** | 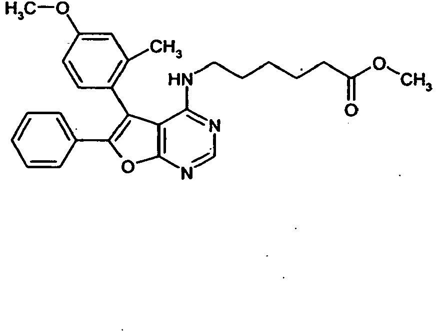 | LC-MS (Methode 5): Rₜ = 2.79 min.; m/z = 460 (M+H)⁺ |
| | | ¹H-NMR (400 MHz, DMSO-d₆): δ = 8.32 (s, 1H), 7.42-7.30 (m, 6H), 7.09 (d, 1H), 6.98 (d, 1H), 4.88 (t, 1H), 3.84 (s, 3H), 3.59 (s, 3H), 3.39-3.30 (m, 2H), 2.26 (t, 2H), 2.06 (s, 3H), 1.49-1.33 (m, 4H), 1.14-1.07 (m, 2H). |
| **24** | 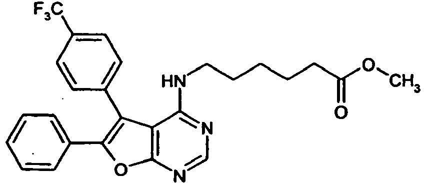 | LC-MS (Methode 2): Rₜ = 2.90 min.; m/z = 484 (M+H)⁺ |
| | | ¹H-NMR (400 MHz, DMSO-d₆): δ = 8.36 (s, 1H), 7.91 (d, 2H), 7.72 (d, 2H), 7.38 (s, 5H), 5.37 (t, 1H), 3.59 (s, 3H), 3.37 (q, 2H), 2.29 (t, 2H), 1.51-1.40 (m, 4H), 1.23-1.14 (m, 2H). |
| **25** | 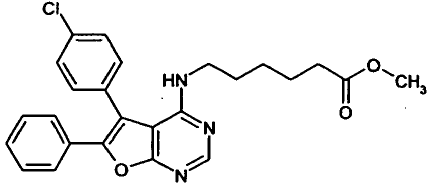 | LC-MS (Methode 5): Rₜ = 3.06 min.; m/z = 450 (M+H)⁺ |
| | | ¹H-NMR (400 MHz, DMSO-d₆): δ = 8.35 (s, 1H), 7.62 (d, 2H), 7.52 (d, 2H), 7.41-7.31 (m, 5H), 3.60 (s, 3H), 3.39 (q, 2H), 2.30 (t, 2H), 1.55-1.41 (m, 4H), 1.24-1.15 (m, 2H). |
| **26** | 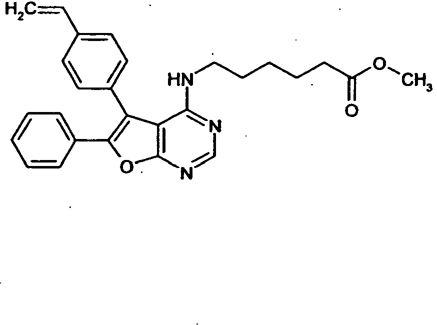 | LC-MS (Methode 2): Rₜ = 2.93 min.; m/z = 442 (M+H)⁺ |
| | | ¹H-NMR (400 MHz, DMSO-d₆): δ = 8.35 (s, 1H), 7.19 (d, 2H), 7.50 (d, 2H), 7.45-7.31 (m, 5H), 6.84 (dd, 1H), 5.99 (d, 1H), 5.39 (d, 1H), 5.15 (d, 1H), 3.58 (s, 3H), 3.37 (q, 2H), 2.25 (t, 2H), 1.49-1.39 (m, 4H), 1.19-1.10 (m, 2H). |
| **27** | 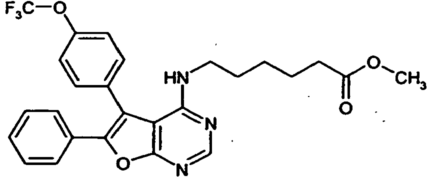 | LC-MS (Methode 2): Rₜ = 2.99 min.; m/z = 500 (M+H)⁺ |
| | | ¹H-NMR (400 MHz, DMSO-d₆): δ = 8.36 (s, 1H), 7.66 (d, 2H), 7.56 (m, 2H), 7.40-7.31 (m, 5H), 5.21 (t, 1H), 3.59 (s, 3H), 3.38 (q, 2H), 2.28 (t, 2H), 1.51-1.39 (m, 4H), 1.22-1.13 (m, 2H). |

Allgemeine Vorschrift B: Hydrolyse von Methylestern zu entsprechenden Carbonsäure-Derivaten

Zu einer Lösung des Methylesters in THF oder THF/Methanol (1:1) (Konzentration ca. 0.05 bis 0.5 mol/l) werden bei RT 1.5 bis 10 eq. Natriumhydroxid als 1 N wässrige Lösung gegeben. Die Mischung wird für einen Zeitraum von 0.5-18 h bei RT gerührt und dann mit 1 N Salzsäure neutralisiert oder schwach angesäuert. Falls dabei ein Feststoff ausfällt, kann das Produkt durch Filtration, Waschen mit Wasser und Trocknen im Hochvakuum isoliert werden, Alternativ wird die Zielverbindung direkt aus dem Rohprodukt, gegebenenfalls nach extraktiver Aufarbeitung mit Dichlormethan, durch präparative RP-HPLC isoliert (Eluent: Wasser/Acetonitril-Gradient).

Die folgenden Beispiele werden gemäß der Allgemeinen Vorschrift B hergestellt:

| **Beispiel** | **Struktur** | **Analytische Daten** |
|---|---|---|
| **28** | 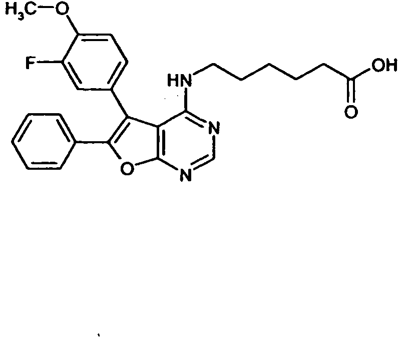 | LC-MS (Methode 4): Rₜ = 2.63 min.; m/z = 450 (M+H)⁺ |
| | | ¹H-NMR (400 MHz, DMSO-d₆): δ = 12.01 (br. s, 1H), 7.47-7.25 (m, 9H), 5.35 (br. s, 1H), 3.96 (s, 3H), 3.42-3.30 (m, 2H), 2.19 (t, 2H), 1.52-1.40 (m, 4H), 1.28-1.16 (m, 2H). |
| **29** | 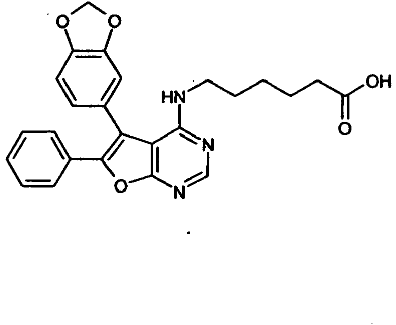 | LC-MS (Methode 5): Rₜ = 2.43 min.; m/z = 446 (M+H)⁺ |
| | | ¹H-NMR (400 MHz, DMSO-d₆): δ = 8.32 (s, 1H), 7.49 (d, 2H), 7.41-7.32 (m, 3H), 7.12 (d, 1H), 7.07 (s, 1H), 6.99 (d, 1H), 6.19 (s, 2H), 5.21 (t, 1H), 3.45-3.25 (m, 2H), 1.82 (t, 2H), 1.45-1.35 (m, 4H), 1.18-1.10 (m, 2H). |
| **30** | 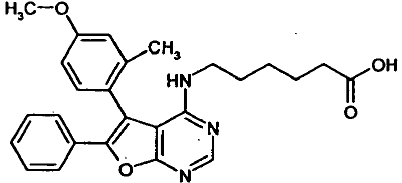 | LC-MS (Methode 4): Rₜ = 2.69 min.; m/z = 446 (M+H)⁺ |
| | | ¹H-NMR (400 MHz, DMSO-d₆): δ = 8.33 (s, 1H), 7.42-7.30 (m, 7H), 7.09 (d, 1H), 6.99 (dd, 1H), 4.82 (t, 1H), 3.86 (s, 3H), 3.40-3.38 (m, 2H), 2.08 (s, 3H), 1.76 (t, 2H), 1.38-1.26 (m, 4H), 1.10-1.01 (m, 2H). |
| **31** | 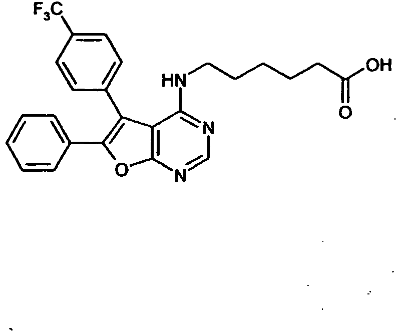 | LC-MS (Methode 5): Rₜ = 2.98 min.; m/z = 470 (M+H)⁺ |
| | | ¹H-NMR (400 MHz, DMSO-d₆): δ = 11.99 (s, 1H), 8.37 (s, 1H), 7.92 (d, 2H), 7.74 (d, 2H), 7.40-7.34 (m, 5H), 5.38 (t, 1H), 3.38 (q, 2H), 2.19 (t, 2H), 1.50-1.40 (m, 4H), 1.21-1.13 (m, 2H). |
| **32** | 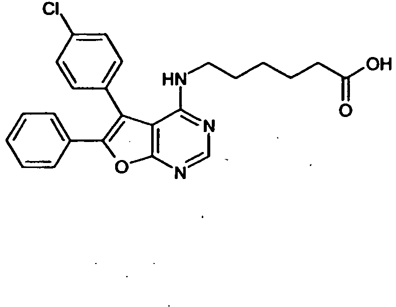 | LC-MS (Methode 4): Rₜ = 2.79 min.; m/z = 436 (M+H)⁺ |
| | | ¹H-NMR (400 MHz, DMSO-d₆): δ = 11.99 (s, 1H), 8.33 (s, 1H), 7.64 (d, 2H), 7.52 (d, 2H), 7.43-7.31 (m, 5H), 6.35 (t, 1H), 3.39 (q, 2H), 2.19 (m, 2H), 1.50-1.40 (m, 4H), 1.24-1.15 (m, 2H). |
| **33** | 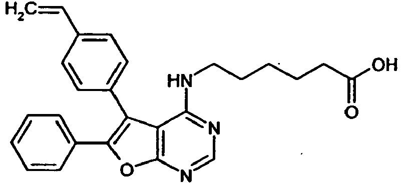 | LC-MS (Methode 4): Rₜ = 2.82 min.; m/z = 428 (M+H)⁺ |
| | | ¹H-NMR (400 MHz, DMSO-d₆): δ = 11.99 (s, 1H), 8.35 (s, 1H), 7.68 (d, 2H), 7.50 (d, 2H), 7.45 (d, 2H), 7.40-7.30 (m, 5H), 6.87 (dd, 1H), 5.99 (d, 1H), 5.39 (d, 1H), 5.15 (t, 1H), 3.42-3.30 (m, 2H), 2.16 (t, 2H), 1.48-1.12 (m, 6H). |
| **34** | 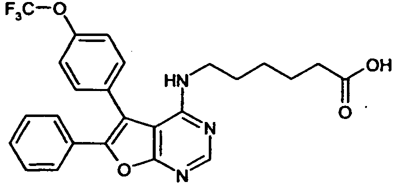 | LC-MS (Methode 5): Rₜ = 2.80 min.; m/z = 486 (M+H)⁺ |
| | | ¹H-NMR (400 MHz, DMSO-d₆): δ = 12.01 (br. s, 1H), 8.35 (s, 1H), 7.67 (br. s, 2H), 7.58 (br. s, 2H), 7.45-7.30 (m, 5H), 5.19 (br. s, 1H), 2.20 (br. s, 2H), 1.51-1.35 (m, 4H), 1.25-1.12 (m, 2H). |

Die folgenden Beispiele werden gemäß der Allgemeinen Vorschrift A (Beschreibung siehe oben) aus den entsprechenden 5-Brom-6-phenylfuro[2,3-d]pyrimidin- und Phenylboronsäure-Derivaten hergestellt:

| **Beispiel** | **Struktur** | **Analytische Daten** |
|---|---|---|
| **35** | 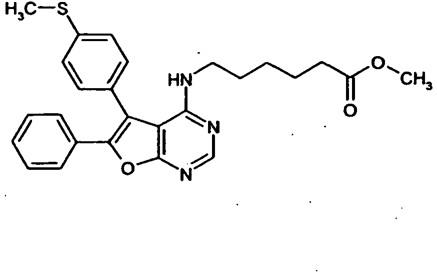 | LC-MS (Methode 5): Rₜ = 3.06 min.; m/z = 462 (M+H)⁺ |
| | | ¹H-NMR (400 MHz, DMSO-d₆): δ = 8.34 (s, 1H), 7.48-7.33 (m, 9H), 5.18 (t, 1H), 3.58 (s, 3H), 3.38 (q, 2H), 2.55 (s, ca. 3H), 2.29 (t, 2H), 1.50-1.40 (m, 4H), 1.20-1.12 (m, 2H). |
| **36** | 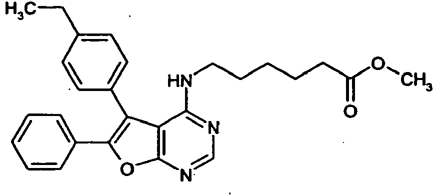 | LC-MS (Methode 8): Rₜ = 3.19 min.; m/z = 444 (M+H)⁺ |
| | | ¹H-NMR (400 MHz, DMSO-d₆): δ = 8.34 (s, 1H), 7.49-7.42 (m, 5H), 7.39-7.28 (m, 4H), 4.93 (t, 1H), 3.35 (m, 2H), 2.75 (q, 2H), 2.28 (t, 2H), 1.49-1.35 (m, 4H), 1.28 (t, 3H), 1.16-1.10 (m, 2H). |
| **37** | 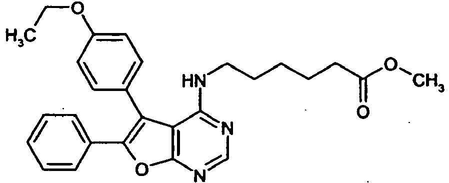 | LC-MS (Methode 8): Rₜ = 3.08 min.; m/z = 460 (M+H)⁺ |
| | | ¹H-NMR (400 MHz, DMSO-d₆): δ = 8.32 (s, 1H), 7.48-7.30 (m, 7H), 7.12 (d, 2H), 5.07 (t, 1H), 4.11 (q, 2H), 3.60 (s, 3H), 3.36 (q, 2H), 2.27 (t, 2H), 1.50-1.38 (m, 4H), 1.40 (t, 3H), 1.18-1.11 (m, 2H). |
| **38** | 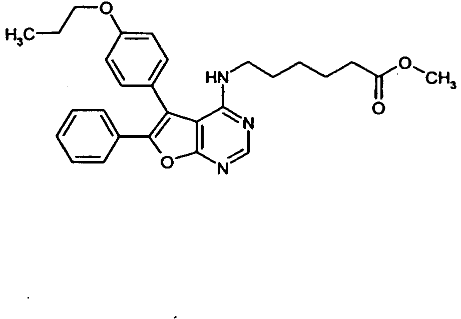 | LC-MS (Methode 8): Rₜ = 3.22 min.; m/z = 474 (M+H)⁺ |
| | | ¹H-NMR (400 MHz, DMSO-d₆): δ = 8.32 (s, 1H), 7.48-7.30 (m, 7H), 7.12 (d, 2H), 5.08 (t, 1H), 4.03 (t, 3H), 3.60 (s, 3H), 3.37 (q, 2H), 2.28 (t, 2H), 1.82-1.77 (m, 2H), 1.50-1.60 (m, 4H), 1.18-1.10 (m, 2H), 1.02 (t, 3H). |
| **39** | 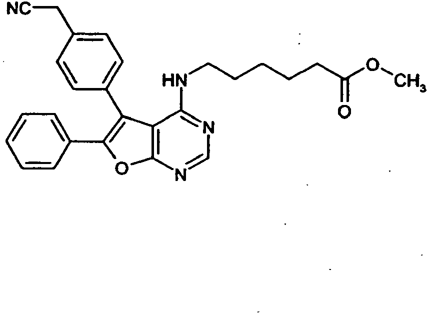 | LC-MS (Methode 8): Rₜ = 2.73 min.; m/z = 455 (M+H)⁺ |
| | | ¹H-NMR (400 MHz, CDCl₃): δ = 8.43 (s, 1H), 7.58-7.48 (m, 6H), 7.30-7.24 (m, ca. 3H), 4.55 (t, 1H), 3.91 (s, 2H), 3.68 (s, 3H), 3.45 (q, 2H), 2.30 (t, 2H), 1.65-1.45 (m, ca. 4H), 1.30-1.22 (m, 2H). |
| **40** | 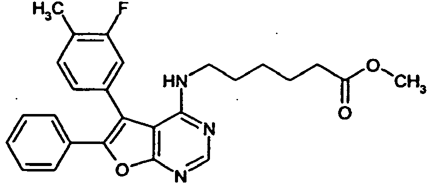 | LC-MS (Methode 2): Rₜ = 2.92 min.; m/z = 448 (M+H)⁺ |
| | | ¹H-NMR (400 MHz, CDCl₃): δ = 8.41 (s, 1H), 7.55-7.50 (m, 2H), 7.38 (t, 1H), 7.32-7.25 (m, ca. 3H), 7.16 (dd, 2H), 4.66 (t, 1H), 3.68 (s, 3H), 3.44 (q, 2H), 2.41 (s, 3H), 2.29 (t, 2H), 1.65-1.45 (m, 4H), 1.30-1.22 (m, 2H). |
| **41** | 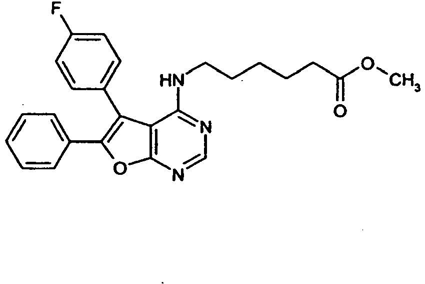 | LC-MS (Methode 2): Rₜ = 2.77 min.; m/z = 434 (M+H)⁺ |
| | | ¹H-NMR (400 MHz, CDCl₃): δ = 8.41 (s, 1H), 7.53-7.47 (m, 4H), 7.31-7.22 (m, ca. 5H), 4.54 (br. s, 1H), 3.69 (s, 3H), 3.45 (q, 2H), 2.29 (t, 2H), 1.67-1.55 (m, ca. 2H), 1.50-1.42 (m, 2H), 1.30-1.22 (m, 2H). |
| **42** | 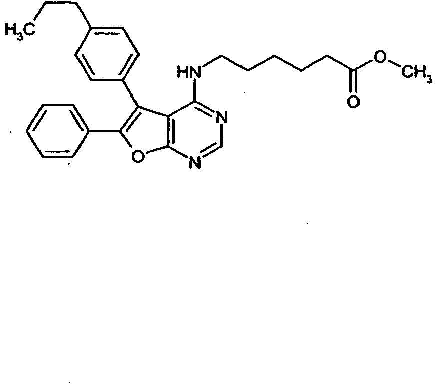 | LC-MS (Methode 8): Rₜ = 3.31 min.; m/z = 458 (M+H)⁺ |
| | | ¹H-NMR (400 MHz, DMSO-d₆): δ = 8.35 (s, 1H), 7.48-7.40 (m, 5H), 7.38-7.30 (m, 4H), 4.89 (t, 1H), 3.59 (s, 3H), 3.35 (q, 2H), 2.69 (t, 2H), 2.25 (t, 2H), 1.69 (q, 2H), 1.50-1.42 (m, 2H), 1.40-1.35 (m, 2H), 1.17-1.11 (m, 2H), 0.95 (t, 3H). |
| **43** | 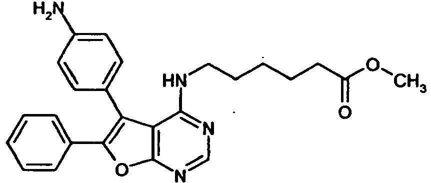 | LC-MS (Methode 5): Rₜ = 2.52 min.; m/z = 431 (M+H)⁺ |
| | | ¹H-NMR (400 MHz, DMSO-d₆): δ = 8.31 (s, 1H), 7.51 (d, 2H), 7.40-7.30 (m, 3H), 7.15 (d, 2H), 6.72 (d, 2H), 5.49 (s, 2H), 5.14 (t, 1H), 3.60 (s, 3H), 3.38 (q, 2H), 2.29 (t, 2H), 1.52-1.38 (m, 4H), 1.22-1.17 (m, 2H). |
| **44** | 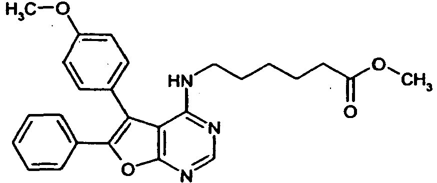 | LC-MS (Methode 5): Rₜ = 2.93 min.; m/z = 446 (M+H)⁺ |
| | | ¹H-NMR (500 MHz, CDCl₃): δ = 8.40 (s, 1H), 7.55 (d, 2H), 7.40 (d, 2H), 7.30-7.23 (m, ca. 3H), 7.07 (d, 2H), 4.68 (br. s, 1H), 3.93 (s, 3H), 3.69 (s, 3H), 3.43 (q, 2H), 2.29 (t, 2H), 1.62-1.55 (m, 2H), 1.50-1.42 (m, 2H), 1.27-1.21 (m, 2H). |

### Beispiel 45

### 6-{[5-(4-Methoxyphenyl)-6-phenylfuro[2,3-d]pyrimidin-4-yl](methyl)amino}hexansäuremethylester

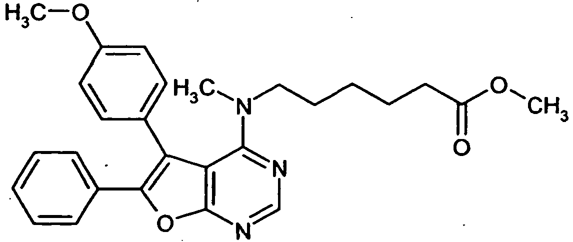

200 mg (0.463 mmol) [(5-Brom-6-phenylfuro[2,3-d]pyrimidin-4-yl)(methyl)amino]hexansäuremethylester werden in 1.25 ml DMSO und 0.125 ml Methanol gelöst. Unter Argon werden bei RT nacheinander 16.2 mg (0.023 mmol) Bis(triphenylphosphin)palladium(II)chlorid, 127 mg (0.925 mmol) Kaliumcarbonat und 105.4 mg (0.694 mmol) 4-Methoxyphenylboronsäure zugegeben. Die Mischung wird unter kräftigem Rühren für ca. 3 h auf 80°C erhitzt. Nach dem Abkühlen wird die Reaktionsmischung direkt durch präparative RP-HPLC (Gradient: Acetonitril/Wasser) gereinigt. Es werden 133.9 mg (63% d. Th.) des Zielprodukts erhalten.
LC-MS (Methode 2): Rₜ = 2.85 min.; m/z = 460 (M+H)⁺
¹H-NMR (400 MHz, DMSO-d₆): δ = 8.33 (s, 1H), 7.40-7.30 (m, 7H), 7.07 (d, 2H), 3.83 (s, 3H), 3.58 (s, 3H), 3.30 (m, ca. 2H), 2.58 (s, 3H), 2.25 (t, 2H), 1.50-1.33 (m, 4H), 1.12-1.03 (m, 2H).

### Beispiel 46

### 6-({5-[4-(Ethylamino)phenyl]-6-phenylfuro[2,3-d]pyrimidin-4-yl}amino)hexansäuremethylester

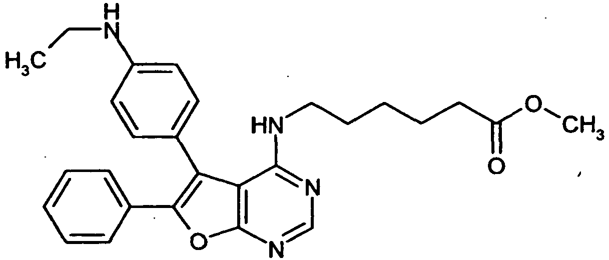

29.2 mg (0.068 mmol) 6-({5-[4-Aminophenyl]-6-phenylfuro[2,3-d]pyrimidin-4-yl}amino)hexansäuremethylester werden in 0.5 ml Acetonitril mit 14 µl (0.102 mmol) Triethylamin vorgelegt und bei 45°C in einer geschlossenen Apparatur über einen Zeitraum von 12 h portionsweise mit einem Überschuss Iodethan versetzt. Nach dem Abkühlen wird die Reaktionsmischung mit Dichlormethan verdünnt, nacheinander mit ges. Natriumhydrogencarbonat-Lösung und ges. Natriumchlorid-Lösung gewaschen, über Natriumsulfat getrocknet und im Vakuum eingeengt. Aus dem Rohproduktgemisch werden nach präparativer RP-HPLC (Gradient: Acetonitril/Wasser) 6.2 mg der Zielverbindung isoliert (19.9% d. Th.).
LC-MS (Methode 5): Rₜ = 2.96 min.; m/z = 459 (M+H)⁺
¹H-NMR (400 MHz, CDCl₃): δ = 8.39 (s, 1H), 7.61 (d, 2H), 7.30-7.21 (m, ca. 5H), 6.73 (d, 2H), 4.83 (t, 1H), 3.88 (br. s, 1H) 3.68 (s, 3H), 3.43 (q, 2H), 3.25 (q, 2H), 2.29 (t, 2H), 1.66-1.60 (m, 2H), 1.52-1.45 (m, 2H), 1.35 (t, 3H), 1.29-1.21 (m, 2H).

### Beispiel 47

### (+)-{[(2S)-3-{[5-(4-Ethylphenyl)-6-phenylfuro[2,3-d]pyrimidin-4-yl]oxy}-2-methylpropyl]oxy}-essigsäure-tert.-butylester

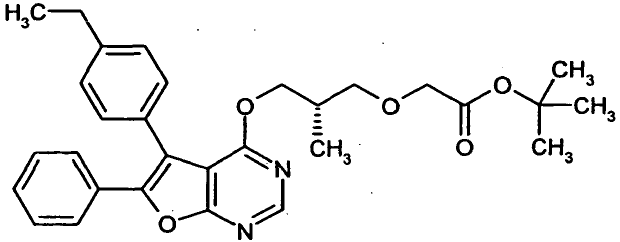

85 mg (0.416 mmol) (+)-{[(2*R*)-3-Hydroxy-2-methylpropyl]oxy}essigsäure-*tert*.-butylester werden in 2 ml abs. THF gelöst, auf 0°C gekühlt und mit 0.21 ml (0.416 mmol) Phosphazen-Base P2-t-Bu (2 M Lösung in THF) versetzt. Nach 10 min bei 0°C werden 126.7 mg (0.378 mmol) 4-Chlor-5-(4-ethylphenyl)-6-phenylfuro[2,3-d]pyrimidin zugegeben, und die Mischung wird 10 min bei 0°C und weitere 2 h bei RT gerührt. Dann wird Wasser zugegeben, mit 1 N Salzsäure auf ca. pH 7- eingestellt und mit Dichlormethan extrahiert. Die organische Phase wird mit ges. Natriumchlorid-Lösung gewaschen, über Natriumsulfat getrocknet und im Vakuum eingeengt. Aus dem Rohprodukt werden durch Chromatographie an Silicagel (Eluent: Cyclohexan/Ethylacetat 1:1) 125.1 mg des Zielprodukts isoliert (65.8% d. Th.).
LC-MS (Methode 9): Rₜ = 4.88 min.; m/z = 503 (M+H)⁺
¹H-NMR (400 MHz, DMSO-d₆): δ = 8.59 (s, 1H), 7.54 (d, 2H), 7.43-7.38 (m, 5H), 7.32 (d, 2H), 4.30 (m, 2H), 3.84 (s, 2H), 3.13 (d, 2H), 2.69 (q, 2H), 1.98 (m, 1H), 1.40 (s, 9H), 1.24 (t, 3H), 0.72 (d, 3H).
[α]_{D}²⁰ = +11°, c = 0.255, Chloroform.

### Beispiel 48

### (-)-{[(2R)-3-{[5-(4-Ethylphenyl)-6-phenylfuro[2,3-d]pyrimidin-4-yl]oxy}-2-methylpropyl]oxy}-essigsäureethylester

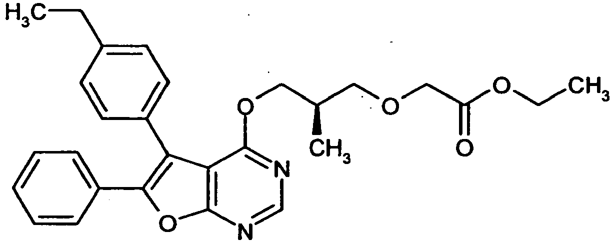

Eine Lösung von 670.3 mg (2.0 mmol) 4-Chlor-5-(4-ethylphenyl)-6-phenylfuro[2,3-d]pyrimidin und 441 mg (2.5 mmol) (-)-{[(2*S*)-3-Hydroxy-2-methylpropyl]oxy}essigsäureethylester in 5.5 ml abs. THF wird auf 0°C gekühlt und mit 2.4 ml (2.4 mmol) Phosphazen-Base P4-t-Bu (1 M Lösung in Hexan) versetzt. Nach Ende der Zugabe wird auf RT erwärmt und 2 h bei RT gerührt, bevor Wasser zur Reaktionsmischung gegeben und mit 1 N Salzsäure neutralisiert wird. Man extrahiert mit Dichlormethan, trocknet die organische Phase über Natriumsulfat und konzentriert im Vakuum. Aus dem Rohprodukt werden nach präparativer RP-HPLC (Gradient: Wasser/Acetonitril) 107.6 mg des Zielprodukts erhalten (65.8% d. Th.).
LC-MS (Methode 8): Rₜ = 3.36 min.; m/z = 475 (M+H)⁺
¹H-NMR (400 MHz, DMSO-d₆): δ = 8.59 (s, 1H), 7.54 (d, 2H), 7.43-7.37 (m, 5H), 7.31 (d, 2H), 4.35-4.27 (m, 2H), 4.09 (q, 2H), 3.97 (s, 2H), 3.19-3.11 (m, 2H), 2.68 (q, 2H), 1.98 (m, 1H), 1.22 (t, 3H), 1.17 (t, 3H), 0.71 (d, 3H).
[α]_{D}²⁰ = -17.1°, c = 0.52, Chloroform.

Die folgenden Beispiele werden gemäß der Allgemeinen Vorschrift B (Hydrolyse von Methyl-oder analog Ethylestern) aus den entsprechenden Carbonsäureestern hergestellt:

| **Beispiel** | **Struktur** | **Analytische Daten** |
|---|---|---|
| **49** | 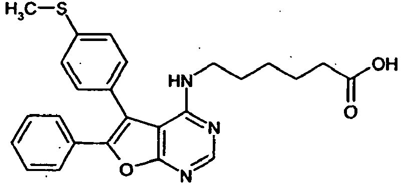 | LC-MS (Methode 8): Rₜ = 2.70 min.; m/z = 448 (M+H)⁺ |
| **50** | 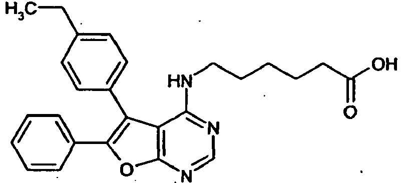 | LC-MS (Methode 2): Rₜ = 2.61 min.; m/z = 430 (M+H)⁺ |
| | | ¹H-NMR (400 MHz, DMSO-d₆): δ = 8.33 (s, 1H), 7.49-7.42 (m, 6H), 7.40-7.30 (m, 3H), 4.89 (br. t, 1H), 3.35 (q, ca. 2H), 2.75 (q, 2H), 1.84 (t, 2H), 1.39-1.30 (in, 4H), 1.28 (t, 3H), 1.11-1.05 (m, 2H). |
| **51** | 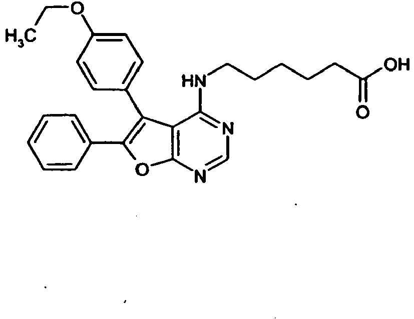 | LC-MS (Methode 5): Rₜ = 2.66 min.; m/z = 446 (M+H)⁺ |
| | | ¹H-NMR (400 MHz, CDCl₃): δ = 8.30 (s, 1H), 7.46-7.41 (m, 2H), 7.29 (d, 2H), 7.20-7.15 (m, 2H), 6.98 (d, 2H), 4.71 (br. t, 1H), 4.04 (q, 2H), 3.35-3.25 (m, 2H), 2.14-2.07 (m, 2H), 1.52-1.43 (m, 2H), 1.40 (t, 3H), 1.40-1.32 (m, 2H), 1.20-1.10 (m, 2H). |
| **52** | 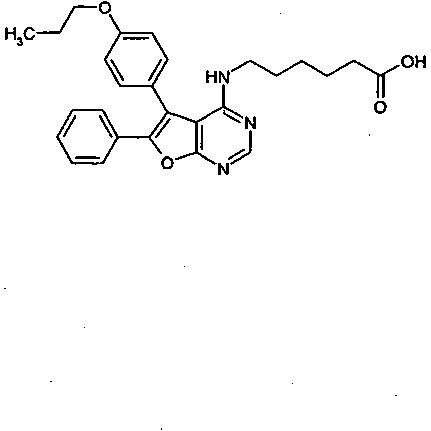 | LC-MS (Methode 8): R, = 2.86 min.; m/z = 460 (M+H)⁺ |
| | | ¹H-NMR (400 MHz, CDCl₃): δ = 8.38 (s, 1H), 7.55 (d, 2H), 7.38 (d, 2H), 7.30-7.23 (m, ca. 3H), 7.08 (d, 2H), 4.74 (t, 1H), 4.03 (t, 2H), 3.40 (q, 2H), 2.30-2.23 (m, 2H), 1.87 (q, 2H), 1.65-1.57 (m, 2H), 1.48-1.41 (m, 2H), 1.30-1.22 (m, ca. 2H), 1.10 (t, 3H). |
| **53** | 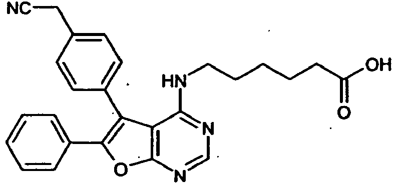 | LC-MS (Methode 8): Rₜ = 2.42 min.; m/z = 441 (M+H)⁺ |
| | | ¹H-NMR (400 MHz, CDCl₃): δ = 8.30 (s, 1H), 7.55-7.40 (m, 7H), 7.27-7.19 (m, 3H), 4.55 (br. t, 1H), 3.98 (s, ca. 2H), 3.37-3.29 (m, 2H), 2.15-2.09 (m, 2H), 1.52-1.42 (m, 2H), 1.40-1.30 (m, 2H), 1.18-1.09 (m, 2H)., |
| **54** | 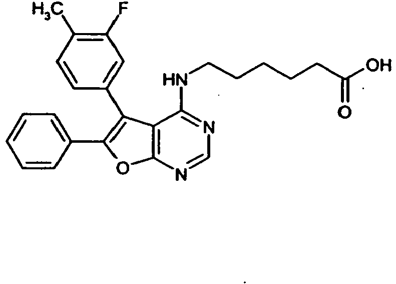 | LC-MS (Methode 2): Rₜ = 2.50 min.; m/z = 434 (M+H)⁺ |
| | | ¹H-NMR (400 MHz, CDCl₃): δ = 8.33 (s, 1H), 7.53-7.22 (m, ca. 6H), 7.15-7.05 (m, 2H), 4.66 (t, 1H), 3.41-3.35 (m, 2H), 2.37 (s, 3H), 2.25-2.15 (m, 2H), 1.58-1.49 (m, 2H), 1.47-1.38 (m, 2H), 1.27-1.18 (m, 2H). |
| **55** | 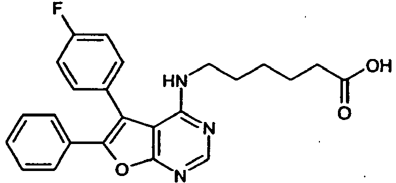 | LC-MS (Methode 5): Rₜ = 2.55 min.; m/z = 420 (M+H)⁺ |
| **56** | 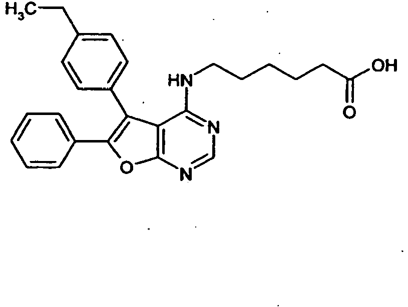 | LC-MS (Methode 2): Rₜ = 2.61 min.; m/z = 430 (M+H)⁺ |
| | | ¹H-NMR (400 MHz, DMSO-d₆): δ = 8.34 (s, 1H), 7.49-7.42 (m, 6H), 7.39-7.30 (m, 3H), 4.39 (t, 1H), 3.33 (q, 2H), 2.73 (q, 2H), 1.82 (t, 2H), 1.39-1.30 (m, 4H), 1.28 (t, 3H), 1.10-1.03 (m, 2H). |
| **57** | 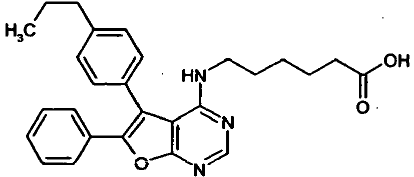 | LC-MS (Methode 8): Rₜ = 3.07 min.; m/z = 444 (M+H)⁺ |
| | | ¹H-NMR (400 MHz, DMSO-d₆): δ = 8.33 (s, 1H), 7.50-7.40 (m, 6H), 7.38-7.30 (m, 3H), 4.85 (t, 1H), 3.35 (q, 2H), 2.70 (t, 2H), 1.82 (t, 2H), 1.72-1.65 (m, 2H), 1.40-1.30 (m, 4H), 1.13-1.04 (m, 2H), 0.93 (t, 3H). |
| **58** | 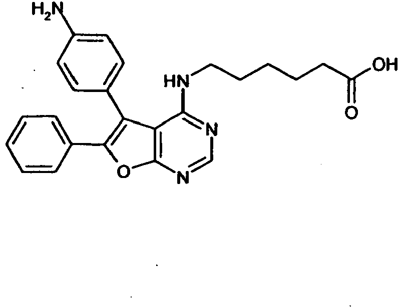 | LC-MS (Methode 2): Rₜ = 2.03 min.; m/z = 416 (M+H)⁺ |
| | | ¹H-NMR (400 MHz, DMSO-d₆): 8 = 8.30 (s, 1H), 7.56 (d, 2H), 7.40-7.28 (m, 3H), 7.14 (d, 2H), 6.78 (d, 2H), 5.60 (br. s, 2H), 5.07 (t, 1H), 3.34 (q, ca. 2H), 1.81 (t, 2H), 1.41-1.32 (m, 4H), 1.16-1.10 (m, 2H). |
| **59** | 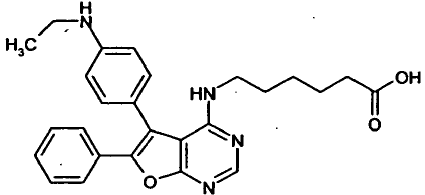 | LC-MS (Methode 5): Rₜ = 2.55 min.; m/z = 445 (M+H)⁺ |
| **60** | 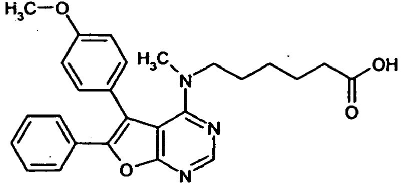 | LC-MS (Methode 2): Rₜ = 2.45 min.; m/z = 446 (M+H)⁺ |
| | | ¹H-NMR (400 MHz, DMSO-d₆): δ = 8.34 (s, 1H), 7.40-7.28 (m, ca. 7H), 7.11-7.06 (m, 2H), 3.83 (s, 3H), 3.27 (m, ca. 2H), 2.58 (s, ca. 3H), 1.89-1.80 (m, 2H), 1.40-1.30 (m, 4H), 1.08-0.98 (m, 2H). |

### Beispiel 61

### (+)-{[(2S)-3-{[5-(4-Ethylphenyl)-6-phenylfuro[2,3-d]pyrimidin-4-yl]oxy}-2-methylpropyl]oxy}-essigsäure

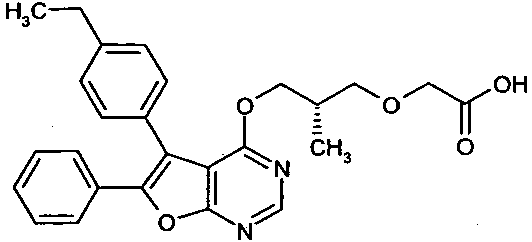

120 mg (0.239 mmol) {[(2*S*)-3-{[5-(4-Ethylphenyl)-6-phenylfuro[2,3-d]pyrimidin-4-yl]oxy}-2-methylpropyl]oxy}essigsäure-*tert*.-butylester werden in 2.0 ml Dichlormethan bei RT mit zunächst 0.46 ml TFA behandelt. Die Mischung wird bei RT ca. 4 h lang gerührt und dabei portionsweise mit mehr TFA versetzt. Die Reaktionsmischung wird danach im Vakuum eingeengt und das Zielprodukt nach präparativer RP-HPLC (Gradient: Acetonitril/Wasser) isoliert. Es werden 106.8 mg (96.1 % d. Th.) erhalten.
LC-MS (Methode 8): Rₜ = 2.98 min.; m/z = 447 (M+H)⁺
¹H-NMR (400 MHz, DMSO-d₆): δ = 8.58 (s, 1H), 7.55 (d, 2H), 7.42-7.37 (m, 5H), 7.30 (d, 2H), 4.30 (m, ca. 2H), 3.89 (s, 2H), 3.20-3.10 (m, 2H), 2.69 (q, 2H), 1.95 (m, 1H), 1.24 (t, 3H), 0.71 (d, 3H).
[α]_{D}²⁰ = +12.8°, c = 0.41, Chloroform.

### Beispiel 62

### (-)-{[(2R)-3-{[5-(4-Ethylphenyl)-6-phenylfuro[2,3-d]pyrimidin-4-yl]oxy}-2-methylpropyl]oxy}-essigsäure

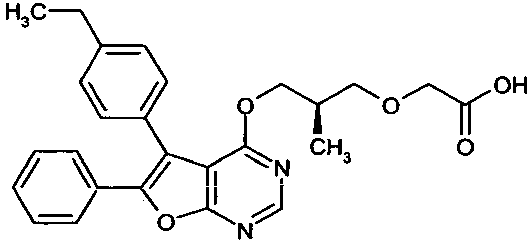

85.3 mg (0.18 mmol) (-)-{[(2*R*)-3-{[5-(4-Ethylphenyl)-6-phenylfuro[2,3-d]pyrimidin-4-yl]oxy}-2-methylpropyl]oxy}essigsäureethylester werden in 1 ml THF gelöst und mit 0.9 ml 1 N Natronlauge versetzt. Die Mischung wird 3.5 h bei RT gerührt, bevor mit 1 N Salzsäure neutralisiert und mit Dichlormethan extrahiert wird. Die organische Phase wird im Vakuum eingeengt, und aus dem Rückstand werden nach präparativer RP-HPLC (Gradient: Acetonitril/Wasser) 18.3 mg des Zielprodukts isoliert (22.8% d. Th.).
LC-MS (Methode 9): Rₜ = 4.02 min.; m/z = 447 (M+H)⁺
¹H-NMR (400 MHz, DMSO-d₆): δ = 8.59 (s, 1H), 7.54 (d, 2H), 7.42-7.36 (m, 5H), 7.32 (d, 2H), 4.30 (m, 2H), 3.85 (s, 2H), 3.20-3.10 (m, 2H), 2.69 (q, 2H), 1.98 (m, 1H), 1.21 (t, 3H), 0.70 (d, 3H).
[α]_{D}²⁰ = -18.5°, c = 0.585, Chloroform.

### Beispiel 63

### (+/-)-6-{[5-(4-Methoxyphenyl)-6-phenylfuro[2,3-d]pyrimidin-4-yl]oxy}heptansäuremethylester

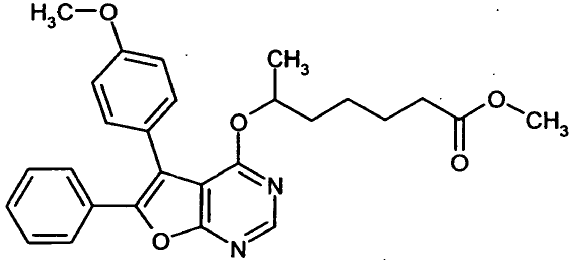

1.902 g (11.9 mmol) (+/-)-6-Hydroxyheptansäuremethylester werden unter Argon in 20 ml THF vorgelegt und auf 0°C abgekühlt. Man gibt 6 ml (11.9 mmol) einer 2 M Lösung der Phosphazen-base P2-*tert*.-Butyl in THF hinzu und rührt 10 min bei RT nach. Dann wird wieder auf 0°C abgekühlt. Man gibt 2.00 g (5.94 mmol) 4-Chlor-5-(4-methoxyphenyl)-6-phenylfuro[2,3-d]pyrimidin zu und rührt anschließend 1 h bei RT nach. Man verdünnt mit Wasser, stellt mit 10%-iger wässriger Zitronensäure-Lösung sauer und extrahiert zweimal mit Ethylacetat. Die Ethylacetat-Phasen werden vereinigt und einmal mit ges. Natriumchlorid-Lösung gewaschen. Man trocknet über Magnesiumsulfat, engt ein und reinigt den Rückstand durch Säulenchromatographie an Silicagel (Laufmittel: Cyclohexan/Ethylacetat 9:1). Man erhält 1.38 g (78.0% d. Th.) der Zielverbindung.
LC-MS (Methode 2): Rₜ = 3.12 min.; m/z = 461 (M+H)⁺
¹H-NMR (400 MHz, DMSO-d₆): δ = 8.50 (s, 1H), 7.62 (m, 2H), 7.36 (d, 2H), 7.30 (m, 3H), 6.94 (d, 2H), 5.35-5.26 (m, 1H), 3.88 (s, 3H), 3.64 (s, 3H), 2.22 (m, 2H), 1.61-1.47 (m, 4H), 1.28 (d, 3H), 1.28-1.15 (m, 2H).

### Beispiel 64

### (+/-)-6-{[5-(4-Methoxyphenyl)-6-phenylfuro[2,3-d]pyrimidin-4-yl]oxy}heptansäure-tert.-butylester

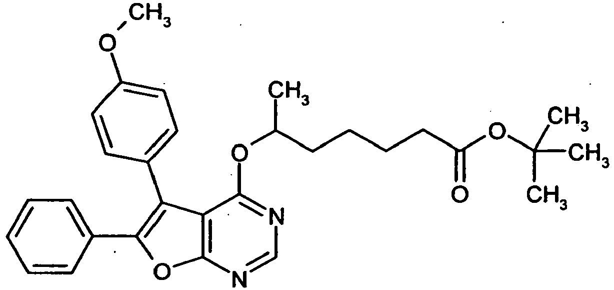

9.010 g (44.54 mmol) (+/-)-6-Hydroxyheptansäure-*tert*.-butylester werden unter Argon in 100 ml TFA vorgelegt und auf 0°C abgekühlt. Man gibt 15.117 g (44.54 mmol) Phosphazenbase P2-Ethyl hinzu und rührt 10 min bei RT nach. Dann wird wieder auf 0°C abgekühlt. Man fügt 10.00 g (29.69 mmol) 4-Chlor-5-(4-methoxyphenyl)-6-phenylfuro[2,3-d]pyrimidin hinzu und rührt anschließend über Nacht bei RT. Man verdünnt danach mit Wasser, stellt mit 10%-iger Zitronensäure-Lösung sauer und extrahiert zweimal mit Ethylacetat. Die Ethylacetat-Phasen werden vereinigt und einmal mit ges. Natriumchlorid-Lösung gewaschen. Man trocknet über Magnesiumsulfat, engt ein und reinigt den Rückstand durch Chromatographie an Silicagel (Laufmittel: Cyclohexan/Ethylacetat 9:1). Man erhält 11.4 g (76.4% d. Th.) der Zielverbindung.
LC-MS (Methode 8): Rₜ = 3.53 min.; m/z = 503 (M+H)⁺
¹H-NMR (400 MHz, DMSO-d₆): δ = 8.49 (s, 1H), 7.62 (m, 2H), 7.36 (d, 2H), 7.30 (m, 3H), 6.94 (d, 2H), 5.36-5.27 (m, 1H), 3.89 (s, 3H), 2.12 (t, 2H), 1.64-1.45 (m, 4H), 1.42 (s, 9H), 1.28 (d, 3H), 1.28-1.12 (m, 2H).

### Trennung der Enantiomere:

Das oben erhaltene Racemat wird durch präparative HPLC an chiraler Phase in die Enantiomere getrennt [Säule: Daicel Chiralpak AD-H, 250 mm x 20 mm; Fluss: 25 ml/min; Detektion: 260 nm; Injektionsvolumen: 1500 µl; Temperatur: 24°C; Eluent: 98% iso-Hexan / 2% 2-Propanol]. Aus 11.4 g des Racemats erhält man so 3.9 g (7.76 mmol) (+)-6-{[5-(4-Methoxyphenyl)-6-phenylfuro-[2,3-d]pyrimidin-4-yl]oxy}heptansäure-*tert*.-butylester *(Beispiel 65*) und 4.8 g (9.45 mmol) (-)-6-{[5-(4-Methoxyphenyl)-6-phenylfuro[2,3-d]pyrimidin-4-yl]oxy}heptansäure-*tert*.-butylester (*Beispiel 66*).

### Beispiel 65

### (+)-6-{[5-(4-Methoxyphenyl)-6-phenylfuro[2,3-d]pyrimidin-4-yl]oxy}heptansäure-tert.-butylester (Enantiomer 1)

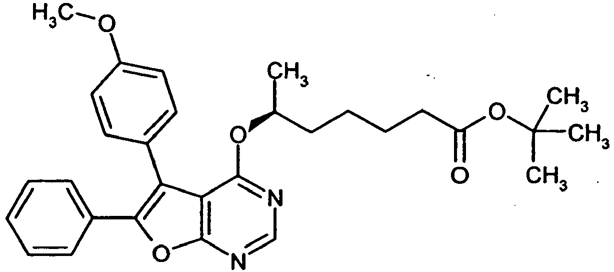

HPLC: Rₜ = 11.76 min; ee >99.5% [Säule: Daicel AD-H, 250 mm x 4 mm; Eluent: Isopropanol/Isohexan 3:97; Fluss: 1 ml/min; UV-Detektion: 250 nm].

### Beispiel 66

### (-)-6-{[5-(4-Methoxyphenyl)-6-phenylfuro[2,3-d]pyrimidin-4-yl]oxy}heptansäure-tert.-butylester (Enantiomer 2)

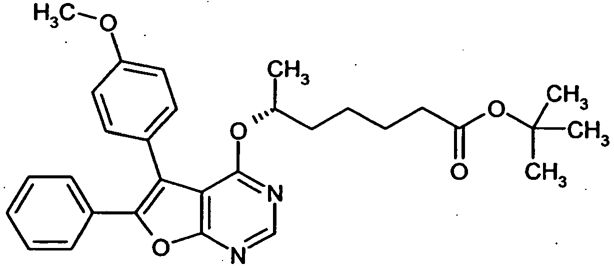

HPLC: Rₜ = 14.00 min; ee > 98.9% [Säule: Daicel AD-H, 250 mm x 4 mm; Eluent: Isopropanol/Isohexan 3:97; Fluss: 1 ml/min; UV-Detektion: 250 nm].

Alternativ kann (-)-6-{[5-(4-Methoxyphenyl)-6-phenylfuro[2,3-d]pyrimidin-4-yl]oxy}heptansäure-*tert*.-butylester durch Reaktion von 4-Chlor-5-(4-methoxyphenyl)-6-phenylfuro[2,3-d]pyrimidin mit (-)-6-Hydroxyheptansäure-*tert*.-butylester hergestellt werden:
Eine Lösung von 5.50 g (27.19 mmol) (-)-6-Hydroxyheptansäure-*tert*.-butylester in 10 ml DMF wird unter Argon auf 0°C abgekühlt und unter Eiskühlung mit 1.054 g (26.36 mmol, 60%-ig) Natriumhydrid versetzt. Die Mischung wird ca. 20 min zwischen 0°C und RT gerührt. Danach werden nach erneutem Abkühlen auf 0°C 5.549 g (16.47 mmol) 4-Chlor-5-(4-methoxyphenyl)-6-phenylfuro[2,3-d]pyrimidin zugesetzt. Nach Ende der Zugabe wird die Mischung langsam auf RT erwärmt und über Nacht bei RT gerührt, bevor Wasser zugegeben und mit 1 N Salzsäure neutral gestellt wird. Die Mischung wird mit Dichlormethan extrahiert. Die organische Phase wird mit ges. Natriumchlorid-Lösung gewaschen, über Natriumsulfat getrocknet und im Vakuum eingeengt. Der Rückstand wird durch Chromatographie an Silicagel gereinigt (Laufmittel: Cyclohexan/Ethylacetat 10:1). Erhalten werden 4.68 g der Zielverbindung (56.5% d. Th.).
   LC-MS (Methode 2): Rₜ = 3.33 min.; m/z = 503 (M+H)⁺
   ¹H-NMR (400 MHz, DMSO-d₆): δ = 8.56 (s, 1H), 7.53 (d, 2H), 7.43-7.35 (m, 5H), 7.02 (d, 2H), 5.29 (m, 1H), 3.83 (s, 3H), 2.10 (t, 2H), 1.54-1.36 (m, 4H), 1.36 (s, 9H), 1.22 (d, 3H), 1.21-1.09 (m, 2H).
   [α]_{D}²⁰ = -61.4°, c = 0.55, Chloroform.

### Beispiel 67

### (+/-)-6-{[5-(4-Methoxyphenyl)-6-phenylfuro[2,3-d]pyrimidin-4-yl]oxy}heptansäure

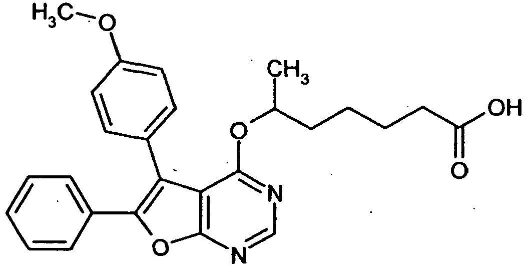

### Methode 1:

Darstellung ausgehend von (+/-)-6-{[5-(4-Methoxyphenyl)-6-phenylfuro[2,3-d]pyrimidin-4-yl]-oxy} heptansäuremethylester:
1.38 g (3.0 mmol) (+/-)-6-{[5-(4-Methoxyphenyl)-6-phenylfuro[2,3-d]pyrimidin-4-yl]oxy}heptansäuremethylester werden in 40 ml THF vorgelegt. Man gibt 30 ml 1 N Natronlauge hinzu und rührt über Nacht bei RT. Man fügt dann ca. 40 ml 1 M Salzsäure bis zu einem pH von ca. 2 zu, verdünnt mit etwas Wasser und extrahiert zweimal mit Ethylacetat. Die Ethylacetat-Phasen werden vereinigt und einmal mit ges. Natriumchlorid-Lösung gewaschen. Man trocknet über Magnesiumsulfat und engt ein. Man erhält 1.34 g (78.0% d. Th.) der Zielverbindung.

### Methode 2:

Darstellung ausgehend von (+/-)-6-{[5-(4-Methoxyphenyl)-6-phenylfuro[2,3-d]pyrimidin-4-yl]-oxy}heptansäure-*tert*.-butylester:
800 mg (1.59 mmol) (+/-)-6-{[5-(4-Methoxyphenyl)-6-phenylfuro[2,3-d]pyrimidin-4-yl]oxy}-heptansäure-*tert*.-butylester werden in 10 ml Dichlormethan gelöst. Man gibt 2.5 ml Trifluoressigsäure hinzu und rührt 2 h bei RT. Man engt danach ein, versetzt den Rückstand mit Petrolether und läßt das Produkt kristallisieren. Man gibt dann noch etwas tert.-Butylmethylether hinzu, verrührt einige Minuten und saugt dann über eine Fritte ab. Man erhält 640 mg (89.9% d. Th.) der Zielverbindung.
   LC-MS (Methode 2): Rₜ = 2.71 min.; m/z = 447 (M+H)⁺
   ¹H-NMR (400 MHz, DMSO-d₆): δ = 8.55 (s, 1H), 7.55 (d, 2H), 7.45-7.35 (m, 5H), 7.02 (d, 2H), 5.31-5.25 (m, 1H), 3.81 (s, 3H), 2.21 (m, 2H), 1.55-1.35 (m, 4H), 1.22 (d, 3H), 1.22-1.05 (m, 2H).

### Trennung der Enantiomere:

1.330 g (+/-)-6-{[5-(4-Methoxyphenyl)-6-phenylfuro[2,3-d]pyrimidin-4-yl]oxy}heptansäure werden in 240 ml Ethanol warm gelöst. Das Racemat wird durch präparative HPLC an chiraler Phase in die Enantiomere getrennt [Säule: Daicel Chiralpak AS-H, 5 µm, 250 mm x 20 mm; Fluss: 15 ml/min; Detektion: 220 nm; Injektionsvolumen: 1000 µl; Temperatur: 30°C; Eluent: 50% iso-Hexan / 50% Ethanol + 0.2% Eisessig + 1% Wasser] (siehe Beispiel 68 und 69).

### Beispiel 68

### (+)-6-{[5-(4-Methoxyphenyl)-6-phenylfuro[2,3-d]pyrimidin-4-yl]oxy}heptansäure (Enantiomer 1)

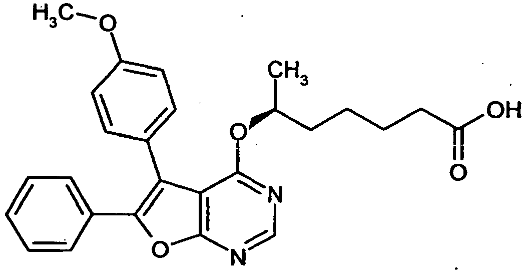

[α]_{D}²⁰ = +86.2°, c = 0.630, Methanol.
LC-MS (Methode 2): Rₜ = 2.68 min.; m/z = 447 (M+H)⁺.

### Beispiel 69

### (-)-6-{[5-(4-Methoxyphenyl)-6-phenylfuro[2,3-d]pyrimidin-4-yl]oxy}heptansäure(Enantiomer 2)

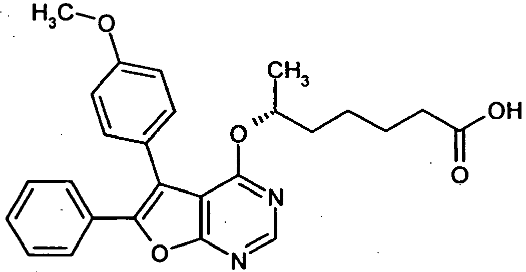

[α]_{D}²⁰ = -79.5°, c = 0.520, Methanol.
LC-MS (Methode 8): Rₜ = 2.93 min.; m/z = 447 (M+H)⁺.

Alternativ lässt sich (-)-6-{[5-(4-Methoxyphenyl)-6-phenylfuro[2,3-d]pyrimidin-4-yl]oxy}heptansäure durch Esterspaltung von (-)-6-{[5-(4-Methoxyphenyl)-6-phenylfuro[2,3-d]pyrimidin-4-yl]-oxy}heptansäure-*tert*.-butylester herstellen:
9.24 g (18.38 mmol) (-)-6-{[5-(4-Methoxyphenyl)-6-phenylfuro[2,3-d]pyrimidin-4-yl]oxy}heptansäure-*tert*.-butylester werden bei RT in 100 ml Dichlormethan gelöst und mit 25 ml TFA versetzt. Nach 2 h bei RT wird die Mischung mit Dichlormethan verdünnt, mehrmals mit Wasser und einmal mit ges. Natriumchlorid-Lösung gewaschen, über Natriumsulfat getrocknet und im Vakuum eingeengt. Der Rückstand wird durch Chromatographie an Silicagel gereinigt (Laufmittel: Cyclohexan/Ethylacetat 5:1 → 2:1, dann Cyclohexan/Ethylacetat 2:1 + 0.5% Essigsäure). Produkthaltige Fraktionen werden im Vakuum eingeengt, der Rückstand wird erneut in Dichlormethan aufgenommen und mehrmals mit Wasser und ges. Natriumchlorid-Lösung gewaschen. Die organische Phase wird über Natriumsulfat getrocknet, eingeengt und im Hochvakuum getrocknet. Erhalten werden 6.89 g der Zielverbindung (83.9% d. Th.).
   LC-MS (Methode 7): Rₜ = 4.10 min.; m/z = 447 (M+H)⁺
   ¹H-NMR (400 MHz, DMSO-d₆): δ = 11.98 (br. s, 1H), 8.57 (s, 1H), 7.55 (d, 2H), 7.45-7.36 (m, 5H), 7.02 (d, 2H), 5.29 (m, 1H), 3.72 (s, 3H), 2.12 (t, 2H), 1.54-1.37 (m, 4H), 1.22 (d, 3H), 1.21-1.08 (m, 2H).
   [α]_{D}²⁰= -70.8°, c = 0.685, Chloroform.

### Beispiel 70

### (-)-6-{[5-(4-Methoxyphenyl)-6-phenylfuro[2,3-d]pyrimidin-4-yl]oxy}heptansäure-Natriumsalz

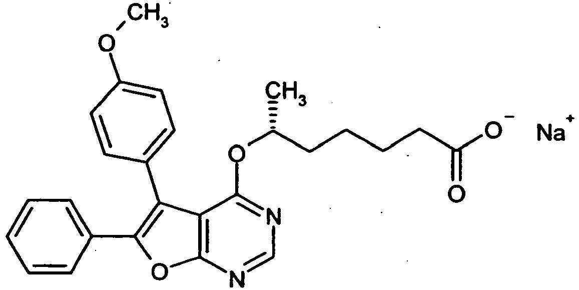

893 mg (2.0 mmol) (-)-6-{[5-(4-Methoxyphenyl)-6-phenylfuro[2,3-d]pyrimidin-4-yl]oxy}heptansäure werden mit 5.0 ml demineralisiertem Wasser (Millipore-Ionenaustauscher) versetzt. Man gibt 2.0 ml (2.0 mmol) 1 N Natronlauge hinzu und rührt 30 min bei RT. Anschließend wird für einige Minuten im Ultraschallbad behandelt. Man setzt 50 ml demineralisiertes Wasser hinzu, filtriert einmal durch einen Papierfilter und wäscht den Filter mit 10 ml demineralisiertem Wasser nach. Man setzt dem Filtrat weitere 200 ml demineralisiertes Wasser zu und lyophilisiert über Nacht. Man erhält 935 mg (99.7% d. Th.) der Zielverbindung.
LC-MS (Methode 8): Rₜ = 2.93 min.; m/z = 447 (M+H)⁺
¹H-NMR (400 MHz, DMSO-d₆): δ = 8.55 (s, 1H), 7.54 (d, 2H), 7.42-7.35 (m, 5H), 7.01 (d, 2H), 5.31-5.24 (m, 1H), 3.82 (s, 3H), 1.79 (t, 2H), 1.52-1.40 (m, 2H), 1.39-1.31 (m, 2H), 1.21 (d, 3H), 1.21-1.06 (m, 2H).
[α]_{D}²⁰ = -32.0°, c = 0.145, Dimethylsulfoxid.

### Beispiel 71

### (-)-6-{[5-(4-Methoxyphenyl)-6-phenylfuro[2,3-d]pyrimidin-4-yl]oxy}heptansäure-Bisethanolaminsalz

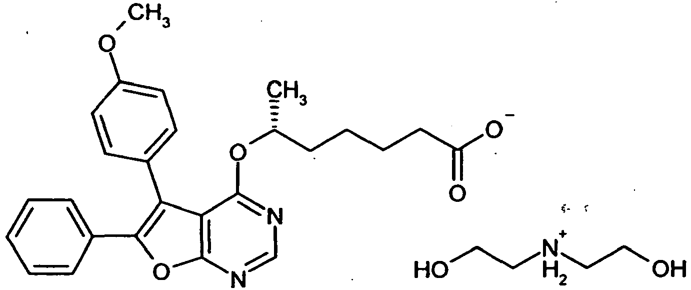

26.8 mg (0.060 mmol) (-)-6-{[5-(4-Methoxyphenyl)-6-phenylfuro[2,3-d]pyrimidin-4-yl]oxy}-heptansäure werden mit 250 µl demineralisiertem Wasser (Millipore-Ionenaustauscher) versetzt. Man gibt 6.3 mg (0.060 mmol) 2,2'-Iminodiethanol hinzu und rührt 30 min bei RT. Anschließend wird für einige Minuten im Ultraschallbad behandelt. Man gibt einige Tropfen Dioxan hinzu, lyophilisiert dann über Nacht und erhält 33.0 mg (99.7% d. Th.) der Zielverbindung.
LC-MS (Methode 8): Rₜ = 2.93 min.; m/z = 447 (M+H)⁺
¹H-NMR (400 MHz, DMSO-d₆): δ = 8.55 (s, 1H), 7.53 (d, 2H), 7.42-7.35 (m, 5H), 7.01 (d, 2H), 5.31-5.24 (m, 1H), 3.82 (s, 3H), 3.44 (t, 4H), 2.61 (t, 3H), 2.10 (t, 2H), 1.52-1.45 (m, 2H), 1.45-1.35 (m, 2H), 1.22 (d, 3H), 1.22-1.06 (m, 2H).
[α]_{D}²⁰ = -36.0°, c = 0.325, Dimethylsulfoxid.

### Beispiel 72

### (+/-)-6-{[5-(4-Ethylphenyl)-6-phenylfuro[2,3-d]pyrimidin-4-yl]oxy}heptansäuremethylester

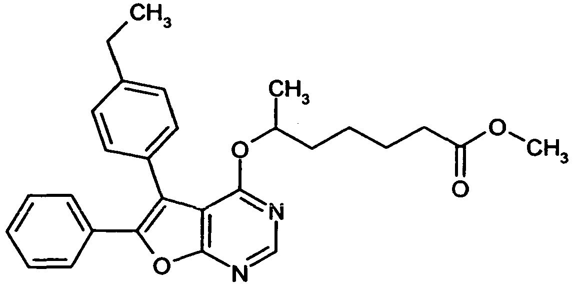

717 mg (4.48 mmol) (+/-)-6-Hydroxyheptansäuremethylester wird unter Argon in 10 ml THF vorgelegt und auf 0°C abgekühlt. Man gibt 2.25 ml (4.48 mmol) einer 2 M Lösung der Phosphazen-base P2-*tert*.-Butyl in THF hinzu und rührt 10 min bei RT nach. Dann wird wieder auf 0°C abgekühlt. Man fügt 1.0 g (2.99 mmol) 4-Chlor-5-(4-ethylphenyl)-6-phenylfuro[2,3-d]pyrimidin hinzu und rührt anschließend über Nacht bei RT nach. Man verdünnt mit Wasser, stellt mit 10%-iger wässriger Zitronensäure-Lösung sauer und extrahiert zweimal mit Ethylacetat. Die Ethylacetat-Phasen werden vereinigt und einmal mit ges. Natriumchlorid-Lösung gewaschen. Man trocknet über Magnesiumsulfat, engt ein und reinigt den Rückstand durch Säulenchromatographie an Silicagel (Laufmittel: Cyclohexan/Ethylacetat 9:1). Man erhält 640 mg (46.7% d. Th.) der Zielverbindung.
LC-MS (Methode 8): Rₜ = 3.44 min.; m/z = 459 (M+H)⁺
¹H-NMR (400 MHz, CDCl₃): δ = 8.50 (s, 1H), 7.64 (m, 2H), 7.38 (d, 2H), 7.31 (m, 3H), 7.24 (d, 2H), 5.35-5.26 (m, 1H), 3.63 (s, 3H), 2.76-2.67 (q, 2H), 2.21 (dd, 2H), 1.60-1.41 (m, 4H), 1.32-1.22 (m, 6H), 1.22-1.10 (m, 2H).

### Beispiel 73

### (+/-)-6-{[5-(4-Ethylphenyl)-6-phenylfuro[2,3-d]pyrimidin-4-yl]oxy}heptansäure

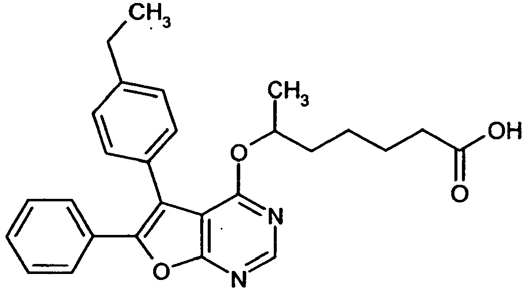

1.38 g (3.0 mmol) (+/-)-6-{[5-(4-Ethylphenyl)-6-phenylfuro[2,3-d]pyrimidin-4-yl]oxy}heptansäuremethylester werden in 25 ml THF vorgelegt. Man gibt 13.8 ml 1 N Natronlauge zu und rührt über Nacht bei RT. Man verdünnt mit Wasser und Ethylacetat und gibt dann 1 M Salzsäure bis zu einem pH-Wert von ca. 2 hinzu. Man trennt die Phasen und extrahiert die wässrige Phase noch zweimal mit Ethylacetat. Die Ethylacetat-Phasen werden vereinigt und einmal mit ges. Natriumchlorid-Lösung gewaschen. Man trocknet über Magnesiumsulfat und engt ein. Man erhält 600 mg (98.2% d. Th.) der Zielverbindung.
LC-MS (Methode 8): Rₜ = 3.32 min.; m/z = 445 (M+H)⁺
¹H-NMR (400 MHz, CDCl₃): δ = 8.51 (s, 1H), 7.62 (m, 2H), 7.37 (d, 2H), 7.30 (m, 3H), 7.23 (m, 2H), 5.34-5.25 (m, 1H), 2.76-2.58 (q, 2H), 2.24 (dd, 2H), 1.59-1.49 (m, 4H), 1.32-1.23 (m, 6H), 1.23-1.12 (m, 2H).

### Trennung der Enantiomere:

600 mg (+/-)-6-{[5-(4-Ethylphenyl)-6-phenylfuro[2,3-d]pyrimidin-4-yl]oxy}heptansäure werden in 20 ml 2-Propanol / 20 ml iso-Hexan gelöst und das Racemat durch präparative HPLC an chiraler Phase in die Enantiomere getrennt [Säule: Daicel Chiralpak AS-H, 5 µm, 250 mm x 20 mm; Fluss: 15 ml/min; Detektion: 220 nm; Injektionsvolumen: 400 µl; Temperatur: 40°C; Eluent: 80% iso-Hexan / 20% 2-Propanol + 0.2% TFA + 1% Wasser] (siehe Beispiel 74 und 75).

### Beispiel 74

### (+)-6-{[5-(4-Ethylphenyl)-6-phenylfuro[2,3-d]pyrimidin-4-yl]oxy}heptansäure (Enantiomer 1)

[α]_{D}²⁰ = +83.4°, c = 0.580, Methanol.
LC-MS (Methode 8): Rₜ = 3.28 min.; m/z = 445 (M+H)⁺.

### Beispiel 75

### (-)-6-{[5-(4-Ethylphenyl)-6-phenylfuro[2,3-d]pyrimidin-4-yl]oxy}heptansäure (Enantiomer 2)

[α]_{D}²⁰ = -81.3°, c = 0.520, Methanol.
LC-MS (Methode 8): Rₜ = 3.17 min.; m/z = 445 (M+H)⁺.

### Beispiel 76

### {[(3R)-3-{[5-(4-Methoxyphenyl)-6-phenylfuro[2,3-d]pyrimidin-4-yl]oxy}butyl]oxy}essigsäure-tert.-butylester

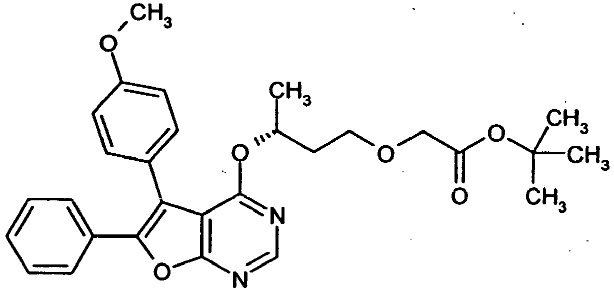

455 mg (2.23 mmol) {[*(3R)*-3-Hydroxybutyl]oxy}essigsäure-*tert*.-butylester (welcher zu ca. 10% {[*(1R)*-3-Hydroxy-1-methylpropyl]oxy}essigsäure-*tert*.-butylester enthält) werden unter Argon in 5 ml THF vorgelegt und auf 0°C abgekühlt. Man gibt 1.15 ml (2.23 mmol) einer 2 M Lösung der Phosphazenbase P2-*tert*.-Butyl in THF hinzu und rührt 10 min bei RT nach. Dann wird wieder auf 0°C abgekühlt. Man fügt 500 mg (1.49 mmol) 4-Chlor-5-(4-methoxyphenyl)-6-phenylfuro[2,3-d]-pyrimidin zu und rührt über Nacht bei RT. Man verdünnt danach mit Wasser, stellt mit 10%-iger wässriger Zitronensäure-Lösung sauer und extrahiert zweimal mit Ethylacetat. Die Ethylacetat-Phasen werden vereinigt und einmal mit ges. Natriumchlorid-Lösung gewaschen. Man trocknet über Magnesiumsulfat und engt ein. Der Rückstand wird durch Chromatographie an Silicagel (Laufmittel: Cyclohexan/Ethylacetat 9:1) gereinigt. Man erhält 450 mg (60.1% d. Th.) der Zielverbindung. Als Nebenprodukt werden 75 mg (9.0% d. Th.) (-)-{[(1*R*)-3-{[5-(4-Methoxyphenyl)-6-phenylfuro[2,3-d]pyrimidin-4-yl]oxy}-1-methylpropyl]oxy}essigsäure-*tert*.-butylester isoliert (siehe Beispiel 77).
LC-MS (Methode 2): Rₜ = 3.15 min.; m/z = 405 (M+H)⁺
¹H-NMR (400 MHz, DMSO-d₆): δ = 8.51 (s, 1H), 7.62 (m, 2H), 7.37 (d, 2H), 7.31 (m, 3H), 6.94 (d, 2H), 5.53-5.45 (m, 1H), 3.72 (s, 2H), 3.47-3.32 (m, 2H), 1.86 (m, 2H), 1.43 (s, 9H), 1.32 (d, 3H).

### Beispiel 77

### (-)-{[(1R)-3-{[5-(4-Methoxyphenyl)-6-phenylfuro[2,3-d]pyrimidin-4-yl]oxy}-1-methylpropyl]-oxy}essigsäure-tert.-butylester

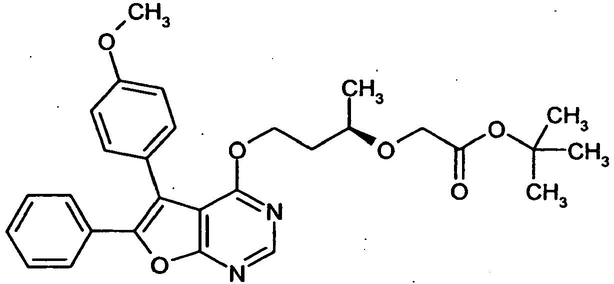

Die Titelverbindung wird als Nebenprodukt bei der Herstellung von Beispiel 76 erhalten.
LC-MS (Methode 5): Rₜ = 3.26 min.; m/z = 405 (M+H)⁺
¹H-NMR (400 MHz, DMSO-d₆): δ = 8.52 (s, 1H), 7.62 (m, 2H), 7.37 (d, 2H), 7.31 (m, 3H), 6.94 (d, 2H), 4.54 (m, 2H), 3.82 (m, 2H), 3.36-3.27 (m, 1H), 1.95-1.83 (m, 1H), 1.79-1.69 (m, 1H), 1.42 (s, 9H), 1.099 (d, 3H).
[α]_{D}²⁰ = -96.8°, c = 0.380, Methanol.

### Beispiel 78

### (-)-{[3-{[5-(4-Methoxyphenyl)-6-phenylfuro[2,3-d]pyrimidin-4-yl]oxy}butyl]oxy}essigsäure

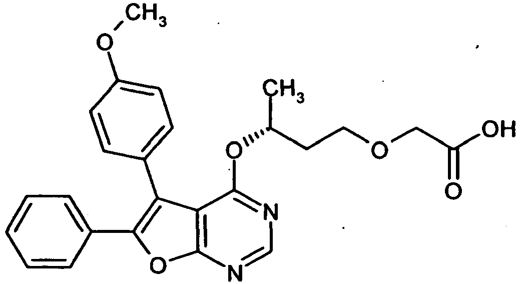

350 mg (0.69 mmol) {[(*3R*)-3-{[5-(4-Methoxyphenyl)-6-phenylfuro[2,3-d]pyrimidin-4-yl]oxy}-butyl]oxy}essigsäure-*tert*.-butylester werden in 7 ml Dichlormethan vorgelegt. Man gibt 1.75 ml TFA hinzu und rührt über Nacht bei RT. Man engt dann ein und reinigt den Rückstand durch Chromatographie an Silicagel (Laufmittel: Cyclohexan/Ethylacetat 1:1). Man erhält 110 mg (35.4% d. Th.) der Zielverbindung.
LC-MS (Methode 10): Rₜ = 2.67 min.; m/z = 449 (M+H)⁺
¹H-NMR (400 MHz, CDCl₃): δ = 8.50 (s, 1H), 7.61 (m, 2H), 7.36 (m, 2H), 7.31 (m, 3H), 6.93 (d, 2H), 5.52 (m, 1H), 3.96 (d, 2H), 3.47-3.36 (m, 2H), 1.92-1.80 (m, 2H), 1.34 (d, 3H).
[α]_{D}²⁰ = -79.6°, c = 0.42, Acetonitril.

### Beispiel 79

### (-)-{[3-{[5-(4-Methoxyphenyl)-6-phenylfuro[2,3-d]pyrimidin-4-yl]oxy}-1-methylpropyl]oxy}-essigsäure

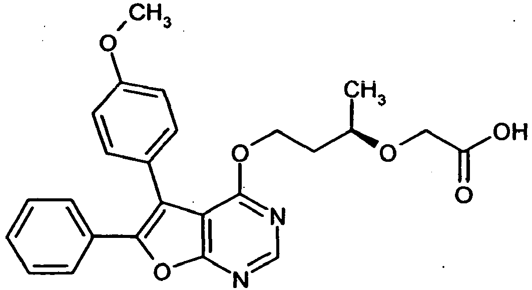

45 mg (0.089 mmol) (-)-{[3-{[5-(4-Methoxyphenyl)-6-phenylfuro[2,3-d]pyrimidin-4-yl]oxy}-1-methylpropyl]oxy}essigsäure-*tert.*-butylester werden in 1 ml Dichlormethan vorgelegt. Man gibt 250 µl TFA hinzu und rührt über Nacht bei RT. Man engt dann ein und reinigt den Rückstand durch Chromatographie an einer Silicagel-Dickschichtplatte (Laufmittel: Cyclohexan/Ethylacetat 1:1). Die Produktzone wird mit Dichlormethan/Methanol 95:5 extrahiert. Man erhält 8 mg (21.5% d. Th.) der Zielverbindung.
LC-MS (Methode 8): Rₜ = 2.65 min.; m/z = 449 (M+H)⁺
¹H-NMR (400 MHz, CDCl₃): δ = 8.49 (s, 1H), 7.58 (m, 2H), 7.34 (m, 2H), 7.27 (m, 3H), 6.94 (d, 2H), 5.58-4.45 (m, 2H), 3.75 (s, 2H), 3.36-3.24 (m, 1H), 1.92-1.81 (m, 1H), 1.81-1.71 (m, 1H), 1.11 (d, 3H).
[α]_{D}²⁰ = -77.1°, c = 0.370, Methanol.

Auf analoge Weise werden die folgenden beiden Verbindungen erhalten:

### Beispiel 80

### (-)-{[3-{[5-(4-Ethylphenyl)-6-phenylfuro[2,3-d]pyrimidin-4-yl]oxy}butyl]oxy}essigsäure

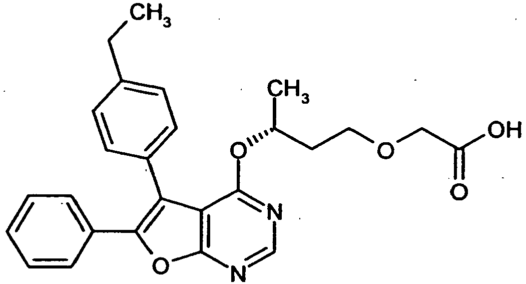

500 mg (0.99 mmol) {[(3*R*)-3-{[5-(4-Ethylphenyl)-6-phenylfuro[2,3-d]pyrimidin-4-yl]oxy}butyl]-oxy}essigsäure-*tert*.-butylester werden analog zur oben beschriebenen Synthesevorschrift mit TFA umgesetzt. Man erhält 447 mg (92.3% d. Th.) der Zielverbindung.
LC-MS (Methode 8): Rₜ = 3.00 min.; m/z = 447 (M+H)⁺
¹H-NMR (400 MHz, CDCl₃): δ = 8.49 (s, 1H), 7.60 (m, 2H), 7.33 (d, 2H), 7.27 (m, 3H), 7.22 (d, 2H), 5.52-5.43 (m, 1H), 3.97-3.87 (dd, 2H), 3.45-3.32 (m, 2H), 2.75-2.68 (q, 2H), 1.90-1.75 (m, 2H), 1.32 (t, 3H), 1.28 (d, 3H).
[α]_{D}²⁰ = -94°, c = 0.530, Methanol.

### Beispiel 81

### (-)-{[3-{[5-(4-Ethylphenyl)-6-phenylfuro[2,3-d]pyrimidin-4-yl]oxy}butyl]oxy}essigsäure-tert.-butylester

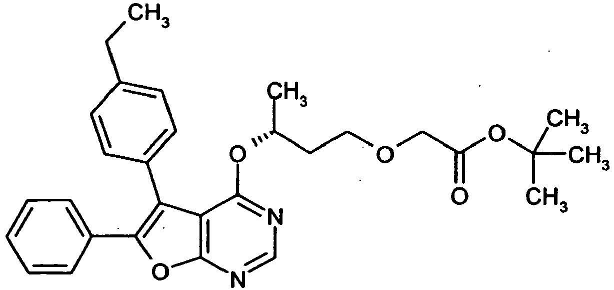

800 mg (2.39 mmol) {[*(3R)*-3-Hydroxybutyl]oxy}essigsäure-*tert*.-butylester (welches zu ca. 10% {[*(1R)*-3-Hydroxy-1-methylpropyl]oxy}essigsäure-*tert*.-butylester enthält) werden analog zur oben beschriebenen Synthesevorschrift mit 4-Chlor-5-(4-ethylphenyl)-6-phenylfuro[2,3-d]pyrimidin umgesetzt. Man erhält 690 mg (57.4% d. Th.) der Zielverbindung.
LC-MS (Methode 2): Rₜ = 3.35 min.; m/z = 503 (M+H)⁺
¹H-NMR (400 MHz, DMSO-d₆): δ = 8.50 (s, 1H), 7.62 (m, 2H), 7.37 (d, 2H), 7.31 (m, 3H), 7.22 (d, 2H), 5.50-5.41 (m, 1H), 3.71 (s, 2H), 3.42-3.28 (m, 2H), 2.75-2.68 (q, 2H), 1.82 (m, 2H), 1.43 (s, 9H), 1.33 (t, 3H), 1.30 (d, 3H).
[α]_{D}²⁰ = -90.7°, c = 0.370, Acetonitril.

### Beispiel 82

### (+)-3-[2-{[5-(4-Methoxyphenyl)-6-phenylfuro[2,3-d]pyrimidin-4-yl]oxy}-1-methylethoxy]propansäure-tert.-butylester

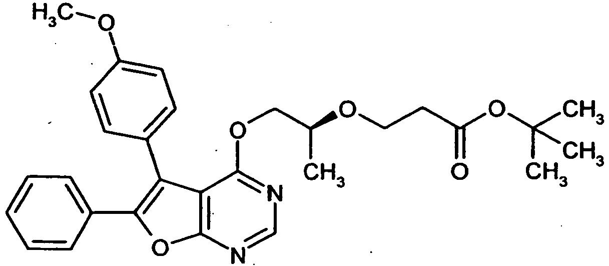

Eine Suspension von 2.548 g (7.57 mmol) 4-Chlor-5-(4-methoxyphenyl)-6-phenylfuro[2,3-d]pyrimidin und 1.70 g (8.32 mmol) (+)-3-[(1*S*)-2-Hydroxy-1-methylethoxy]propansäure-*tert*.-butylester in 8 ml DMF wird auf 0°C abgekühlt und portionsweise über 30 min mit 272 mg (6.81 mmol, 60%-ig) Natriumhydrid versetzt. Danach werden 0.5 ml abs. THF zugefügt, und die Mischung wird 10 min bei 0°C gerührt, bevor etwas Essigsäure zugesetzt und die Mischung auf Wasser gegeben wird. Die wässrige Phase wird dreimal mit Dichlormethan extrahiert. Man vereinigt die organischen Phasen, trocknet über Magnesiumsulfat und konzentriert im Vakuum. Das Rohprodukt kann entweder durch Chromatographie an Silicagel (Laufmittel: Cyclohexan/Ethylacetat 10:1 → 8:1) oder durch präparative RP-HPLC (Gradient: Acetonitril/Wasser) gereinigt werden. Es werden 2.27 g (59.5% d. Th.) der Zielverbindung erhalten.
LC-MS (Methode 8): Rₜ = 3.27 min.; m/z = 505 (M+H)⁺
¹H-NMR (400 MHz, DMSO-d₆): δ = 8.60 (s, 1H), 7.54 (d, 2H), 7.45-7.39 (m, 5H), 7.03 (d, 2H), 4.38-4.32 (m, 2H), 3.81 (s, 3H), 3.65-3.59 (m, 1H), 3.50-3.40 (m, 2H), 2.28 (t, 2H), 1.35 (s, 9H), 1.00 (d, 3H).
[α]_{D}²⁰ = +22.4°, c = 0.515, Chloroform.

### Beispiel 83

### (+)-3-{[(2S)-2-{[5-(4-Methoxyphenyl)-6-phenylfuro[2,3-d]pyrimidin-4-yl]oxy}-1-methylethoxy]-oxy}propansäure

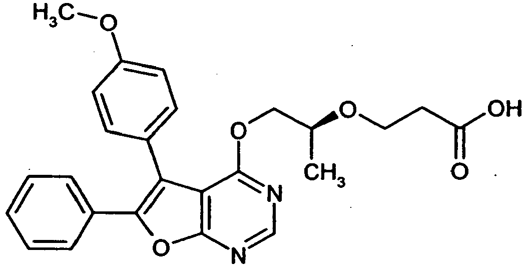

3.17 g (6.28 mmol) (+)-3-[2-{[5-(4-Methoxyphenyl)-6-phenylfuro[2,3-d]pyrimidin-4-yl]oxy}-1-methylethoxy]propansäure-*tert*.-butylester werden in 21 ml Dichlormethan gelöst und bei RT mit 12.1 ml TFA versetzt. Die Reaktionsmischung wird 2.5 h bei RT gerührt und dann vorsichtig im Vakuum eingeengt. Der Rückstand wird in Dichlormethan aufgenommen, mit Wasser gewaschen, über Natriumsulfat getrocknet und im Vakuum eingeengt. Der Rückstand wird durch Chromatographie an Silicagel (Laufmittel: Dichlormethan/Aceton 10:1 → 3:1) gereinigt. Die produkthaltigen Fraktionen werden vereinigt und im Vakuum konzentriert. Der anfallende Rückstand wird in Petrolether verrührt. Nach Filtration und Trocknen im Hochvakuum werden 1.77 g (62.8% d. Th.) des Zielprodukts erhalten.
LC-MS (Methode 8): Rₜ = 2.63 min.; m/z = 449 (M+H)⁺
¹H-NMR (400 MHz, DMSO-d₆): δ = 12.1 (s, 1H), 8.60 (s, 1H), 7.54 (d, 2H), 7.43-7.37 (m, 5H), 7.03 (d, 2H), 4.38-4.30 (m, 2H), 3.82 (s, 3H), 3.67-3.60 (m, 1H), 3.53-3.44 (m, 2H), 2.31 (t, 2H), 1.00 (d, 3H).
[α]_{D}²⁰ = +30.6°, c = 0.495, Chloroform.

### Beispiel 84

### (+)-3-[2-{[5-(4-Ethylphenyl)-6-phenylfuro[2,3-d]pyrimidin-4-yl]oxy}-1-methylethoxy]propansäure

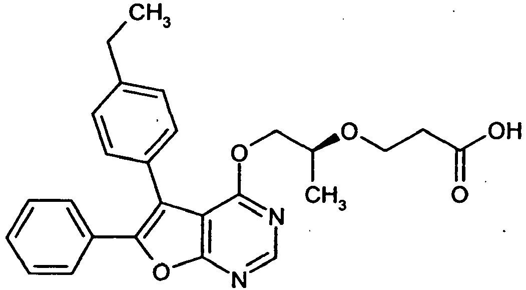

1.14 g (3.05 mmol) eines Gemisches aus *(2S)*-1-{[5-(4-Ethylphenyl)-6-phenylfuro[2,3-d]pyrimidin-4-yl]oxy}propan-2-ol und *(2S)*-2-{[5-(4-Ethylphenyl)-6-phenylfuro[2,3-d]pyrimidin-4-yl]oxy}propan-1-ol werden zusammen mit 1.951 g (15.22 mmol) Acrylsäure-*tert*.-butylester und 207 mg (0.609 mmol) Tetra-*n*-butylammoniumhydrogensulfat in 10 ml Dichlormethan vorgelegt und auf 0°C abgekühlt. Man gibt 2.5 ml einer 50%-igen Natronlauge hinzu und rührt 1 h kräftig bei 0°C. Man verdünnt danach mit Dichlormethan und stellt mit 10%-iger Zitronensäure-Lösung schwach sauer. Man trennt die Phasen, extrahiert die wässrige Phase einmal mit Dichlormethan, vereinigt die organischen Phasen, trocknet über Magnesiumsulfat und engt ein. Der erhaltene Rückstand wird in 30 ml Dichlormethan gelöst. Man gibt 7.5 ml TFA hinzu und rührt 1 h bei RT. Das Gemisch wird dann eingeengt und der Rückstand im Hochvakuum getrocknet. Man reinigt zunächst durch Chromatographie an Silicagel (Laufmittel: Cyclohexan/Ethylacetat 1:1) und erhält so 1.15 g eines Regioisomeren-Gemisches.

Das erhaltene Regioisomeren-Gemisch (1.15 g) wird in einem Gemisch aus 5 ml Isohexan und 5 ml Ethylacetat gelöst und durch Chromatographie an chiraler Phase in die Isomere getrennt [Säule: chirale Kieselgel-Phase basierend auf dem Selektor Poly(*N*-methacryloyl-*L*-Leucin-*tert*.-butylamid), 500 mm x 30 mm; Fluss: 50 ml/min; Detektion: 260 nm; Injektionsvolumen: 300 µl; Temperatur: 24°C; Eluent: Isohexan/Ethylacetat 1:1]. Man erhält auf diese Weise 287 mg (25.6% d. Th.) der Titelverbindung sowie 255 mg (22.8% d. Th.) des Regioisomers (+)-3-{[2-{[5-(4-Ethylphenyl)-6-phenylfuro[2,3-d]pyrimidin-4-yl]oxy}propyl]oxy}propansäure (siehe Beispiel 85).
[α]_{D}²⁰ = +50.1 °, c = 0.500, Methanol.
LC-MS (Methode 8): Rₜ = 2.82 min.; m/z = 447 (M+H)⁺ .
¹H-NMR (400 MHz, DMSO-d₆): δ = 12.12 (br. s, 1H), 8.49 (s, 1H), 7.52 (d, 2H), 7.39 (m, 5H), 7.30 (d, 2H), 4.31 (m, 2H), 3.63-3.55 (m, 1H), 3.53-3.38 (m, 2H), 2.72-2.65 (q, 2H), 2.30 (t, 2H), 1.22 (t, 3H), 0.93 (d, 3H).

### Beispiel 85

### (+)-3-{[2-{[5-(4-Ethylphenyl)-6-phenylfuro[2,3-d]pyrimidin-4-yl]oxy}propyl]oxy}propansäwe

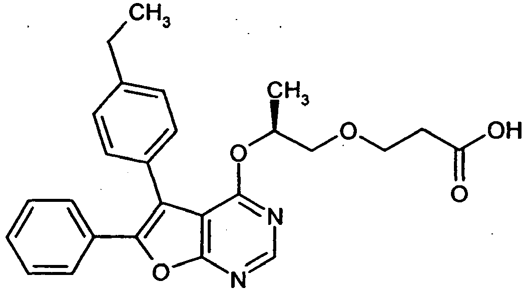

Zur Herstellung siehe oben unter Beispiel 84.
LC-MS (Methode 8): Rₜ = 2.85 min.; m/z = 447 (M+H)⁺
¹H-NMR (400 MHz, DMSO-d₆): δ = 12.12 (br. s, 1H), 8.49 (s, 1H), 7.53 (d, 2H), 7.39 (m, 5H), 7.29 (d, 2H), 5.45-5.36 (m, 1H), 3.52-3.32 (m, 4H), 2.72-2.65 (q, 2H), 2.30 (t, 2H), 1.22 (t, 3H), 1.20 (d, 3H).
[α]_{D}²⁰ = +46.0°, c = 0.590, Methanol.

Die in der folgenden Tabelle aufgeführten Verbindungen werden analog zur oben beschriebenen Synthese hergestellt. Die Trennung der Regioisomeren wird im Einzelnen wie folgt durchgeführt:

### Beispiel 86 und Beispiel 87:

1.00 g (1.99 mmol) eines Gemisches aus (-)-3-[2-{[5-(4-Ethylphenyl)-6-phenylfuro[2,3-d]pyrimidin-4-yl]oxy}-1-methylethoxy]propansäure (Beispiel 86) und (-)-3-{[2-{[5-(4-Ethylphenyl)-6-phenylfuro[2,3-d]pyrimidin-4-yl]oxy}propyl]oxy}propansäure (Beispiel 87) werden in einem Gemisch aus 5 ml Isohexan und 5 ml Ethylacetat gelöst und durch Chromatographie an chiraler Phase in die Isomere getrennt; Säule: chirale Kieselgel-Phase basierend auf dem Selektor Poly(N-methacryloyl-L-Leucin-dicyclopropylmethylamid), 680 mm x 40 mm; Fluss: 50 ml/min; Detektion: 260 nm; Injektionsvolumen: 1700 µl; Temperatur: 24°C; Eluent: 50% Isohexan / 50% Ethylacetat.

### Beispiel 83 und Beispiel 88:

7.80 g (15.45 mmol) eines Gemisches aus (+)-3-[2-{[5-(4-Methoxyphenyl)-6-phenylfuro[2,3-d]-pyrimidin-4-yl]oxy}-1-methylethoxy]propansäure (Beispiel 83) und (+)-3-{[2-{[5-(4-Methoxyphenyl)-6-phenylfuro[2,3-d]pyrimidin-4-yl]oxy}propyl]oxy}propansäure (Beispiel 88) werden in einem Gemisch aus 50 ml Isohexan und 50 ml Ethylacetat gelöst und durch Chromatographie an chiraler Phase in die Isomere getrennt; Säule: chirale Kieselgel-Phase basierend auf dem Selektor Poly(*N*-methacryloyl-*L*-Leucin-1-menthylamid), 250 mm x 30 mm; Fluss: 50 ml/min; Detektion: 260 nm; Injektionsvolumen: 400 µl; Temperatur: 24°C; Eluent: 50% Isohexan / 50% Ethylacetat.

### Beispiel 83 kann auch auf alternative Weise hergestellt werden (Beschreibung siehe oben).

### Beispiel 89 und Beispiel 90:

250 mg (0.56 mmol) eines Gemisches aus (-)-3-[2-{[5-(4-Methoxyphenyl)-6-phenylfuro[2,3-d]-pyrimidin-4-yl]oxy}-1-methylethoxy]propansäure (Beispiel 89) und (-)-3-{[2-{[5-(4-Methoxyphenyl)-6-phenylfuro[2,3-d]pyrimidin-4-yl]oxy}propyl]oxy}propansäure (Beispiel 90) werden in einem Gemisch aus 2 ml Isohexan und 2 ml Ethylacetat gelöst und durch Chromatographie an chiraler Phase in die Isomere getrennt; Säule: chirale Kieselgel-Phase basierend auf dem Selektor Poly(*N*-methacryloyl-*L*-Leucin-dicyclopropylmethylamid), 680 mm x 40 mm; Fluss: 50 ml/min; Detektion: 260 nm; Injektionsvolumen: 4000 µl; Temperatur: 24°C; Eluent: t = 0 min 60% Iso-hexan / 40% Ethylacetat → t = 13 min 45% Isohexan / 55% Ethylacetat.

| **Beispiel** | **Struktur** | **Analytische Daten** |
|---|---|---|
| **86** | 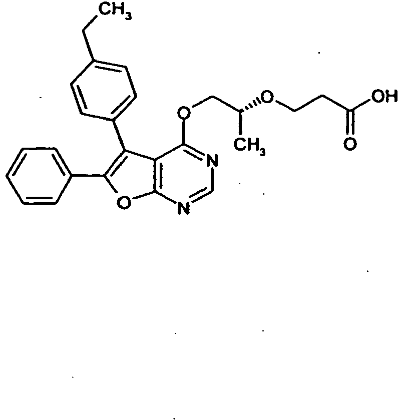 | [α]_{D}²⁰ = -44.4°, c = 0.475, Methanol; |
| | | LC-MS (Methode 7): Rₜ = 4.12. min.; m/z = 447 (M+H)⁺ |
| | | ¹H-NMR (400 MHz, CDCl₃): δ = 8.51 (s, 1H), 7.60 (m, 2H), 7.38 (d, 2H), 7.31-7.22 (m, 5H), 4.40-4.30 (m, 2H), 3.69-3.61 (m, 1H), 3.56-3.43 (m, 2H), 2.70 (q, 2H), 2.42 (t, 2H), 1.29 (t, 3H), 1.06 (d, 3H). |
| **87** | 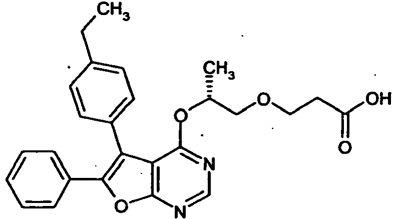 | [α]_{D}²⁰ = -45.2°, c = 0.430, Methanol; |
| | | LC-MS (Methode 7): Rₜ = 4.16 min.; m/z = 447 (M+H)⁺ |
| | | ¹H-NMR (400 MHz, CDCl₃): δ = 8.51 (s, 1H), 7.61 (m, 2H), 7.38 (d, 2H), 7.31-7.28 (m, 3H), 7.22 (d, 2H), 5.55-5.45 (m, 1H), 3.55-3.45 (m, 4H), 2.70 (q, 2H), 2.43 (t, 2H), 1.29 (t, 3H), 1.25 (d, 3H). |
| **83** | 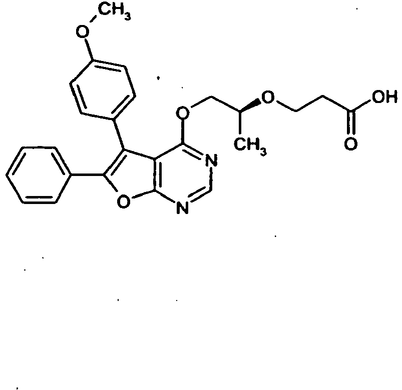 | [α]_{D}²⁰ = +59.6°, c = 0.432, Methanol; |
| | | LC-MS (Methode 2): Rₜ = 2.41 min.; m/z = 449 (M+H)⁺ |
| | | ¹H-NMR (400 MHz, CDCl₃): δ = 8.51 (s, 1H), 7.60 (m, 2H), 7.38 (d, 2H), 7.31-7.28 (m, 3H), 6.95 (d, 2H), 4.49 (d, 2H), 3.86 (s, 3H), 3.75-3.65 (m, 1H), 3.61-3.45 (m, 2H), 2.43 (t, 2H), 1.12 (d, 3H). |
| **88** | 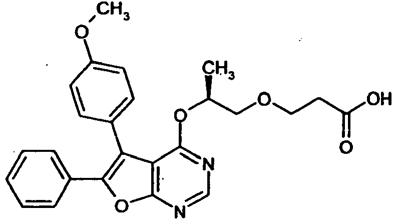 | [α]_{D}²⁰ = +48.1 °, c = 0.425, Acetonitril; |
| | | LC-MS (Methode 8): Rₜ = 2.73 min.; m/z = 449 (M+H)⁺ |
| | | ¹H-NMR (400 MHz, CDCl₃): δ = 8.51 (s, 1H), 7.60 (m, 2H), 7.39 (d, 2H), 7.31-7.27 (m, 3H), 6.93 (d, 2H), 5.55-5.48 (m, 1H), 3.86 (s, 3H), 3.60-3.48 (m, 4H), 2.48 (t, 2H), 1.26 (d, 3H). |
| **89** | 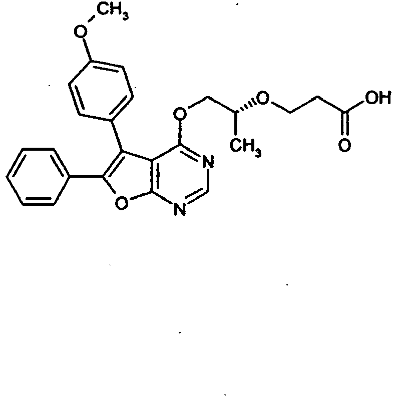 | [α]_{D}²⁰ =-41.1°, c = 0.3655, Acetonitril; |
| | | LC-MS (Methode 2): Rₜ = 2.41 min.; m/z = 449 (M+H)⁺ |
| | | ¹H-NMR (400 MHz, CDCl₃): δ = 8.51 (s, 1H), 7.60 (m, 2H), 7.38 (d, 2H), 7.31-7.28 (m, 3H), 6.95 (d, 2H), 4.49 (d, 2H), 3.86 (s, 3H), 3.75-3.65 (m, 1H), 3.61-3.45 (m, 2H), 2.43 (t, 2H), 1.12 (d, 3H). |
| **90** | 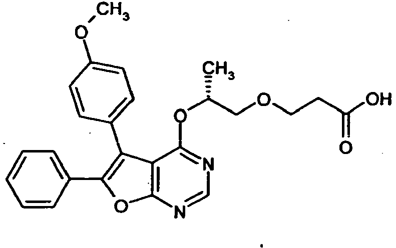 | [α]_{D}²⁰ = -31.5°, c = 0.415, Acetonitril; |
| | | LC-MS (Methode 8): Rₜ = 2.73 min.; m/z = 449 (M+H)⁺ |
| | | ¹H-NMR (400 MHz, CDCl₃): δ = 8.51 (s, 1H), 7.60 (m, 2H), 7.39 (d, 2H), 7.31-7.27 (m, 3H), 6.93 (d, 2H), 5.55-5.48 (m, 1H), 3.86 (s, 3H), 3.60-3.48 (m, 4H), - 2.48 (t, 2H), 1.26 (d, 3H). |

### Beispiel 91

### (+/-)-4-[(2-{[5-(4-Methoxyphenyl)-6-phenylfuro[2,3-d]pyrimidin-4-yl]oxy}propyl)(methyl)-amino]butansäuremethylester

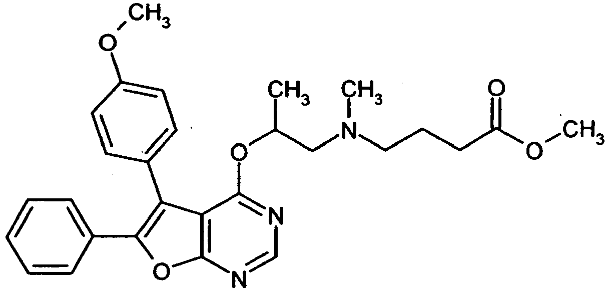

25 mg (0.064 mmol) (+/-)-2-{[5-(4-Methoxyphenyl)-6-phenylfuro[2,3-d]pyrimidin-4-yl]oxy}-*N-*methylpropan-1-amin werden mit 13 mg (0.128 mmol) Triethylamin in 250 µl Dichlormethan vorgelegt. Man gibt 23.2 mg (0.128 mmol) 4-Brombuttersäuremethylester hinzu und rührt über Nacht bei RT. Es werden erneut die gleichen Mengen an Triethylamin und 4-Brombuttersäuremethylester zugegeben und für weitere 24 h bei RT gerührt. Man engt danach ein und reinigt den Rückstand durch Dickschicht-Chromatographie an Silicagel (Laufmittel: Dichlormethan/Methanol 95:5). Die produkthaltige Zone wird mit Dichlormethan/Methanol 9:1 extrahiert. Man erhält 22.4 mg der Zielverbindung als Rohprodukt.
LC-MS (Methode 8): Rₜ = 1.84 min.; m/z = 490 (M+H)⁺.

### Beispiel 92

### (+/-)-4-[(2-{[5-(4-Methoxyphenyl)-6-phenylfuro[2,3-d]pyrimidin-4-yl]oxy}propyl)(methyl)-amino]butansäure

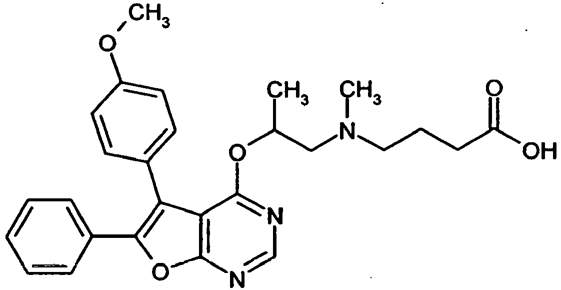

20 mg (0.027 mmol) (+/-)-4-[(2-{[5-(4-Methoxyphenyl)-6-phenylfuro[2,3-d]pyrimidin-4-yl]oxy}-propyl)(methyl)amino]butansäuremethylester werden in 0.8 ml THF vorgelegt. Man gibt 0.27 ml (0.27 mmol) 1 N Natronlauge hinzu und rührt über Nacht bei RT. Man engt danach ein und reinigt den Rückstand durch Dickschicht-Chromatographie an Silicagel (Laufmittel: Dichlormethan/Methanol 9:1). Die Produktzone wird mit Dichlormethan/Methanol 7:3 extrahiert. Man erhält 8.5 mg (66.3% d. Th.) der Zielverbindung.
LC-MS (Methode 10): Rₜ = 1.70 min.; m/z = 476 (M+H)⁺
¹H-NMR (400 MHz, DMSO-d₆): δ = 8.57 (s, 1H), 7.52 (m, 2H), 7.43-7.35 (m, 5H), 7.02 (d, 2H), 5.57-5.48 (m, 1H), 3.81 (s, 3H), 3.6-3.4 (br. s, 2H), 2.36-2.25 (br. s, 2H), 2.14-2.06 (m, 5H), 1.57-1.45 (m, 2H), 1.22 (d, 3H).

### Beispiel 93

### 3-[2-{[5-(4-Methoxyphenyl)-6-phenylfuro[2,3-d]pyrimidin-4-yl]amino}-1-methylethoxy]propansäure

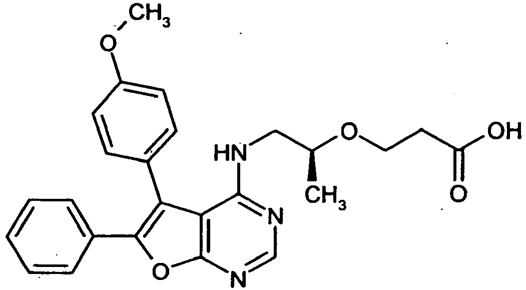

100 mg (0.27 mmol) (+)-1-{[5-(4-Methoxyphenyl)-6-phenylfuro[2,3-d]pyrimidin-4-yl]amino}propan-2-ol werden mit 170 mg (1.33 mmol) Acrylsäure-*tert*.-butylester und 18.1 mg (0.053 mmol) Tetra-*n*-butylammoniumhydrogensulfat in 2 ml Dichlormethan vorgelegt und auf 0°C abgekühlt. Dann werden 250 µl 50%-ige Natronlauge zugegeben und die Mischung bei 0°C 1 h lang kräftig gerührt. Man läßt auf RT kommen und rührt über Nacht bei RT nach. Dann wird mit Dichlormethan und Wasser verdünnt. Man stellt mit 10%-iger Zitronensäure-Lösung sauer und trennt die Phasen. Die wässrige Phase wird einmal mit Dichlormethan rückextrahiert. Die organischen Phasen werden vereinigt, einmal mit ges. Natriumchlorid-Lösung gewaschen, über Magnesiumsulfat getrocknet und eingeengt. Man löst den so erhaltenen Rückstand in 2.5 ml Dichlormethan, gibt 600 µl Trifluoressigsäure hinzu und rührt 2 h bei RT nach. Man engt dann ein und reinigt den Rückstand durch zweimalige Chromatographie über eine Silicagel-Dickschichtplatte (Laufmittel: Dichlormethan/Methanol 9:1). Die Produktzone wird mit Dichlormethan/Methanol 9:1 extrahiert. Nach Einengen und Trocknen erhält man 38 mg (42.8% d. Th.) der Zielverbindung.
LC-MS (Methode 8): Rₜ = 2.41 min.; m/z = 448 (M+H)⁺
¹H-NMR (400 MHz, CDCl₃): δ = 8.32 (s, 1H), 7.44 (m, 4H), 7.38-7.30 (m, 3H), 7.13 (d, 2H), 5.18 (t, 1H), 3.84 (s, 3H), 3.66-3.45 (m, 3H), 3.39-3.15 (m, 2H), 2.26 (m, 2H), 1.01 (d, 3H).

### Beispiel 94

### (3-{[5-(4-Methoxyphenyl)-6-phenylfuro[2,3-d]pyrimidin-4-yl]oxy}-2,2-dimethylpropoxy)essigsäure-tert.-butylester

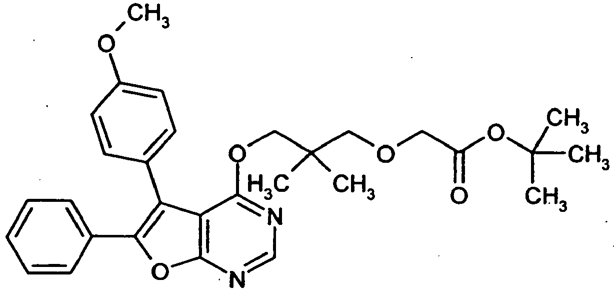

300 mg (0.742 mmol) 3-{[5-(4-Methoxyphenyl)-6-phenylfuro[2,3-d]pyrimidin-4-yl]oxy}-2,2-dimethylpropan-1-ol werden mit 723 mg (3.71 mmol) Bromessigsäure-*tert*.-butylester und 50 mg (0.148 mmol) Tetra-*n*-butylammoniumhydrogensulfat in 6 ml Dichlormethan vorgelegt. Man kühlt auf 0°C. Dann gibt man 750 µl 50%-ige Natronlauge hinzu und rührt einige Minuten heftig bei 0°C. Man läßt unter kräftigem Rühren auf RT kommen und rührt kräftig weiter über Nacht. Man verdünnt dann mit Dichlormethan und stellt mit 10%-iger Zitronensäure-Lösung schwach sauer. Die Phasen werden getrennt und die wässrige Phase einmal mit Dichlormethan extrahiert. Die vereinigten organischen Phasen werden einmal mit ges. Natriumchlorid-Lösung gewaschen, über Magnesiumsulfat getrocknet und eingeengt. Man reinigt den Rückstand durch Chromatographie an Silicagel (Laufmittel: Cyclohexan/Ethylacetat 9:1). Es werden 295 mg (76.7% d. Th.) der Zielverbindung erhalten.
LC-MS (Methode 8): Rₜ = 3.33 min.; m/z = 419 (M+H)⁺
¹H-NMR (400 MHz, CDCl₃): δ = 8.51 (s, 1H), 7.61 (m, 2H), 7.49 (d, 2H), 7.30 (m, 3H), 6.98 (d, 2H), 4.21 (s, 2H), 3.87 (s, 3H), 3.77 (s, 2H), 3.02 (s, 2H), 1.44 (s, 9H), 0.82 (s, 6H).

### Beispiel 95

### (3-{[5-(4-Methoxyphenyl)-6-phenylfuro[2,3-d]pyrimidin-4-yl]oxy}-2,2-dimethylpropoxy)essigsäure

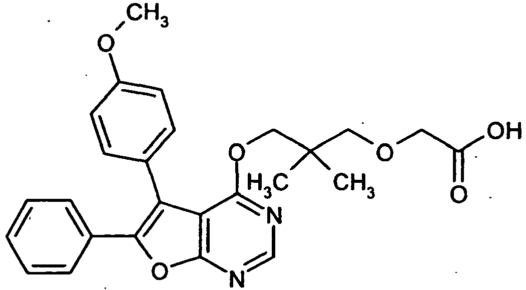

280 mg (0.54 mmol) (3-{[5-(4-Methoxyphenyl)-6-phenylfuro[2,3-d]pyrimidin-4-yl]oxy}-2,2-di-methylpropoxy)essigsäure-*tert*.-butylester werden in 8 ml Dichlormethan vorgelegt. Man gibt 2 ml Trifluoressigsäure hinzu und rührt 1 h bei RT. Man engt dann ein und verrührt den Rückstand mit Petrolether. Der Feststoff wird über eine Fritte abgesaugt und im Hochvakuum getrocknet. Man erhält 220 mg (88.1% d. Th.) der Zielverbindung.
LC-MS (Methode 7): Rₜ = 4.03 min.; m/z = 463 (M+H)⁺
¹H-NMR (400 MHz, CDCl₃): δ = 8.56 (s, 1H), 7.54 (d, 2H), 7.42-7.35 (m, 5H), 7.02 (d, 2H), 4.12 (s, 2H), 3.86 (s, 2H), 3.81 (s, 3H), 3.01 (s, 2H), 0.72 (s, 6H).

### Beispiel 96

### (3-{[5-(4-Methoxyphenyl)-6-phenylfuro[2,3-d]pyrimidin-4-yl]oxy}propoxy)essigsäure

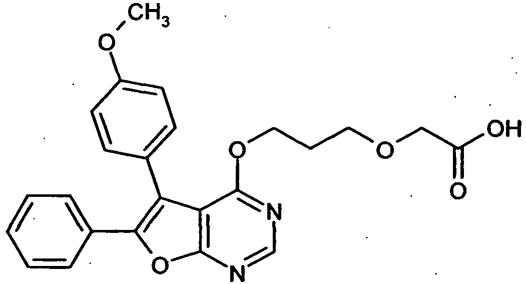

200 mg (0.53 mmol) 3-{[5-(4-Methoxyphenyl)-6-phenylfuro[2,3-d]pyrimidin-4-yl]oxy}propan-1-ol werden mit 518 mg (2.66 mmol) Bromessigsäure-*tert*.-butylester und 36 mg (0.106 mmol) Tetra-*n*-butylammoniumhydrogensulfat in 5 ml Dichlormethan vorgelegt und auf 0°C abgekühlt. Man gibt 1.0 ml 50%-ige Natronlauge hinzu und rührt kräftig bei 0°C. Man lässt dann auf RT kommen und rührt über Nacht kräftig nach. Man verdünnt danach mit Dichlormethan und Wasser, stellt mit 10%-iger Zitronensäure-Lösung sauer und trennt die Phasen. Die wässrige Phase wird einmal mit Dichlormethan rückextrahiert. Die organischen Phasen werden vereinigt, einmal mit ges. Natriumchlorid-Lösung gewaschen, über Magnesiumsulfat getrocknet und eingeengt. Der Rückstand wird in 5 ml Dichlormethan gelöst. Man gibt 1.25 ml TFA hinzu und rührt 2 h bei RT. Man engt dann ein und trocknet im Hochvakuum. Der Rückstand wird chromatographisch über eine Silicagel-Dickschichtplatte gereinigt (Laufmittel: Dichlormethan/Methanol 95:5). Die Produktzone wird mit Dichlormethan/Methanol 9:1 extrahiert. Man erhält 50 mg (23.0% d. Th.) der Zielverbindung.
LC-MS (Methode 7): Rₜ = 3.68 min.; m/z = 435 (M+H)⁺
¹H-NMR (400 MHz, CDCl₃): δ = 12.58 (br. s, 1H), 8.59 (s, 1H), 7.54 (d, 2H), 7.42-7.35 (m, 5H), 7.02 (d, 2H), 4.43 (t, 2H), 3.82 (s, 3H), 3.39-3.31 (m, 4H), 1.84-1.78 (m, 2H).

### Beispiel 97

### (-)-4-[2-{[5-(4-Methoxyphenyl)-6-phenylfuro[2,3-d]pyrimidin-4-yl]amino}-1-methylethoxy]buttersäure

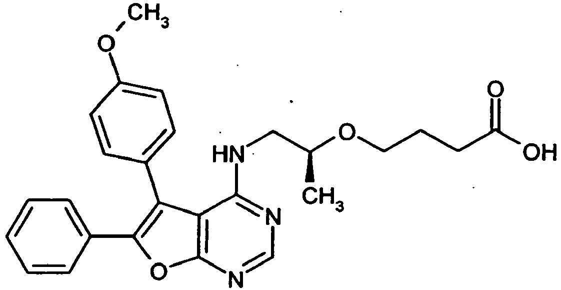

110 mg (0.29 mmol) (+)-1-{[5-(4-Methoxyphenyl)-6-phenylfuro[2,3-d]pyrimidin-4-yl]amino}propan-2-ol werden mit 327 mg (1.47 mmol) 4-Brombuttersäure-*tert*.-butylester und 20 mg (0.059 mmol) Tetra-*n*-butylammoniumhydrogensulfat in 2 ml Dichlormethan vorgelegt und auf 0°C abgekühlt. Dann werden 500 µl 50%-ige Natronlauge zugegeben und zwei Tage bei RT gerührt. Es werden nochmals die gleichen Mengen an 4-Brombuttersäure-*tert*.-butylester, Tetra-*n*-butylammoniumhydrogensulfat und 50%-iger Natronlauge zugesetzt und weitere 24 h bei RT gerührt. Danach wird 24 h lang unter Rückfluss erhitzt. Man lässt abkühlen und verdünnt mit Dichlormethan und Wasser. Man stellt mit 10%-iger Zitronensäure-Lösung sauer und trennt die Phasen. Die wässrige Phase wird einmal mit Dichlormethan rückextrahiert. Die organischen Phasen werden vereinigt, einmal mit ges. Natriumchlorid-Lösung gewaschen, über Magnesiumsulfat getrocknet und eingeengt. Man reinigt den Rückstand durch präparative HPLC. Das so erhaltene Produkt (30 mg) wird in 1 ml Dichlormethan gelöst. Man gibt 250 µl Trifluoressigsäure hinzu und rührt über Nacht bei RT. Man engt dann ein und reinigt den Rückstand chromatographisch über eine Silicagel-Dickschichtplatte (Laufmittel: Dichlormethan/Methanol 95:5). Die Produktzone wird mit Dichlormethan/Methanol 9:1 extrahiert. Man erhält 25 mg (21.2% d. Th.) der Zielverbindung.
LC-MS (Methode 7): Rₜ = 3.64 min.; m/z = 462 (M+H)⁺
¹H-NMR (400 MHz, CDCl₃): δ = 8.39 (s, 1H), 7.51 (m, 2H), 7.41 (d, 2H), 7.26 (m, 3H), 7.06 (d, 2H), 5.12 (t, 1H), 3.89 (s, 3H), 3.77-3.70 (m, 1H), 3.55-3.47 (m, 1H), 3.42-3.38 (m, 1H), 3.28-3.20 (m, 2H), 2.31 (t, 2H), 1.76-1.67 (m, 2H), 1.09 (d, 3H).
[α]_{D}²⁰ = -20.0°, c = 0.077, Acetonitril.

### Beispiel 98

### 3-{[(1R,2R)-2-{[5-(4-Methoxyphenyl)-6-phenylfuro[2,3-d]pyrimidin-4-yl]oxy}-1-methylpropyl]-oxy}propionsäure-tert.-butylester

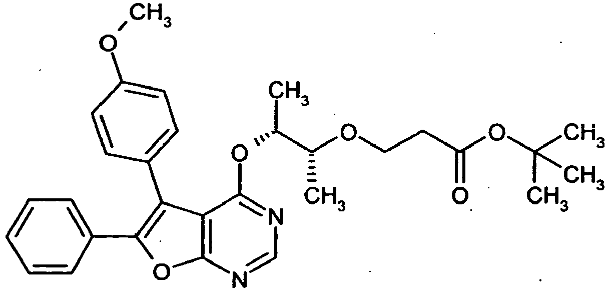

Eine Lösung von 535 mg (1.37 mmol) (*2R,3R*)-3-{[5-{4-Methoxyphenyl)-6-phenylfuro[2,3-d]pyrimidin-4-yl]oxy}butan-2-ol in 5 ml THF wird mit 157 mg (1.37 mmol) Kalium-*tert*.-butylat versetzt. Nach 15 min Rühren bei RT werden 1.0 ml (878 mg, 6.65 mmol) Acrylsäure-*tert*.-butylester zugegeben. Nach drei Stunden werden 10 ml Wasser zugesetzt, und das Reaktionsgemisch wird im Vakuum eingeengt. Der Rückstand wird mittels präparativer RP-HPLC (Gradient: Wasser/Acetonitril) gereinigt. Es werden 346 mg (47% d. Th.) des gewünschten Produkts erhalten.
LC-MS (Methode 7): Rₜ = 4.83 min.; m/z = 519 (M+H)⁺
¹H-NMR (400 MHz, CDCl₃): δ = 8.56 (s, 1H), 7.55 (d, 2H), 7.40-7.35 (m, 5H), 7.01 (d, 2H), 5.29 (dt, 1H), 3.81 (s, 3H), 3.63-3.40 (m, 3H), 2.26 (t, 2H), 1.33 (s, 9H), 1.15 (d, 3H), 0.88 (d, 3H).

### Beispiel 99

### 4-{[(2R)-2-{[5-(4-Ethylphenyl)-6-phenylfuro[2,3-d]pyrimidin-4-yl]oxy}propyl](methyl)amino}-buttersäuremethylester

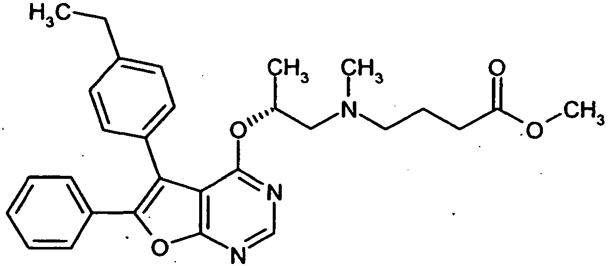

Eine Lösung von 1000 mg (2.31 mmol) (*2R*)-2-{[5-(4-Ethylphenyl)-6-phenylfuro[2,3-d]pyrimidin-4-yl]oxy}-*N*-methylpropan-1-ammoniumformiat in 20 ml THF wird mit 797 mg (5.77 mmol) Kaliumcarbonat versetzt. Nach Zugabe von 0.35 ml (501 mg, 2.77 mmol) 4-Brombuttersäuremethylester und 34 mg (0.09 mmol) Tetra-n-butylammoniumiodid wird das Reaktionsgemisch 16 h bei 80°C gerührt. Nach dem Abkühlen auf Raumtemperatur werden die anorganischen Salze abfiltriert und mit THF gewaschen. Das Filtrat wird im Vakuum eingeengt. Der Rückstand wird in Acetonitril aufgenommen und mittels präparativer RP-HPLC (Gradient: Wasser/Acetonitril/Ammoniak) gereinigt. Man erhält 308 mg (73% Reinheit, 20% d. Th.) des gewünschten Produkts.
LC-MS (Methode 8): Rₜ =1.91 min.; m/z = 488 (M+H)⁺.

### Beispiel 100

### (6R)-6-{[6-(2-Fluorphenyl)-5-(4-methoxyphenyl)furo[2,3-d]pyrimidin-4-yl]oxy}heptansäure-tert.-butylester

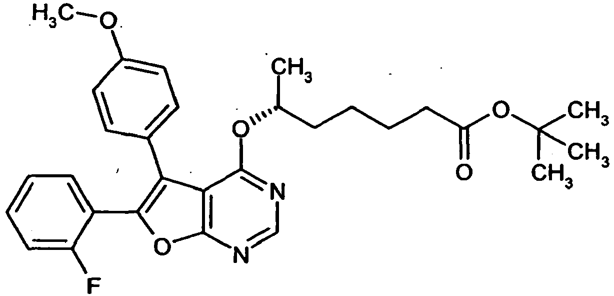

Eine Lösung von 350 mg (1.73 mmol) (*6R*)-6-Hydroxyheptansäure-*tert*.-butylester in 5 ml THF wird unter Eiskühlung mit 87 mg (2.16 mmol) Natriumhydrid (60%-ige Dispersion in Mineralöl) versetzt. Nach zehn Minuten Rühren unter Eiskühlung werden eine Lösung von 644 mg (1.82 mmol) 4-Chlor-6-(2-fluorphenyl)-5-(-methoxyphenyl)furo[2,3-d]pyrimidin in 5 ml THF sowie 32 mg (0.09 mmol) Tetra-*n*-butylammoniumiodid zugegeben. Das Reaktionsgemisch wird 48 bei RT gerührt. Nach Zugabe von Wasser und Essigsäureethylester wird die abgetrennte organische Phase mit 1 N Salzsäure gewaschen und im Vakuum eingeengt. Der Rückstand wird in Acetonitril/DMSO aufgenommen und mittels präparativer RP-HPLC (Gradient: Wasser/Acetonitril) gereinigt. Es werden 425 mg (47% d. Th.) des gewünschten Produkts erhalten.
LC-MS (Methode 8): Rₜ = 3.37 min.; m/z = 521 (M+H)⁺
¹H-NMR (400 MHz, DMSO-d₆): δ = 8.60 (s, 1H), 7.55-7.50 (m, 2H), 7.34-7.28 (m, 4H), 6.93-6.91 (m, 2H), 5.41-5.34 (m, 1H), 3.77 (s, 3H), 2.10 (t, 2H), 1.60-1.55 (m, 2H), 1.46-1.39 (m, 2H), 1.34 (s, 9H), 1.28 (d, 3H), 1.25-1.15 (m, 2H).

### Beispiel 101

### 4-{[(2S)-2-{[6-(2-Fluorphenyl)-5-(4-methoxyphenyl)furo[2,3-d]pyrimidin-4-yl]oxy}propyl]-(methyl)amino}buttersäure-tert.-butylester

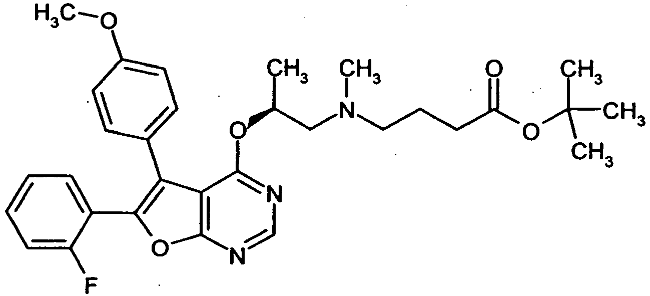

Eine Lösung von 100 mg (0.43 mmol) 4-{[(*2S*)-2-Hydroxypropyl](methyl)amino}buttersäure-*tert*.-butylester in 1 ml THF wird unter Eiskühlung mit 22 mg (0.54 mmol) Natriumhydrid (60%-ige Dispersion in Mineralöl) versetzt. Nach zehn Minuten Rühren unter Eiskühlung werden eine Lösung von 161 mg (0.45 mmol) 4-Chlor-6-(2-fluorphenyl)-5-(4-methoxyphenyl)furo[2,3-d]pyrimidin in 2 ml THF sowie 8 mg (0.02 mmol) Tetra-*n*-butylammoniumiodid zugegeben. Das Reaktionsgemisch wird 16 Stunden bei Raumtemperatur gerührt. Nach Zugabe von Wasser und Essigsäureethylester wird die abgetrennte organische Phase mit 1 N Salzsäure gewaschen und im Vakuum eingeengt. Der Rückstand wird in Acetonitril/DMSO aufgenommen und mittels präparativer RP-HPLC (Gradient: Wasser/Acetonitril) gereinigt. Es werden 114 mg (93% Reinheit, 45% d. Th.) des gewünschten Produkts erhalten.
LC-MS (Methode 8): Rₜ = 1.90 min.; m/z = 550 (M+H)⁺
¹H-NMR (400 MHz, DMSO-d₆): δ = 8.60 (s, 1H), 7.55-7.51 (m, 2H), 7.33-7.28 (m, 4H), 6.93-6.91 (m, 2H), 5.59-5.51 (m, 1H), 3.77 (s, 3H), 2.40-2.29 (m, 2H), 2.25-2.22 (m, 2H), 2.08 (s, 3H), 2.05-2.00 (m, 2H), 1.53-1.42 (m, 2H), 1.32 (s, 9H), 1.27 (d, 3H).

### Beispiel 102

### 6-{[5-(4-Ethylphenyl)-6-(2-fluorphenyl)furo[2,3-d]pyrimidin-4-yl]oxy}heptansäure-tert.-butylester

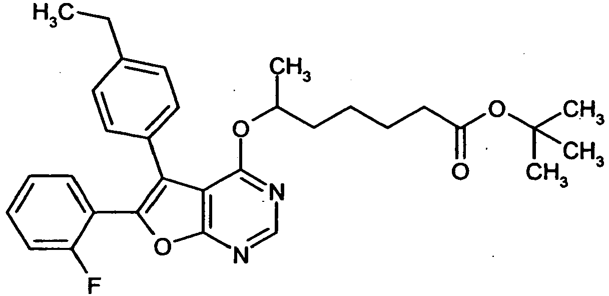

Eine Lösung von 200 mg (0.99 mmol) 6-Hydroxyheptansäure-*tert*.-butylester in 5 ml THF wird mit 49 mg (1.24 mmol) Natriumhydrid (60%-ige Dispersion in Mineralöl) versetzt. Nach zehn Minuten Rühren werden eine Lösung von 407 mg (90% Reinheit, 1.04 mmol) 4-Chlor-5-(4-ethylphenyl)-6-(2-fluorphenyl)furo[2,3-d]pyrimidin in 5 ml THF sowie 18 mg (0.05 mmol) Tetra-*n*-butylammoniumiodid zugegeben. Das Reaktionsgemisch wird 40 Stunden bei 75°C gerührt. Nach Zugabe von Wasser und Essigsäureethylester wird die abgetrennte organische Phase mit 1 N Salzsäure gewaschen und im Vakuum eingeengt. Der Rückstand wird in Acetonitril/DMSO aufgenommen und mittels präparativer RP-HPLC (Gradient: Wasser/Acetonitril) gereinigt. Es werden 104 mg (19% d. Th.) des gewünschten Produkts (Racemat) erhalten.
LC-MS (Methode 8): Rₜ = 3.59 min.; m/z = 519 (M+H)⁺
¹H-NMR (400 MHz, DMSO-d₆): δ = 8.61 (s, 1H), 7.55-7.51 (m, 2H), 7.33-7.27 (m, 4H), 7.20-7.18 (m, 2H), 5.39-5.31 (m, 1H), 2.63 (q, 2H), 2.08 (t, 2H), 1.60-1.50 (m, 2H), 1.45-1.37 (m, 2H), 1.34 (s, 9H), 1.28 (d, 3H), 1.24-1.16 (m, 5H).

### Beispiel 103

### (6R)-6-{[5-(4-Ethylphenyl)-6-(2-fluorphenyl)furo[2,3-d]pyrimidin-4-yl]oxy}heptansäure-tert.-butylester

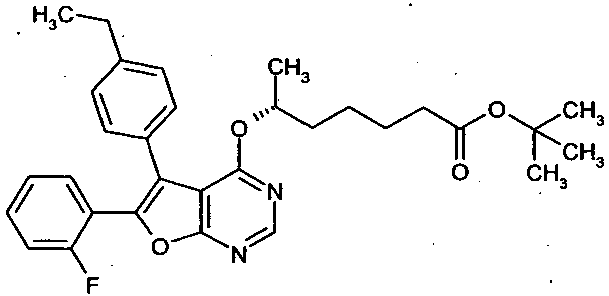

Eine Lösung von 350 mg (1.73 mmol) (*6R*)-6-Hydroxyheptansäure-*tert*.-butylester in 5 ml THF wird unter Eiskühlung mit 87 mg (2.16 mmol) Natriumhydrid (60%-ige Dispersion in Mineralöl) versetzt. Nach zehn Minuten Rühren unter Eiskühlung werden eine Lösung von 712 mg (90% Reinheit, 1.82 mmol) 4-Chlor-5-(4-ethylphenyl)-6-(2-fluorphenyl)furo[2,3-d]pyrimidin in 5 ml THF sowie 32 mg (0.09 mmol) Tetra-*n*-butylammoniumiodid zugegeben. Das Reaktionsgemisch wird 48 Stunden bei Raumtemperatur gerührt. Nach Zugabe von Wasser und Essigsäureethylester wird die abgetrennte organische Phase mit 1 N Salzsäure gewaschen und im Vakuum eingeengt. Der Rückstand wird in Acetonitril/DMSO aufgenommen und mittels präparativer RP-HPLC (Gradient: Wasser/Acetonitril) gereinigt. Es werden 459 mg (51% d. Th.) des gewünschten Produkts erhalten.
LC-MS (Methode 8): Rₜ = 3.51 min.; m/z = 519 (M+H)⁺
¹H-NMR (400 MHz, DMSO-d₆): δ = 8.61 (s, 1H), 7.55-7.51 (m, 2H), 7.33-7.27 (m, 4H), 7.20-7.18 (m, 2H), 5.39-5.31 (m, 1H), 2.63 (q, 2H), 2.08 (t, 2H), 1.60-1.50 (m, 2H), 1.45-1.37 (m, 2H), 1.34 (s, 9H), 1.28 (d, 3H), 1.24-1.16 (m, 5H).

### Beispiel 104

### (3-{[5-(4-Methoxyphenyl)-6-phenylfuro[2,3-d]pyrimidin-4-yl]oxy}-1-methylbutoxy)essigsäure-tert.-butylester

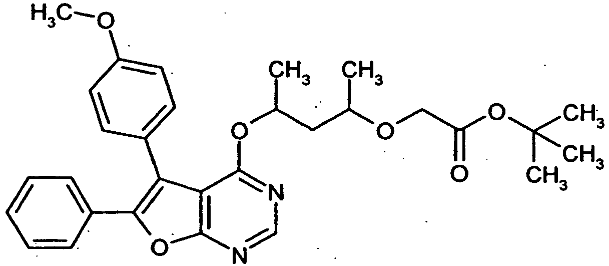

Eine Lösung von 2.19 g (5.41 mmol) 4-{[5-(4-Methoxyphenyl)-6-phenylfuro[2,3-d]pyrimidin-4-yl]oxy}pentan-2-ol in 20 ml Toluol wird mit 4.8 ml einer 11.25 N Natronlauge versetzt. Nach Zugabe von 184 mg (0.54 mmol) Tetra-*n*-butylammoniumhydrogensulfat und 2.11 g (10.83 mmol) Bromessigsäure-*tert*.-butylester wird das Reaktionsgemisch 15 h bei 70°C gerührt. Nach Abkühlen auf Raumtemperatur wird mit konzentrierter Salzsäure auf pH 7 eingestellt. Es wird dreimal mit je 50 ml Dichlormethan extrahiert. Die vereinigten organischen Extrakte werden mit ges. wässriger Natriumchlorid-Lösung gewaschen, über Natriumsulfat getrocknet und filtriert. Das Filtrat wird im Vakuum eingeengt. Der Rückstand wird in Essigsäureethylester aufgenommen und mittels Flash-Chromatographie an Kieselgel (Laufmittel: Essigsäureethylester/Methanol 1:0, 5:1) gereinigt. Es werden 0.08 g (92% Reinheit, 3% d. Th.) des gewünschten Produkts als racemisches Diastereomerengemisch erhalten.
LC-MS (Methode 8): Rₜ = 3.33 min.; m/z = 519 (M+H)⁺
¹H-NMR (400 MHz, DMSO-d₆): [Minder-Stereoisomer in Klammern] δ = 8.57 (s, 1H), [8.56, s, 1H], 7.55-7.51 (m, 2H), 7.43-7.37 (m, 5H), 7.04-7.01 (m, 2H), 5.54-5.46 (m, 1H), [5.39-5.30, m, 1H], 3.83-3.81 (m, 5H), 3.42-3.36 (m, 1H), 1.87-1.80 (m, 1H), 1.56-1.49 (m, 1H), 1.39 (s, 9H), [1.34, d, 3H], 1.27 (d, 3H), 1.00 (d, 3H), [0.89, d, 3H].

### Beispiel 105

### [2-({[5-(4-Ethylphenyl)-6-phenylfuro[2,3-d]pyrimidin-4-yl]oxy}methyl)-3,3-dimethylbutoxy]-essigsäure-tert.-butylester

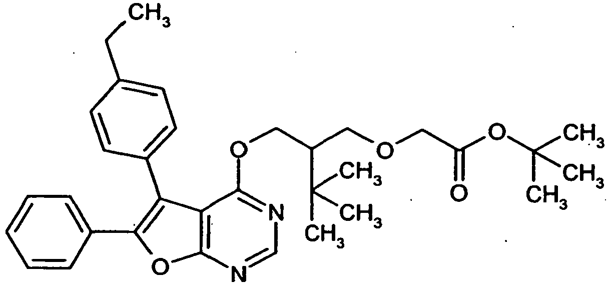

Eine Lösung von 265 mg (0.62 mmol) 2-({[5-(4-Ethylphenyl)-6-phenylfuro[2,3-d]pyrimidin-4-yl]-oxy}methyl)-3,3-dimethylbutan-1-ol in 10 ml Toluol wird mit 0.5 ml einer 11.25 N Natronlauge versetzt. Nach Zugabe von 21 mg (0.06 mmol) Tetra-*n*-butylammoniumhydrogensulfat und 240 mg (1.23 mmol) 2-Bromessigsäure-*tert*.-butylester wird das Reaktionsgemisch 16 h bei 70°C gerührt. Nach Abkühlen auf Raumtemperatur wird mit 1 N Salzsäure neutralisiert und mit Essigsäureethylester extrahiert. Die organische Phase wird mit ges. wässriger Natriumchlorid-Lösung gewaschen, über Natriumsulfat getrocknet und filtriert. Das Filtrat wird im Vakuum eingeengt. Der Rückstand wird mittels präparativer RP-HPLC (Gradient: Wasser/Acetonitril) gereinigt. Es werden 170 mg (51% d. Th.) des gewünschten Produkts (Racemat) erhalten.
LC-MS (Methode 9): Rₜ = 5.34 min.; m/z = 545 (M+H)⁺
¹H-NMR (400 MHz, DMSO-d₆): δ = 8.60 (s, 1H), 7.53-7.51 (m, 2H), 7.39-7.36 (m, 5H), 7.31-7.29 (m, 2H), 4.54-4.45 (m, 2H), 3.83 (dd, 2H), 3.30 (s, 2H), 2.69 (q, 2H), 1.55-1.49 (m, 1H), 1.39 (s, 9H), 1.24 (t, 3H), 0.73 (s, 9H).

### Beispiel 106

### 3-(2-{[5-(4-Methoxyphenyl)-6-phenylfuro[2,3-d]pyrimidin-4-yl]oxy}-1-methylpropoxy)propionsäure-tert.-butylester

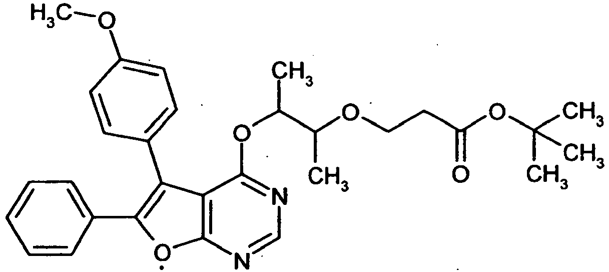

Eine Mischung aus 900 mg (2.31 mmol) 3-{(5-(4-Methoxyphenyl)-6-phenylfuro[2,3-d]pyrimidin-4-yl]oxy}butan-2-ol, 1477 mg (11.53 mmol) Acrylsäure-*tert*.-butylester und 157 mg (0.46 mmol) Tetra-*n*-butylammoniumhydrogensulfat in 10 ml Dichlorethan wird bei 0°C mit 2.2 ml 45%-iger Natronlauge versetzt und eine Stunde bei dieser Temperatur gerührt. Nach weiteren 16 Stunden bei Raumtemperatur wird das Reaktionsgemisch filtriert und das Filtrat im Vakuum eingeengt. Das Rohprodukt wird mittels präparativer RP-HPLC (Gradient: Wasser/Acetonitril) gereinigt. Es werden 690 mg (57% d. Th.) des gewünschten Produkts als (*R,S*/*S,R*)-Racemat erhalten.
LC-MS (Methode 7): Rₜ = 4.82 min.; m/z = 519 (M+H)⁺
¹H-NMR (400 MHz, DMSO-d₆): δ = 8.57 (s, 1H), 7.53-7.50 (m, 2H), 7.42-7.36 (m, 5H), 7.03-6.99 (m, 2H), 5.43-5.37 (m, 1H), 3.81 (s, 3H), 3.49-3.45 (m, 1H), 3.41 (t, 2H), 2.24 (t, 2H), 1.31 (s, 9H), 1.19 (d, 3H), 0.88 (d, 3H).

### Beispiel 107

### 6-({5-(4-Methoxyphenyl)-6-[2-(trifluormethyl)phenyl]furo[2,3-d]pyrimidin-4-yl}amino)hexansäuremethylester

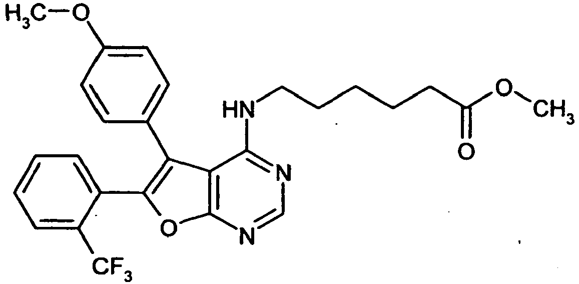

Ein Gemisch aus 224 mg (0.50 mmol) 6-{[6-Brom-5-(4-methoxyphenyl)furo[2,3-d]pyrimidin-4-yl]amino}hexansäuremethylester und 29 mg (0.03 mmol) Tetrakis(triphenylphosphin)palladium(0) in 2.5 ml 1,2-Dimethoxyethan wird mit 0.5 ml einer 2 M wässrigen Kaliumcarbonat-Lösung versetzt. Anschließend werden 119 mg (0.63 mmol) (2-Trifluormethyl)phenylboronsäure zugegeben und der Ansatz 15 h unter Rückfluss gerührt. Das Reaktionsgemisch wird filtriert und direkt mittels präparativer RP-HPLC (Gradient: Wasser/Acetonitril) gereinigt. Es werden 118 mg (46% d. Th.) des gewünschten Produkts erhalten.
LC-MS (Methode 2): Rₜ = 2.74 min.; m/z = 514 (M+H)⁺
¹H-NMR (400 MHz, DMSO-d₆): δ = 8.36 (s, 1H), 7.89 (dd, 1H), 7.69-7.63 (m, 2H), 7.44 (dd, 1H), 7.23 (d, 2H), 6.98 (d, 2H), 5.50 (t, NH), 3.76 (s, 3H), 3.57 (s, 3H), 3.41 (q, 2H), 2.29 (t, 2H), 1.55-1.44 (m, 4H), 1.28-1.19 (m, 2H).

### Beispiel 108

### 6-{[6-(2-Chlorphenyl)-5-(4-methoxyphenyl)furo[2,3-d]pyrimidin-4-yl]amino} hexansäuremethylester

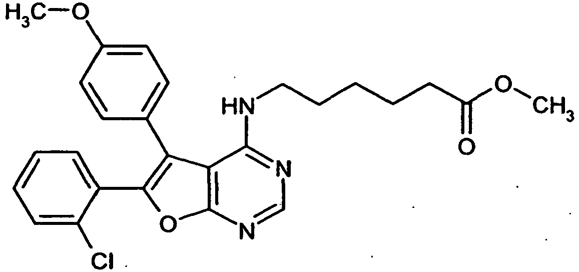

Ein Gemisch aus 220 mg (0.49 mmol) 6-{[6-Brom-5-(4-methoxyphenyl)furo[2,3-d]pyrimidin-4-yl]amino}hexansäuremethylester und 17 mg (0.03 mmol) Bis(triphenylphosphin)palladium(II)-chlorid in 2.2 ml Toluol wird mit 229 mg (1.08 mmol) Kaliumphosphat versetzt. Anschließend werden 176 mg (0.74 mmol) (2-Chlorphenyl)boronsäurepinacolester zugegeben und der Ansatz 15 h bei 80°C gerührt. Das Reaktionsgemisch wird direkt mittels präparativer RP-HPLC (Gradient: Wasser/Acetonitril) gereinigt. Es werden 101 mg (42% d. Th.) des gewünschten Produkts erhalten.
LC-MS (Methode 10): Rₜ = 2.81 min.; m/z = 481 (M+H)⁺
¹H-NMR (400 MHz, DMSO-d₆): δ = 8.36 (s, 1H), 7.58 (dd, 1H), 7.50-7.44 (m, 2H), 7.36 (dd, 1H), 7.24 (d, 2H), 6.98 (d, 2H), 5.54 (t, NH), 3.77 (s, 3H), 3.58 (s, 3H), 3.42 (q, 2H), 2.29 (t, 2H), 1.55-1.45 (m, 4H), 1.27-1.19 (m, 2H).

### Beispiel 109

### 6-{[6-(2,6-Difluorphenyl)-5-(4-methoxyphenyl)furo[2,3-d]pyrimidin-4-yl]lamino}hexansäuremethylester

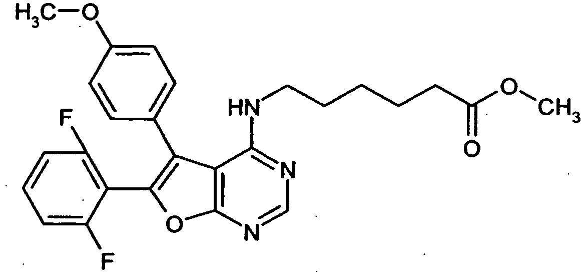

150 mg (0.34 mmol) 6-{[6-Brom-5-(4-methoxyphenyl)furo[2,3-d]pyrimidin-4-yl]amino}hexansäuremethylester und 106 mg (0.70 mmol) (2,6-Difluorphenyl)boronsäure werden in 3.5 ml Toluol sowie 1.0 ml Ethanol gelöst und mit 0.34 ml einer 2 M wässrigen Natriumcarbonat-Lösung sowie 25 mg (0.03 mmol) 1,1'-Bis(diphenylphosphano)ferrocenpalladium(II)chlorid versetzt. Anschließend wird 15 h bei 70°C gerührt. Das Reaktionsgemisch wird direkt mittels präparativer RP-HPLC (Gradient: Wasser/Acetonitril) gereinigt. Es werden 13 mg (8% d. Th.) des gewünschten Produkts erhalten.
LC-MS (Methode 10): Rₜ = 2.72 min.; m/z = 482 (M+H)⁺
¹H-NMR (300 MHz, DMSO-d₆): δ = 8.37 (s, 1H), 7.63-7.57 (m, 1H), 7.26-7.10 (m, 4H), 6.98 (d, 2H), 5.65 (t, NH), 3.78 (s, 3H), 3.57 (s, 3H), 3.42 (q, 2H), 2.29 (t, 2H), 1.55-1.47 (m, 4H), 1.28-1.24 (m, 2H).

### Beispiel 110

### 6-{[6-(2-Methoxyphenyl)-5-(4-methoxyphenyl)furo[2,3-d]pyrimidin-4-yl]amino}hexansäuremethylester

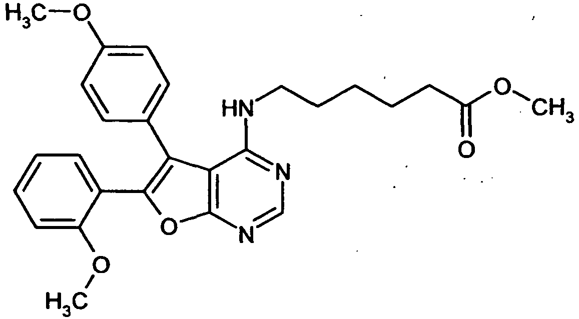

Ein Gemisch aus 200 mg (0.45 mmol) 6-{[6-Brom-5-(4-methoxyphenyl)furo[2,3-dipyrimidin-4-yl]amino}hexansäuremethylester und 16 mg (0.02 mmol) Bis(triphenylphosphin)palladium(II)-chlorid in 10 ml Dimethylsulfoxid wird mit 0.45 ml einer 2 M wässrigen Natriumcarbonat-Lösung versetzt. Anschließend werden 85 mg (0.56 mmol) (2-Methoxyphenyl)boronsäure zugegeben und der Ansatz 15 h bei 80°C gerührt. Das Reaktionsgemisch wird filtriert und direkt mittels präparativer RP-HPLC (Gradient: Wasser/Acetonitril) gereinigt. Es werden 63 mg (42% d. Th.) des gewünschten Produkts erhalten.
LC-MS (Methode 10): Rₜ = 2.72 min.; m/z = 476 (M+H)⁺
¹H-NMR (400 MHz, DMSO-d₆): δ = 8.32 (s, 1H), 7.42 (dd, 1H), 7.30 (dd, 1H), 7.23 (d, 2H), 7.05 (d, 1H), 7.00-6.94 (m, 3H), 5.37 (t, NH), 3.78 (s, 3H), 3.58 (s, 3H), 3.54 (s, 3H), 3.41 (q, 2H), 2.28 (t, 2H), 1.54-1.43 (m, 4H), 1.26-1.18 (m, 2H).

### Beispiel 111

### 6-{[5-(4-Methoxyphenyl)-6-(2-vinylphenyl)furo[2,3-d]pyrimidin-4-yl]amino}hexansäuremethylester

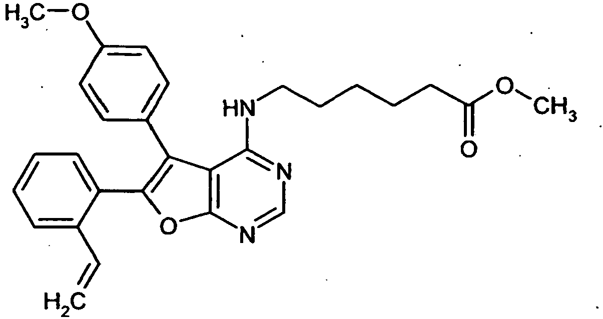

Ein Gemisch aus 224 mg (0.50 mmol) 6-{[6-Brom-5-(4-methoxyphenyl)furo[2,3-d]pyrimidin-4-yl]amino}hexansäuremethylester und 29 mg (0.03 mmol) Tetrakis(triphenylphosphin)palladium(0) in 2.5 ml 1,2-Dimethoxyethan wird mit 0.5 ml einer 2 M wässrigen Kaliumcarbonat-Lösung versetzt. Anschließend werden 92 mg (0.63 mmol) (2-Vinylphenyl)boronsäure zugegeben und der Ansatz 15 h unter Rückfluss gerührt. Das Reaktionsgemisch wird filtriert und direkt mittels präparativer RP-HPLC (Gradient: Wasser/Acetonitril) gereinigt. Es werden 82 mg (35% d. Th.) des gewünschten Produkts erhalten.
LC-MS (Methode 2): Rₜ = 2.77 min.; m/z = 472 (M+H)⁺
¹H-NMR (400 MHz, DMSO-d₆): δ = 8.35 (s, 1H), 7.70 (d, 1H), 7.46-7.42 (m, 1H), 7.30 (d, 2H), 7.23 (d, 2H), 6.97 (d, 2H), 6.61 (dd, 1H), 5.72 (d, 1H), 5.48 (t, NH), 5.17 (d, 1H), 3.76 (s, 3H), 3.58 (s, 3H), 3.42 (q, 2H), 2.29 (t, 2H), 1.55-1.46 (m, 4H), 1.27-1.22 (m, 2H).

### Beispiel 112

### 6-{[6-(2-Ethylphenyl)-5-(4-methoxyphenyl)furo[2,3-d]pyrimidin-4-yl]amino}hexansäuremethylester

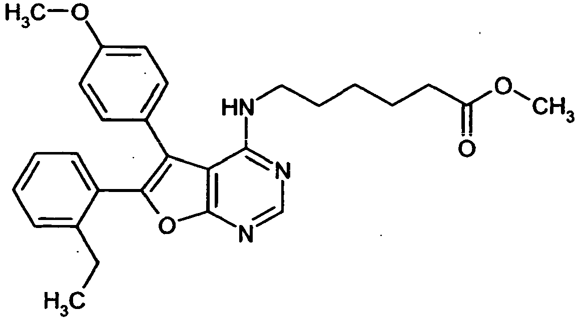

Ein Gemisch aus 224 mg (0.50 mmol) 6-{[6-Brom-5-(4-methoxyphenyl)furo[2,3-d]pyrimidin-4-yl]amino}hexansäuremethylester und 18 mg (0.03 mmol) Bis(triphenylphosphin)palladium(II)-chlorid in 11.2 ml DMSO wird mit 0.50 ml einer 2 M wässrigen Natriumcarbonat-Lösung versetzt. Anschließend werden 187 mg (1.25 mmol) (2-Ethylphenyl)boronsäure zugegeben und der Ansatz 15 h bei 80°C gerührt. Das Reaktionsgemisch wird filtriert und direkt mittels präparativer RP-HPLC (Gradient: Wasser/Acetonitril) gereinigt. Es werden 69 mg (29% d. Th.) des gewünschten Produkts erhalten.
LC-MS (Methode 2): Rₜ = 2.83 min.; m/z = 474 (M+H)⁺
¹H-NMR (400 MHz, DMSO-d₆): δ = 8.33 (s, 1H), 7.39-7.31 (m, 2H), 7.26-7.17 (m, 4H), 6.98 (d, 2H), 5.42 (t, NH), 3.76 (s, 3H), 3.58 (s, 3H), 3.41 (q, 2H), 2.49 (q, 2H), 2.29 (t, 2H), 1.55-1.44 (m, 4H), 1.27-1.19 (m, 2H), 1.00 (t, 3H).

### Beispiel 113

### 6-{[6-(2-Fluorphenyl)-5-(4-methoxyphenyl)furo[2,3-d]pyrimidin-4-yl]amino}hexansäuremethylester

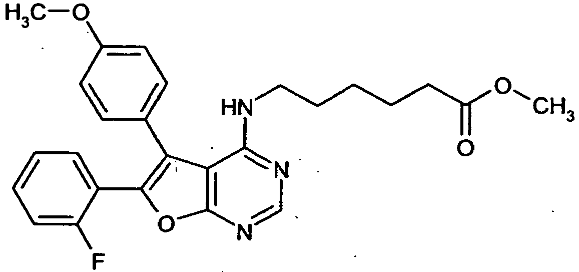

Ein Gemisch aus 100 mg (0.22 mmol) 6-{[6-Brom-5-(4-methoxyphenyl)furo[2,3-d]pyrimidin-4-yl]amino}hexansäuremethylester und 8 mg (0.01 mmol) Bis(triphenylphosphin)palladium(II)-chlorid in 5.0 ml DMSO wird mit 0.22 ml einer 2 M wässrigen Natriumcarbonat-Lösung versetzt. Anschließend werden 39 mg (0.28 mmol) (2-Fluorphenyl)boronsäure zugegeben und der Ansatz 15 h bei 80°C gerührt. Das Reaktionsgemisch wird filtriert und direkt mittels präparativer RP-HPLC (Gradient: Wasser/Acetonitril) gereinigt. Es werden 69 mg (29% d. Th.) des gewünschten Produkts erhalten.
LC-MS (Methode 5): Rₜ = 2.82 min.; m/z = 464 (M+H)⁺
¹H-NMR (400 MHz, DMSO-d₆): δ = 8.35 (s, 1H), 7.48-7.41 (m, 2H), 7.31 (d, 2H), 7.26-7.21 (m, 2H), 7.03 (d, 2H), 5.43 (t, NH), 3.80 (s, 3H), 3.58 (s, 3H), 3.41 (q, 2H), 2.29 (t, 2H), 1.54-1.42 (m, 4H), 1.22-1.18 (m, 2H).

### Beispiel 114

### 6-{[5-(4-Methoxyphenyl)-6-(2-methylphenyl)furo[2,3-d]pyrimidin-4-yl]amino}hexansäuremethylester

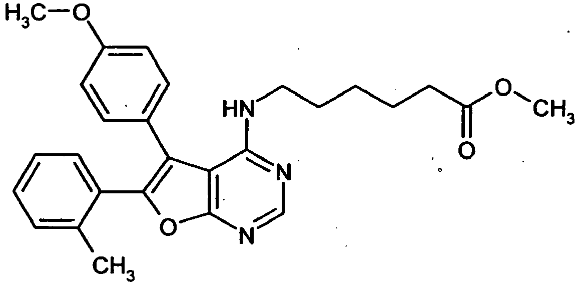

Ein Gemisch aus 224 mg (0.50 mmol) 6-{[6-Brom-5-(4-methoxyphenyl)furo[2,3-d]pyrimidin-4-yl]amino}hexansäuremethylester und 29 mg (0.03 mmol) Tetrakis(triphenylphosphin)palladium(0) in 2,5 ml 1,2-Dimethoxyethan wird mit 0.5 ml einer 2 M wässrigen Kaliumcarbonat-Lösung versetzt. Anschließend werden 85 mg (0.63 mmol) (2-Methylphenyl)boronsäure zugegeben und der Ansatz 15 h unter Rückfluss gerührt. Das Reaktionsgemisch wird filtriert und direkt mittels präparativer RP-HPLC (Gradient: Wasser/Acetonitril) gereinigt. Es werden 68 mg (30% d. Th.) des gewünschten Produkts erhalten.
LC-MS (Methode 8): Rₜ = 2.97 min.; m/z = 460 (M+H)⁺
¹H-NMR (400 MHz, DMSO-d₆): δ = 8.32 (s, 1H), 7.34-7.14 (m, 6H), 6.98 (d, 2H), 5.44 (t, NH), 3.77 (s, 3H), 3.57 (s, 3H), 3.42 (q, 2H), 2.29 (t, 2H), 2.10 (s, 3H), 1.55-1.44 (m, 4H), 1.27-1.19 (m, 2H).

### Beispiel 115

### 6-{[6-(2-Fluor-6-methoxyphenyl)-5-(4-methoxyphenyl)furo[2,3-d]pyrimidin-4-yl]amino}hexansäuremethylester

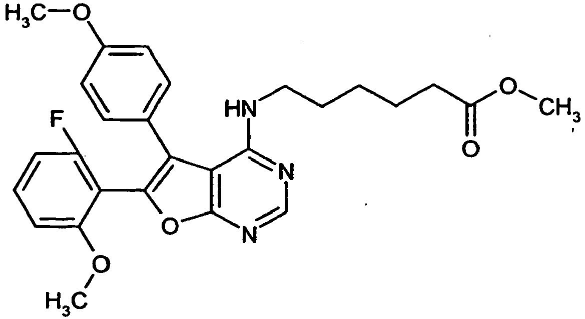

150 mg (0.34 mmol) 6-{[6-Brom-5-(4-methoxyphenyl)furo[2,3-d]pyrimidin-4-yl]amino}hexansäuremethylester und 142 mg (0.84 mmol) (2-Fluor-6-methoxyphenyl)boronsäure werden in 2.0 ml 1,2-Dimethoxyethan gelöst und mit 0.34 ml einer 2 M wässrigen Natriumcarbonat-Lösung sowie 24 mg (0.03 mmol) 1,1'-Bis(diphenylphosphano)ferrocenpalladium(II)chlorid versetzt. Anschließend wird 15 h bei 80°C gerührt. Das Reaktionsgemisch wird direkt mittels präparativer RP-HPLC (Gradient: Wasser/Acetonitril) gereinigt. Es werden 56 mg (34% d. Th.) des gewünschten Produkts erhalten.
LC-MS (Methode 2): Rₜ = 2.57 min.; m/z = 494 (M+H)⁺
¹H-NMR (400 MHz, DMSO-d₆): δ = 8.33 (s, 1H), 7.48 (dd, 1H), 7.20 (d, 2H), 6.98-6.94 (m, 3H), 6.86 (dd, 1H), 5.55 (t, NH), 3.76 (s, 3H), 3.67 (s, 3H), 3.57 (s, 3H), 3.42 (q, 2H), 2.29 (t, 2H), 1.55-1.45 (m, 4H), 1.28-1.21 (m, 2H).

### Beispiel 116

### 6-({5-(4-Methoxyphenyl)-6-[2-(trifluormethyl)phenyl]furo[2,3-d]pyrimidin-4-yl}amino)hexansäure

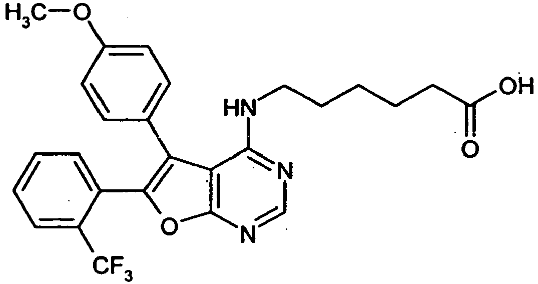

85 mg (0.17 mmol) 6-({5-(4-Methoxyphenyl)-6-[2-(trifluormethyl)phenyl]furo[2,3-d]pyrimidin-4-yl}amino)hexansäuremethylester werden in 2.5 ml Dioxan gelöst und mit 0.5 ml 1 N Natronlauge versetzt. Es wird 16 h bei RT gerührt, dann 0.5 ml 1 N Salzsäure zugegeben und mit 6 ml Essigsäureethylester versetzt. Die organische Phase wird abgetrennt, über Natriumsulfat getrocknet, filtriert und eingeengt. Es werden 68 mg (82% d. Th.) der Zielverbindung erhalten.
LC-MS (Methode 2): Rₜ = 2.38 min.; m/z = 514 (M+H)⁺
¹H-NMR (400 MHz, DMSO-d₆): δ = 11.98 (s, 1H), 8.35 (s, 1H), 7.89 (dd, 1H), 7.69-7.63 (m, 2H), 7.44 (dd, 1H), 7.23 (d, 2H), 6.98 (d, 2H), 5.50 (t, NH), 3.76 (s, 3H), 3.42 (q, 2H), 2.19 (t, 2H), 1.52-1.44 (m, 4H), 1.27-1.20 (m, 2H).

### Beispiel 117

### 6-{[6-(2-Chlorphenyl)-5-(4-methoxyphenyl)furo[2,3-d]pyrimidin-4-yl]amino}hexansäure

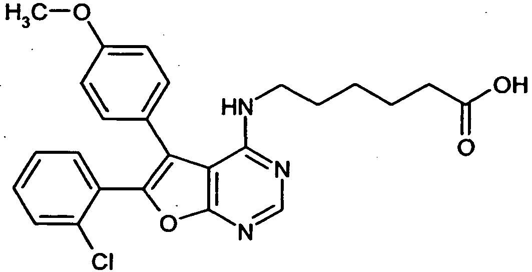

65 mg (0.14 mmol) 6-{[6-(2-Chlorphenyl)-5-(4-methoxyphenyl)furo[2,3-d]pyrimidin-4-yl]amino}-hexansäuremethylester werden in 2.5 ml Dioxan gelöst und mit 0.5 ml 1 N Natronlauge versetzt. Es wird 16 h bei RT gerührt, dann 0.5 ml 1 N Salzsäure zugegeben und mit 6 ml Essigsäureethylester versetzt. Die organische Phase wird abgetrennt, über Natriumsulfat getrocknet, filtriert und eingeengt. Es werden 44 mg (70% d. Th.) der Zielverbindung erhalten.
LC-MS (Methode 2): Rₜ = 2.34 min.; m/z = 467 (M+H)⁺
¹H-NMR (400 MHz, DMSO-d₆): δ = 12.00 (s, 1H), 8.36 (s, 1H), 7.56 (d, 1H), 7.49-7.44 (m, 2H), 7.39-7.35 (m, 1H), 7.24 (d, 2H), 6.99 (d, 2H), 5.55 (t, NH), 3.77 (s, 3H), 3.42 (q, 2H), 2.20 (t, 2H), 1.53-1.45 (m, 4H), 1.27-1.20 (m, 2H).

### Beispiel 118

### 6-{[6-(2-Methoxyphenyl)-5-(4-methoxyphenyl)furo[2,3-d]pyrimidin-4-yl]amino}hexansäure

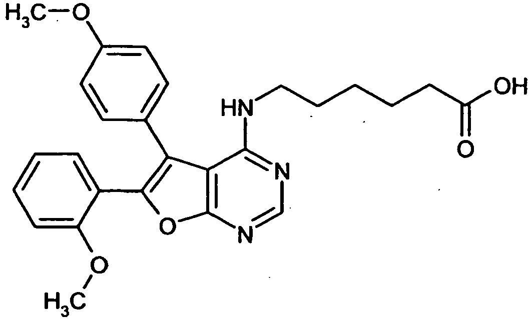

55 mg (0.12 mmol) 6-{[6-(2-Methoxyphenyl)-5-(4-methoxyphenyl)furo[2,3-d]pyrimidin-4-yl]-amino}hexansäuremethylester werden in 2.5 ml Dioxan gelöst und mit 0.5 ml 1 N Natronlauge versetzt. Es wird 16 h bei RT gerührt, dann 0.5 ml 1 N Salzsäure zugegeben und mit 6 ml Essigsäureethylester versetzt. Die organische Phase wird abgetrennt, über Natriumsulfat getrocknet, filtriert und eingeengt. Es werden 42 mg (77% d. Th.) der Zielverbindung erhalten.
LC-MS (Methode 2): Rₜ = 2.25 min.; m/z = 462 (M+H)⁺
¹H-NMR (300 MHz, DMSO-d₆): δ = 12.00 (s, 1H), 8.32 (s, 1H), 7.42-7.37 (m, 1H), 7.29 (dd, 1H), 7.23 (d, 2H), 7.05 (d, 1H), 6.99 (d, 2H), 6.97-6.93 (m, 1H), 5.37 (t, NH), 3.77 (s, 3H), 3.41 (q, 2H), 2.20 (t, 2H), 1.53-1.43 (m, 4H), 1.27-1.20 (m, 2H).

### Beispiel 119

### 6-{[5-(4-Methoxyphenyl)-6-(2-vinylphenyl)furo[2,3-d]pyrimidin-4-yl]amino}hexansäure

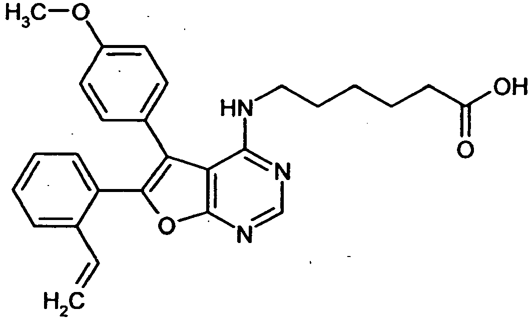

Die Titelverbindung entsteht als Nebenprodukt bei der Synthese von 6-{[5-(4-Methoxyphenyl)-6-(2-vinylphenyl)furo[2,3-d]pyrimidin-4-yl]amino}hexansäuremethylester (Beispiel 111) und wird mittels präparativer RP-HPLC (Gradient: Wasser/Acetonitril) isoliert. Es werden 36 mg (16% d. Th.) der Titelverbindung erhalten.
LC-MS (Methode 10): Rₜ = 2.58 min.; m/z= 458 (M+H)⁺
¹H-NMR (400 MHz, DMSO-d₆): δ = 8.33 (s, 1H), 7.69 (d, 1H), 7.45-7.40 (m, 1H), 7.31-7.26 (m, 2H), 7.22 (d, 2H), 6.98 (d, 2H), 6.61 (dd, 1H), 5.70 (d, 1H), 5.41 (t, NH), 5.15 (d, 1H), 3.76 (s, 3H), 3.41 (q, 2H), 1.90 (t, 2H), 1.48-1.36 (m, 4H), 1.22-1.15 (m, 2H).

### Beispiel 120

### 6-{[6-(2-Ethylphenyl)-5-(4-methoxyphenyl)furo[2,3-d]pyrimidin-4-yl]amino}hexansäure

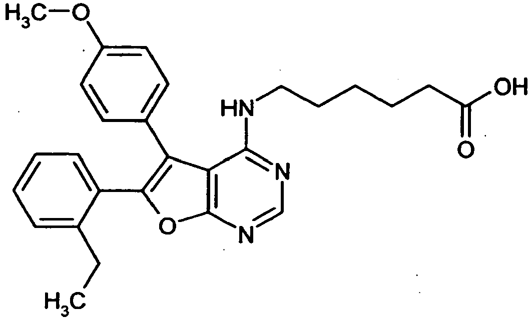

45 mg (0.10 mmol) 6-{[6-(2-Ethylphenyl)-5-(4-methoxyphenyl)furo[2,3-d]pyrimidin-4-yl]amino}-hexansäuremethylester werden in 2.0 ml Dioxan gelöst und mit 0.5 ml 1 N Natronlauge versetzt. Es wird 16 h bei RT gerührt, dann 0.5 ml 1 N Salzsäure zugegeben und mit 5 ml Essigsäureethylester versetzt. Die organische Phase wird abgetrennt, über Natriumsulfat getrocknet, filtriert und eingeengt. Es werden 38 mg (87% d. Th.) der Zielverbindung erhalten.
LC-MS (Methode 10): Rₜ = 2.58 min.; m/z = 460 (M+H)⁺
¹H-NMR (400 MHz, DMSO-d₆): δ = 12.00 (br. s, 1H), 8.33 (s, 1H), 7.39-7.31 (m, 2H), 7.26-7.16 (m, 4H), 6.98 (d, 2H), 5.43 (t, NH), 3.76 (s, 3H), 3.41 (q, 2H), 2.49 (q, 2H), 2.19 (t, 2H), 1.52-1.45 (m, 4H), 1.28-1.16 (m, 2H), 1.00 (t, 3H).

### Beispiel 121

### 6-{[6-(2-Fluorphenyl)-5-(4-methoxyphenyl)furo[2,3-d]pyrimidin-4-yl]amino}hexansäure

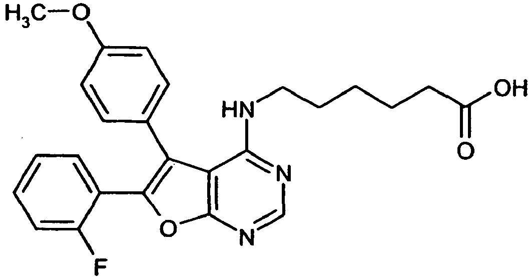

25 mg (0.05 mmol) 6-{[6-(2-Fluorphenyl)-5-(4-methoxyphenyl)furo[2,3-d]pyrimidin-4-yl]amino}-hexansäuremethylester werden in 1.0 ml Dioxan gelöst und mit 0.16 ml 1 N Natronlauge versetzt. Es wird 16 h bei RT gerührt, dann 0.17 ml 1 N Salzsäure zugegeben und mit 2 ml Wasser sowie 5 ml Dichlormethan versetzt. Die organische Phase wird abgetrennt, über Natriumsulfat getrocknet, filtriert und eingeengt. Es werden 23 mg (92% d. Th.) der Zielverbindung erhalten.
LC-MS (Methode 8): Rₜ = 2.52 min.; m/z = 450 (M+H)⁺
¹H-NMR (400 MHz, DMSO-d₆): δ =.12.01 (br. s, 1H), 8.35 (s, 1H), 7.49-7.42 (m, 2H), 7.31 (d, 2H), 7.26-7.21 (m, 2H), 7.03 (d, 2H), 5.41 (t, NH), 3.80 (s, 3H), 3.41 (q, 2H), 2.29 (t, 2H), 1.51-1.42 (m, 4H), 1.27-1.18 (m, 2H).

### Beispiel 122

### 6-{[5-4-Methoxyphenyl)-6-(2-methylphenyl)furo[2,3-d]pyrimidin-4-yl]amino}hexansäure

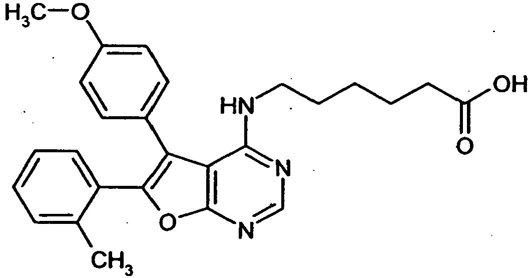

65 mg (0.14 mmol) 6-{[5-(4-Methoxyphenyl)-6-(2-methylphenyl)furo[2,3-d]pyrimidin-4-yl]-amino}hexansäuremethylester werden in 2.5 ml Dioxan gelöst und mit 0.50 ml 1 N Natronlauge versetzt. Es wird 16 h bei RT gerührt, dann 0.50 ml 1 N Salzsäure zugegeben und mit 6 ml Essigsäuremethylester versetzt. Die organische Phase wird abgetrennt, über Natriumsulfat getrocknet, filtriert und eingeengt. Es werden 53 mg (82% d. Th.) der Zielverbindung erhalten.
LC-MS (Methode 2): Rₜ = 2.35 min.; m/z = 446 (M+H)⁺
¹H-NMR (400 MHz, DMSO-d₆): δ = 12.00 (br. s, 1H), 8.34 (s, 1H), 7.34-7.16 (m, 6H), 6.98 (d, 2H), 5.45 (t, NH), 3.77 (s, 3H), 3.42 (q, 2H), 2.29 (t, 2H), 2.10 (s, 3H), 1.50-1.45 (m, 4H), 1.28-1.20 (m, 2H).

### Beispiel 123

### 6-{[6-(2-Fluor-6-methoxyphenyl)-5-(4-methoxyphenyl)furo[2,3-d]pyrimidin-4-yl]amino}hexansäure

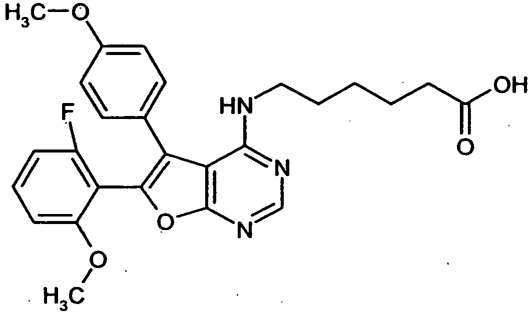

42 mg (0.09 mmol) 6-{[6-(2-Fluor-6-methoxyphenyl)-5-(4-methoxyphenyl)furo[2,3-d]pyrimidin-4-yl]amino}hexansäuremethylester werden in 1.0 ml Dioxan gelöst und mit 0.26 ml 1 N Natronlauge versetzt. Es wird 16 h bei RT gerührt, dann 0.26 ml 1 N Salzsäure zugegeben und mit 2 ml Wasser sowie 5 ml Dichlormethan versetzt. Die organische Phase wird abgetrennt, über Natriumsulfat getrocknet, filtriert und eingeengt. Es werden 39 mg (96% d. Th.) der Zielverbindung erhalten.
LC-MS (Methode 2): Rₜ = 2.22 min.; m/z = 480 (M+H)⁺
¹H-NMR (400 MHz, DMSO-d₆): δ = 11.99 (s, 1H), 8.33 (s, 1H), 7.48 (dd, 1H), 7.20 (d, 2H), 6.98-6.94 (m, 3H), 6.85 (dd, 1H), 5.55 (t, NH), 3.76 (s, 3H), 3.68 (s, 3H), 3.42 (q, 2H), 2.19 (t, 2H), 1.53-1.45 (m, 4H), 1.29-1.21 (m, 2H).

### Beispiel 124

### (3-{[5-(4-Methoxyphenyl)-6-phenylfuro[2,3-d]pyrimidin-4-yl]oxy}-2-methylpropoxy)essigsäure-tert.-butylester

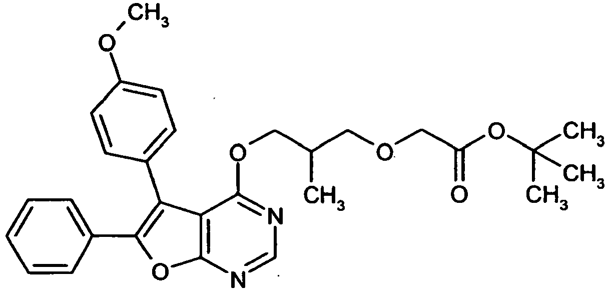

Eine Lösung von 500 mg (1.28 mmol) 3-{[5-(4-Methoxyphenyl)-6-phenylfuro[2,3-d]pyrimidin-4-yl]oxy}-2-methylpropan-1-ol in 10 ml Toluol wird bei 70°C mit 1.14 ml einer 12.5 N Natronlauge versetzt. Nach Zugabe von 44 mg (0.13 mmol) Tetra-*n*-butylammoniumhydrogensulfat und 500 mg (2.56 mmol) Bromessigsäure-*tert*.-butylester wird das Reaktionsgemisch 20 h bei 70°C gerührt. Nach Abkühlen auf Raumtemperatur wird mit konzentrierter Salzsäure auf pH 7 eingestellt und dreimal mit je 50 ml Dichlormethan extrahiert. Die vereinigten organischen Extrakte werden mit ges. wässriger Natriumchlorid-Lösung gewaschen, über Natriumsulfat getrocknet, filtriert und im Vakuum eingeengt. Das Rohprodukt wird mittels präparativer RP-HPLC (Gradient: Wasser/Acetonitril) gereinigt. Es werden 298 mg (46% d. Th.) des gewünschten Produkts als Racemat erhalten.
LC-MS (Methode 8): Rₜ = 3.41 min.; m/z = 505 (M+H)⁺
¹H-NMR (400 MHz, DMSO-d₆): δ = 8.58 (s, 1H), 7.54 (dd, 2H), 7.51-7.37 (m, 5H), 7.03 (d, 2H), 4.35-4.26 (m, 2H), 3.85 (s, 2H), 3.82 (s, 3H), 3.19 (d, 2H), 2.02-1.97 (m, 1H), 1.39 (s, 9H), 0.76 (d, 3H).

### Beispiel 125

### (3-{[5-(4-Methoxyphenyl)-6-phenylfuro[2,3-d]pyrimidin-4-yl]oxy}-2-methylpropoxy)essigsäure-tert.-butylester (Enantiomer 1)

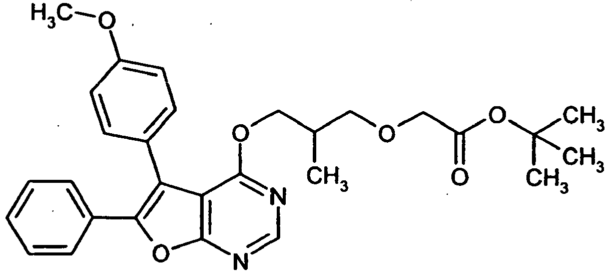

298 mg (0.59 mmol) *rac*.-(3-{[5-(4-Methoxyphenyl)-6-phenylfuro[2,3-d]pyrimidin-4-yl]oxy}-2-methylpropoxy)essigsäure-*tert*.-butylester werden mittels Chromatographie an chiraler Phase in die Enantiomere getrennt [Säule: Daicel Chiralpak IA, 250 mm x 20 mm; Fluss: 15 ml/min; Detektion: 220 nm; Temperatur: 30°C; Eluent: 50% iso-Hexan / 50% *tert*.-Butylmethylether]. Auf diese Weise werden 51 mg (17% d. Th.) von Enantiomer 1 erhalten.
HPLC: Rₜ = 7.46 min. [Säulenmaterial wie oben, 250 mm x 4.6 mm; Fluss: 1 ml/min; Eluent: 50% iso-Hexan / 50% tert.-Butylmethylether; Temperatur: 25°C]
LC-MS (Methode 2): Rₜ = 3.14 min.; m/z = 505 (M+H)⁺
¹H-NMR (400 MHz, DMSO-d₆): δ = 8.58 (s, 1H), 7.55-7.54 (m, 2H), 7.42-7.38 (m, 5H), 7.04-7.03 (m, 2H), 4.35-4.27 (m, 2H), 3.83 (s, 3H), 1.39 (s, 9H), 0.77 (d, 3H).

### Beispiel 126

### (3-{[5-(4-Methoxyphenyl)-6-phenylfuro[2,3-d]pyrimidin-4-yl]oxy}-2-methylpropoxy)essigsäure-tert.-butylester (Enantiomer 2)

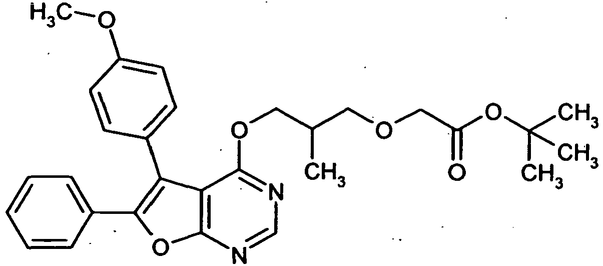

298 mg (0.59 mmol) *rac*.-(3-{[5-(4-Methoxyphenyl)-6-phenylfuro[2,3-d]pyrimidin-4-yl]oxy}-2-methylpropoxy)essigsäure-*tert.*-butylester werden mittels Chromatographie an chiraler Phase in die Enantiomere getrennt [Säule: Daicel Chiralpak IA, 250 mm x 20 mm; Fluss: 15 ml/min; Detektion: 220 nm; Temperatur: 30°C; Eluent: 50% iso-Hexan / 50% *tert.*-Butylmethylether]. Auf diese Weise werden 56 mg (19% d. Th.) von Enantiomer 2 erhalten.
HPLC: Rₜ = 7.94 min. [Säulenmaterial wie oben, 250 mm x 4.6 mm; Fluss: 1 ml/min; Eluent: 50% iso-Hexan / 50% *tert.*-Butylmethylether; Temperatur: 25°C]
LC-MS (Methode 8): Rₜ = 3.36 min.; m/z = 505 (M+H)⁺
¹H-NMR (400 MHz, DMSO-d₆): δ = 8.58 (s, 1H), 7.55-7.54 (m, 2H), 7.42-7.38 (m, 5H), 7.04-7.03 (m, 2H); 4.35-4.27 (m, 2H), 3.83 (s, 3H), 1.39 (s, 9H), 0:77 (d, 3H).

### Beispiel 127

### (3-{[5-(4-Methoxyphenyl)-6-phenylfuro[2,3-d]pyrimidin-4-yl]oxy}-2-methylpropoxy)essigsäure

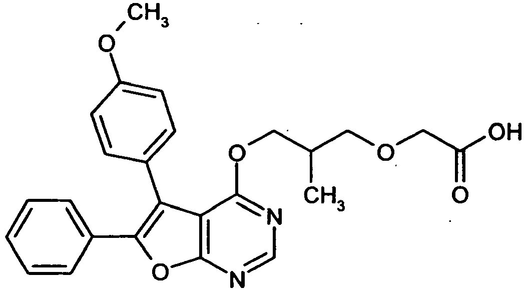

70 mg (0.14 mmol) *rac*.-(3-{[5-(4-Methoxyphenyl)-6-phenylfuro[2,3-d]pyrimidin-4-yl]oxy}-2-methylpropoxy)essigsäure-*tert*.-butylester werden in 2.0 ml 4 N Chlorwasserstoff in Dioxan gelöst und 16 h bei RT gerührt. Es wird direkt mittels präparativer RP-HPLC (Gradient: Wasser/Acetonitril) aufgereinigt. Es werden 48 mg (76% d. Th.) des gewünschten Produkts als Racemat erhalten.
LC-MS (Methode 7): Rₜ = 3.86 min.; m/z = 449 (M+H)⁺
¹H-NMR (400 MHz, DMSO-d₆): δ = 12.64 (br. s, 1H), 8.58 (s, 1H), 7.55 (d, 2H), 7.41-7.36 (m, 5H), 7.03 (d, 2H), 4.35-4.27 (m, 2H), 3.88 (s, 2H), 3.81 (s, 3H), 3.19 (d, 2H), 2.01-1.97 (m, 1H), 0.76 (d, 3H).

### Beispiel 128

### (+)-(3-{[5-(4-Methoxyphenyl)-6-phenylfuro[2,3-d]pyrimidin-4-yl]oxy}-2-methylpropoxy)essigsäure (Enantiomer 1)

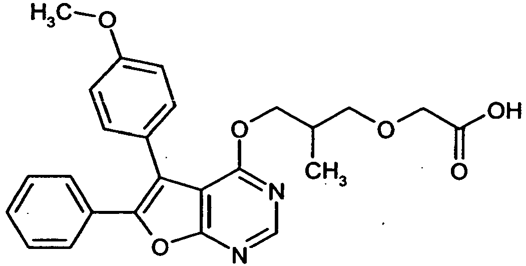

41 mg (0.08 mmol) (3-{[5-(4-Methoxyphenyl)-6-phenylfuro[2,3-d]pyrimidin-4-yl]oxy}-2-methyl-propoxy)essigsäure-*tert*.-butylester (Enantiomer 1) werden in 0.5 ml Dioxan gelöst, mit 0.2 ml 4 N Chlorwasserstoff in Dioxan versetzt und 48 h bei RT gerührt. Die Reaktionslösung wird danach mit weiteren 0.4 ml 4 N Chlorwasserstoff in Dioxan versetzt und erneut 16 h bei RT gerührt. Nach Einengen der Reaktionslösung im Vakuum wird der Rückstand mittels präparativer RP-HPLC (Gradient: Wasser/Acetonitril) aufgereinigt. Es werden 27 mg (74% d. Th.) des gewünschten Produkts als Enantiomer 1 erhalten.
LC-MS (Methode 8): Rₜ = 2.67 min.; m/z = 449 (M+H)⁺
¹H-NMR (400 MHz, DMSO-d₆): δ = 8.57 (s, 1H), 7.56-7.53 (m, 2H), 7.42-7.37 (m, 5H), 7.05-7.01 (m, 2H), 4.41-4.37 (m, 1H), 4.24-4.20 (m, 1H), 3.82 (s, 3H), 3.42 (s, 2H), 3.21-3.14 (m, 2H), 2.00-1.93 (m, 1H), 0.71 (d, 3H)
[α]_{D}²⁰ = +21°, c = 0.400, Chloroform.

### Beispiel 129

### (-)-(3-{[5-(4-Methoxyphenyl)-6-phenylfuro[2,3-d]pyrimidin-4-yl)oxy}-2-methylpropoxy)essigsäure (Enantiomer 2)

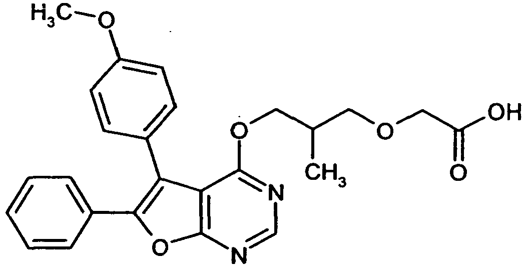

45 mg (0.09 mmol) (3-{[5-(4-Methoxyphenyl)-6-phenylfuro[2,3-d]pyrimidin-4-yl]oxy}-2-methyl-propoxy)essigsäure-*tert*.-butylester (Enantiomer 2) werden in 0.5 ml Dioxan gelöst, mit 0.2 ml 4 N Chlorwasserstoff in Dioxan versetzt und 48 h bei RT gerührt. Die Reaktionslösung wird danach mit weiteren 0.4 ml 4 N Chlorwasserstoff in Dioxan versetzt und erneut 16 h bei RT gerührt. Nach Einengen der Reaktionslösung im Vakuum wird der Rückstand mittels präparativer RP-HPLC (Gradient: Wasser/Acetonitril) aufgereinigt. Es werden 21 mg (53% d. Th.) des gewünschten Produkts als Enantiomer 2 erhalten.
LC-MS (Methode 2): Rₜ = 2.45 min.; m/z = 449 (M+H)⁺
¹H-NMR (400 MHz, DMSO-d₆): δ = 8.57 (s, 1H), 7.56-7.53 (m, 2H), 7.42-7.37 (m, 5H), 7.05-7.01 (m, 2H), 4.41-4.37 (m; 1H), 4.24-4.20 (m, 1H), 3.82 (s, 3H), 3.42 (s, 2H), 3.21-3.14 (m, 2H), 2.00-1.93 (m, 1H), 0.71 (d, 3H).

### Beispiel 130

### 3-{[(1R,2R)-2-{[5-(4-Methoxyphenyl)-6-phenylfuro[2,3-d]pyrimidin-4-yl]oxy}-1-methylpropyl]-oxy}propionsäure

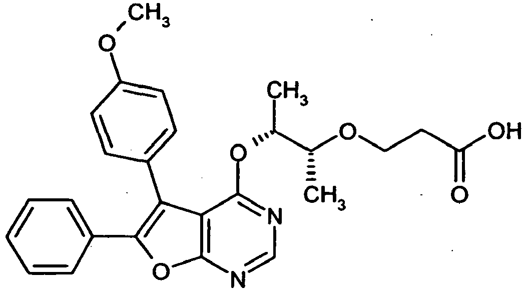

60 mg (0.12 mmol) 3-([(1*R,2R*)-2-{[5-(4-Methoxyphenyl)-6-phenylfuro[2,3-d]pyrimidin-4-yl]-oxy}-1-methylpropyl]oxy}propionsäure-*tert*.-butylester werden mit 1.2 ml 4 N Chlorwasserstoff in Dioxan versetzt und 16 h bei RT gerührt. Mit 1 N Natronlauge wird dann pH 7 eingestellt, die wässrige Phase dreimal mit je 10 ml Dichlormethan extrahiert und die vereinigten organischen Extrakte über Natriumsulfat getrocknet, filtriert und im Vakuum eingeengt. Es werden 52 mg (93% d. Th.) des gewünschten Produkts erhalten.
LC-MS (Methode 7): Rₜ = 4.83 min.; m/z = 519 (M+H)⁺
¹H-NMR (400 MHz DMSO-d₆): δ = 8.57 (s, 1H), 7.55 (d, 2H), 7.40-7.35 (m, 5H), 7.01 (d, 2H), 5.35 (dt, 1H), 3.81 (s, 3H), 3.52-3.40 (m, 3H), 2.26 (t, 2H), 1.16 (d, 3H), 0.88 (d, 3H).

### Beispiel 131

### 6-{[6-(3-Fluorphenyl)-5-(4-methoxyphenyl)furo[2,3-d]pyrimidin-4-yl]amino}hexansäuremethylester

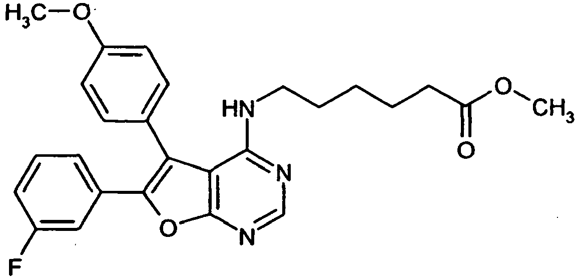

Ein Gemisch aus 100 mg (0.22 mmol) 6-{[6-Brom-5-(4-methoxyphenyl)furo[2,3-d]pyrimidin-4-yl]amino}hexansäuremethylester, 47 mg (0.34 mmol) 3-Fluorphenylboronsäure und 145 mg (0.45 mmol) Cäsiumcarbonat in 5 ml Dioxan wird mit 3 mg (0.01 mmol) *trans*-Bis(dicyclohexylamin)-palladium(II)acetat [T. Bin, J. Org. Chem. 2004, 69, 4330-4335] versetzt und 16 h bei 80°C gerührt. Nach Zugabe von 95 mg (0.45 mmol) Kaliumphosphat, weiteren 47 mg (0.34 mmol) 3-Fluorphenylboronsäure und einer Spatelspitze *trans*-Bis(dicyclohexylamin)palladium(II)acetat wird das Reaktionsgemisch für weitere 4 h bei 80°C gerührt. Das Reaktionsgemisch wird dann filtriert und direkt mittels präparativer RP-HPLC (Gradient: Wasser/Acetonitril) gereinigt. Es werden 57 mg (53% d. Th.) des gewünschten Produkts erhalten.
LC-MS (Methode 2): Rₜ = 2.81 min.; m/z = 464 (M+H)⁺
¹H-NMR (400 MHz, DMSO-d₆): δ = 8.34 (s, 1H), 7.47-7.45 (m, 2H), 7.42-7.39 (m, 1H), 7.28-7.26 (m, 1H), 7.19-7.15 (m, 4H), 5.14 (t, NH), 3.86 (s, 3H), 3.58 (s, 3H), 3.38-3.30 (m, 2H), 2.26 (t, 2H), 1.51-1.37 (m, 4H), 1.18-1.11 (m, 2H).

### Beispiel 132

### 6-{[6-(3-Fluorphenyl)-5-(4-methoxyphenyl)furo[2,3-d]pyrimidin-4-yl]amino}hexansäure

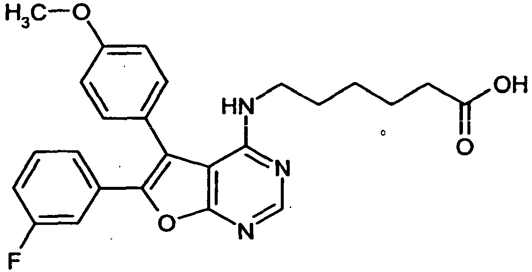

Eine Lösung von 45 mg (0.01 mmol) 6-{[6-(3-Fluorphenyl)-5-(4-methoxyphenyl)furo[2,3-d]pyrimidin-4-yl]amino}hexansäuremethylester in 2.5 ml Dioxan wird mit 0.5 ml 1 N Natronlauge versetzt und 16 h bei RT gerührt. Nach Zugabe von 0.75 ml 1 N Salzsäure wird die Reaktionslösung im Vakuum eingeengt. Der Rückstand wird in Diethylether verrührt, filtriert und im Vakuum getrocknet. Es werden 42 mg (93% d. Th.) des gewünschten Produkts erhalten.
LC-MS (Methode 5): R, = 2.59 min.; m/z = 450 (M+H)⁺
¹H-NMR (400 MHz, DMSO-d₆): δ = 12.00 (br. s, 1H), 8.35 (s, 1H), 7.47-7.45 (m, 2H), 7.42-7.39 (m, 1H), 7.28-7.26 (m, 1H), 7.19-7.15 (m, 4H), 5.15 (t, NH), 3.86 (s, 3H), 3.39-3.34 (m, 2H), 2.17 (t, 2H), 1.48-1.37 (m, 4H), 1.19-1.11 (m, 2H).

### Beispiel 133

### 4-{[(2R)-2-{[5-(4-Ethylphenyl)-6-phenylfuro[2,3-d]pyrimidin-4-yl]oxy}propyl](methyl)amino}-buttersäure

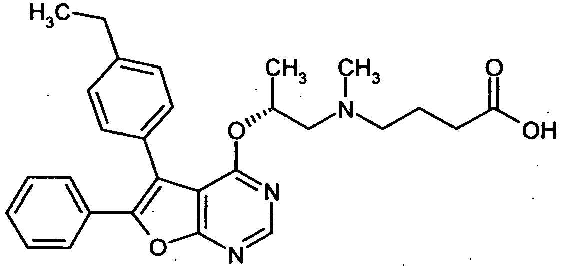

Eine Lösung von 100 mg (73% Reinheit, 0.15 mmol) 4-{[(*2R*)-2-{[5-(4-Ethylphenyl)-6-phenyl-furo[2,3-d]pyrimidin-4-yl]oxy}propyl](methyl)amino}buttersäuremethylester in 3 ml Dioxan wird mit 0.6 ml 1 N Natronlauge versetzt und 16 h bei RT gerührt. Nach Zugabe von 0.7 ml 1 N Salzsäure wird mit Essigsäureethylester extrahiert. Die wässrige Phase wird noch zweimal mit Essigsäureethylester nachextrahiert. Die vereinigten organischen Phasen werden über Natriumsulfat getrocknet und filtriert. Das Filtrat wird im Vakuum eingeengt. Der ölige Rückstand wird in Acetonitril aufgenommen und mittels präparativer RP-HPLC (Gradient: Wasser/Acetonitril) gereinigt. Es werden 57 mg (80% d. Th.) des gewünschten Produkts erhalten.
LC-MS (Methode 8): R, = 1.88 min.; m/z = 474 (M+H)⁺
¹H-NMR (400 MHz, DMSO-d₆): δ = 12.17 (br. s, 1H), 8.57 (s, 1H), 7.55-7.53 (m, 2H), 7.42-7.37 (m, 5H), 7.30-7.28 (m, 2H), 5.50-5.42 (m, 1H), 2.69 (q, 2H), 2.44-2.30 (m, 2H), 2.20 (t, 2H), 2.06 (t, 2H), 2.01 (s, 3H), 1.52-1.41 (m, 2H), 1.26-1.19 (m, 6H).

### Beispiel 134

### 6-{[6-(4-Fluorphenyl)-5-(4-methoxyphenyl)furo[2,3-d]pyrimidin-4-yl]amino}hexansäuremethylester

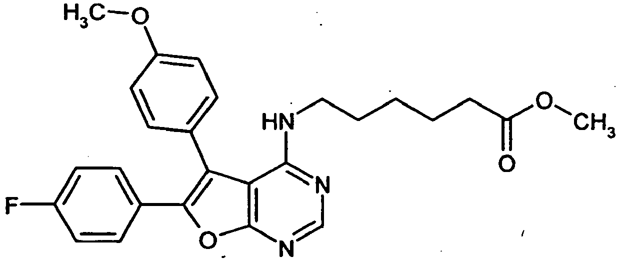

Ein Gemisch aus 100 mg (0.22 mmol) 6-{[6-Brom-5-(4-methoxyphenyl)furo[2,3-d]pyrimidin-4-yl]amino}hexansäuremethylester, 47 mg (0.34 mmol) 4-Fluorphenylboronsäure und 95 mg (0.45 mmol) Kaliumphosphat in 5 ml Dioxan wird mit 3 mg (0.01 mmol) *trans*-Bis(dicyclohexylamin-palladium(II)acetat [T. Bin, J. Org. Chem. 2004, 69, 4330-4335] versetzt und 21 h bei 80°C gerührt. Nach Filtration des Feststoffs wird das Filtrat im Vakuum eingeengt. Der Rückstand wird mittels präparativer RP-HPLC (Gradient: Wasser/Acetonitril) gereinigt. Es werden 59 mg (57% d. Th.) des gewünschten Produkts erhalten.
LC-MS (Methode 8): Rₜ = 3.06 min.; m/z = 464 (M+H)⁺
¹H-NMR (400 MHz, DMSO-d₆): δ = 8.32 (s, 1H), 7.50-7.42 (m, 4H), 7.26-7.20 (m, 2H), 7.16-7.12 (m, 2H), 5.09 (t, NH), 3.85 (s, 3H), 3.58 (s, 3H), 3.38-3.30 (m, 2H), 2.26 (t, 2H), 1.51-1.37 (m, 4H), 1.18-1.11 (m, 2H).

### Beispiel 135

### 6-{[6-(4-Fluorphenyl)-5-(4-methoxyphenyl)furo[2,3-d]pyrimidin-4-yl]amino}hexansäureethylester

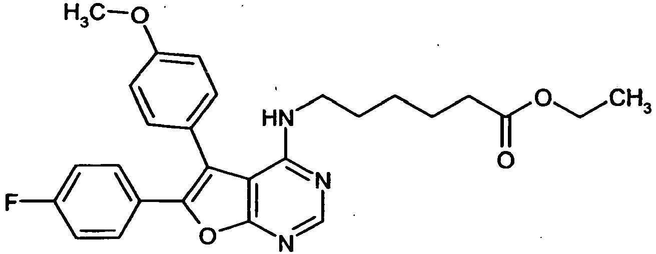

Ein Gemisch aus 100 mg (0.22 mmol) 6-{[6-Brom-5-(4-methoxyphenyl)furo[2,3-d]pyrimidin-4-yl]amino}hexansäuremethylester, 37 mg (0.27 mmol) 4-Fluorphenylboronsäure und 95 mg (0.45 mmol) Kaliumphosphat in 5 ml Ethanol wird mit 3 mg (0.01 mmol) *trans*-Bis(dicyclohexylamin)-palladium(II)acetat [T. Bin, J. Org. Chem. 2004, 69, 4330-4335] versetzt und zunächst 16 h bei RT und dann 3 h bei 80°C gerührt. Nach Filtration des Feststoffs wird das Filtrat im Vakuum eingeengt. Der Rückstand wird mittels präparativer RP-HPLC (Gradient: Wasser/Acetonitril) gereinigt. Es werden 28 mg (26% d. Th.) des gewünschten Produkts erhalten.
LC-MS (Methode 5): Rₜ = 3.07 min.; m/z = 478 (M+H)⁺
¹H-NMR (400 MHz, DMSO-d₆): δ = 8.32 (s, 1H), 7.49-7.43 (m, 4H), 7.25-7.20 (m, 2H), 7.15-7.13 (m, 2H), 5.09 (t, NH), 4.04 (q, 2H), 3.85 (s, 3H), 3.38-3.30 (m, 2H), 2.24 (t, 2H), 1.51-1.37 (m, 4H), 1.18-1.11 (m, 5H).

### Beispiel 136

### 6-{[6-(4-Fluorphenyl)-5-(4-methoxyphenyl)furo[2,3-d]pyrimidin-4-yl]amino}hexansäure

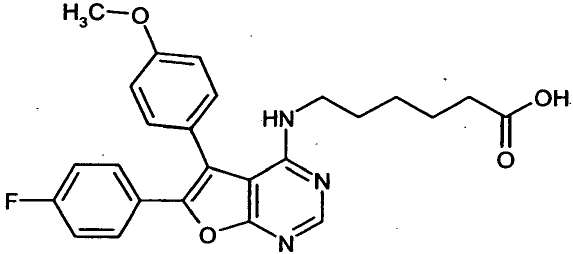

Eine Lösung von 169 mg (0.37 mmol) 6-{[6-(4-Fluorphenyl)-5-(4-methoxyphenyl)furo[2,3-d]pyrimidin-4-yl]amino}hexansäuremethylester in 5 ml Dioxan wird mit 1 ml 1 N Natronlauge versetzt und 16 h bei RT gerührt. Nach Zugabe von 3 ml 1 N Salzsäure wird die Reaktionslösung im Vakuum eingeengt. Der Rückstand wird in Diethylether verrührt, abfiltriert und im Vakuum getrocknet. Es werden 165 mg (99% d. Th.) des gewünschten Produkts erhalten.
LC-MS (Methode 2): Rₜ = 2.39 min.; m/z = 450 (M+H)⁺
¹H-NMR (400 MHz, DMSO-d₆): δ = 14.25-10.15 (br. s, CO₂H), 8.33 (s, 1H), 7.49-7.43 (m, 4H), 7.25-7.21 (m, 2H), 7.15-7.13 (m, 2H), 5.13 (br. t, NH), 3.85 (s, 3H), 3.39-3.34 (m, 2H), 2.17 (t, 2H), 1.48-1.37 (m, 4H), 1.19-1.11 (m, 2H).

### Beispiel 137

### (6R)-6-{[6-(2-Fluorphenyl)-5-(4-methoxyphenyl)furo[2,3-d]pyrimidin-4-yl]oxy}heptansäure

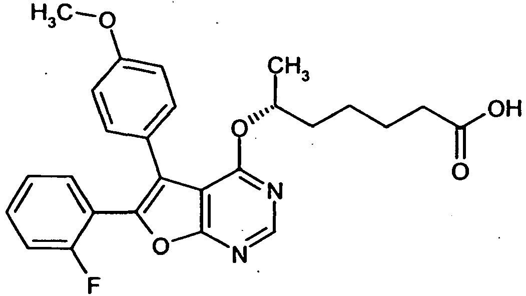

373 mg (0.72 mmol) (6*R*)-6-{[6-(2-Fluorphenyl)-5-(4-methoxyphenyl)furo[2,3-d]pyrimidin-4-yl]-oxy}heptansäure-*tert.*-butylester werden in 4 ml 4 N Chlorwasserstoff in Dioxan gelöst und 16 h bei RT gerührt. Nach Einengen der Reaktionslösung im Vakuum wird der Rückstand mittels präparativer RP-HPLC (Gradient: Wasser/Acetonitril) aufgereinigt. Es werden 171 mg (51% d. Th.) des gewünschten Produkts erhalten.
LC-MS (Methode 8): Rₜ = 2.78 min.; m/z = 465 (M+H)⁺
¹H-NMR (400 MHz, DMSO-d₆): δ = 11.98 (br. s, 1H), 8.60 (s, 1H), 7.55-7.50 (m, 2H), 7.33-7.28 (m, 4H), 6.94-6.91 (m, 2H), 5.40-5.33 (m, 1H), 3.77 (s, 3H), 2.14 (t, 2H), 1.60-1.55 (m, 2H), 1.48-1.40 (m, 2H), 1.31-1.17 (m, 3H), 1.28 (d, 3H).

### Beispiel 138

### (+)-4-{[(2S)-2-{[6-(2-Fluorphenyl)-5-(4-methoxyphenyl)furo[2,3-d]pyrimidin-4-yl]oxy}propyl]-(methyl)amino} buttersäure

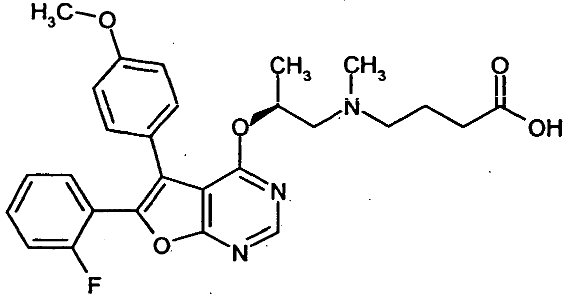

102 mg (93% Reinheit, 0.19 mmol) 4-{[(*2S*)-2-{[6-(2-Fluorphenyl)-5-(4-methoxyphenyl)furo[2,3-d]pyrimidin-4-yl]oxy}propyl](methyl)amino}buttersäure-*tert.*-butylester werden in 2 ml 4 N Chlorwasserstoff in Dioxan gelöst und 16 h bei RT gerührt. Nach Einengen der Reaktionslösung im Vakuum wird der Rückstand mittels präparativer RP-HPLC (Gradient: Wasser/Acetonitril) aufgereinigt. Es werden 48 mg (52% d. Th.) des gewünschten Produkts erhalten.
LC-MS (Methode 8): Rₜ = 1.68 min.; m/z = 494 (M+H)⁺
¹H-NMR (400 MHz, DMSO-d₆): δ = 12.11 (br. s, 1H), 8.60 (s, 1H), 7.55-7.50 (m, 2H), 7.33-7.28 (m, 4H), 6.93-6.91 (m, 2H), 5.60-5.53 (m, 1H), 3.77 (s, 3H), 2.47-2.37 (m, 2H), 2.31-2.33 (m, 2H), 2.13-2.05 (m, 5H), 1.54-1.46 (m, 2H), 1.28 (d, 3H).
[α]_{D}²⁰ = +123°, c = 0.260, Chloroform.

### Beispiel 139

### 4-tert.-Butoxy-N-[(2S)-2-{[5-(4-ethylphenyl)-6-phenylfuro[2,3-d]pyrimidin-4-yl]oxy}propyl]-N-methyl-4-oxobutan-1-ammoniumchlorid

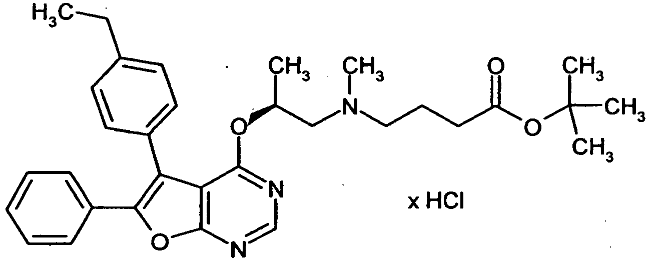

Eine Lösung von 200 mg (0.87 mmol) 4-{[(*2S*)-2-Hydrooypropyl](methyl)amino}buttersäure-*tert.-*butylester in 2 ml THF wird unter Eiskühlung mit 43 mg (1.08 mmol) Natriumhydrid (60%-ige Dispersion in Mineralöl) versetzt. Nach zehn Minuten Rühren unter Eiskühlung werden eine Lösung von 338 mg (0.91 mmol) 4-Chlor-5-(4-ethylphenyl)-6-phenylfuro[2,3-d]pyrimidin in 3 ml THF sowie 16 mg (0.04 mmol) Tetra-*n*-butylammoniumiodid zugegeben. Das Reaktionsgemisch wird 16 h bei RT gerührt. Nach Zugabe von Wasser und Essigsäureethylester wird die abgetrennte organische Phase mit 1 N Salzsäure gewaschen und im Vakuum eingeengt. Der Rückstand wird in Acetonitril/DMSO aufgenommen und mittels präparativer RP-HPLC (Gradient: Wasser/Acetonitril) gereinigt. Es werden 94 mg (19% d. Th.) des gewünschten Produkts erhalten.
LC-MS (Methode 8): Rₜ = 2.06 min.; m/z = 530 (M-HCl+H)⁺
¹H-NMR (400 MHz, DMSO-d₆): δ = 9.77-9.55 (m, NH), 8.65 (s, 1H), 7.56-7.51 (m, 2H), 7.44-7.40 (m, 4H), 7.35-7.32 (m, 2H), 5.80-5.64 (m, 1H), 3.16-2.76 (m, 4H), 2.71 (q, 2H), 2.66-2.59 (m, 2H), 2.33-2.29 (m, 2H), 2.24-2.16 (m, 2H), 1.77-1.53 (m, 2H), 1.38-1.33 (m, 12H), 1.09 (t, 3H).

### Beispiel 140

### (+)-4-{[(2S)-2-{[5-(4-Ethylphenyl)-6-phenylfuro[2,3-d]pyrimidin-4-yl]oxy}propyl](methyl)-amino}buttersäure

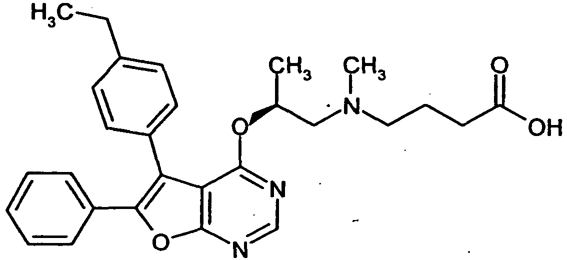

93 mg (0.16 mmol) 4-*tert*.-Butoxy-*N*-[(*2S*)-2-{[5-(4-ethylphenyl)-6-phenylfuro[2,3-d]pyrimidin-4-yl]oxy}propyl]-*N*-methyl-4-oxobutan-1-ammoniumchlorid werden in 3 ml 4 N Chlorwasserstoff in Dioxan gelöst und 16 h bei RT gerührt. Nach Einengen der Reaktionslösung im Vakuum wird der Rückstand mittels präparativer RP-HPLC (Gradient: Wasser/Acetonitril) aufgereinigt. Es werden 50 mg (64% d. Th.) des gewünschten Produkts erhalten.
LC-MS (Methode 8): Rₜ = 1.87 min.; m/z = 474 (M+H)⁺ .
¹H-NMR (400 MHz, DMSO-d₆): δ = 12.13 (br. s, 1H), 8.57 (s, 1H), 7.55-7.52 (m, 2H), 7.42-7.37 (m, 5H), 7.31-7.29 (m, 2H), 5.53-5.46 (m, 1H), 2.68 (q, 2H), 2.50-2.42 (m, 2H), 2.32-2.21 (m, 2H), 2.10-2.02 (m, 5H), 1.51-1.42 (m, 2H), 1.26-1.21 (m, 6H).
[α]_{D}²⁰ = +171°, c = 0.200, Chloroform.

### Beispiel 141

### 6-{[5-(4-Ethylphenyl)-6-(2-fluorphenyl)furo[2,3-d]pyrimidin-4-yl]oxy}heptansäure

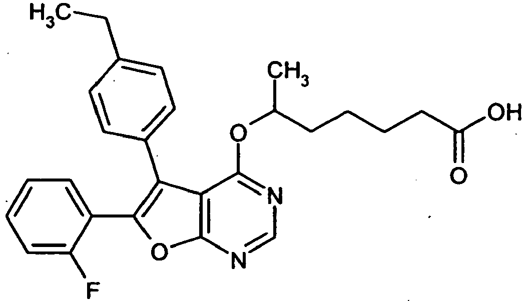

80 mg (0.15 mmol) 6-{[5-(4-Ethylphenyl)-6-(2-fluorphenyl)furo[2,3-d]pyrimidin-4-yl]oxy}heptansäure-*tert.*-butylester werden in 2 ml 4 N Chlorwasserstoff in Dioxan gelöst und 16 h bei RT gerührt. Nach Einengen der Reaktionslösung im Vakuum wird der Rückstand mittels präparativer RP-HPLC (Gradient: Wasser/Acetonitril) aufgereinigt. Es werden 12 mg (16% d. Th.) des gewünschten Produkts als Racemat erhalten.
LC-MS (Methode 8): Rₜ = 3.06 min.; m/z = 463 (M+H)⁺
¹H-NMR (400 MHz, DMSO-d₆): δ = 11.98 (br. s, 1H), 8.61 (s, 1H), 7.55-7.51 (m, 2H), 7.33-7.28 (m, 4H), 7.21-7.19 (m, 2H), 5.38-5.32 (m, 1H), 2.63 (q, 2H), 2.02 (t, 2H), 1.57-1.52 (m, 2H), 1.43-1.37 (m, 2H), 1.27-1.18 (m, 8H).

### Beispiel 142

### (-)-(6R)-6-{[5-(4-Ethylphenyl)-6-(2-fluorphenyl)furo[2,3-d]pyrimidin-4-yl]oxy}heptansäure

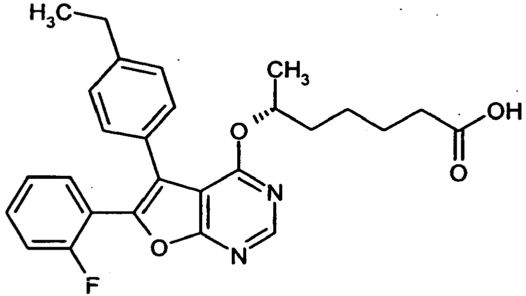

420 mg (0.81 mmol) (*6R*)-6-{[5-(4-Ethylphenyl)-6-(2-fluorphenyl)furo[2,3-d]pyrimidin-4-yl]oxy}-heptansäure-*tert*.-butylester werden in 5.3 ml 4 N Chlorwasserstoff in Dioxan gelöst und 16 h bei RT gerührt. Nach Einengen der Reaktionslösung im Vakuum wird der Rückstand mittels präparativer RP-HPLC (Gradient: Wasser/Acetonitril) aufgereinigt. Es werden 200 mg (53% d. Th.) des gewünschten Produkts erhalten.
LC-MS (Methode 8): Rₜ = 3.03 min.; m/z = 463 (M+H)⁺
¹H-NMR (400 MHz, DMSO-d₆): δ = 11.97 (br. s, 1H), 8.61 (s, 1H), 7.56-7.50 (m, 2H), 7.33-7.27 (m, 4H), 7.21-7.19 (m, 2H), 5.39-5.31 (m, 1H), 2.63 (q, 2H), 2.12 (t, 2H), 1.58-1.53 (m, 2H), 1.47-1.38 (m, 2H), 1.28-1.18 (m, 8H).
[α]_{D}²⁰ = -62°, c = 0.390, Chloroform.

### Beispiel 143

### (+)-(6S)-6-{[5-(4-Ethylphenyl)-6-(2-fluorphenyl)furo[2,3-d]pyrimidin-4-yl]oxy}heptansäure

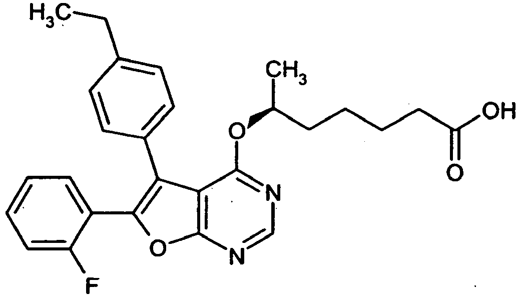

50 mg (0.11 mmol) *rac*.-6-{[5-(4-Ethylphenyl)-6-(2-fluorphenyl)furo[2,3-d]pyrimidin-4-yl]oxy}-heptansäure werden mittels chromatographischer Enantiomerentrennung getrennt [Säule: Daicel Chiralpak AD-H, 5 µm, 250 mm x 20 mm; Fluss: 20 ml/min; Detektion: 245 nm; Temperatur: 25°C; Eluent: 93% iso-Hexan / 7% Ethanol]. Es werden 8 mg (16% d. Th.) des gewünschten enantiomerenreinen Produkts erhalten.
HPLC: Rₜ = 5.65 min. [Säule: Daicel Chiralpak AD-H, 5 µm, 250 mm x 4 mm; Fluss: 1 ml/min; Detektion: 245 nm; Temperatur: 25°C; Eluent: 85% iso-Hexan / 15% Ethanol]
¹H-NMR (400 MHz, DMSO-d₆): δ = 11.97 (br. s, 1H), 8.61 (s, 1H), 7.56-7.50 (m, 2H), 7.33-7.28 (m, 4H), 7.21-7.19 (m, 2H), 5.39-5.31 (m, 1H), 2.63 (q, 2H), 2.12 (t, 2H), 1.58-1.53 (m, 2H), 1.47-1.39 (m, 2H), 1.28-1.18 (m, 8H).
[α]_{D}²⁰ = +50°, c = 0.235, Chloroform.

### Beispiel 144

### (3-{[5-(4-Methoxyphenyl)-6-phenylfuro[2,3-d]pyrimidin-4-yl]oxy}-1-methylbutoxy)essigsäure

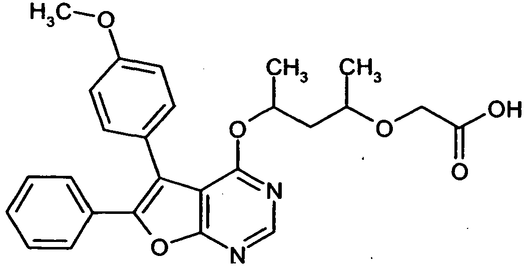

Eine Lösung von 2.19 g (5.41 mmol) 4-{[5-(4-Methoxyphenyl)-6-phenylfuro[2,3-d]pyrimidin-4-yl]oxy}pentan-2-ol in 20 ml Toluol wird mit 4.8 ml einer 11.25 N Natronlauge versetzt. Nach Zugabe von 184 mg (0.54 mmol) Tetra-n-butylammoniumhydrogensulfat und 2.11 g (10.83 mmol) Bromessigsäure-tert.-butylester wird das Reaktionsgemisch 15 h bei 70°C gerührt. Nach Abkühlen auf Raumtemperatur wird mit konzentrierter Salzsäure auf pH 7 eingestellt und dreimal mit je 50 ml Dichlormethan extrahiert. Die vereinigten organischen Extrakte werden mit ges. wässriger Natriumchlorid-Lösung gewaschen, über Natriumsulfat getrocknet und filtriert. Das Filtrat wird im Vakuum eingeengt. Der Rückstand wird in Essigsäureethylester aufgenommen und mittels Flash-Chromatographie an Kieselgel (Laufmittel: zunächst Essigsäureethylester, dann Essigsäureethylester/Methanol 5:1) aufgereinigt. Die hierbei erhaltene Produktfraktion wird nochmals mittels präparativer RP-HPLC (Gradient: Wasser/Acetonitril) gereinigt. Es werden 0.29 g (11% d. Th.) des gewünschten Produkts als racemisches Diastereomerengemisch erhalten.
LC-MS (Methode 8): Rₜ = 2.76 min.; m/z = 463 (M+H)⁺
¹H-NMR (400 MHz, DMSO-d₆): [Minder-Stereoisomer in Klammern] δ = 12.49 (br. s, 1H), 8.57 (s, 1H), [8.55, s, 1H], 7.55-7.52 (m, 2H), 7.42-7.37 (m, 5H), 7.04-6.99 (m, 2H), 5.53-5.46 (m, 1H), [5.41-5.34, m, 1H], 3.88 (d, 2H), 3.82 (s, 3H), 3.47-3.39 (m, 1H), 1.89-1.82 (m, 1H), 1.55-1.48 (m, 1H), 1.28 (d, 3H), 1.00 (d, 3H).

### Beispiel 145

### (3-{[5-(4-Methoxyphenyl)-6-phenylfuro[2,3-d]pyrimidin-4-yl]oxy}-1-methylbutoxy)essigsäure (Enantiomer 1)

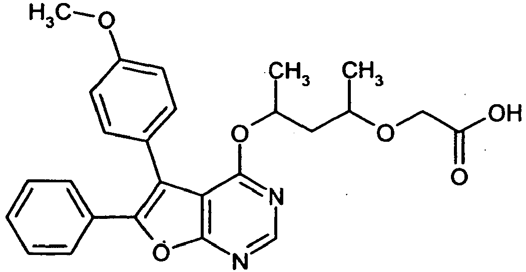

280 mg (0.61 mmol) (3-{[5-(4-Methoxyphenyl)-6-phenylfuro[2,3-d]pyrimidin-4-yl]oxy}-1-methyl-butoxy)essigsäure werden mittels chromatographischer Stereoisomeren-Trennung aufgetrennt [Säule: chirale Kieselgel-Phase basierend auf dem Selektor Poly(*N*-methacryloyl-*L*-Leucin-di-cyclopropylmethylamid), 670 mm x 40 mm; Fluss: 80 ml/min; Detektion: 260 nm; Temperatur: 24°C; Eluent: 60% iso-Hexan / 40% Essigsäureethylester]. Es werden 108 mg (39% d. Th.) des diastereomerenreinen Enantiomers 1 erhalten.
HPLC: R, = 3.40 min. [Säulenmaterial wie oben, 250 mm x 4.6 mm; Fluss: 2 ml/min; Eluent: 50% iso-Hexan / 50% Essigsäureethylester; Temperatur: 25°C]
LC-MS (Methode 9): Rₜ = 3.79 min.; m/z = 463 (M+H)⁺
¹H-NMR (400 MHz, DMSO-d₆): δ = 12.50 (br. s, 1H), 8.57 (s, 1H), 7.55-7.52 (m, 2H), 7.42-7.37 (m, 5H), 7.03-7.00 (m, 2H), 5.53-5.45 (m, 1H), 3.88 (d, 2H), 3.81 (s, 3H), 3.47-3.39 (m, 1H), 1.88-1.81 (m, 1H), 1.55-1.48 (m, 1H), 1.28 (d, 3H), 1.00 (d, 3H).

### Beispiel 146

### (3-{[5-(4-Methoxyphenyl)-6-phenylfuro[2,3-d]pyrimidin-4-yl]oxy}-1-methylbutoxy)essigsäure (Enantiomer 2)

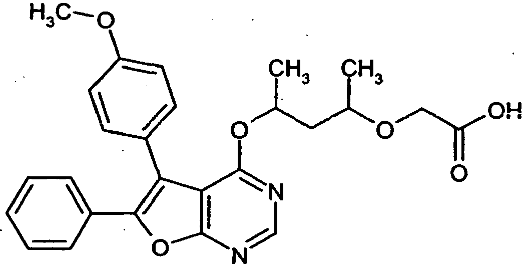

280 mg (0.61 mmol) (3-{[5-(4-Methoxyphenyl)-6-phenylfuro[2,3-d]pyrimidin-4-yl]oxy}-1-methyl-butoxy)essigsäure werden mittels chromatographischer Stereoisomeren-Trennung aufgetrennt [Säule: chirale Kieselgel-Phase basierend auf dem Selektor Poly(*N*-methacryloyl-*L*-Leucin-di-cyclopropylmethylamid), 670 mm x 40 mm; Fluss: 80 ml/min; Detektion: 260 nm; Temperatur: 24°C; Eluent: 60% iso-Hexan / 40% Essigsäureethylester]. Es werden 116 mg (41% d. Th.) des diastereomerenreinen Enantiomers 2 erhalten.
HPLC: Rₜ = 3.80 min. [Säulenmaterial wie oben, 250 mm x 4.6 mm; Fluss: 2 ml/min; Eluent: 50% iso-Hexan / 50% Essigsäureethylester; Temperatur: 25°C]
LC-MS (Methode 9): Rₜ = 3.78 min.; m/z = 463 (M+H)⁺
¹H-NMR (400 MHz, DMSO-d₆): δ = 12.50 (br. s, 1H), 8.57 (s, 1H), 7.55-7.52 (m, 2H), 7.42-7.37 (m, 5H), 7.03-7.00 (m, 2H), 5.53-5.45 (m, 1H), 3.88 (d, 2H), 3.81 (s, 3H), 3.47-3.39 (m, 1H), 1.88-1.81 (m, 1H), 1.55-1.48 (m, 1H), 1.28 (d, 3H), 1.00 (d, 3H).

### Beispiel 147 *

### [2-({[5-(4-'Ethylphenyl)-6-phenylfuro[2,3-d]pyrimidin-4-yl]oxy}methyl)-3,3-dimethylbutoxy]-essigsäure

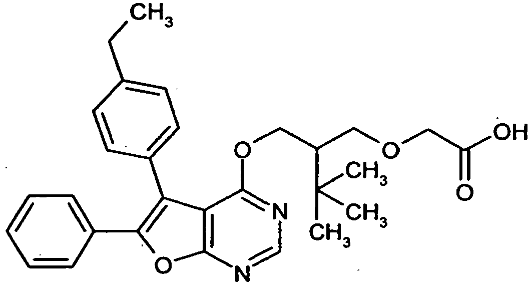

155 mg (0.29 mmol) [2-({[5-(4-Ethylphenyl)-6-phenylfuro[2,3-d]pyrimidin-4-yl]oxy}methyl)-3,3-dimethylbutoxy]essigsäure-*tert*.-butylester werden mit 1.0 ml 4 N Chlorwasserstoff in Dioxan versetzt und 48 h bei RT gerührt. Nach Einengen der Reaktionslösung im Vakuum wird der Rückstand mittels präparativer RP-HPLC (Gradient: Wasser/Acetonitril) aufgereinigt. Es werden 122 mg (88% d. Th.) des gewünschten Produkts (Racemat) erhalten.
LC-MS (Methode 9): Rₜ = 4.47 min.; m/z = 489 (M+H)⁺
¹H-NMR (400 MHz, DMSO-d₆): δ = 12.57 (br. s, 1H), 8.59 (s, 1H), 7.53-7.51 (m, 2H), 7.39-7.36 (m, 5H), 7.31-7.29 (m, 2H), 4.53 (dd, 1H), 4.47 (dd, 1H), 3.88 (dd, 2H), 3.38-3.29 (m, 2H), 2.68 (q, 2H), 1.54-1.49 (m, 1H), 1.24 (t, 3H), 0.71 (s, 9H).

### Beispiel 148

### 3-(2-{[5-(4-Methoxyphenyl)-6-phenylfuro[2,3-d]pyrimidin-4-yl]oxy}-1-methylpropoxy)propionsäure

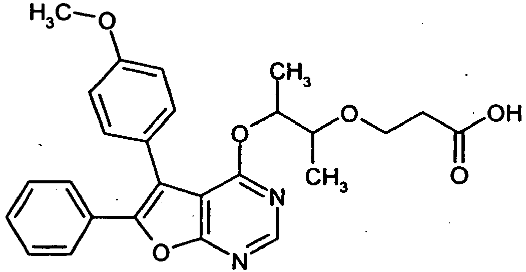

500 mg (0.96 mmol) 3-(2-{[5-(4-Methoxyphenyl)-6-phenylfuro[2,3-d]pyrimidin-4-yl]oxy}-1-methylpropoxy)propionsäure-*tert*.-butylester werden mit 4.0 ml 4 N Chlorwasserstoff in Dioxan versetzt und 16 h bei RT gerührt. Nach Einengen der Reaktionslösung im Vakuum wird der Rückstand mittels präparativer RP-HPLC (Gradient: Wasser/Acetonitril) aufgereinigt. Es werden 249 mg (56% d. Th.) des gewünschten Produkts als (*R,S*/*S,R*)-Racemat erhalten.
LC-MS (Methode 8): Rₜ = 2.72 min.; m/z = 463 (M+H)⁺
¹H-NMR (400 MHz, DMSO-d₆): δ = 12.09 (br. s, 1H), 8.57 (s, 1H), 7.54-7.51 (m, 2H), 7.42-7.36 (m, 5H), 7.03-6.99 (m, 2H), 5.41-5.34 (m, 1H), 3.81 (s, 3H), 3.50-3.41 (m, 3H), 2.27 (t, 2H), 1.18 (d, 3H), 0.88 (d, 3H).

### Beispiel 149

### 3-(2-{[5-(4-Methoxyphenyl)-6-phenylfuro[2,3-d]pyrimidin-4-yl]oxy}-1-methylpropoxy)propionsäure (Enantiomer 1)

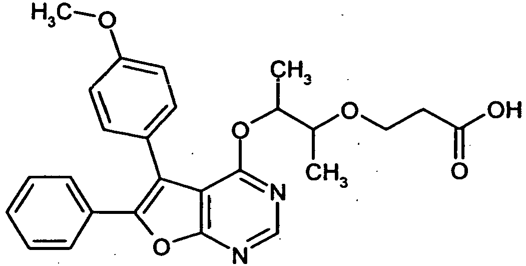

240 mg (0.46 mmol) 3-(2-{[5-(4-Methoxyphenyl)-6-phenylfuro[2,3-d]pyrimidin-4-yl]oxy}-1-methylpropoxy)propionsäure ((*R*,*SlS*,*R*)-Racemat) werden mittels Chromatographie an chiraler Phase in die Enantiomere getrennt [Säule: chirale Kieselgel-Phase basierend auf dem Selektor Poly(*N*-methacryloyl-*L*-Leucin-dicyclopropylmethylamid), 670 mm x 40 mm; Fluss: 80 ml/min; Detektion: 260 nm; Temperatur: 24°C; Eluent: 60% iso-Hexan / 40% Essigsäureethylester]. Es werden 119 mg (50% d. Th.) von Enantiomer 1 erhalten.
HPLC: Rₜ = 3.60 min. [Säulenmaterial wie oben, 250 mm x 4.6 mm; Fluss: 2 ml/min; Eluent: 50% iso-Hexan / 50% Essigsäureethylester; Temperatur: 25°C]
LC-MS (Methode 8): Rₜ = 2.81 min.; m/z = 463 (M+H)⁺
¹H-NMR (400 MHz, DMSO-d₆): δ = 12.06 (br. s, 1H), 8.57 (s, 1H), 7.54-7.51 (m, 2H), 7.41-7.36 (m, 5H), 7.03-6.99 (m, 2H), 5.41-5.36 (m, 1H), 3.81 (s, 3H), 3.50-3.41 (m, 3H), 2.27 (t, 2H), 1.18 (d, 3H), 0.88 (d, 3H).

### Beispiel 150

### 3-(2-{[5-(4-Methoxyphenyl)-6-phenylfuro[2,3-d]pyrimidin-4-yl]oxy}-1-methylpropoxy)propionsäure (Enantiomer 2)

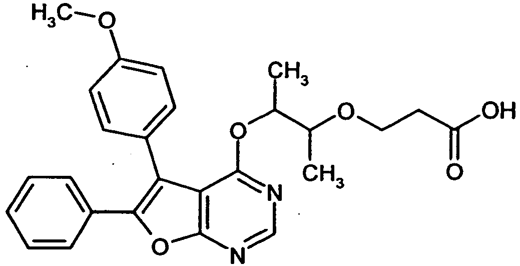

240 mg (0.46 mmol) 3-(2-{[5-(4-Methoxyphenyl)-6-phenylfuro[2,3-d]pyrimidin-4-yl]oxy}-1-methylpropoxy)propionsäure ((*R*,*S*/*S*,*R*)-Racemat) werden mittels Chromatographie an chiraler Phase in die Enantiomere getrennt [Säule: chirale Kieselgel-Phase basierend auf dem Selektor Poly(*N*-methacryloyl-*L*-Leucin-dicyclopropylmethylamid), 670 mm x 40 mm; Fluss: 80 ml/min; Detektion: 260 nm; Temperatur: 24°C; Eluent: 60% iso-Hexan / 40% Essigsäureethylester]. Es werden 110 mg (46% d. Th.) von Enantiomer 2 erhalten.
HPLC: Rₜ = 4.31 min. [Säulenmaterial wie oben, 250 mm x 4.6 mm; Fluss: 2 ml/min; Eluent: 50% iso-Hexan / 50% Essigsäureethylester; Temperatur: 25°C]
LC-MS (Methode 8): Rₜ = 2.80 min.; m/z = 463 (M+H)⁺
¹H-NMR (400 MHz, DMSO-d₆): δ = 12.06 (br. s, 1H), 8.57 (s, 1H), 7.54-7.51 (m, 2H), 7.41-7.36 (m, 5H), 7.03-6.99 (m, 2H), 5.41-5.36 (m, 1H), 3.81 (s, 3H), 3.50-3.41 (m, 3H), 2.27 (t, 2H), 1.18 (d, 3H), 0.88 (d, 3H).

### Beispiel 151

### (-)-{[(2R)-3-{[5-(4-Ethylphenyl)-6-(2-fluorphenyl)furo[2,3-d]pyrimidin-4-yl]oxy}-2-methylpropyl]oxy}essigsäureethylester

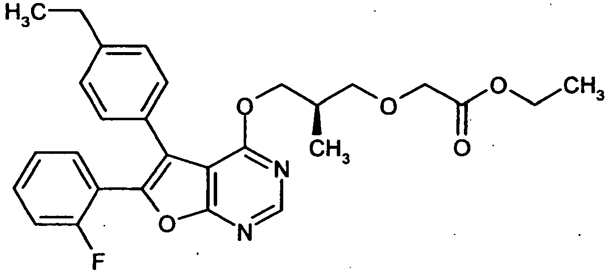

Eine Lösung von 397 mg (90% Reinheit, 1.01 mmol) 4-Chlor-5-(4-ethylphenyl)-6-(2-fluorphenyl)-furo[2,3-d]pyrimidin und 250 mg (2.5 mmol) (-)-{[(2*S*)-3-Hydroxy-2-methylpropyl]oxy}essigsäureethylester in 2.8 ml abs. THF wird auf 0°C gekühlt und mit 1.27 ml (1.27 mmol) Phosphazen-Base P4-t-Bu (1 M Lösung in Hexan) versetzt. Nach Ende der Zugabe wird auf RT erwärmt und 2.5 h bei RT gerührt, bevor Wasser zur Reaktionsmischung gegeben und mit 1 N Salzsäure neutralisiert wird. Man extrahiert mit Dichlormethan, trocknet die organische Phase über Natriumsulfat und konzentriert im Vakuum. Das Rohprodukt wird mittels präparativer RP-HPLC (Gradient: Wasser/Acetonitril) aufgereinigt. Die erhaltenen Produktfraktionen werden vereinigt, im Vakuum eingeengt und der Rückstand durch abermalige Chromatographie an Silicagel (Gradient: Cyclohexan/Ethylacetat 30:1 → 5:1) weiter gereinigt. Erhalten werden 121.1 mg (24.3% d. Th.) des Zielprodukts.
LC-MS (Methode 8): Rₜ = 3.32 min.; m/z = 493 (M+H)⁺
¹H-NMR (400 MHz, DMSO-d₆): δ = 8.63 (s, 1H), 7.58-7.51 (m, 2H), 7.35-7.28 (m, 4H), 7.20 (d, 2H), 4.40 (dd, 1H), 4.35 (dd, 1H), 4.09 (q, 2H), 3.99 (s, 2H), 3.28 (d, 2H), 2.63 (q, 2H), 2.08 (m, 1H), 1.20-1.14 (m, 6H), 0.71 (d, 3H).
[α]_{D}²⁰ = -9.1 °, c = 0.455, Chloroform.

### Beispiel 152

### (-)-{[(2R)-3-{[5-(4-Methoxyphenyl)-6-(2-fluorphenyl)furo[2,3-d]pyrimidin-4-yl]oxy}-2-methylpropyl]oxy}essigsäureethylester

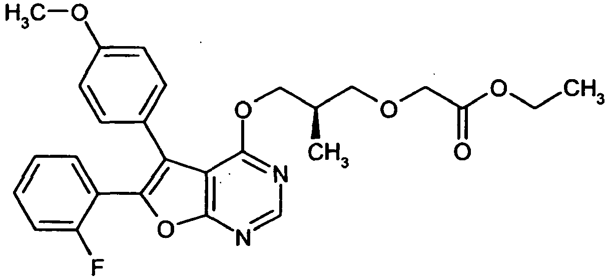

Eine Lösung von 359.5 mg (1.01 mmol) 4-Chlor-5-(4-methoxyphenyl)-6-(2-fluorphenyl)furo[2,3-d]pyrimidin und 250 mg (2.5 mmol) (-)-{[(2*S*)-3-Hydroxy-2-methylpropyl]oxy}essigsäureethylester in 2.8 ml abs. THF wird auf 0°C gekühlt und mit 1.27 ml (1.27 mmol) Phosphazen-Base P4-t-Bu (1 M Lösung in Hexan) versetzt. Nach Ende der Zugabe wird auf RT erwärmt und 2.5 h bei RT gerührt, bevor Wasser zur Reaktionsmischung gegeben und mit 1 N Salzsäure neutralisiert wird. Man extrahiert mit Dichlormethan, trocknet die organische Phase über Natriumsulfat und konzentriert im Vakuum. Das Rohprodukt wird mittels präparativer RP-HPLC (Gradient: Wasser/Acetonitril) gereinigt. Erhalten werden 77.6 mg (15.5% d. Th.) des Zielprodukts.
LC-MS (Methode 9): Rₜ = 4.11 min.; m/z = 495 (M+H)⁺
¹H-NMR (400 MHz, DMSO-d₆): δ = 8.62 (s, 1H), 7.55-7.50 (m, 2H), 7.34-7.28 (m, 4H), 6.94 (d, 2H), 4.44 (dd, 1H), 4.37 (dd, 1H), 4.06 (q, 2H), 4.02 (s, 2H), 3.78 (s, 3H), 3.32-3.28 (m, 2H), 2.09 (m, 1H), 1.18 (t, 3H), 0.85 (d, 3H).
[α]_{D}²⁰ = -4.4°, c = 0_{.}58, Chloroform.

### Beispiel 153

### (+)-3-[(1S)-2-{[5-(4-Ethylphenyl)-6-(2-fluorphenyl)furo[2,3-d]pyrimidin-4-yl]oxy}-1-methyl-ethoxy]propansäure-tert.-butylester

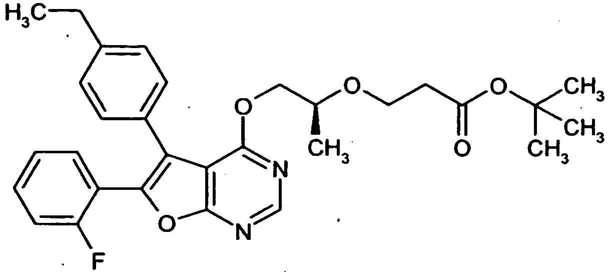

Eine Lösung von 487.1 mg (90% Reinheit, 1.24 mmol) 4-Chlor-5-(4-ethylphenyl)-6-(2-fluorphenyl)furo[2,3-d]pyrimidin und 300 mg (ca. 1.47 mmol) (+)-3-[(1*S*)-2-Hydroxy-1-methylethoxy]-propansäure-*tert*.-butylester in 3.5 ml abs. THF wird auf -20°C gekühlt und mit 1.13 ml (1.13 mmol) Phosphazen-Base P4-t-Bu (1 M Lösung in Hexan) versetzt. Nach Ende der Zugabe wird langsam auf RT erwärmt und 2 h bei RT gerührt, bevor Wasser zur Reaktionsmischung gegeben und mit 1 N Salzsäure neutralisiert wird. Man extrahiert mit Dichlormethan, trocknet die organische Phase über Natriumsulfat und konzentriert im Vakuum. Das Rohprodukt wird mittels präparativer RP-HPLC (Gradient: Wasser/Acetonitril) gereinigt. Es werden 101.2 mg (15.6% d. Th.) des Zielprodukts erhalten.
LC-MS (Methode 9): Rₜ = 4.67 min.; m/z = 521 (M+H)⁺
¹H-NMR (400 MHz, DMSO-d₆): δ = 8.64 (s, 1H), 7.56-7.51 (m, 2H), 7.35-7.29 (m, 4H), 7.20 (d, 2H), 4.43 (dd, 1H), 4.38 (dd, 1H), 3.75-3.67 (m, 1H), 3.33-3.45 (m, 2H), 2.62 (q, 2H), 2.29 (t, 2H), 1.32 (s, 9H), 1.20 (t, 3H), 1.03 (d, 2H).
[α_{]D}²⁰ = +19.5°, c = 0.47, Chloroform.

### Beispiel 154

### (-)-{[(2R)-3-{[5-(4-Ethylphenyl)-6-(2-fluorphenyl)furo[2,3-d]pyrimidin-4-yl]oxy}-2-methylpropyl]oxy}essigsäure

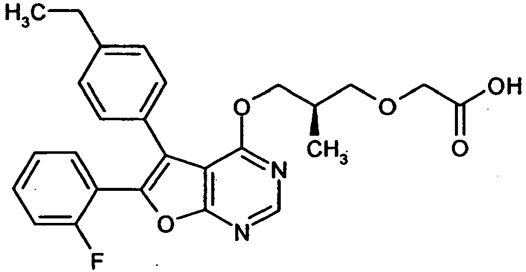

95.0 mg (0.19 mmol) (-)-{[(2*R*)-3-{[5-(4-Ethylphenyl)-6-(2-fluorphenyl)furo[2,3-d]pyrimidin-4-yl]oxy}-2-methylpropyl]oxy}essigsäureethylester werden in 1 ml THF und 0.3 ml Methanol gelöst und mit 0.96 ml 1 N Natronlauge versetzt. Die Mischung wird 30 min bei RT gerührt, bevor mit 1 N Salzsäure neutralisiert und mit Dichlormethan extrahiert wird. Die organische Phase wird im Vakuum eingeengt, und aus dem Rückstand werden nach präparativer RP-HPLC (Gradient: Acetonitril/Wasser) 57.1 mg des Zielprodukts isoliert (63.7 % d. Th.).
LC-MS (Methode 8): Rₜ = 2.98 min.; m/z = 465 (M+H)⁺
¹H-NMR (400 MHz, DMSO-d₆): δ = 8.62 (s, 1H), 7.58-7.51 (m, 2H), 7.35-7.28 (m, 4H), 7.21 (d, 2H), 4.45 (dd, 1H), 4.28 (dd, 1H), 3.45 (s, 2H), 3.23 (d, 2H), 2.63 (q, 2H), 2.04 (m, 1H), 1.20 (t, 3H), 0.78 (d, 3H).
[α]_{D}²⁰ = -32.5°, c = 0.505, Chloroform.

### Beispiel 155

### (-)-{[(2R)-3-{[5-(4-Methoxyphenyl)-6-(2-fluorphenyl)furo[2,3-d]pyrimidin-4-yl]oxy}-2-methylpropyl]oxy}essigsäure

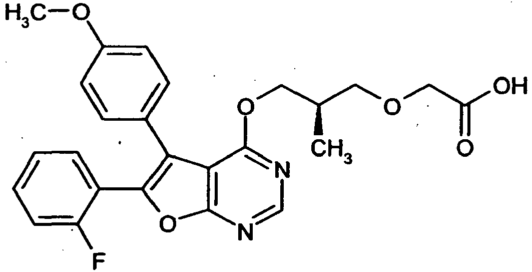

80.5 mg (0.16 mmol) (-)-{[(2*R*)-3-{[5-(4-Methoxyphenyl)-6-(2-fluorphenyl)furo[2,3-d]pyrimidin-4-yl]oxy}-2-methylpropyl]oxy}essigsäureethylester werden in 0.9 ml THF und 0.25 ml Methanol gelöst und mit 0.81 ml 1 N Natronlauge versetzt. Die Mischung wird 30 min bei RT gerührt, bevor mit 1 N Salzsäure neutralisiert und mit Dichlormethan extrahiert wird. Die organische Phase wird im Vakuum eingeengt, und aus dem Rückstand werden nach präparativer RP-HPLC (Gradient: Acetonitril/Wasser) 17.2 mg des Zielprodukts isoliert (22.7 % d. Th.).
LC-MS (Methode 8): Rₜ = 2.73 min.; m/z = 467 (M+H)⁺
¹H-NMR (400 MHz, DMSO-d₆): δ = 8.62 (s, 1H), 7.57-7.50 (m, 2H), 7.35-7.28 (m, 4H), 6.95 (d, 2H), 4.47 (dd, 1H), 4.30 (dd, 1H), 3.79 (s, 3H), 3.47 (s, 2H), 3.28 (d, 2H), 2.05 (m, 1H), 0.79 (d, 3H).
[α]_{D}²⁰ = -16.8°, c = 0.45, Chloroform.

### Beispiel 156

### 3-[(1S)-2-{[5-(4-Ethylphenyl)-6-(2-fluorphenyl)furo[2,3-d]pyrimidin-4-yl]oxy}-1-methylethoxy]-propionsäure

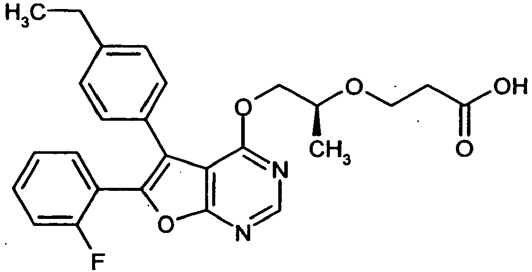

78.5 mg (0.151 mmol) (+)-3-[(1*S*)-2-{[5-(4-Ethylphenyl)-6-(2-fluorphenyl)furo[2,3-d]pyrimidin-4-yl]oxy}-1-methylethoxy]propansäure-*tert*.-butylester werden in 0.5 ml Dichlormethan gelöst und bei RT mit 0.17 ml TFA versetzt. Nach 1.5 h bei RT werden weitere 0.17 ml TFA zugesetzt, und die Reaktionsmischung wird eine weitere halbe Stunde bei RT gerührt, bevor im Vakuum eingeengt wird. Der Rückstand wird in Dichlormethan aufgenommen, mit Wasser gewaschen, über Natriumsulfat getrocknet und im Vakuum eingeengt. Das Rohprodukt wird durch RP-HPLC (Gradient: Acetonitril/Wasser) gereinigt. Es werden 61.8 mg (88.2% d. Th.) des Zielprodukts erhalten.
LC-MS (Methode 8): Rₜ = 2.87 min.; m/z = 465 (M+H)⁺
¹H-NMR (400 MHz, DMSO-d₆): δ = 12.12 (br. s, 1H), 8.63 (s, 1H), 7.57-7.50 (m, 2H), 7.35-7.28 (m, 4H), 7.21 (d, 2H), 4.45-4.35 (m, 2H), 3.73-3.68 (m, 1H), 3.60-3.47 (m, 2H), 2.64 (q, 2H), 2.34 (t, 2H), 1.19 (t, 3H), 1.04 (d, 3H).
[α]_{D}²⁰ = +30.5°, c = 0.48, Chloroform.

### Beispiel 157

### (+/-)-2-Methoxy-3-{[5-(4-methoxyphenyl)-phenylfuro[2,3-d]pyrimidin-4-yl]oxy}propoxy)essigsäure-tert.-butylester

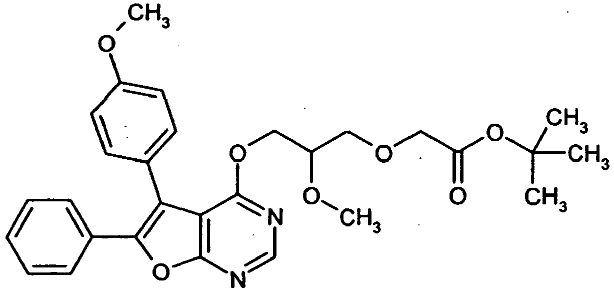

550 mg (1.35 mmol) (+/-)-2-Methoxy-3-{[5-(4-methoxyphenyl)-6-phenylfuro[2,3-d]pyrimidin-4-yl]oxy}propan-1-ol werden mit 1.0 ml (6.77 mmol) Bromessigsäure-*tert*.-butylester und 92 mg (0.27 mmol) Tetrabutylammoniumhydrogensulfat in 15 ml Dichlormethan vorgelegt und auf 0°C abgekühlt. Man gibt 2.75 ml 50%-ige Natronlauge hinzu und rührt einige Minuten bei 0°C. Man lässt dann auf RT kommen und rührt über Nacht bei RT. Anschließend verdünnt man mit Dichlormethan und Wasser, stellt mit 10%-iger Zitronensäure-Lösung sauer und trennt die Phasen. Die wässrige Phase wird einmal mit Dichlormethan nachextrahiert. Die Dichlormethan-Phasen werden vereinigt und einmal mit ges. Natriumchlorid-Lösung gewaschen. Man trocknet über Magnesiumsulfat, engt ein und reinigt den Rückstand durch Chromatographie an Silicagel (Laufmittel: Cyclohexan/Ethylacetat 85:15). Es werden 550 mg (78.1% d. Th.) der Zielverbindung erhalten.
LC-MS (Methode 7): Rₜ = 4.50 min.; m/z = 521 (M+H)⁺
¹H-NMR (400 MHz, CDCl₃): δ = 8.52 (s, 1H), 7.61 (m, 2H), 7.41 (d, 2H), 7.30 (m, 3H), 6.98 (d, 2H), 4.67-4.62 (m, 1H), 4.50-4.44 (m, 1H), 3.90 (s, 2H), 3.87 (s, 3H), 3.60 (m, 1H), 3.52-3.45 (m, 2H), 3.31 (s, 3H), 1.47 (s; 9H).

### Beispiel 158

### (+/-)-(2-Methoxy-3-{[5-(4-methoxyphenyl)-6-phenylfuro[2,3-d]pyrimidin-4-yl]oxy}propoxy)essigsäure

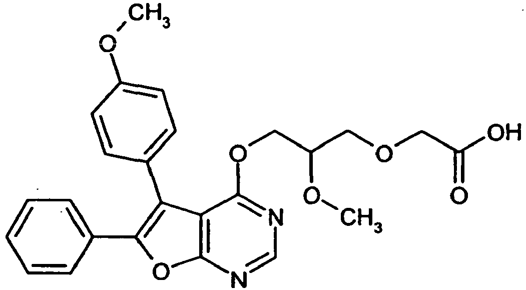

500 mg (0.96 mmol) (+/-)-2-Methoxy-3-{[5-(4-methoxyphenyl)-6-phenylfuro[2,3-d]pyrimidin-4-yl]oxy}propoxy)essigsäure-*tert*.-butytester werden in 10 ml Dichlormethan gelöst. Man gibt 2.5 ml (32.4 mmol) Trifluoressigsäure hinzu und rührt 1 h bei RT. Man engt danach ein und trocknet im Hochvakuum. Es werden 390 mg (87.4% d.Th.) der Zielverbindung erhalten.
LC-MS (Methode 9): Rₜ = 3.46 min.; m/z = 465 (M+H)⁺
¹H-NMR (400 MHz, CDCl₃): δ = 8.52 (s, 1H), 7.61 (m, 2H), 7.45 (d, 2H), 7.40 (m, 3H), 6.97 (d, 2H), 4.70-4.65 (m, 1H), 4.46-4.42 (m, 1H), 4.10-3.96 (dd, 2H), 3.88 (s, 3H), 3.64-3.53 (m, 2H), 3.36 (s, 3H), 3.36-3.30 (m, 1H).

### Beispiel 159

### 6-{[5-(4-Methoxyphenyl)-6-phenylfuro[2,3-d]pyrimidin-4-yl]amino}heptansäuremethylester

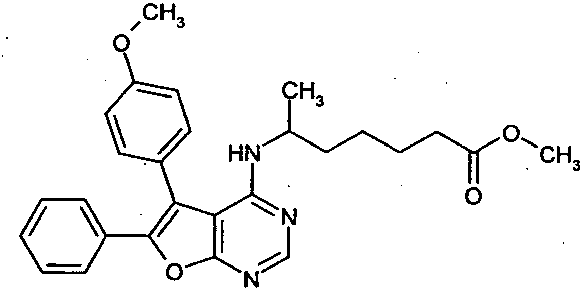

1.00 g (3.80 mmol) 6-{[*(1R)*-1-Phenylethyl]amino}heptansäuremethylester werden in Gegenwart von 100 mg Palladium auf Kohle (10%-ig) in 10 ml Methanol und 1 ml Essigsäure bei Normaldruck über Nacht hydriert. Man saugt danach über Kieselgur ab, wäscht mit Methanol nach und engt ein. Da laut HPLC die Umsetzung noch nicht vollständig ist, hydriert man erneut in 10 ml Methanol und 1 ml Essigsäure in Gegenwart von 100 mg Palladium auf Kohle (10%-ig) bei 4 bar über einen Zeitraum von 6 h. Man saugt wiederum über Kieselgur ab, wäscht mit Methanol nach und engt ein (HPLC: kein Edukt mehr vorhanden). Der so erhaltene 6-Aminoheptansäuremethylester wird als Rohprodukt direkt weiter umgesetzt.

200 mg (0.59 mmol) 4-Chlor-5-(4-methoxyphenyl)-6-phenylfuro[2,3-d]pyrimidin in 1 ml DMSO werden mit 260 mg (ca. 1.19 mmol) des oben erhaltenen 6-Aminoheptansäuremethylesters und mit 307 mg (2.38 mmol) *N,N-*Diisopropylamin versetzt und über Nacht bei 100°C gerührt. Man lässt danach abkühlen und engt ein. Der Rückstand wird durch präparative HPLC gereinigt: Man erhält 80 mg (29.3% d. Th.) der Zielverbindung.
LC-MS (Methode 7): Rₜ = 4.32 min.; m/z = 460 (M+H)⁺
¹H-NMR (400 MHz, CDCl₃): δ = 8.39 (s, 1H), 7.55 (m, 2H), 7.39 (d, 2H), 7.27 (m, 3H), 7.08 (d, 2H), 4.52 (d, 1H), 4.28-4.17 (m, 1H), 3.91 (s, 3H), 3.65 (s, 3H), 2.27 (t, 2H), 1.62-1.52 (m, 2H), 1.44-1.13 (m, 4H), 1.06 (d, 3H).
HPLC [Säule: Daicel Chiralpak AD 250 mm x 2 mm; Eluent: 99% n-Heptan, 1% Ethanol mit 0.2% Trifluoressigsäure; Fluss: 0.2 ml/min; Detektion: 322 nm; Temperatur 25°C]:
Enantiomer 1: Rₜ = 29.8 min, 18.6%
Enantiomer 2: Rₜ = 32.7 min, 81.4%
ee = 62.8%.

### Beispiel 160

### 6-{[5-(4-Methoxyphenyl)-6-phenylfuro[2,3-d]pyrimidin-4-yl]amino}heptansäure

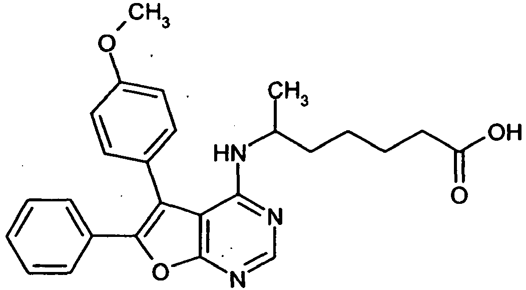

65 mg (0.141 mmol) 6-{[5-(4-Methoxyphenyl)-6-phenylfuro[2,3-d]pyrimidin-4-yl]amino}heptansäuremethylester werden in 2.5 ml THF vorgelegt. Man gibt 1.42 ml (1.42 mmol) 1 N Natronlauge hinzu und rührt über Nacht bei RT. Man verdünnt danach mit *tert*.-Butylmethylether und stellt mit 10%-iger Zitronensäure-Lösung auf ca. pH 5-6 ein. Man extrahiert die wässrige Phase einmal mit *tert*.-Butylmethylether, trocknet die vereinigten organischen Phasen über Magnesiumsulfat und engt ein. Es werden 59.5 mg (94.4% d. Th.) der Zielverbindung erhalten.
LC-MS (Methode 8): Rₜ = 2.75 min.; m/z = 446 (M+H)⁺
¹H-NMR (400 MHz, CDCl₃): δ = 8.39 (s, 1H), 7.53 (m, 2H), 7.39 (d, 2H), 7.26 (m, 3H), 7.05 (d, 2H), 4.51 (d, 1H), 4.26-4.17 (m, 1H), 3.91 (s, 3H), 2.30 (t, 2H), 1.64-1.54 (m, 2H), 1.40-1.15 (m, 4H), 1.06 (d, 3H).

### B. Bewertung der pharmakologischen Wirksamkeit

Die pharmakologische Wirkung der erfindungsgemäßen Verbindungen kann in folgenden Assays gezeigt werden:

### B-1. Bindungsstudien mit Prostacyclin-Rezeptoren (IP-Rezeptoren) von humanen Thrombozytenmembranen

Zur Gewinnung von Thrombozytenmembranen werden 50 ml Humanblut (Buffy coats mit CDP-Stabilizer, Fa. Maco Pharma, Langen) für 20 min bei 160 x g zentrifugiert. Der Überstand (plättchenreiches Plasma, PRP) wird abgenommen und anschließend nochmals bei 2000 x g für 10 min bei Raumtemperatur zentrifugiert. Das Sediment wird in 50 mM Tris-(hydroxymethyl)-amino-methan, welches mit 1 N Salzsäure auf einen pH-Wert von 7.4 eingestellt ist, re-suspendiert und bei -20°C über Nacht aufbewahrt. Am folgenden Tag wird die Suspension bei 80000 x g und 4°C 30 min lang zentrifugiert. Der Überstand wird verworfen. Das Sediment wird in 50 mM Tris-(hydroxymethyl)-aminomethan/Salzsäure, 0.25 mM Ethylendiamintetraessigsäure (EDTA), pH 7.4 re-suspendiert und danach nochmals bei 80000 x g und 4°C für 30 min zentrifugiert. Das Membransediment wird in Bindungspuffer (50 mM Tris-(hydroxymethyl)-aminomethan/Salzsäure, 5 mM Magnesiumchlorid, pH 7.4) aufgenommen und bis zum Bindungsversuch bei -70°C gelagert.

Für den Bindungsversuch werden 3 nM ³H-Iloprost (592 GBq/mmol, Fa. AmershamBioscience) 60 min lang mit 300-1000 µg/ml humanen Thrombozytenmembranen pro Ansatz (max. 0.2 ml) in Gegenwart der Testsubstanzen bei Raumtemperatur inkubiert. Nach dem Abstoppen werden die Membranen mit kaltem Bindungspuffer versetzt und mit 0.1% Rinderserumalbumin gewaschen. Nach Zugabe von Ultima Gold-Szintillator wird die an den Membranen gebundene Radioaktivität mittels eines Szintillationszählers quantifiziert. Die nicht-spezifische Bindung wird als Radioaktivität in Gegenwart von 1 µM Iloprost (Fa. Cayman Chemical, Ann Arbor) definiert und beträgt in der Regel < 25% der gebundenen Gesamt-Radioaktivität. Die Bindungsdaten (IC₅₀-Werte) werden mittels des Programmes GraphPad Prism Version 3.02 bestimmt.

Repräsentative Ergebnisse zu den erfindungsgemäßen Verbindungen sind in Tabelle 1 aufgeführt:

**Tabelle 1**

| **Beispiel Nr.** | **IC₅₀ [nM]** |
|---|---|
| 1 | 206 |
| 4 | 34 |
| 14 | 49 |
| 15 | 82 |
| 33 | 64 |
| 36 | 217 |
| 45 | 895 |
| 49 | 159 |
| 63 | 37 |
| 69 | 9 |
| 80 | 22 |
| 83 | 20 |
| 85 | 470 |
| 92 | 219 |
| 95 | 10 |
| 113 | 51 |
| 117 | 84 |
| 122 | 48 |
| 128 | 33 |
| 138 | 53 |
| 140 | 52 |
| 142 | 2.5 |
| 146 | 7 |
| 152 | 3.7 |
| 154 | 3.8 |
| 156 | 13 |

### B-2. IP-Rezeptor-Stimulierung auf Ganzzellen

Die IP-agonistische Wirkung von Testsubstanzen wird mit Hilfe der humanen Erythroleukämie-Zelllinie (HEL), die den IP-Rezeptor endogen exprimiert, bestimmt [Murray, R., FEBS Letters 1989, 1: 172-174]. Dazu werden die Suspensionszellen (4 x 10⁷ Zellen/ml) in Puffer [10 mm HEPES (4-(2-Hydroxyethyl)-1-piperazinethansulfonsäure) / PBS (Phosphat-gepufferte Salzlösung, Fa. Oxoid, UK)], 1 mM Calciumchlorid, 1 mM Magnesiumchlorid, 1 mM IBMx (3-Isobutyl-1-methylxanthin), pH 7.4, mit der jeweiligen Testsubstanz 5 Minuten lang bei 30°C inkubiert. Anschließend wird die Reaktion durch Zugabe von 4°C kaltem Ethanol gestoppt und die Ansätze weitere 30 Minuten bei 4°C gelagert. Danach werden die Proben bei 10000 x g und 4°C zentrifugiert. Der resultierende Überstand wird verworfen und das Sediment zur Bestimmung der Konzentration an cyclischem Adenosinmonophosphat (cAMP) in einem kommerziell erhältlichen cAMP-Radioimmunoassay (Fa. IBL, Hamburg) eingesetzt. IP-Agonisten führen in diesem Test zu einem Anstieg der cAMP-Konzentration, IP-Antagonisten sind wirkunglos. Die effektive Konzentration (EC₅₀-Werte) wird mittels des Programmes GraphPad Prism Version 3.02 bestimmt.

### B-3. Thrombozytenaggregationshemmung in vitro

Zur Bestimmung der Thrombozytenaggregationshemmung wird Blut von gesunden Probanden beiderlei Geschlechts verwendet. Einem Teil 3.8%-iger Natriumcitrat-Lösung als Koagulans werden 9 Teile Blut zugemischt. Das Blut wird mit 900 U/min für 20 min zentrifugiert. Der pH-Wert des gewonnenen plättchenreichen Plasmas wird mit ACD-Lösung (Natriumcitrat/Citronensäure/Glucose) auf pH 6.5 eingestellt. Die Thrombozyten werden anschließend abzentrifugiert, in Puffer aufgenommen und erneut abzentrifugiert. Der Thrombozyten-Niederschlag wird in Puffer aufgenommen und zusätzlich mit 2 mmol/l Calciumchlorid re-suspendiert.

Für die Aggregationsmessungen werden Aliquots der Thrombozytensuspension mit der Prüfsubstanz 10 min lang bei 37°C inkubiert. Anschließend wird die Aggregation durch Zugabe von ADP induziert und mittels der turbidometrischen Methode nach Born im Aggregometer bei 37°C bestimmt [Born G.V.R., J. Physiol. (London) 168, 178-179 (1963)].

### B-4. Blutdruckmessung an narkotisierten Ratten

Männliche Wistar-Ratten mit einem Körpergewicht von 300-350 g werden mit Thiopental (100 mg/kg i.p.) anästhesiert. Nach der Tracheotomie wird die Arteria femoralis zur Blutdruckmessung katheterisiert. Die zu prüfenden Substanzen werden als Lösung oral mittels Schlundsonde oder über die Femoralvene intravenös in einem geeigneten Vehikel verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 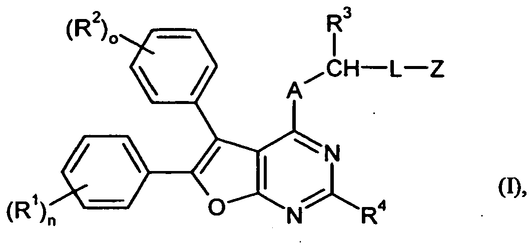 in welcher
A für O, S oder N-R⁵ steht, worin
R⁵ Wasserstoff, (C₁-C₆)-Alkyl, (C₃-C₇)-Cycloalkyl oder (C₄-C₇)-Cycloalkenyl bedeutet,
L für (C₁-C₇)-Alkandiyl oder (C₂-C₇)-Alkendiyl, welche ein- oder zweifach mit Fluor substituiert sein können, oder für eine Gruppe der Formel *-L¹-Q-L² steht, worin
* die Verknüpfungsstelle mit der CHR³-Gruppe,
L¹ (C₁-C₅)-Alkandiyl, welches mit (C₁-C₄)-Alkyl oder (C₁-C₄)-Alkoxy substituiert sein kann,
L² eine Bindung oder (C₁-C₃)-Alkandiyl, welches ein- oder zweifach mit Fluor substituiert sein kann,
und
Q O oder N-R⁶, worin
R⁶ Wasserstoff, (C₁-C₆)-Alkyl oder (C₃-C₇)-Cycloalkyl darstellt,
bedeuten,
Z für eine Gruppe der Formel 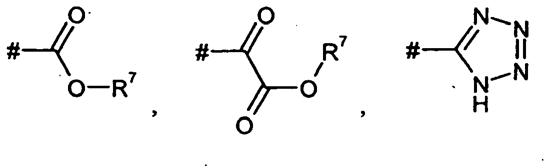 oder 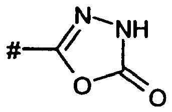 steht, worin
# die Verknüpfungsstelle mit der Gruppe L
und
R⁷ Wasserstoff oder (C₁-C₄)-Alkyl bedeutet,
R¹ und R² unabhängig voneinander für einen Substituenten ausgewählt aus der Reihe Halogen, Cyano, Nitro, (C₁-C₆)-Alkyl, (C₂-C₆)-Alkenyl, (C₂-C₄)-Alkinyl, (C₃-C₇)-Cycloalkyl, (C₄-C₇)-Cycloalkenyl, (C₁-C₆)-Alkoxy, Trifluormethyl, Trifluormethoxy, (C₁-C₆)-Alkylthio, (C₁-C₆)-Acyl, Amino, Mono-(C₁-C₆)-alkylamino, Di-(C₁-C₆)-alkylamino und (C₁-C₆)-Acylamino stehen,
wobei (C₁-C₆)-Alkyl und (C₁-C₆)-Alkoxy ihrerseits jeweils mit Cyano, Hydroxy, (C₁-C₄)-Alkoxy, (C₁-C₄)-Alkylthio, Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂-O-, -O-CH₂-CH₂-O- oder -O-CF₂-CF₂-O- bilden,
n und o unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
R³ für Wasserstoff oder (C₁-C₄)-Alkyl, welches mit Hydroxy oder Amino substituiert sein kann, steht
und
R⁴ für Wasserstoff, (C₁-C₄)-Alkyl oder Cyclopropyl steht,
sowie ihre Salze, Solvate und Solvate der Salze.

2. Verbindung der Formel (I) nach Anspruch 1, in welcher
A für O, S oder N-R⁵ steht, worin
R⁵ Wasserstoff, (C₁-C₆)-Alkyl, (C₃-C₇)-Cycloalkyl oder (C₄-C₇)-Cycloalkenyl bedeutet,
L für (C₁-C₇)-Alkandiyl oder (C₂-C₇)-Alkendiyl, welche ein- oder zweifach mit Fluor substituiert sein können, oder für eine Gruppe der Formel *-L¹-Q-L² steht, worin
* die Verknüpfungsstelle mit der CHR³-Gruppe,
L¹ (C₁-C₅)-Alkandiyl,
L² eine Bindung oder (C₁-C₃)-Alkandiyl, welches ein- oder zweifach mit Fluor substituiert sein kann,
und
Q O oder N-R⁶, worin
R⁶ Wasserstoff, (C₁-C₆)-Alkyl oder (C₃-C₇)-Cycloalkyl darstellt,
bedeuten,
Z für eine Gruppe der Formel 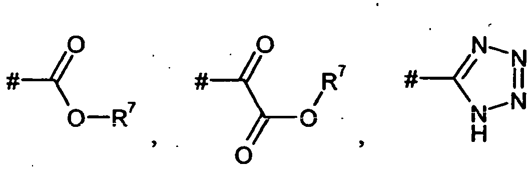 oder 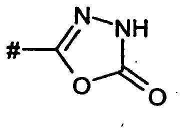 steht, worin
# die Verknüpfungsstelle mit der Gruppe L
und
R⁷ Wasserstoff oder (C₁-C₄)-Alkyl bedeutet,
R¹ und R² unabhängig voneinander für einen Substituenten ausgewählt aus der Reihe Halogen, Cyano, Nitro, (C₁-C₆)-Alkyl, (C₂-C₆)-Alkenyl, (C₂-C₄)-Alkinyl, (C₃-C₇)-Cycloalkyl, (C₄-C₇)-Cycloalkenyl, (C₁-C₆)-Alkoxy, Trifluormethyl, Trifluormethoxy, (C₁-C₆)-Alkylthio, (C₁-C₆)-Acyl, Amino, Mono-(C₁-C₆)-alkylamino, Di-(C₁-C₆)-alkylamino und (C₁-C₆)-Acylamino stehen,
wobei (C₁-C₆)-Alkyl und (C₁-C₆)-Alkoxy ihrerseits jeweils mit Hydroxy, (C₁-C₄)-Alkoxy, Amino, Mono- oder Di-(C₁-C₄)-alkylamino substituiert sein können,
oder
zwei an benachbarte Kohlenstoffatome des jeweiligen Phenylrings gebundene Reste R¹ und/oder R² zusammen eine Gruppe der Formel -O-CH₂-O-, -O-CHF-O-, -O-CF₂-O-, -O-CH₂-CH₂-O- oder -O-CF₂-CF₂-O- bilden,
n und o unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
R³ für Wasserstoff oder (C₁-C₄)-Alkyl, welches mit Hydroxy oder Amino substituiert sein kann, steht
und
R⁴ für Wasserstoff, (C₁-C₄)-Alkyl oder Cyclopropyl steht,
sowie ihre Salze, Solvate und Solvate der Salze.

3. Verbindung der Formel (I) nach Anspruch 1 oder 2, in welcher
A für O oder N-R⁵ steht, worin
R⁵ Wasserstoff, (C₁-C₄)-Alkyl oder (C₃-C₆)-Cycloalkyl bedeutet,
L für (C₃-C₇)-Alkandiyl oder (C₃-C₇)-Alkendiyl, welche ein- oder zweifach mit Fluor substituiert sein können, oder für eine Gruppe der Formel *-L¹-Q-L² steht, worin
* die Verknüpfungsstelle mit der CHR³-Gruppe,
L¹ (C₁-C₃)-Alkandiyl,
L² (C₁-C₃)-Alkandiyl, welches ein- oder zweifach mit Fluor substituiert sein kann,
und
Q O oder N-R⁶, worin
R⁶ Wasserstoff, (C₁-C₃)-Alkyl oder Cyclopropyl darstellt,
bedeuten,
Z für eine Gruppe der Formel 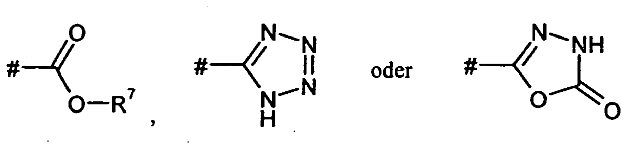 steht, worin
# die Verknüpfungsstelle mit der Gruppe L
und
R⁷ Wasserstoff, Methyl oder Ethyl bedeutet,
R¹ und R² unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄)-Alkoxy, trifluormethyl, Trifluormethoxy, (C₁-C₄)-Alkylthio, (C₁-C₅)-Acyl, Amino, Mono-(C₁-C₄)-alkylamino, Di-(C₁-C₄)-alkylamino und (C₁-C₄)-Acylamino stehen
oder
zwei an benachbarte Kohlenstoffatome des jeweiligen Phenylrings gebundene Reste R¹ und/oder R² zusammen eine Gruppe der Formel -O-CH₂-O-, -O-CHF-O-oder -O-CF₂-O- bilden,
n und o unabhängig voneinander für die Zahl 0, 1, 2 oder 3 stehen,
wobei für den Fall, dass R¹ oder R² mehrfach auftreten, ihre Bedeutungen jeweils gleich oder verschieden sein können,
R³ für Wasserstoff oder (C₁-C₃)-Alkyl, welches mit Hydroxy oder Amino substituiert sein kann, steht
und
R⁴ für Wasserstoff oder (C₁-C₃)-Alkyl steht,
sowie ihre Salze, Solvate und Solvate der Salze.

4. Verbindung der Formel (I) nach Anspruch 1, 2 oder 3, in welcher
A für O oder NH steht,
L für (C₃-C₇)-Alkandiyl, (C₃-C₇)-Alkendiyl oder für eine Gruppe der Formel *-L¹-O-L² steht, worin
* die Verknüpfungsstelle mit der CHR³-Gruppe
und
L¹ und L² unabhängig voneinander (C₁-C₃)-Alkandiyl bedeuten,
Z für eine Gruppe der Formel 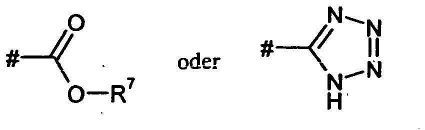 steht, worin
# die Verknüpfungsstelle mit der Gruppe L
und
R⁷ Wasserstoff, Methyl oder Ethyl bedeutet,
R¹ und R² unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄)-Alkoxy, Trifluormethyl, Trifluormethoxy, (C₁-C₄)-Alkylthio, (C₁-C₅)-Acyl, Amino, Mono-(C₁-C₄)-alkylamino, Di-(C₁-C₄)-alkylamino und (C₁-C₄)-Acylamino stehen
oder
zwei an benachbarte Kohlenstoffatome des jeweiligen Phenylrings gebundene Reste R¹ und/oder R² zusammen eine Gruppe der Formel -O-CH₂-O-, -O-CHF-O-oder -O-CF₂-O- bilden,
n und o unabhängig voneinander für die Zahl 0, 1 oder 2 stehen,
wobei für den Fall, dass R¹ oder R² zweifach auftreten, ihre Bedeutungen jeweils gleich oder verschieden sein können,
R³ für Wasserstoff, Methyl oder Ethyl steht
und
R⁴ für Wasserstoff steht,
sowie ihre Salze, Solvate und Solvate der Salze.

5. Verbindung der Formel (I) nach Anspruch 1, 2 oder 3, in welcher
A für O oder NH steht,
L für eine Gruppe der Formel *-L¹-N(CH₃)-L² steht, worin
* die Verknüpfungsstelle mit der CHR³-Gruppe
und
L¹ und L² unabhängig voneinander (C₁-C₃)-Alkandiyl bedeuten,
Z für eine Gruppe der Formel 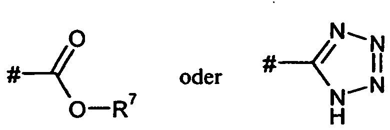 steht, worin
# die Verknüpfungsstelle mit der Gruppe L
und
R⁷ Wasserstoff, Methyl oder Ethyl bedeutet,
R¹ und R² unabhängig voneinander für einen Substituenten ausgewählt aus der Reihe Fluor, Chlor, Cyano, (C₁-C₅)-Alkyl, (C₂-C₅)-Alkenyl, (C₃-C₆)-Cycloalkyl, (C₄-C₆)-Cycloalkenyl, (C₁-C₄)-Alkoxy, Trifluormethyl, Trifluormethoxy, (C₁-C₄)-Alkylthio, (C₁-C₅)-Acyl, Amino, Mono-(C₁-C₄)-alkylamino, Di-(C₁-C₄)-alkylamino und (C₁-C₄)-Acylamino stehen
oder
zwei an benachbarte Kohlenstoffatome des jeweiligen Phenylrings gebundene Reste R¹ und/oder R² zusammen eine Gruppe der Formel -O-CH₂-O-, -O-CHF-O-oder -O-CF₂-O- bilden,
n und o unabhängig voneinander für die Zahl 0, 1 oder 2 stehen,
wobei für den Fall, dass R¹ oder R² zweifach auftreten, ihre Bedeutungen jeweils gleich oder verschieden sein können,
R³ für Wasserstoff, Methyl oder Ethyl steht
und
R⁴ für Wasserstoff steht,
sowie ihre Salze, Solvate und Solvate der Salze.

6. Verbindung der Formel (I) nach einem der Ansprüche 1 bis 5, in welcher
A für O oder NH steht,
L für (C₃-C₇)-Alkandiyl, (C₃-C₇)-Alkendiyl oder für eine Gruppe der Formel *-L¹-Q-L² steht, worin
* die Verknüpfungsstelle mit der CHR³-Gruppe,
L¹ und L² unabhängig voneinander (C₁-C₃)-Alkandiyl
und
Q O oder N(CH₃) bedeuten,
Z für eine Gruppe der Formel 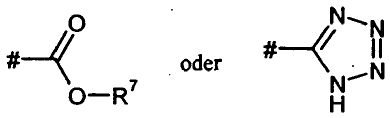 steht, worin
# die Verknüpfungsstelle mit der Gruppe L
und
R⁷ Wasserstoff, Methyl oder Ethyl bedeutet,
R¹ für einen Substituenten ausgewählt aus der Reihe Fluor, Chlor, Methyl, Ethyl, Vinyl, Trifluormethyl und Methoxy steht,
R² für einen Substituenten ausgewählt aus der Reihe Fluor, Chlor, Cyano, Methyl, Ethyl, n-Propyl, Vinyl, Trifluormethyl, Methoxy, Ethoxy, Trifluormethoxy, Methylthio, Ethylthio, Amino, Methylamino und Ethylamino steht,
n und o unabhängig voneinander für die Zahl 0, 1 oder 2 stehen,
wobei für den Fall, dass R¹ oder R² zweifach auftreten, ihre Bedeutungen jeweils gleich oder verschieden sein können,
R³ für Wasserstoff, Methyl oder Ethyl steht
und
R⁴ für Wasserstoff steht,
sowie ihre Salze, Solvate und Solvate der Salze.

7. Verfahren zur Herstellung von Verbindungen der Formel (I), wie in den Ansprüchen 1 bis 6 definiert, **dadurch gekennzeichnet, dass** man entweder
[A] Verbindungen der Formel (II) 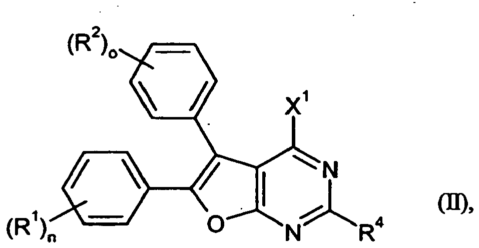 in welcher R¹, R², R⁴, n und o jeweils die in den Ansprüchen 1 bis 6 angegebenen Bedeutungen haben
und
X¹ für eine Abgangsgruppe wie beispielsweise Halogen, insbesondere für Chlor steht,
in Gegenwart einer Base gegebenenfalls in einem inerten Lösungsmittel mit einer Verbindung der Formel (III) 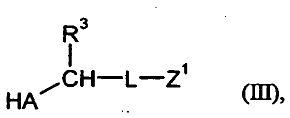 in welcher A, L und R³ jeweils die in den Ansprüchen 1 bis 6 angegebenen Bedeutungen haben
und
Z¹ für Cyano oder eine Gruppe der Formel -[C(O)]_{y}COOR^{7A} steht, worin
y die Zahl 0 oder 1
und
R^{7A} (C₁-C₄)-Alkyl bedeutet,
zu Verbindungen der Formel (IV) 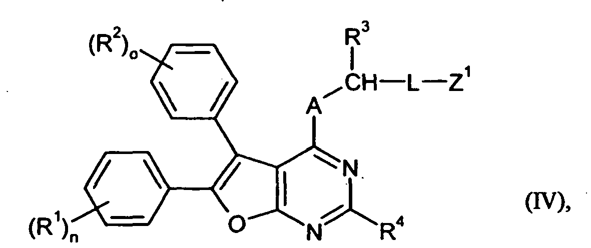 in welcher A, L, Z¹, R¹, R², R³, R⁴, n und o jeweils die oben angegebenen Bedeutungen haben,
umsetzt
oder
[B] Verbindungen der Formel (V-1) 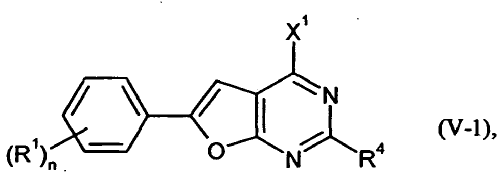 in welcher R¹, R⁴, X¹ und n jeweils die in den Ansprüchen 1 bis 6 angegebenen Bedeutungen haben,
in Gegenwart einer Base gegebenenfalls in einem inerten Lösungsmittel mit einer Verbindung der Formel (III) zu Verbindungen der Formel (VI-1) 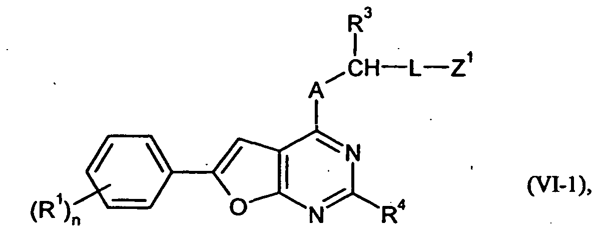 in welcher A, L, Z¹, R¹, R³, R⁴ und n jeweils die oben angegebenen Bedeutungen haben,
umsetzt, dann in einem inerten Lösungsmittel zu Verbindungen der Formel (VII-1) 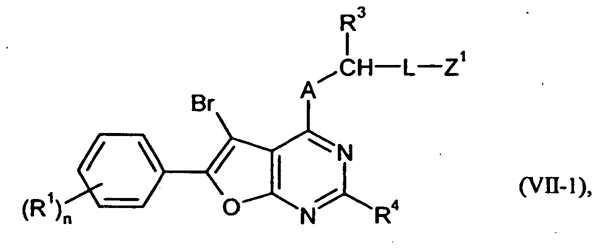 in welcher A, L, Z¹, R¹, R³, R⁴ und n jeweils die oben angegebenen Bedeutungen haben,
bromiert und diese anschließend in einem inerten Lösungsmittel in Gegenwart einer Base und eines geeigneten Palladium-Katalysators mit einer Phenylboronsäure der Formel (VIII-1) 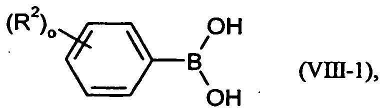 in welcher R² und o die in den Ansprüchen 1 bis 6 angegebenen Bedeutungen haben,
zu Verbindungen der Formel (IV) kuppelt
oder
[C] Verbindungen der Formel (V-2) 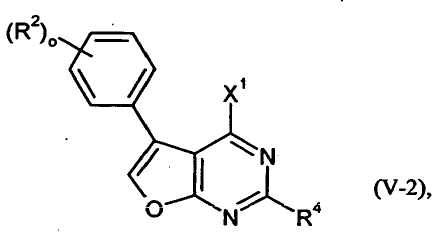 in welcher R², R⁴, X¹ und o jeweils die in den Ansprüchen 1 bis 6 angegebenen Bedeutungen haben,
in Gegenwart einer Base gegebenenfalls in einem inerten Lösungsmittel mit einer Verbindung der Formel (III) zu Verbindungen der Formel (VI-2) 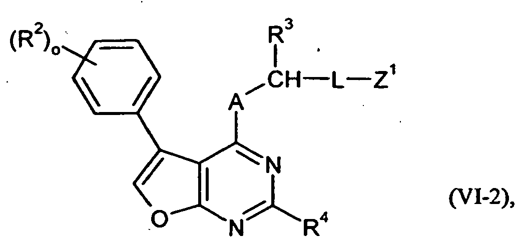 in welcher A, L, Z¹, R², R³, R⁴ und o jeweils die oben angegebenen Bedeutungen haben,
umsetzt, dann in einem inerten Lösungsmitteln zu Verbindungen der Formel (VII-2) 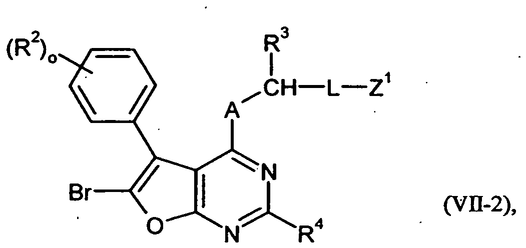 in welcher A, L, Z¹, R², R³, R⁴ und o jeweils die oben angegebenen Bedeutungen haben,
bromiert und diese anschließend in einem inerten Lösungsmittel in Gegenwart einer Base und eines geeigneten Palladium-Katalysators mit einer Phenylboronsäure der Formel (VIII-2) 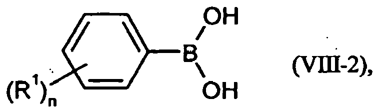 in welcher R¹ und n die in den Ansprüchen 1 bis 6 angegebenen Bedeutungen haben,
zu Verbindungen der Formel (IV) kuppelt,
und die jeweils resultierenden Verbindungen der Formel (IV) dann durch Hydrolyse der Ester- bzw. Cyano-Gruppe Z¹ in die Carbonsäuren der Formel (I-A) 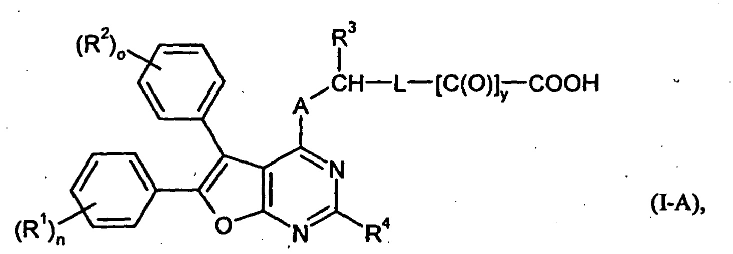 in welcher A, L, R¹, R², R³, R⁴, n, o und y jeweils die oben angegebenen Bedeutungen haben,
überführt und diese gegebenenfalls mit den entsprechenden (i) Lösungsmitteln und/oder (ii) Basen oder Säuren zu ihren Solvaten, Salzen und/oder Solvaten der Salze umsetzt.

8. Verbindung der Formel (I), wie in einem der Ansprüche 1 bis 6 definiert, zur Behandlung und/oder Prophylaxe von Krankheiten.

9. Verwendung einer Verbindung der Formel (I), wie in einem der Ansprüche 1 bis 6 definiert, zur Herstellung eines Arzneimittels zur Behandlung und/oder Prophylaxe von kardiovaskulären Erkrankungen.

10. Arzneimittel enthaltend eine Verbindung der Formel (I), wie in einem der Ansprüche 1 bis 6 definiert, in Kombination mit einem inerten, nicht-toxischen, pharmazeutisch geeigneten Hilfsstoff.

11. Arzneimittel enthaltend eine Verbindung der Formel (I), wie in einem der Ansprüche 1 bis 6 definiert, in Kombination mit einem weiteren Wirkstoff.

12. Arzneimittel nach Anspruch 10 oder 11 zur Behandlung und/oder Prophylaxe von kardiovaskulären Erkrankungen.

13. Verbindungen zur Behandlung und/oder Prophylaxe von kardiovaskulären Erkrankungen bei Menschen und Tieren gemäβ einer Verbindung der Formel (I), wie in einem der Ansprüche 1 bis 6 definiert, oder eines Arzneimittels, wie in einem der Ansprüche 10 bis 12 definiert.

## Claims

1. Compound of formula (I) 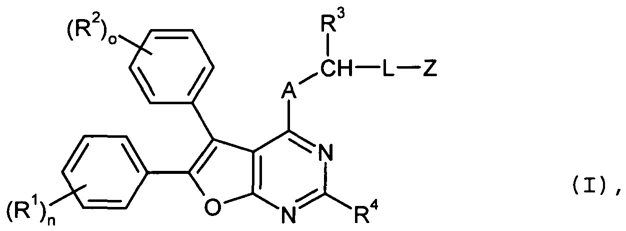 in which
A stands for O, S or N-R⁵, where
R⁵ denotes hydrogen, (C₁-C₆) alkyl, (C₃-C₇) cycloalkyl or (C₄-C₇) cycloalkenyl,
L stands for (C₁-C₇) alkanediyl or (C₂-C₇) alkenediyl, which can be substituted singly or doubly with fluorine, or for a group of formula *-L¹-Q-L², where
* denotes the point of linkage with the CHR³ group,
L¹ denotes (C₁-C₅) alkanediyl, which can be substituted with (C₁-C₄) alkyl or (C₁-C₄) alkoxy,
L² denotes a bond or (C₁-C₃) alkanediyl, which can be substituted singly or doubly with fluorine,
and
Q denotes O or N-R⁶, where
R⁶ represents hydrogen, (C₁-C₆) alkyl
or (C₃-C₇) cycloalkyl,
Z stands for a group of formula 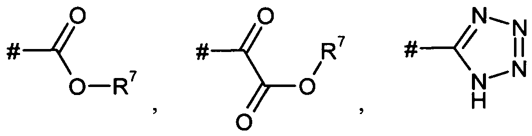 or 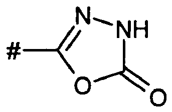 where
# denotes the point of linkage with group L
and
R⁷ denotes hydrogen or (C₁-C₄) alkyl,
R¹ and R², independently of one another, stand for a substituent selected from the group comprising halogen, cyano, nitro, (C₁-C₆) alkyl, (C₂-C₆) alkenyl, (C₂-C₄) alkinyl, (C₃-C₇) cycloalkyl, (C₄-C₇) cycloalkenyl, (C₁-C₆) alkoxy, trifluoromethyl, trifluoromethoxy, (C₁-C₆) alkylthio, (C₁-C₆) acyl, amino, mono-(C₁-C₆) alkylamino, di -(C₁-C₆) alkylamino and (C₁-C₆) acylamino,
in which (C₁-C₆) alkyl and (C₁-C₆) alkoxy can in their turn each be substituted with cyano, hydroxy, (C₁-C₄) alkoxy, (C₁-C₄) alkylthio, amino, mono- or di-(C₁-C₄) alkylamino,
or
two residues R¹ and/or R² bound to adjacent carbon atoms of the respective phenyl ring together form a group of formula -O-CH₂-O-, -O-CHF-O-, -O-CF₂-O-, -O-CH₂-CH₂-O- or -O-CF₂-CF₂-O-,
n and o, independently of one another, stand for the number 0, 1, 2 or 3,
and for the case when R¹ or R² occurs more than once, their meanings can in each case be identical or different,
R³ stands for hydrogen or (C₁-C₄) alkyl, which can be substituted with hydroxy or amino,
and
R⁴ stands for hydrogen, (C₁-C₄) alkyl or cyclopropyl,
and their salts, solvates and solvates of the salts.

2. Compound of formula (I) according to Claim 1, in which
A stands for O, S or N-R⁵, where
R⁵ denotes hydrogen, (C₁-C₆) alkyl, (C₃-C₇) cycloalkyl or (C₄-C₇) cycloalkenyl,
L stands for (C₁-C₇) alkanediyl or (C₂-C₇) alkenediyl, which can be substituted singly or doubly with fluorine, or for a group of formula *-L¹-Q-L², where
* denotes the point of linkage with the CHR³ group,
L¹ denotes (C₁-C₅) alkanediyl,
L² denotes a bond or (C₁-C₃) alkanediyl, which can be substituted singly or doubly with fluorine,
and
Q denotes O or N-R⁶, where
R⁶ represents hydrogen, (C₁-C₆) alkyl
or (C₃-C₇) cycloalkyl,
Z stands for a group of formula 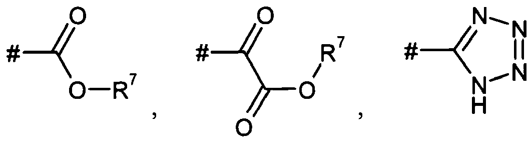 or 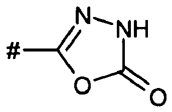 where
# denotes the point of linkage with group L
and
R⁷ denotes hydrogen or (C₁-C₄) alkyl,
R¹ and R², independently of one another, stand for a substituent selected from the group comprising halogen, cyano, nitro, (C₁-C₆) alkyl, (C₂-C₆) alkenyl, (C₂-C₄) alkinyl, (C₃-C₇) cycloalkyl, (C₄-C₇) cycloalkenyl, (C₁-C₆) alkoxy, trifluoromethyl, trifluoromethoxy, (C₁-C₆) alkylthio, (C₁-C₆) acyl, amino, mono-(C₁-C₆) alkylamino, di-(C₁₋C₆) alkylamino and (C₁-C₆) acylamino,
and (C₁-C₆) alkyl and (C₁-C₆) alkoxy in their turn can each be substituted with hydroxy, (C₁-C₄) alkoxy, amino, mono- or di- (C₁-C₄) alkylamino,
or
two residues R¹ and/or R² bound to adjacent carbon atoms of the respective phenyl ring together form a group of formula -O-CH₂-O-, -O-CHF-O-, -O-CF₂-O-, -O-CH₂-CH₂-O- or -O-CF₂-CF₂-O-,
n and o, independently of one another, stand for the number 0, 1, 2 or 3,
and for the case when R¹ or R² occurs more than once, their meanings can in each case be identical or different,
R³ stands for hydrogen or (C₁-C₄) alkyl, which can be substituted with hydroxy or amino,
and
R⁴ stands for hydrogen, (C₁-C₄) alkyl or cyclopropyl,
and their salts, solvates and solvates of the salts.

3. Compound of formula (I) according to Claim 1 or 2, in which
A stands for O or N-R⁵, where
R⁵ denotes hydrogen, (C₁-C₄) alkyl or (C₃-C₆) cycloalkyl,
L stands for (C₃- C₇) alkanediyl or (C₃-C7) alkenediyl, which can be substituted singly or doubly with fluorine, or for a group of formula *-L¹-Q-L², where
* denotes the point of linkage with the CHR³ group,
L¹ denotes (C₁-C₃) alkanediyl,
L² denotes (C₁-C₃) alkanediyl, which can be substituted singly or doubly with fluorine,
and
Q denotes O or N-R⁶, where
R⁶ represents hydrogen, (C₁-C₃) alkyl or cyclopropyl,
Z stands for a group of formula 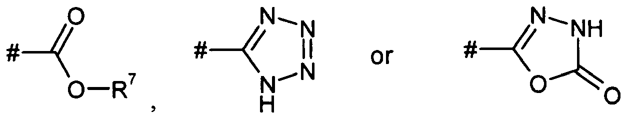 where
# denotes the point of linkage with group L
and
R⁷ denotes hydrogen, methyl or ethyl,
R¹ and R², independently of one another, stand for a substituent selected from the group comprising fluorine, chlorine, cyano, (C₁-C₅) alkyl, (C₂-C₅) alkenyl, (C₃-C₆) cycloalkyl, (C₄-C₆) cycloalkenyl, (C₁-C₄) alkoxy, trifluoromethyl, trifluoromethoxy, (C₁-C₄) alkylthio, (C₁-C₅) acyl, amino, mono-(C₁-C₄) alkylamino, di-(C₁-C₄) alkylamino and (C₁-C₄) acylamino
or
two residues R¹ and/or R² bound to adjacent carbon atoms of the respective phenyl ring together form a group of formula -O-CH₂-O-, -O-CHF-O- or -O-CF₂-O-,
n and o, independently of one another, stand for the number 0, 1, 2 or 3,
and for the case when R¹ or R² occurs more than once, their meanings can in each case be identical or different,
R³ stands for hydrogen or (C₁-C₃) alkyl, which can be substituted with hydroxy or amino,
and
R⁴ stands for hydrogen or (C₁-C₃) alkyl,
and their salts, solvates and solvates of the salts.

4. Compound of formula (I) according to Claim 1, 2 or 3, in which
A stands for O or NH,
L stands for (C₃-C₇) alkanediyl, (C₃-C₇) alkenediyl or for a group of formula *-L¹-O-L², where
* denotes the point of linkage with the CHR³ group
and
L¹ and L², independently of one another, denote (C₁-C₃) alkanediyl,
Z stands for a group of formula 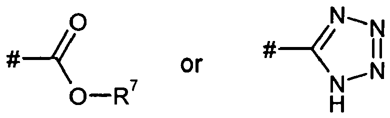 where
# denotes the point of linkage with group L
and
R⁷ denotes hydrogen, methyl or ethyl,
R¹ and R², independently of one another, stand for a substituent selected from the group comprising fluorine, chlorine, cyano, (C₁-C₅) alkyl, (C₂-C₅) alkenyl, (C₃-C₆) cycloalkyl, (C₄-C₆) cycloalkenyl, (C₁-C₄) alkoxy,
trifluoromethyl, trifluoromethoxy, (C₁-C₄) alkylthio, (C₁-C₅) acyl, amino, mono-(C₁-C₄) alkylamino, di-(C₁-C₄) alkylamino and (C₁-C₄) acylamino
or
two residues R¹ and/or R² bound to adjacent carbon atoms of the respective phenyl ring together form a group of formula -O-CH₂-O-, -O-CHF-O- or -O-CF₂-O-,
n and o, independently of one another, stand for the number 0, 1 or 2,
and for the case when R¹ or R² occurs twice, their meanings can in each case be identical or different,
R³ stands for hydrogen, methyl or ethyl
and
R⁴ stands for hydrogen,
and their salts, solvates and solvates of the salts.

5. Compound of formula (I) according to Claim 1, 2 or 3, in which
A stands for O or NH,
L stands for a group of formula *-L¹-N(CH₃)-L², where
* denotes the point of linkage with the CHR³ group
and
L¹ and L², independently of one another, denote (C₁-C₃) alkanediyl,
Z stands for a group of formula 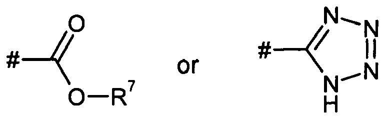 where
# denotes the point of linkage with group L
and
R⁷ denotes hydrogen, methyl or ethyl,
R¹ and R², independently of one another, stand for a substituent selected from the group comprising fluorine, chlorine, cyano, (C₁-C₅) alkyl, (C₂-C₅) alkenyl, (C₃-C₆) cycloalkyl, (C₄-C₆) cycloalkenyl, (C₁-C₄) alkoxy, trifluoromethyl, trifluoromethoxy, (C₁-C₄) alkylthio, (C₁-C₅) acyl, amino, mono-(C₁-C₄) alkylamino, di-(C₁-C₄) alkylamino and (C₁-C₄) acylamino
or
two residues R¹ and/or R² bound to adjacent carbon atoms of the respective phenyl ring together form a group of formula -O-CH₂-O-, -O-CHF-O- or -O-CF₂-O-,
n and o, independently of one another, stand for the number 0, 1 or 2,
and for the case when R¹ or R² occurs twice, their meanings can in each case be identical or different,
R³ stands for hydrogen, methyl or ethyl
and
R⁴ stands for hydrogen,
and their salts, solvates and solvates of the salts.

6. Compound of formula (I) according to one of the
Claims 1 to 5, in which
A stands for O or NH,
L stands for (C₃-C₇) alkanediyl, (C₃-C₇) alkenediyl or for a group of formula *-L¹-Q-L², where
* denotes the point of linkage with the CHR³ group,
L¹ and L², independently of one another, denote (C₁-C₃) alkanediyl
and
Q denotes O or N(CH₃),
Z stands for a group of formula 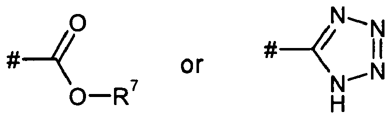 where
# denotes the point of linkage with group L
and
R⁷ denotes hydrogen, methyl or ethyl,
R¹ stands for a substituent selected from the group comprising fluorine, chlorine, methyl, ethyl, vinyl, trifluoromethyl and methoxy,
R² stands for a substituent selected from the group comprising fluorine, chlorine, cyano, methyl, ethyl, n-propyl, vinyl, trifluoromethyl, methoxy, ethoxy, trifluoromethoxy, methylthio, ethylthio, amino, methylamino and ethylamino,
n and o, independently of one another, stand for the number 0, 1 or 2,
and for the case when R¹ or R² occurs twice, their meanings can in each case be identical or different,
R³ stands for hydrogen, methyl or ethyl
and
R⁴ stands for hydrogen,
and their salts, solvates and solvates of the salts.

7. Method of production of compounds of formula (I), as defined in Claims 1 to 6, **characterized in that** either
[A] Compounds of formula (II) 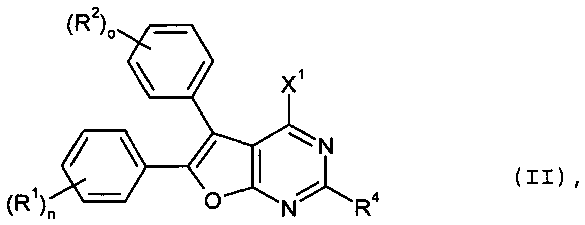 in which R¹, R², R⁴, n and o have the respective meanings given in Claims 1 to 6 and
X¹ stands for a leaving group such as halogen, especially for chlorine,
in the presence of a base if necessary in an inert solvent with a compound of formula (III) 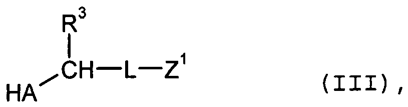 in which A, L and R³ have the respective meanings given in Claims 1 to 6
and
Z¹ stands for cyano or a group of formula - [C(O)]_{y}-COOR^{7A}, where
y denotes the number 0 or 1
and
R^{7A} denotes (C₁-C₄) alkyl,
are converted to compounds of formula (IV) 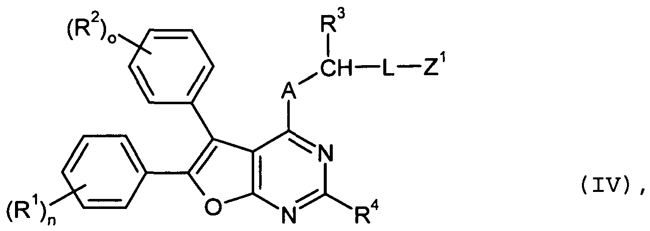 in which A, L, Z¹, R¹, R², R³, R⁴, n and o have the respective meanings given above,
or
[B] Compounds of formula (V-1) 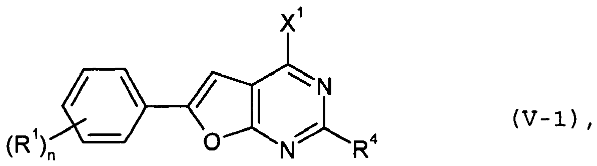 in which R¹, R⁴, X¹ and n have the respective meanings given in Claims 1 to 6,
are reacted in the presence of a base if necessary in an inert solvent with a compound of formula (III) to compounds of formula (VI-1) 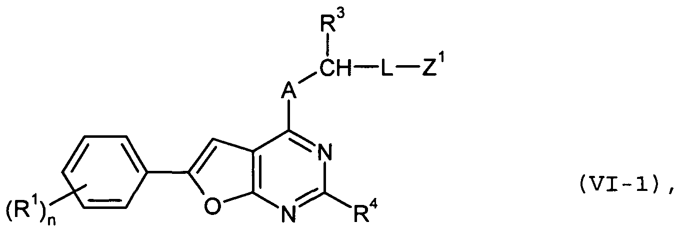 in which A, L, Z¹, R¹, R³, R⁴ and n have the respective meanings given above,
then brominated in an inert solvent to compounds of formula (VII-1) 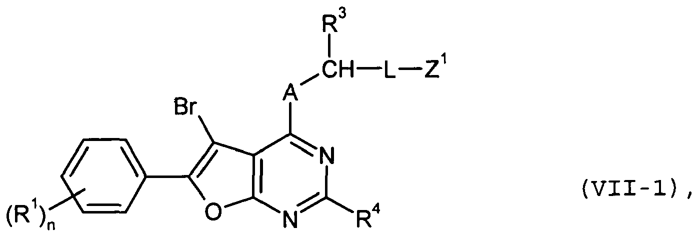 in which A, L, Z¹, R¹, R³, R⁴ and n have the respective meanings given above,
and these are then coupled in an inert solvent in the presence of a base and a suitable palladium catalyst with a phenylboronic acid of formula (VIII-1) 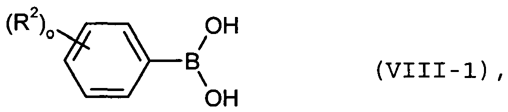 in which R² and o have the meanings given in Claims 1 to 6,
to compounds of formula (IV)
or
[C] Compounds of formula (V-2) 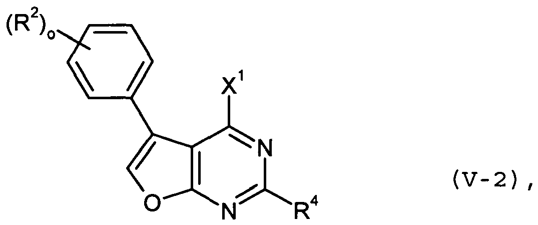 in which R², R⁴, X¹ and o have the respective meanings given in Claims 1 to 6,
are reacted in the presence of a base if necessary in an inert solvent with a compound of formula (III) to compounds of formula (VI-2) 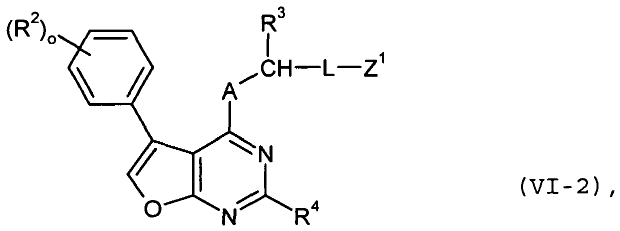 in which A, L, Z¹, R², R³, R⁴ and o have the respective meanings given above,
then brominated in an inert solvent to compounds of formula (VII-2) 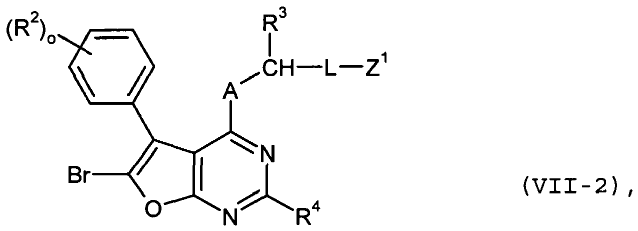 in which A, L, Z¹, R², R³, R⁴ and o have the respective meanings given above,
and these are then coupled in an inert solvent in the presence of a base and a suitable palladium catalyst with a phenylboronic acid of formula (VIII-2) 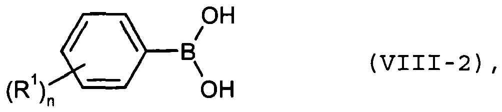 in which R¹ and n have the meanings given in Claims 1 to 6,
to compounds of formula (IV),
and in each case the resultant compounds of formula (IV) are then converted by hydrolysis of the ester or cyano group Z¹ to the carboxylic acids of formula (I-A) 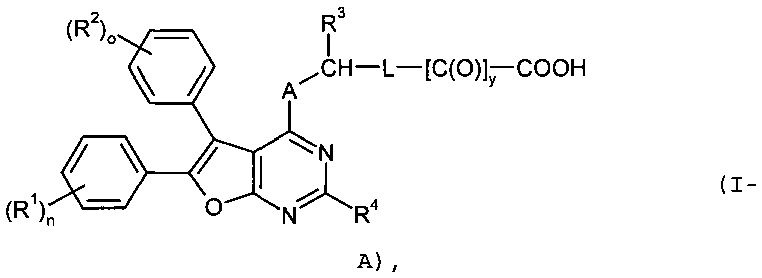 in which A, L, R¹, R², R³, R⁴, n, o and y have the respective meanings given above,
and these are converted if necessary with the corresponding (i) solvents and/or (ii) bases or acids to their solvates, salts and/or solvates of the salts.

8. Compound of formula (I), as defined in one of the Claims 1 to 6, for the treatment and/or prophylaxis of diseases.

9. Use of a compound of formula (I), as defined in one of the Claims 1 to 6, for the production of a medicinal product for the treatment and/or prophylaxis of cardiovascular diseases.

10. Medicinal product containing a compound of formula (I), as defined in one of the Claims 1 to 6, in combination with an inert, nontoxic, pharmaceutically acceptable excipient.

11. Medicinal product containing a compound of formula (I), as defined in one of the Claims 1 to 6, in combination with another active substance.

12. Medicinal product according to Claim 10 or 11 for the treatment and/or prophylaxis of cardiovascular diseases.

13. Compounds for the treatment and/or prophylaxis of cardiovascular diseases in humans and animals according to a compound of formula (I), as defined in one of the Claims 1 to 6, or to a medicinal product, as defined in one of the Claims 10 to 12.

## Revendications

1. Composé de formule (I) 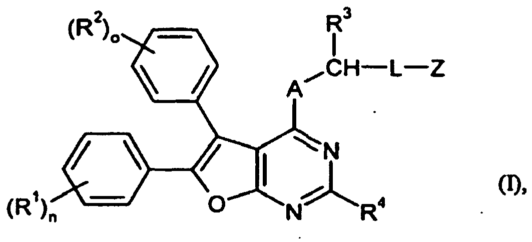 dans laquelle
A représente O, S ou N-R⁵,
R⁵ signifiant hydrogène, alkyle en (C₁-C₆), cycloalkyle en (C₃-C₇) ou cycloalcényle en (C₄-C₇),
L représente alcanediyle en (C₁-C₇) ou alcènediyle en (C₂-C₇), qui peuvent être substitués une ou deux fois avec fluor, ou un groupe de formule *-L¹-Q-L²,
* signifiant l'emplacement de liaison avec le groupe CHR³,
L¹ signifiant alcanediyle en (C₁-C₅), qui peut être substitué avec alkyle en (C₁-C₄) ou alcoxy en (C₁-C₄),
L² signifiant une liaison ou alcanediyle en (C₁-C₃), qui peut être substitué une ou deux fois avec fluor,
et
Q signifiant O ou N-R⁶,
R⁶ représentant hydrogène, alkyle en (C₁-C₆) ou cycloalkyle en (C₃-C₇),
Z représente un groupe de formule 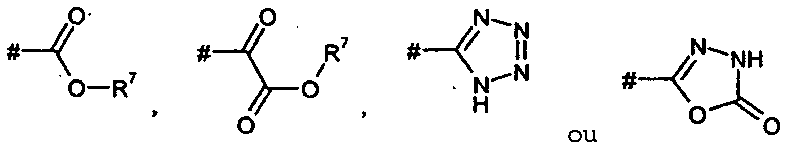
# signifiant l'emplacement de liaison avec le groupe L
et
R⁷ signifiant hydrogène ou alkyle en (C₁-C₄),
R¹ et R² représentent indépendamment l'un de l'autre un substituant choisi dans la série halogène, cyano, nitro, alkyle en (C₁-C₆), alcényle en (C₂-C₆), alcynyle en (C₂-C₄), cycloalkyle en (C₃-C₇), cycloalcényle en (C₄-C₇), alcoxy en (C₁-C₆), trifluorométhyle, trifluorométhoxy, alkylthio en (C₁-C₆), acyle en (C₁-C₆), amino, mono-alkylamino en (C₁-C₆), di-alkylamino en (C₁-C₆) et acylamino en (C₁-C₆),
alkyle en (C₁-C₆) et alcoxy en (C₁-C₆) pouvant chacun de leur côté être substitués avec cyano, hydroxy, alcoxy en (C₁-C₄), alkylthio en (C₁-C₄), amino, mono- ou di-alkylamino en (C₁-C₄),
ou
deux radicaux R¹ et/ou R² reliés à des atomes de carbone voisins du cycle phényle respectif forment ensemble un groupe de formule -O-CH₂-O-, -O-CHF-O-, -O-CF₂-O-, -O-CH₂-CH₂-O- ou -O-CF₂-CF₂-O-,
n et o représentent indépendamment l'un de l'autre le nombre 0, 1, 2 ou 3, lorsque R¹ ou R² apparaissent à plusieurs reprises, leurs significations pouvant à chaque fois être identiques ou différentes,
R³ représente hydrogène ou alkyle en (C₁-C₄), qui peut être substitué avec hydroxy ou amino,
et
R⁴ représente hydrogène, alkyle en (C₁-C₄) ou cyclopropyle,
ainsi que leurs sels, solvates et solvates des sels.

2. Composé de formule (I) selon la revendication 1, dans lequel
A représente O, S ou N-R⁵,
R⁵ signifiant hydrogène, alkyle en (C₁-C₆), cycloalkyle en (C₃-C₇) ou cycloalcényle en (C₄-C₇),
L représente alcanediyle en (C₁-C₇) ou alcènediyle en (C₂-C₇), qui peuvent être substitués une ou deux fois avec fluor, ou un groupe de formule *-L¹-Q-L²,
* signifiant l'emplacement de liaison avec le groupe CHR³,
L¹ signifiant alcanediyle en (C₁-C₅),
L² signifiant une liaison ou alcanediyle en (C₁-C₃), qui peut être substitué une ou deux fois avec fluor,
et
Q signifiant O ou N-R⁶,
R⁶ représentant hydrogène, alkyle en (C₁-C₆) ou cycloalkyle en (C₃-C₇),
Z représente un groupe de formule 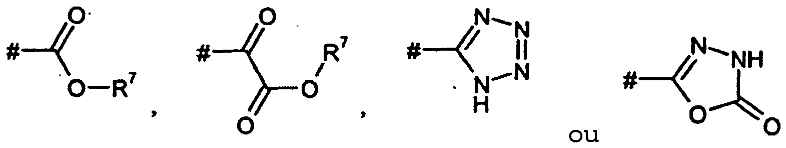
# signifiant l'emplacement de liaison avec le groupe L
et
R⁷ signifiant hydrogène ou alkyle en (C₁-C₄),
R¹ et R² représentent indépendamment l'un de l'autre un substituant choisi dans la série halogène, cyano, nitro, alkyle en (C₁-C₆), alcényle en (C₂-C₆), alcynyle en (C₂-C₄), cycloalkyle en (C₃-C₇), cycloalcényle en (C₄-C₇), alcoxy en (C₁-C₆), trifluorométhyle, trifluorométhoxy, alkylthio en (C₁-C₆), acyle en (C₁-C₆), amino, mono-alkylamino en (C₁-C₆), di-alkylamino en (C₁-C₆) et acylamino en (C₁-C₆),
alkyle en (C₁-C₆) et alcoxy en (C₁-C₆) pouvant chacun de leur côté être substitués avec hydroxy, alcoxy en (C₁-C₄), amino, mono- ou di-alkylamino en (C₁-C₄),
ou
deux radicaux R¹ et/ou R² reliés à des atomes de carbone voisins du cycle phényle respectif forment ensemble un groupe de formule -O-CH₂-O-, -O-CHF-O-, -O-CF₂-O-, -O-CH₂-CH₂-O- ou -O-CF₂-CF₂-O-,
n et o représentent indépendamment l'un de l'autre le nombre 0, 1, 2 ou 3, lorsque R¹ ou R² apparaissent à plusieurs reprises, leurs significations pouvant à chaque fois être identiques ou différentes,
R³ représente hydrogène ou alkyle en (C₁-C₄), qui peut être substitué avec hydroxy ou amino,
et
R⁴ représente hydrogène, alkyle en (C₁-C₄) ou cyclopropyle,
ainsi que leurs sels, solvates et solvates des sels.

3. Composé de formule (I) selon la revendication 1 ou 2, dans lequel
A représente O ou N-R⁵,
R⁵ signifiant hydrogène, alkyle en (C₁-C₄) ou cycloalkyle en (C₃-C₆),
L représente alcanediyle en (C₃-C₇) ou alcènediyle en (C₃-C₇), qui peuvent être substitués une ou deux fois avec fluor, ou un groupe de formule *-L¹-Q-L²,
* signifiant l'emplacement de liaison avec le groupe CHR³,
L¹ signifiant alcanediyle en (C₁-C₃),
L² signifiant alcanediyle en (C₁-C₃), qui peut être substitué une ou deux fois avec fluor,
et
Q signifiant O ou N-R⁶,
R⁶ représentant hydrogène, alkyle en (C₁-C₃) ou cyclopropyle,
Z représente un groupe de formule 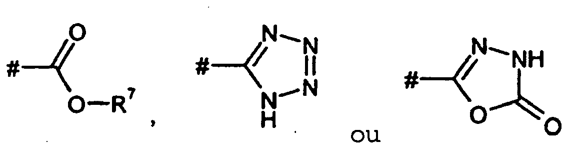
# signifiant l'emplacement de liaison avec le groupe L
et
R⁷ signifiant hydrogène, méthyle ou éthyle,
R¹ et R² représentent indépendamment l'un de l'autre un substituant choisi dans la série fluor, chlore, cyano, alkyle en (C₁-C₅), alcényle en (C₂-C₅), cycloalkyle en (C₃-C₆), cycloalcényle en (C₄-C₆), alcoxy en (C₁-C₄), trifluorométhyle, trifluorométhoxy, alkylthio en (C₁-C₄), acyle en (C₁-C₅), amino, mono-alkylamino en (C₁-C₄), di-alkylamino en (C₁-C₄) et acylamino en (C₁-C₄), ou
deux radicaux R¹ et/ou R² reliés à des atomes de carbone voisins du cycle phényle respectif forment ensemble un groupe de formule -O-CH₂-O-, -O-CHF-O-ou -O-CF₂-O-,
n et o représentent indépendamment l'un de l'autre le nombre 0, 1, 2 ou 3,
lorsque R¹ ou R² apparaissent à plusieurs reprises, leurs significations pouvant à chaque fois être identiques ou différentes,
R³ représente hydrogène ou alkyle en (C₁-C₃), qui peut être substitué avec hydroxy ou amino
et
R⁴ représente hydrogène ou alkyle en (C₁-C₃),
ainsi que leurs sels, solvates et solvates des sels.

4. Composé de formule (I) selon la revendication 1, 2 ou 3, dans lequel
A représente O ou NH,
L représente alcanediyle en (C₃-C₇), alcènediyle en (C₃-C₇) ou un groupe de formule *-L¹-O-L²,
* signifiant l'emplacement de liaison avec le groupe CHR³,
L¹ et L² signifiant indépendamment l'un de l'autre alcanediyle en (C₁-C₃),
Z représente un groupe de formule 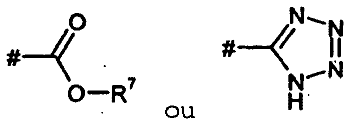
# signifiant l'emplacement de liaison avec le groupe L
et
R⁷ signifiant hydrogène, méthyle ou éthyle,
R¹ et R² représentent indépendamment l'un de l'autre un substituant choisi dans la série fluor, chlore, cyano, alkyle en (C₁-C₅), alcényle en (C₂-C₅), cycloalkyle en (C₃-C₆), cycloalcényle en (C₄-C₆), alcoxy en (C₁-C₄), trifluorométhyle, trifluorométhoxy, alkylthio en (C₁-C₄), acyle en (C₁-C₅), amino, mono-alkylamino en (C₁-C₄), di-alkylamino en (C₁-C₄) et acylamino en (C₁-C₄), ou
deux radicaux R¹ et/ou R² reliés à des atomes de carbone voisins du cycle phényle respectif forment ensemble un groupe de formule -O-CH₂-O-, -O-CHF-O-ou -O-CF₂-O-,
n et o représentent indépendamment l'un de l'autre le nombre 0, 1 ou 2,
lorsque R¹ ou R² apparaissent à deux reprises, leurs significations pouvant à chaque fois être identiques ou différentes,
R³ représente hydrogène, méthyle ou éthyle
et
R⁴ représente hydrogène,
ainsi que leurs sels, solvates et solvates des sels.

5. Composé de formule (I) selon la revendication 1, 2 ou 3, dans lequel
A représente O ou NH,
L représente un groupe de formule *-L¹-N(CH₃)-L²_{,}
* signifiant l'emplacement de liaison avec le groupe CHR³,
et
L¹ et L² signifiant indépendamment l'un de l'autre alcanediyle en (C₁-C₃),
Z représente un groupe de formule 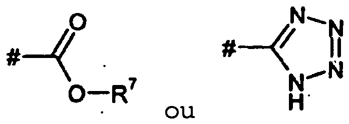
# signifiant l'emplacement de liaison avec le groupe L
et
R⁷ signifiant hydrogène, méthyle ou éthyle,
R¹ et R² représentent indépendamment l'un de l'autre un substituant choisi dans la série fluor, chlore, cyano, alkyle en (C₁-C₅), alcényle en (C₂-C₅), cycloalkyle en (C₃-C₆), cycloalcényle en (C₄-C₆), alcoxy en (C₁-C₄), trifluorométhyle, trifluorométhoxy, alkylthio en (C₁-C₄), acyle en (C₁-C₅), amino, mono-alkylamino en (C₁-C₄), di-alkylamino en (C₁-C₄) et acylamino en (C₁-C₄), ou
deux radicaux R¹ et/ou R² reliés à des atomes de carbone voisins du cycle phényle respectif forment ensemble un groupe de formule -O-CH₂-O-, -O-CHF-O-ou -O-CF₂-O-,
n et o représentent indépendamment l'un de l'autre le nombre 0, 1 ou 2,
lorsque R¹ ou R² apparaissent à deux reprises, leurs significations pouvant à chaque fois être identiques ou différentes,
R³ représente hydrogène, méthyle ou éthyle
et
R⁴ représente hydrogène,
ainsi que leurs sels, solvates et solvates des sels.

6. Composé de formule (I) selon l'une quelconque des revendications 1 à 5, dans lequel
A représente O ou NH,
L représente alcanediyle en (C₃-C₇), alcènediyle en (C₃-C₇) ou un groupe de formule *-L¹-Q-L²,
* signifiant l'emplacement de liaison avec le groupe CHR³,
L¹ et L² signifiant indépendamment l'un de l'autre alcanediyle en (C₁-C₃),
et
Q signifiant O ou N(CH₃),
Z représente un groupe de formule 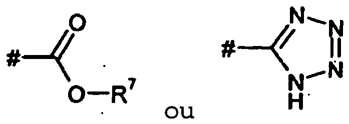
# signifiant l'emplacement de liaison avec le groupe L
et
R⁷ signifiant hydrogène, méthyle ou éthyle,
R¹ représente un substituant choisi dans la série fluor, chlore, méthyle, éthyle, vinyle, trifluorométhyle et méthoxy,
R² représente un substituant choisi dans la série fluor, chlore, cyano, méthyle, éthyle, n-propyle, vinyle, trifluorométhyle, méthoxy, éthoxy, trifluorométhoxy, méthylthio, éthylthio, amino, méthylamino et éthylamino,
n et o représentent indépendamment l'un de l'autre le nombre 0, 1 ou 2,
lorsque R¹ ou R² apparaissent à deux reprises, leurs significations pouvant à chaque fois être identiques ou différentes,
R³ représente hydrogène, méthyle ou éthyle
et
R⁴ représente hydrogène,
ainsi que leurs sels, solvates et solvates des sels.

7. Procédé de fabrication de composés de formule (I), tels que définis dans les revendications 1 à 6, **caractérisé en ce que** soit
[A] des composés de formule (II) 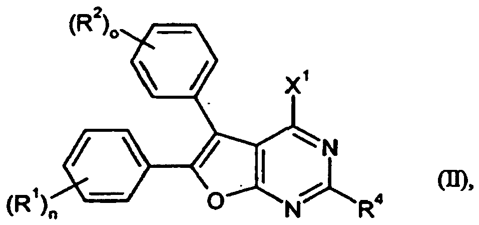 dans laquelle R¹, R², R⁴, n et o ont chacun les significations indiquées dans les revendications 1 à 6,
et
X¹ représente un groupe partant, tel que par exemple halogène, notamment chlore,
sont mis en réaction en présence d'une base, éventuellement dans un solvant inerte, avec un composé de formule (III) 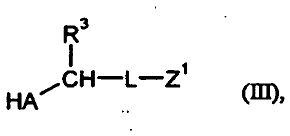 dans laquelle A, L et R³ ont chacun les significations indiquées dans les revendications 1 à 6
et
Z¹ représente cyano ou un groupe de formule - [C(O)]_{y}-COOR^{7A},
y signifiant le nombre 0 ou 1
et
R^{7A} signifiant alkyle en (C₁-C₄),
pour former des composés de formule (IV) 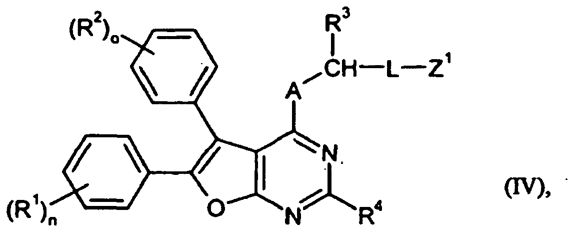 dans laquelle A, L, Z¹, R¹, R², R³, R⁴, n et o ont chacun les significations indiquées précédemment,
soit
[B] des composés de formule (V-1) 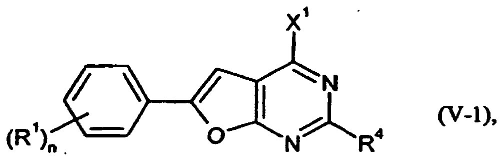 dans laquelle R¹, R⁴, X¹ et n ont chacun les significations indiquées dans les revendications 1 à 6,
sont mis en réaction en présence d'une base, éventuellement dans un solvant inerte, avec un composé de formule (III) pour former des composés de formule (VI-1) 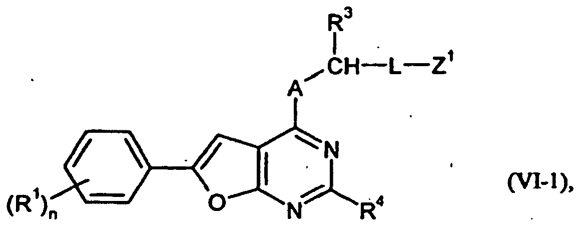 dans laquelle A, L, Z¹, R¹, R³, R⁴ et n ont chacun les significations indiquées précédemment,
puis bromés dans un solvant inerte pour former des composés de formule (VII-1) 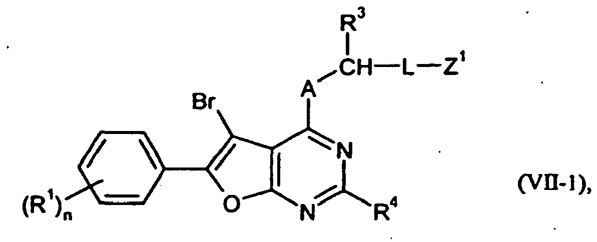 dans laquelle A, L, Z¹, R¹, R³, R⁴ et n ont chacun les significations indiquées précédemment,
puis ceux-ci sont couplés dans un solvant inerte en présence d'une base et d'un catalyseur de palladium approprié avec un acide phénylboronique de formule (VIII-1) 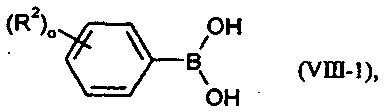 dans laquelle R² et o ont les significations indiquées dans les revendications 1 à 6,
pour former des composés de formule (IV),
soit
[C] des composés de formule (V-2) 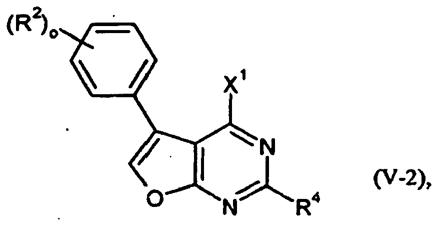 dans laquelle R², R⁴, X¹ et o ont chacun les significations indiquées dans les revendications 1 à 6,
sont mis en réaction en présence d'une base, éventuellement dans un solvant inerte, avec un composé de formule (III) pour former des composés de formule (VI-2) 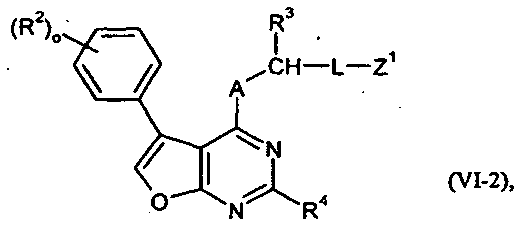 dans laquelle A, L, Z¹, R², R³, R⁴ et o ont chacun les significations indiquées précédemment,
puis bromés dans un solvant inerte pour former des composés de formule (VII-2) 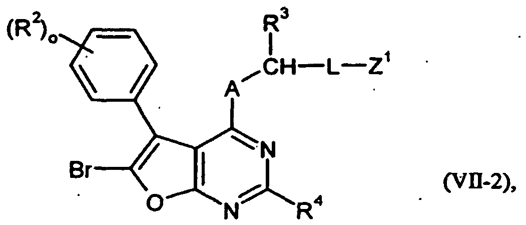 dans laquelle A, L, Z¹, R², R³, R⁴ et o ont chacun les significations indiquées précédemment,
puis ceux-ci sont couplés dans un solvant inerte en présence d'une base et d'un catalyseur de palladium approprié avec un acide phénylboronique de formule (VIII-2) 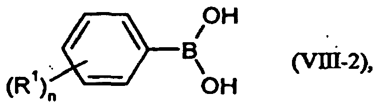 dans laquelle R¹ et n ont les significations indiquées dans les revendications 1 à 6,
pour former des composés de formule (IV),
et les composés de formule (IV) résultants à chaque fois sont ensuite transformés par hydrolyse du groupe ester ou cyano Z¹ en les acides carboxyliques de formule (I-A) 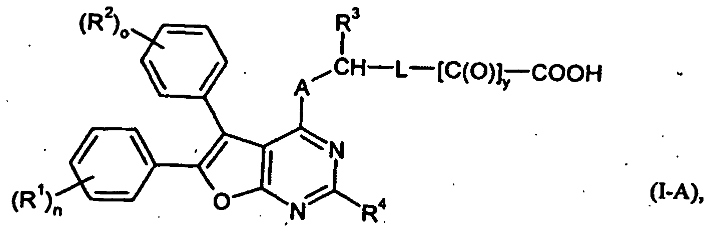 dans laquelle A, L, R¹, R², R³, R⁴, n, o et y ont chacun les significations indiquées précédemment,
et ceux-ci sont éventuellement transformés en leurs solvates, sels et/ou solvates des sels avec les (i) solvants et/ou (ii) bases ou acides correspondants.

8. Composés de formule (I), tels que définis dans l'une quelconque des revendications 1 à 6, pour le traitement et/ou la prophylaxie de maladies.

9. Utilisation d'un composé de formule (I), tel que défini dans l'une quelconque des revendications 1 à 6, pour la fabrication d'un médicament pour le traitement et/ou la prophylaxie de maladies cardiovasculaires.

10. Médicament contenant un composé de formule (I), tel que défini dans l'une quelconque des revendications 1 à 6, en combinaison avec un adjuvant inerte, non toxique, pharmaceutiquement approprié.

11. Médicament contenant un composé de formule (I), tel que défini dans l'une quelconque des revendications 1 à 6, en combinaison avec un agent actif supplémentaire.

12. Médicament selon la revendication 10 ou 11 pour le traitement et/ou la prophylaxie de maladies cardiovasculaires.

13. Composés pour le traitement et/ou la prophylaxie de maladies cardiovasculaires chez les hommes et les animaux selon un composé de formule (I), tel que défini dans l'une quelconque des revendications 1 à 6, ou un médicament, tel que défini dans l'une quelconque des revendications 10 à 12.
